(19) Europäisches Patentamt
European Patent Office
Office européen des brevets

(11) EP 1 684 202 A1

(12) # EUROPEAN PATENT APPLICATION
published in accordance with Art. 158(3) EPC

(43) Date of publication:
**26.07.2006 Bulletin 2006/30**

(21) Application number: **04792509.4**

(22) Date of filing: **15.10.2004**

(51) Int Cl.:
***G06F 19/00*** *(2006.01)*

(86) International application number:
**PCT/JP2004/015292**

(87) International publication number:
**WO 2005/036443 (21.04.2005 Gazette 2005/16)**

(84) Designated Contracting States:
**AT BE BG CH CY CZ DE DK EE ES FI FR GB GR HU IE IT LI LU MC NL PL PT RO SE SI SK TR**

(30) Priority: **15.10.2003 JP 2003355716**

(71) Applicant: **Signpost Corporation**
**Osaka 532-0004 (JP)**

(72) Inventor: **YAMASAKI, Yoshimitsu**
**Nishinomiya-shi, Hyogo 662-0084 (JP)**

(74) Representative: **Müller-Boré & Partner**
**Patentanwälte**
**Grafinger Strasse 2**
**81671 München (DE)**

(54) **METHOD OF DETERMINING GENETIC POLYMORPHISM FOR JUDGMENT OF DEGREE OF DISEASE RISK, METHOD OF JUDGING DEGREE OF DISEASE RISK, AND JUDGMENT ARRAY**

(57) The present invention provides a method of assessing disease risk, which enables accurate assessment of disease risk (i.e., the likelihood of onset of a disease, the probability of progression of the disease, etc.) and is available for the prevention and treatment of disease; and a method of determining genetic polymorphisms for assessing disease risk, usable for such disease risk assessment and like purposes. It also provides an array, a kit for analyzing a genetic polymorphism, an apparatus for assessing disease risk, etc., for use in disease risk assessment. The main characteristic of the risk assessment is the use of polymorphisms or polymorphism set(s) negatively associated with (resistant to) a target disease, and especially the combined use of polymorphisms or polymorphism set(s) negatively associated with the disease and polymorphism(s) or polymorphism set(s) positively associated with the diseases.

[FIG. 74]

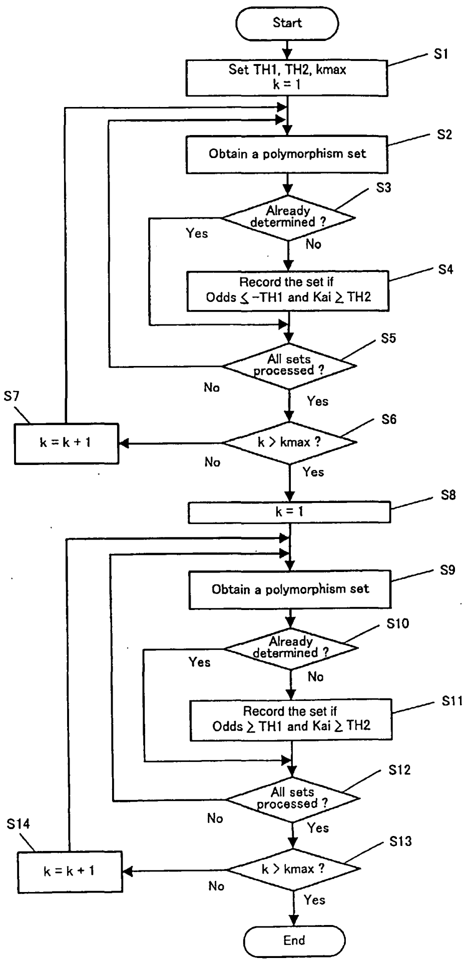


EP 1 684 202 A1

## Description

### TECHNICAL FIELD

The present invention relates to a method of determining genetic polymorphisms for assessing disease risk. The present invention also relates to a method of assessing the disease risk of each subject by using polymorphisms determined by said method. More specifically, it relates to a method of assessing disease risk, applicable to the prevention, treatment, and diagnosis of various diseases, and also relates to an apparatus and program for assessing disease risk, usable in performing the method.

The present invention further relates to an array for assessing disease risk, a method of assessing disease risk, a genetic marker, and an analysis kit, that are usable in assessing risks of arteriosclerotic diseases that arise from diabetes.

### BACKGROUND ART

Known risk factors for the onset of arteriosclerosis (ischemic heart disease) include environmental factors such as hypertension, diabetes, hyperlipidemia, obesity, smoking, etc. Family history is another such risk factor. Developments in molecular biological techniques in recent years have revealed a variety of polymorphisms present in arteriosclerosis-related genes, and their involvement in illness has been studied.

If the "disease risk", such as the likelihood that a subject will get a disease, the probability that such a disease will progress, etc., could be assessed with respect to arteriosclerosis and various other diseases based on information about the genotypes, etc., of disease-related polymorphisms existing in a subject, it would then be possible to take some proactive measures to prevent the onset of such diseases and suppress their progress. That is, those assessed to be at high risk could take daily precautions to prevent the disease. The possibility of onset of the disease and the likely degree of its progress could also be predicted, which would make it possible to provide more detailed diagnosis and treatment suitable for each subject.

In prior clinical studies of genetic polymorphisms including SNPs regarding arteriosclerosis and other diseases, the ratio of patients to healthy persons in a group with one genotype and such a ratio in a group with another genotype have been determined with respect to single kinds of genetic polymorphisms, and the odds ratio of the probability of disease has thereby been calculated (non-patent document 1). However, in such examination methods, because most polymorphic variants do not show significant differences, it is impossible to accurately predict disease risk, such as the likeliness of onset and progression, from such genetic polymorphisms.

Further, there have been no test methods which make it possible to highly accurately predict disease risk (the likeliness of onset and progression) in a significant proportion of test subjects.

The applicants filed an international patent application (PCT/IB03/01368) on April 14, 2003, disclosing a method wherein combinations of a plurality of genetic polymorphisms that exhibit a significant positive association with carotid artery intima-media thickness are used to assess arteriosclerosis. References (non-patent documents 2 to 6) cited in the international search report for this international patent application mention polymorphisms having a significant positive association with carotid artery intima-media thickness, but none of them describes the idea of using polymorphisms having a "negative association", as opposed to a "positive association", for risk degree assessment.

Non-patent document 1: Yamada Y, Izawa H, Ichihara S, Takatsu F, Ishihara H, Hirayama H, Sone T, Tanaka M, Yokota M., "Prediction of the risk of myocardial infarction from polymorphisms in candidate genes", *N. Engl. J. Med.*, 2002, 347 (24), pp. 1916-1923

Non-patent document 2: Rauramaa R, et al., *Arterioscler Thromb Vasc Biol.,* Dec. 2000, vol. 20, no. 12, pp. 2657-2662

Non-patent document 3: Chapman CM, et al., *Arteriosclerosis,* Nov. 2001, vol. 159, no. 1, pp. 209-217

Non-patent document 4: McQuillan BM, et al., *Circulation,* May 11, 1999, vol. 99, no. 18, pp. 2383-2388

Non-patent document 5: Terry JG, et al., *Stroke,* Oct. 1996, vol. 27, no. 10, pp. 1755-1759

Non-patent document 6: Castellano M, et al, *Circulation,* Jun. 1, 1995, vol. 91, no. 11, pp. 2721-2724

### DISCLOSURE OF THE INVENTION

A purpose of the present invention is to solve problems of the prior art in assessing disease risk (the likelihood of onset of a disease, the probability of progression of the disease, etc.) and to achieve the following objects.

The first object of the present invention is to provide a method of determining genetic polymorphisms for disease risk assessment that are unique to various diseases. The second object of the invention is to provide a method of assessing disease risk, which is capable of assessing the likelihood of onset of a disease and the probability of progression of the disease, and is applicable to the prevention and treatment of the disease, and also provide an apparatus for assessing disease risk and a program for assessing disease risk, which can be used to perform the method. The third object of the invention is to provide, with respect to diabetic arteriosclerosis in particular, an array for assessing disease risk, a method of assessing disease risk, a genetic marker, and an analysis kit for detecting a polymorphism or polymorphism set peculiar to the disease.

In a study of diabetics, the present inventors quantitatively analyzed the association between certain genetic polymorphisms and carotid artery intima-media thickness, which is an indicator of arteriosclerosis, and found the existence of polymorphisms having a negative association (resistance) with carotid artery intima-media thickness, and thereby confirmed that the likelihood that a subject will not develop arteriosclerosis (the likelihood of no onset) can be explained by employing at least one combination of two or more of such polymorphisms (polymorphism set). They further found that when such at least one negative (resistant) polymorphism set is employed in combination with at least one polymorphism or polymorphism set having a positive association (sensitivity) with carotid artery intima-media thickness, and their relevance to arteriosclerosis is evaluated, the risk of arteriosclerosis can be assessed with higher accuracy than when using only positive (sensitive) polymorphism(s) or polymorphism set(s).

In further research based on the above findings, the present inventors confirmed that the combined use of one or more disease-sensitive polymorphisms (or one or more disease-sensitive polymorphism sets) with disease-resistant polymorphisms (or one or more disease-resistant polymorphisms sets) can increase the accuracy of assessing the risk of not only arteriosclerosis but also various other diseases. Further, during this research, the inventors became convinced that the method for determining disease-related sensitive polymorphisms (or sensitive polymorphisms sets) and disease-resistant polymorphisms (or resistant polymorphisms sets) can be applied to other diseases as well as arteriosclerosis, and established a method of determining polymorphisms (sensitive polymorphisms and resistant polymorphisms) unique to each disease.

The present invention has been accomplished based on the above findings, and means for solving the above problems are as follows.

(1) A method of determining genetic polymorphisms for assessing disease risk, comprising:

a first step of selecting, from preselected genetic polymorphisms, a predetermined number of genetic polymorphisms each having a specified genotype to thereby obtain a genetic polymorphism set;
a second step of using a group comprising elements, each element being a combination of a disease index value with genetic polymorphisms each having a genotype, to calculate an association between a disease index and the obtained genetic genetic polymorphism set and a statistical significance of the association; and,
if the calculated association is negative and significant, a third step of employing the genetic polymorphism or genetic polymorphisms that form the genetic polymorphism set as a genetic polymorphism or genetic polymorphisms for assessing disease risk.

(2) An array for assessing arteriosclerosis risk, comprising:

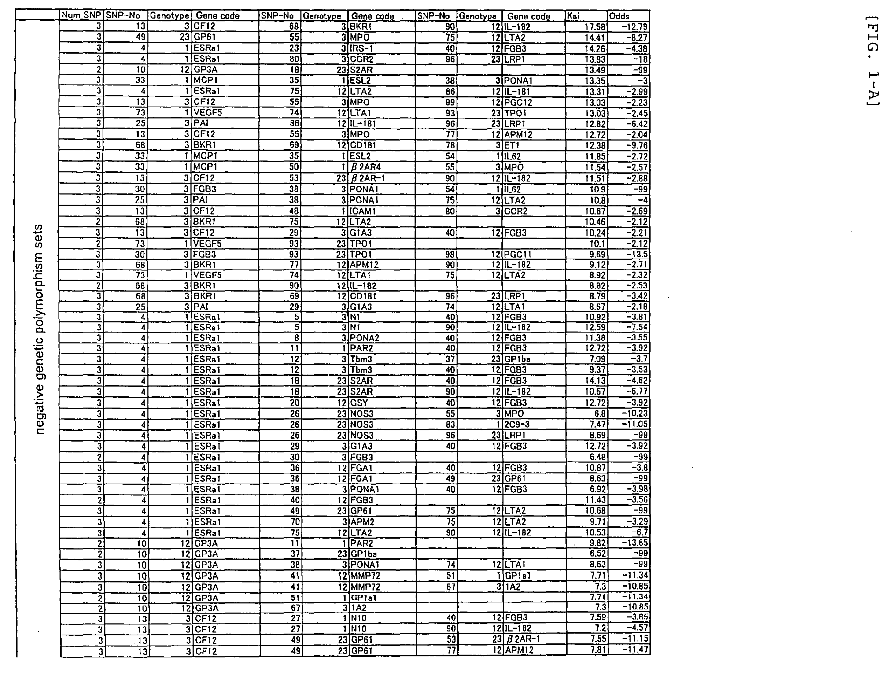

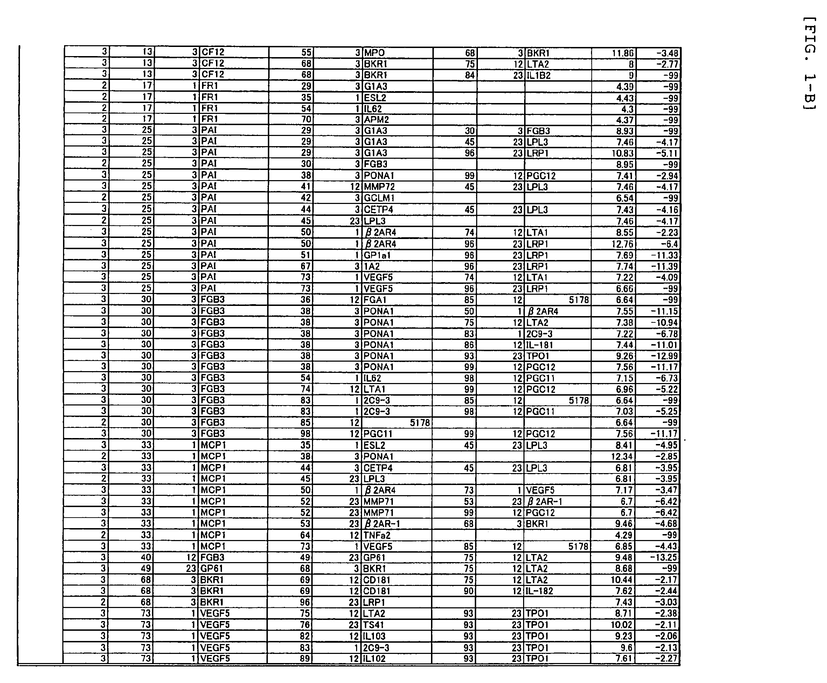

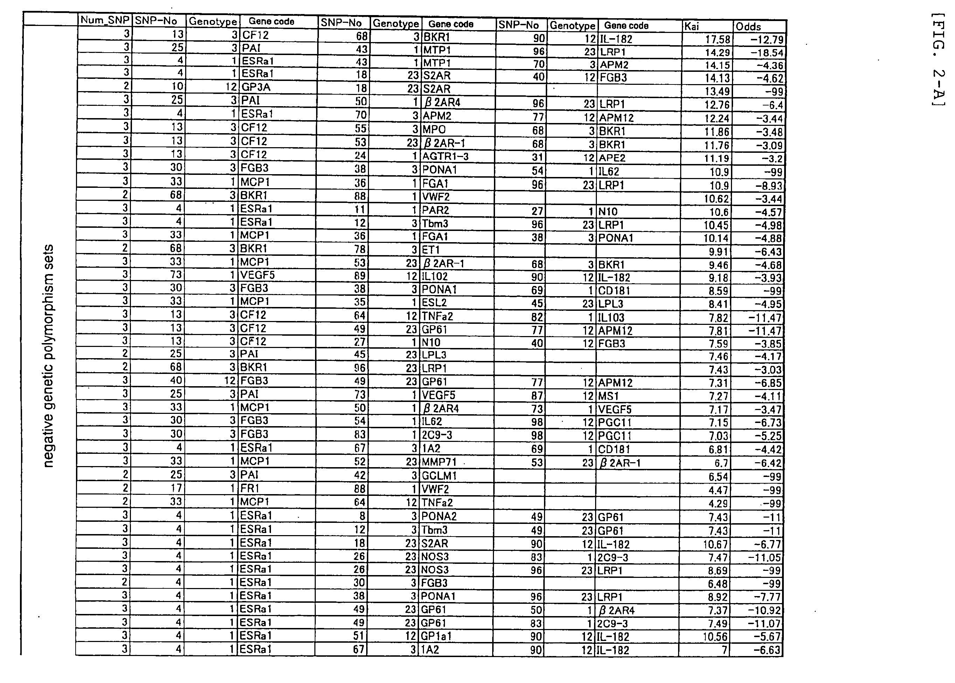

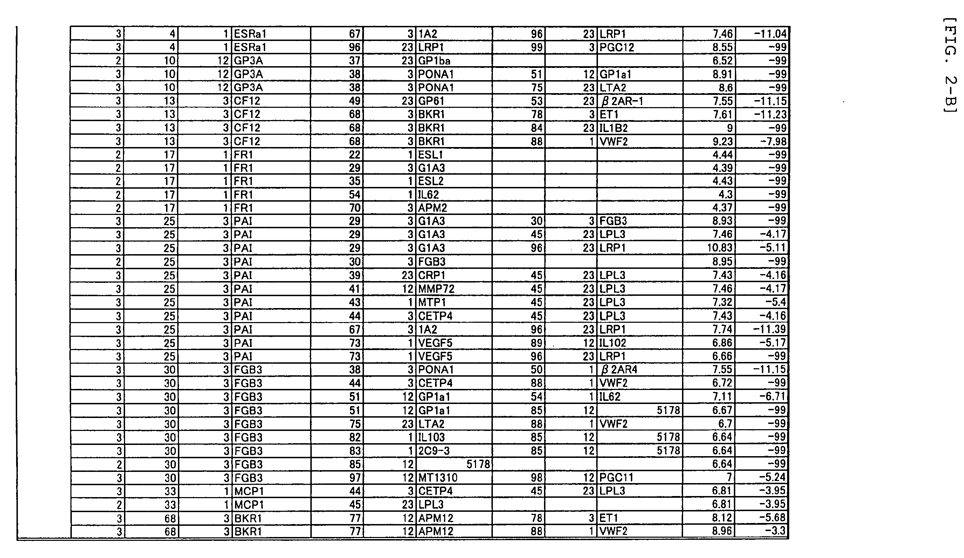

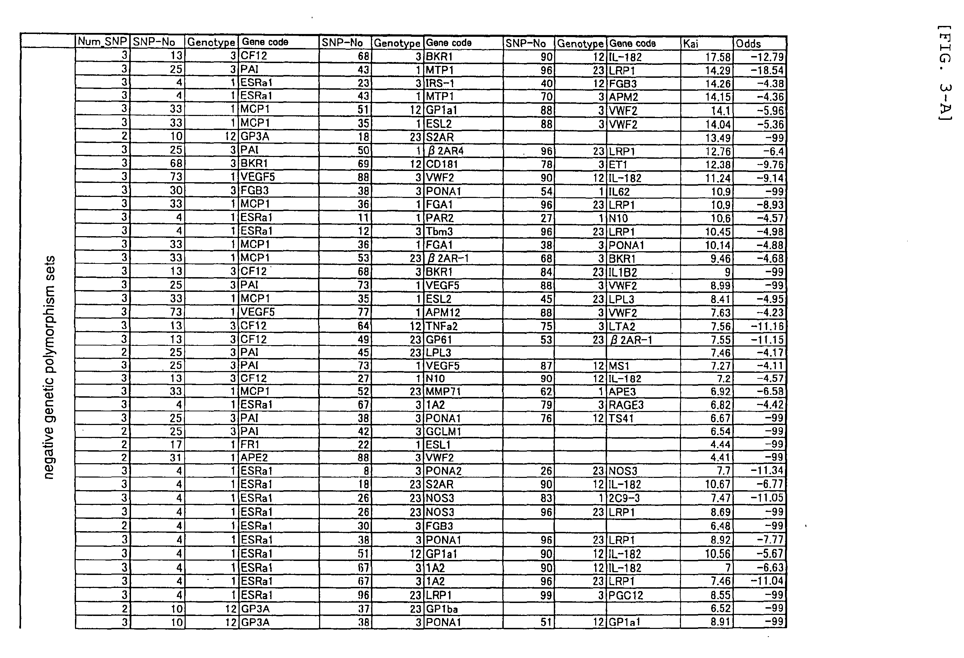

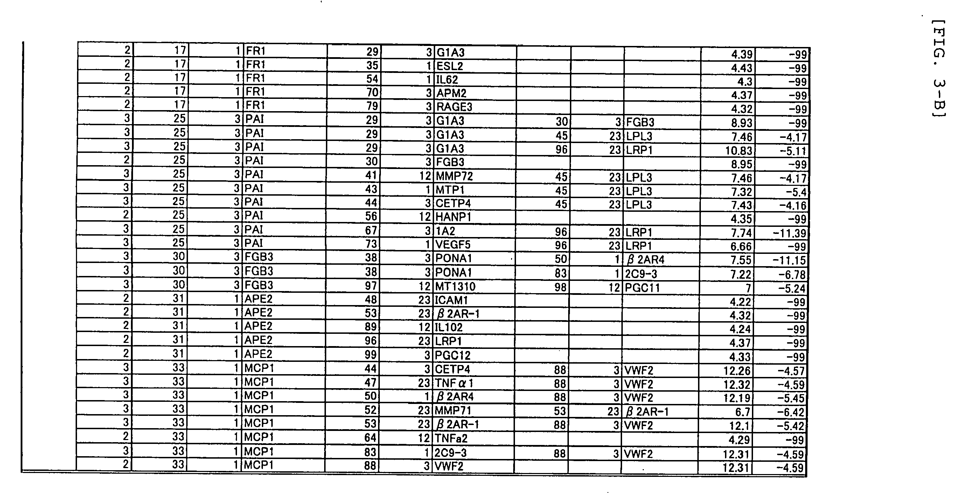

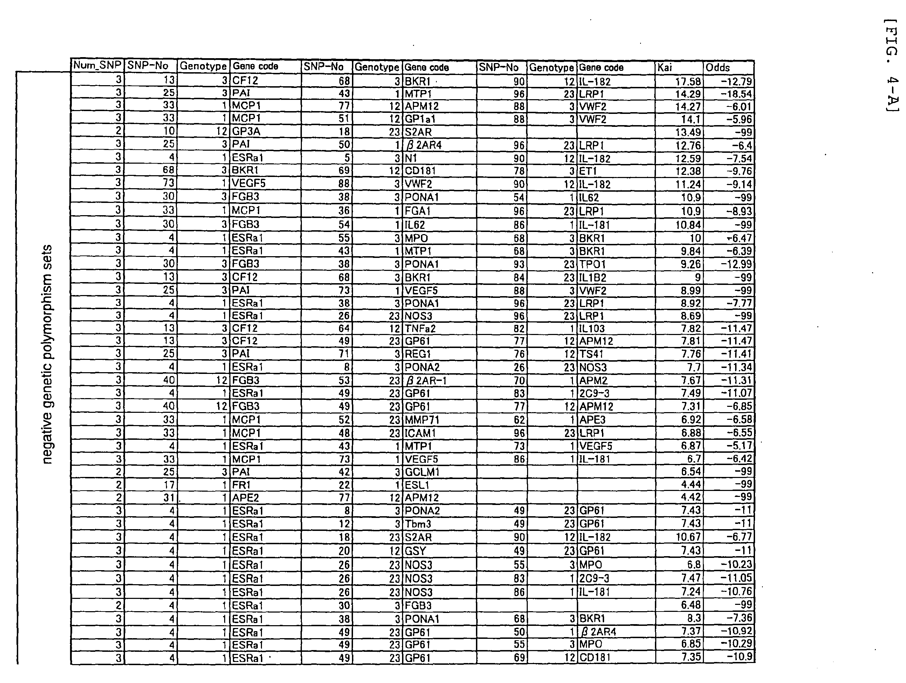

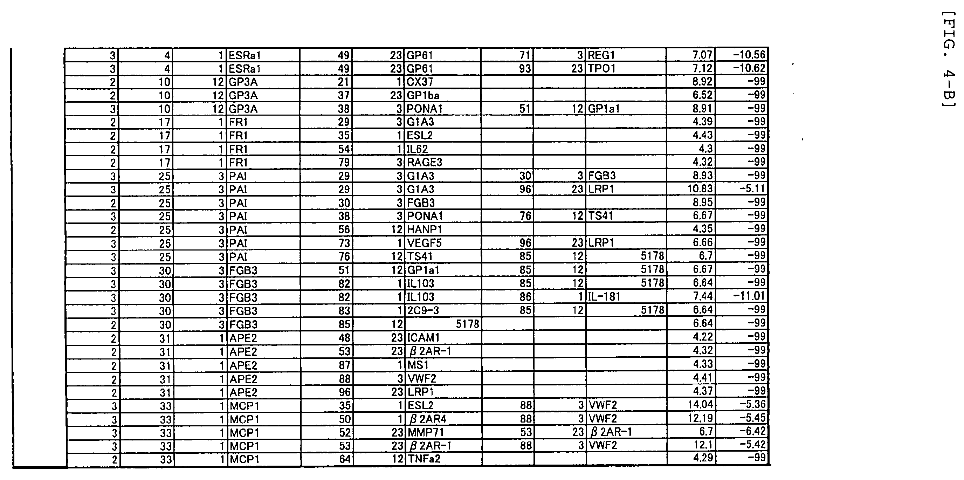

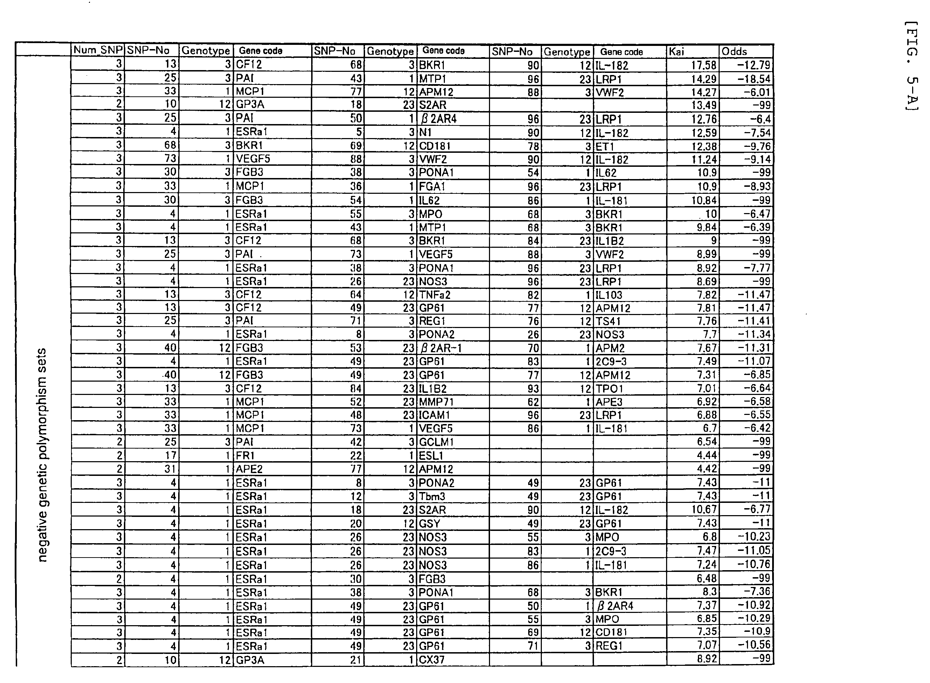

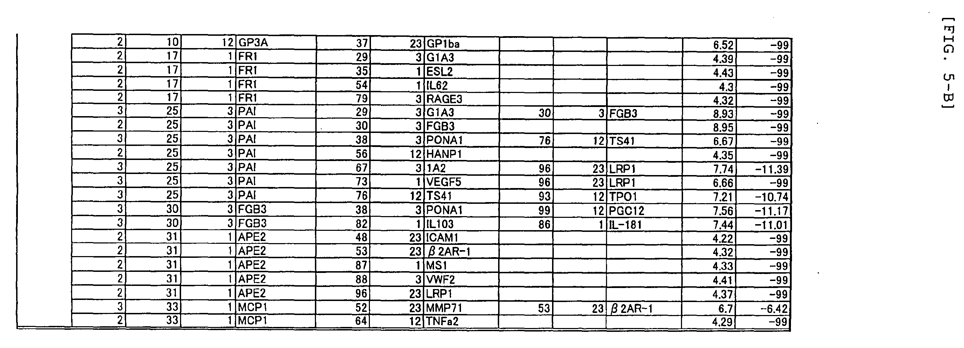

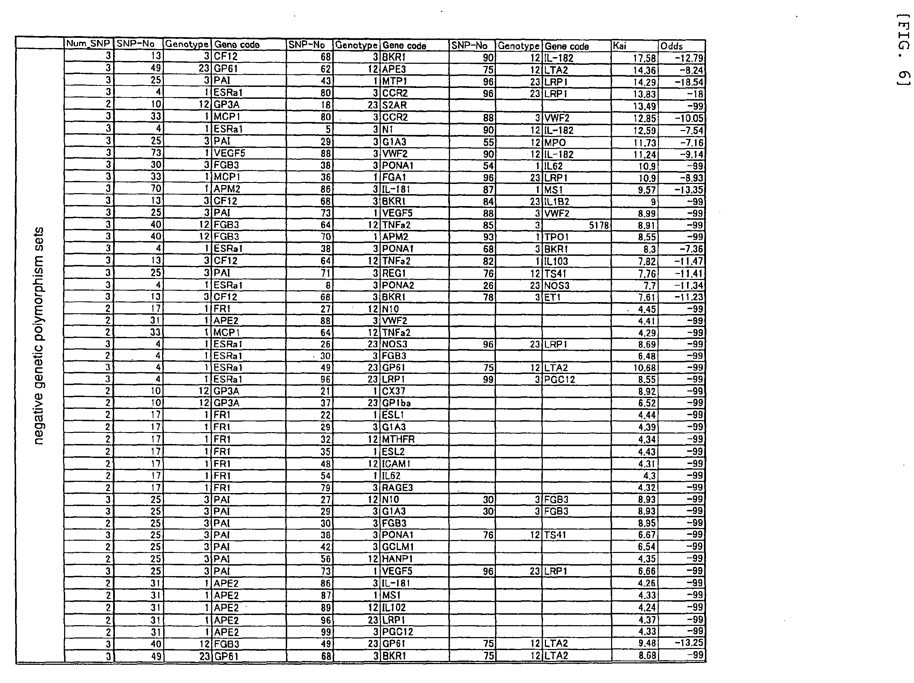

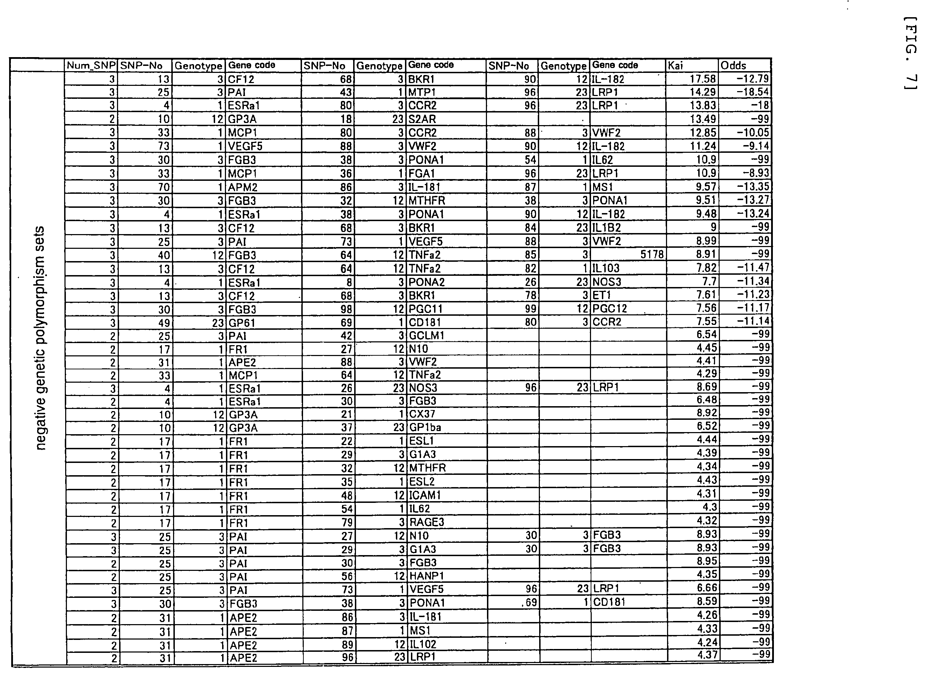

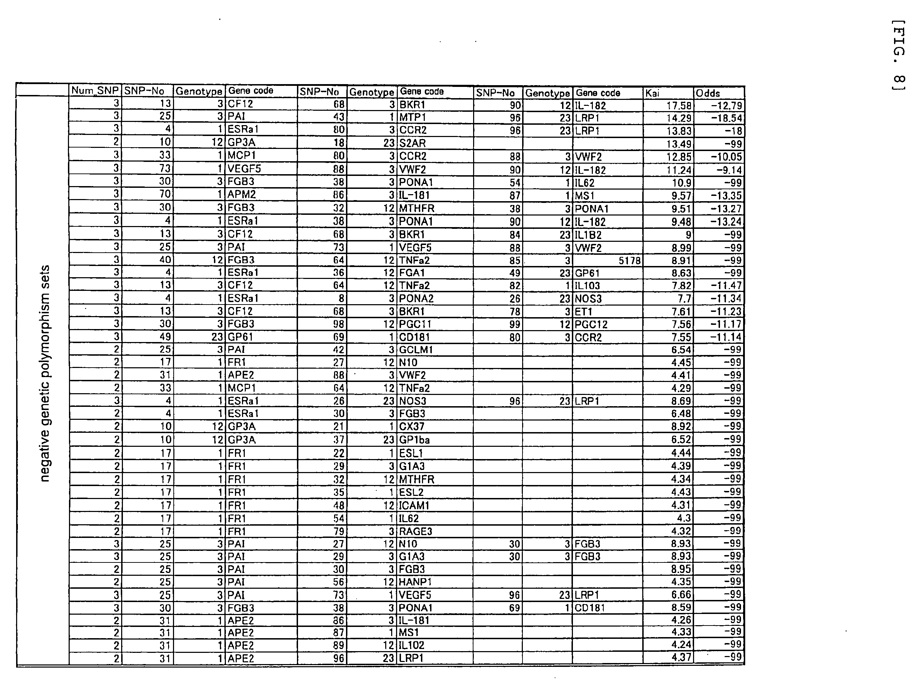

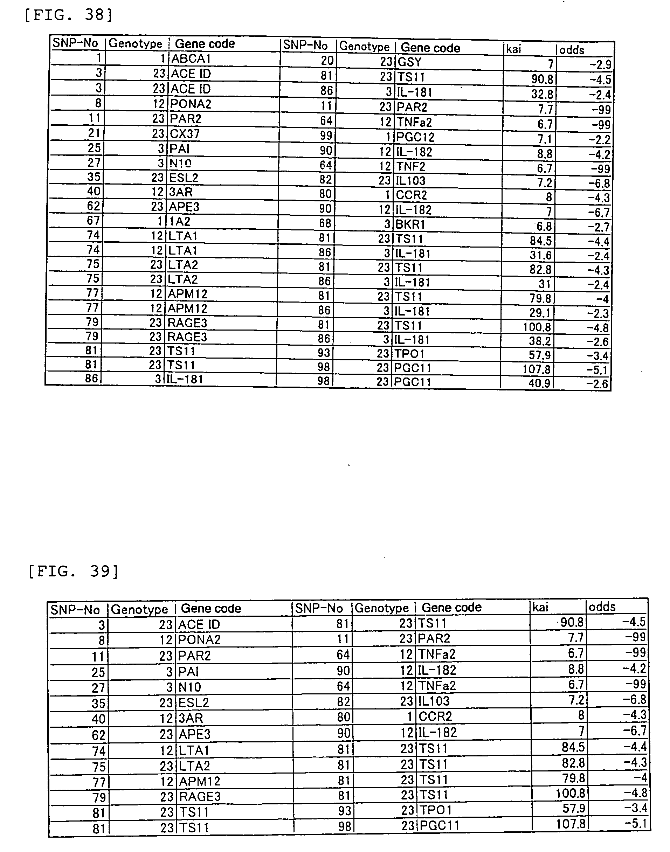

a probe or probes for detecting one or more genetic polymorphisms of at least one genetic polymorphism set selected from the negative (resistant) genetic polymorphism sets shown in any one of FIGS. 1 to 9, or
a probe or probes for detecting one or more genetic polymorphisms of at least one genetic polymorphism set selected from the negative (resistant) genetic polymorphism sets shown in any one of FIGS. 38 to 43; or
an array for assessing myocardial infarction risk, comprising:

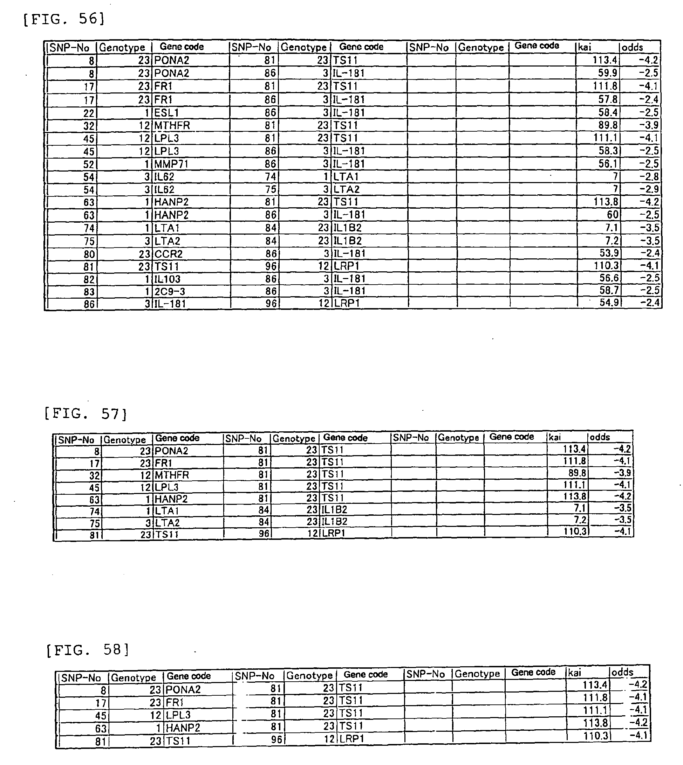

a probe or probes for detecting one or more genetic polymorphisms of at least one genetic polymorphism set selected from the negative (resistant) genetic polymorphism sets shown in any one of FIGS. 56 to 58.

(3) A method of assessing disease risk, comprising:

a step (b) of checking a genetic polymorphism detected in a specimen against one or more genetic polymorphisms negatively associated with a disease index, or against one or more genetic polymorphism sets negatively associated with a disease index, said method further optionally comprising:

a step (b') of checking a genetic polymorphism detected in the specimen against one or more genetic polymorphisms positively associated with a disease index, or against one or more genetic polymorphism sets positively associated with a disease index, and
a step (c) of, based on the result of step (b'), comparing the negative association with the positive association of a set of tdetected genetic polymorphisms to thereby calculate the balance.

(4) An apparatus for assessing disease risk, comprising:

a storage unit for recording a reference table in which first genetic polymorphism sets, each consisting of one or more genetic polymorphisms each having a genotype, are each related to a positive or negative association between the set and a disease index;
an interface for obtaining from a specimen genetic polymorphisms having genotypes; and
a processor for checking a second genetic polymorphism set comprising a predetermined number of genetic polymorphisms obtained from a specimen against said first genetic polymorphism sets in the reference table, wherein:

the processor, if the checking finds an agreement between the second genetic polymorphism set and any first genetic polymorphism set, determines the balance of the specimen toward positive or negative association based on the association of such a first genetic polymorphism set.

(5) A program for assessing disease risk, the program enabling a computer to perform the functions of:

receiving an input concerning genetic polymorphisms having genotypes obtained from a specimen;
recording, in a storage unit, a reference table in which first genetic polymorphism sets, each consisting of one or more genetic polymorphisms each having a genotype, are each related to a positive or negative association between a set and a disease index;
checking a second genetic polymorphism set consisting of a predetermined number of the inputted genetic polymorphisms obtained from the specimen against said one or more first genetic polymorphism sets in the reference table; and
if the checking finds an agreement between the second genetic polymorphism set and any first genetic polymorphism set, determining the balance of the specimen toward positive or negative association based on the association of such a first genetic polymorphism set.

(6) A computer-readable recording medium having stored thereon a program for assessing disease risk, the program enabling a computer to perform the functions of:

receiving an input concerning genetic polymorphisms each having a genotype obtained from a specimen;
recording, in a storage unit, a reference table in which first genetic polymorphism sets, each consisting of one or more genetic polymorphisms each having a genotype, are each related to a positive or negative association between a set and a disease index;
checking a second genetic polymorphism set consisting of a predetermined number of the inputted genetic polymorphisms obtained from the specimen against said one or more first genetic polymorphism sets in the reference table; and,
if the checking finds an agreement between the second genetic polymorphism set and any first genetic polymorphism set, determining the balance of the specimen toward positive or negative association based on the association of such a first genetic polymorphism set.

(7) A genetic marker resistant to arteriosclerosis comprising:

one or more genetic polymorphisms of at least one genetic polymorphism set selected from the negative genetic polymorphism sets shown in any one of FIGS. 1 to 9, or
one or more genetic polymorphisms of at least one genetic polymorphism set selected from the negative genetic polymorphism sets shown in any one of FIGS. 38 to 43; or
a genetic marker resistant to myocardial infarction comprising:

one or more genetic polymorphisms of at least one genetic polymorphism set selected from the negative genetic polymorphism sets shown in any one of FIGS. 56 to 58.

(8) A kit for analyzing genetic polymorphisms resistant to arteriosclerosis, comprising:

(i) at least one primer pair capable of specifically amplifying a gene or genes that contain one or more genetic polymorphisms of at least one genetic polymorphism set selected from the negative genetic polymorphism sets shown in any one of FIGS. 1 to 9, or
at least one nucleic acid probe capable of specifically hybridizing with said gene or genes; or
(ii) at least one primer pair capable of specifically amplifying a gene or genes that contain one or more genetic polymorphisms of at least one genetic polymorphism set selected from the negative genetic polymorphism sets shown in any one of FIGS. 38 to 43, or
at least one nucleic acid probe capable of specifically hybridizing with said gene or genes.

(9) A kit for analyzing genetic polymorphisms resistant to myocardial infarction, comprising:

at least one primer pair capable of specifically amplifying a gene or genes that contain one or more genetic polymorphisms of at least one genetic polymorphism set selected from the negative genetic polymorphism sets shown in any one of FIGS. 56 to 58;
or at least one nucleic acid probe capable of specifically hybridizing with said gene or genes.

BRIEF DESCRIPTION OF DRAWINGS

[FIG. 1-A] shows a list of polymorphism sets negatively associated with arteriosclerosis (Odds = -2 or less).
[FIG. 1-B] is a continuation of the above FIG. 1-A.
[FIG. 2-A] shows a list of polymorphism sets negatively associated with arteriosclerosis (Odds = -3 or less).
[FIG. 2-B] is a continuation of the above FIG. 2-A.
[FIG. 3-A] shows a list of polymorphism sets negatively associated with arteriosclerosis (Odds = -4 or less).
[FIG. 3-B] is a continuation of the above FIG. 3-A.
[FIG. 4-A] shows a list of polymorphism sets negatively associated with arteriosclerosis (Odds = -5 or less).
[FIG. 4-B] is a continuation of the above FIG. 4-A.
[FIG. 5-A] shows a list of polymorphism sets negatively associated with arteriosclerosis (Odds = -6 or less).
[FIG. 5-B] is a continuation of the above FIG. 5-A.
[FIG. 6] shows a list of polymorphism sets negatively associated with arteriosclerosis (Odds = -7 or less).
[FIG. 7] shows a list of polymorphism sets negatively associated with arteriosclerosis (Odds = -8 or less).
[FIG. 8] shows a list of polymorphism sets negatively associated with arteriosclerosis (Odds = -9 or less).
[FIG. 9] shows a list of polymorphism sets negatively associated with arteriosclerosis (Odds = -10 or less).
[FIG. 10] shows a list of polymorphisms that form polymorphism sets negatively associated with arteriosclerosis (Odds = -2 or less).
[FIG. 11] shows a list of polymorphisms that form polymorphism sets negatively associated with arteriosclerosis (Odds = -3 or less).
[FIG. 12] shows a list of polymorphisms that form polymorphism sets negatively associated with arteriosclerosis (Odds = -4 or less).
[FIG. 13] shows a list of polymorphisms that form polymorphism sets negatively associated with arteriosclerosis (Odds = -5 or less).
[FIG. 14] shows a list of polymorphisms that form polymorphism sets negatively associated with arteriosclerosis (Odds = -6 or less).
[FIG. 15] shows a list of polymorphisms that form polymorphism sets negatively associated with arteriosclerosis (Odds = -7 or less).
[FIG. 16] shows a list of polymorphisms that form polymorphism sets negatively associated with arteriosclerosis (Odds = -8 or less).
[FIG. 17] shows a list of polymorphisms that form polymorphism sets negatively associated with arteriosclerosis (Odds = -9 or less).
[FIG. 18] shows a list of polymorphisms that form polymorphism sets negatively associated with arteriosclerosis (Odds = -10 or less).
[FIG. 19-A] shows a list of polymorphism sets positively associated with arteriosclerosis (Odds = 2 or more).
[FIG. 19-B] is a continuation of the above FIG. 19-A.
[FIG. 19-C] is a continuation of the above FIG. 19-B.
[FIG. 20-A] shows a list of polymorphism sets positively associated with arteriosclerosis (Odds = 3 or more).
[FIG. 20-B] is a continuation of the above FIG. 20-A.
[FIG. 21] shows a list of polymorphism sets positively associated with arteriosclerosis (Odds = 4 or more).
[FIG. 22] shows a list of polymorphism sets positively associated with arteriosclerosis (Odds = 5 or more).
[FIG. 23] shows a list of polymorphism sets positively associated with arteriosclerosis (Odds = 6 or more).
[FIG. 24] shows a list of polymorphism sets positively associated with arteriosclerosis (Odds = 7 or more).
[FIG. 25] shows a list of polymorphism sets positively associated with arteriosclerosis (Odds = 8 or more).
[FIG. 26] shows a list of polymorphism sets positively associated with arteriosclerosis (Odds = 9 or more).
[FIG. 27] shows a list of polymorphism sets positively associated with arteriosclerosis (Odds = 10 or more).

[FIG. 28] shows a list of polymorphisms that form polymorphism sets positively associated with arteriosclerosis (Odds = 2 or more).
[FIG. 29] shows a list of polymorphisms that form polymorphism sets positively associated with arteriosclerosis (Odds = 3 or more).
[FIG. 30] shows a list of polymorphisms that form polymorphism sets positively associated with arteriosclerosis (Odds = 4 or more).
[FIG. 31] shows a list of polymorphisms that form polymorphism sets positively associated with arteriosclerosis (Odds = 5 or more).
[FIG. 32] shows a list of polymorphisms that form polymorphism sets positively associated with arteriosclerosis (Odds = 6 or more).
[FIG. 33] shows a list of polymorphisms that form polymorphism sets positively associated with arteriosclerosis (Odds = 7 or more).
[FIG. 34] shows a list of polymorphisms that form polymorphism sets positively associated with arteriosclerosis (Odds = 8 or more).
[FIG. 35] shows a list of polymorphisms that form polymorphism sets positively associated with arteriosclerosis (Odds = 9 or more).
[FIG. 36] shows a list of polymorphisms that form polymorphism sets positively associated with arteriosclerosis (Odds = 10 or more).
[FIG. 37-A] shows a list of arteriosclerosis-associated polymorphisms.
[FIG. 37-B] is a continuation of the above FIG. 37-A.
[FIG. 38] shows a list of polymorphism sets negatively associated with arteriosclerosis (Odds = -2 or less).
[FIG. 39] shows a list of polymorphism sets negatively associated with arteriosclerosis (Odds = -3 or less).
[FIG. 40] shows a list of polymorphism sets negatively associated with arteriosclerosis (Odds = -4 or less).
[FIG. 41] shows a list of polymorphism sets negatively associated with arteriosclerosis (Odds = -5 or less).
[FIG. 42] shows a list of polymorphism sets negatively associated with arteriosclerosis (Odds = -6 or less).
[FIG. 43] shows a list of polymorphism sets negatively associated with arteriosclerosis (Odds = -7 or less).
[FIG. 44] shows a list of polymorphisms that form polymorphism sets negatively associated with arteriosclerosis (Odds = -2 or less).
[FIG. 45] shows a list of polymorphisms that form polymorphism sets negatively associated with arteriosclerosis (Odds = -3 or less).
[FIG. 46] shows a list of polymorphisms that form polymorphism sets negatively associated with arteriosclerosis (Odds = -4 or less).
[FIG. 47] shows a list of polymorphisms that form polymorphism sets negatively associated with arteriosclerosis (Odds = -5 or less).
[FIG. 48-A] shows a list of polymorphism sets positively associated with arteriosclerosis (Odds = 2 or more).
[FIG. 48-B] is a continuation of the above FIG 48-A.
[FIG. 49-A] shows a list of polymorphism sets positively associated with arteriosclerosis (Odds = 3 or more).
[FIG. 49-B] is a continuation of the above FIG. 49-A.
[FIG. 50] shows a list of polymorphism sets positively associated with arteriosclerosis (Odds = 4 or more).
[FIG. 51] shows a list of polymorphism sets positively associated with arteriosclerosis (Odds = 5 or more).
[FIG. 52] shows a list of polymorphisms that form polymorphism sets positively associated with arteriosclerosis (Odds = 2 or more).
[FIG. 53] shows a list of polymorphisms that form polymorphism sets positively associated with arteriosclerosis (Odds = 3 or more).
[FIG. 54] shows a list of polymorphisms that form polymorphism sets positively associated with arteriosclerosis (Odds = 4 or more).
[FIG. 55] shows a list of polymorphisms that form polymorphism sets positively associated with arteriosclerosis (Odds = 5 or more).
[FIG. 56] shows a list of polymorphism sets negatively associated with myocardial infarction (Odds = -2 or less).
[FIG. 57] shows a list of polymorphism sets negatively associated with myocardial infarction (Odds = -3 or less).
[FIG. 58] shows a list of polymorphism sets negatively associated with myocardial infarction (Odds = -4 or less).
[FIG. 59] shows a list of polymorphisms that form polymorphism sets negatively associated with myocardial infarction (Odds = -2 or less).
[FIG. 60] shows a list of polymorphisms that form polymorphism sets negatively associated with myocardial infarction (Odds = -3 or less).
[FIG. 61] shows a list of polymorphisms that form polymorphism sets negatively associated with myocardial infarction (Odds = -4 or less).
[FIG. 62] shows a list of polymorphisms that form polymorphism sets negatively associated with myocardial infarction (Odds = -5 or less).

[FIG. 63] shows a list of polymorphism sets positively associated with myocardial infarction (Odds = 2 or more).

[FIG. 64] shows a list of polymorphism sets positively associated with myocardial infarction (Odds = 3 or more).

[FIG. 65] shows a list of polymorphism sets positively associated with myocardial infarction (Odds = 4 or more).

[FIG. 66] shows a list of polymorphism sets positively associated with myocardial infarction (Odds = 5 or more).

[FIG. 67] shows a list of polymorphism sets positively associated with myocardial infarction (Odds = 6 or more).

[FIG. 68] shows a list of polymorphism sets positively associated with myocardial infarction (Odds = 7 or more).

[FIG. 69] shows a list of polymorphism sets positively associated with myocardial infarction (Odds = 8 or more).

[FIG. 70] shows a list of polymorphisms that form polymorphism sets positively associated with myocardial infarction (Odds = 2 or more).

[FIG. 71] shows a list of polymorphisms that form polymorphism sets positively associated with myocardial infarction (Odds = 3 or more).

[FIG. 72] shows a list of polymorphisms that form polymorphism sets positively associated with myocardial infarction (Odds = 4 or more).

[FIG. 73] shows a list of polymorphisms that form polymorphism sets positively associated with myocardial infarction (Odds = 5 or more).

[FIG. 74] shows a flow chart illustrating a method of determining polymorphisms for assessing disease risk according to one embodiment of the present invention.

[FIG. 75] shows a block diagram illustrating the entire system including an apparatus for assessing disease risk according to one embodiment of the present invention.

[FIG. 76] shows a flow chart illustrating the processing of risk assessment performed by an apparatus for assessing disease risk according to one embodiment of the present invention.

[FIG. 77] graphically shows the results of Example 1. The graph is obtained by equating "sensitivity (accuracy)" and "false-positive (inaccuracy)" with the agreement and the disagreement, respectively, between the invention assessment results and the clinical examination result.

□ represents the predicted sensitivity (accuracy) and false-positive (inaccuracy) calculated using only sensitive "explicative SSNPs"; Δ represents those calculated using only resistant "explicative SSNP"; and • represents those calculated using both sensitive "explicative SSNPs" and resistant "explicative SSNPs".

## BEST MODE FOR CARRYING OUT THE INVENTION

The present invention is described below in more detail.

(Terms)

A "polymorphism" is a variation in a gene wherein two or more alternative alleles are present at one genetic locus. It more specifically means a mutation that occurs in a population at a certain frequency or more. Genetic mutations used herein are not limited to those in a region transcribed into RNA but includes identifiable variations in all the DNA in the human genome, including promoters, enhancers, and like control regions. The human genomic DNA of individuals is 99.9% identical, with the remaining 0.1% causing such variations, which may lead to personal differences in sensitivity to specific diseases, reactivity to drugs and environmental factors, etc. Presence of a genetic polymorphism does not necessarily result in differences in phenotype. SNPs (Single Nucleotide Polymorphisms) are one type of genetic polymorphism, but the target polymorphisms of the present invention are not limited thereto.

In this specification, genotypes are categorized such that "1" indicates a homozygous polymorphism showing, among the exchangeable bases, the base which comes first in alphabetical order (A, C, G, T), "2" indicates a heterozygous polymorphism, and "3" indicates a homozygous polymorphism showing, among the exchangeable bases, the base which comes last in alphabetical order. For example, the genotype of a polymorphism described as NCP-1(A-2518G) is type 1 when it is A/A homozygous, type 2 when it is heterozygous (A/G), and type 3 when it is G/G homozygous. "12" means genotypes 1 and 2, while "23" is genotypes 2 and 3.

A "polymorphism set" means a combination of a plurality of polymorphisms. A plurality of polymorphisms herein means two or more polymorphisms having different genetic loci. "Polymorphism" used herein reflects and includes genotype. That is, a "polymorphism" in the present invention means a polymorphism having a specific genotype.

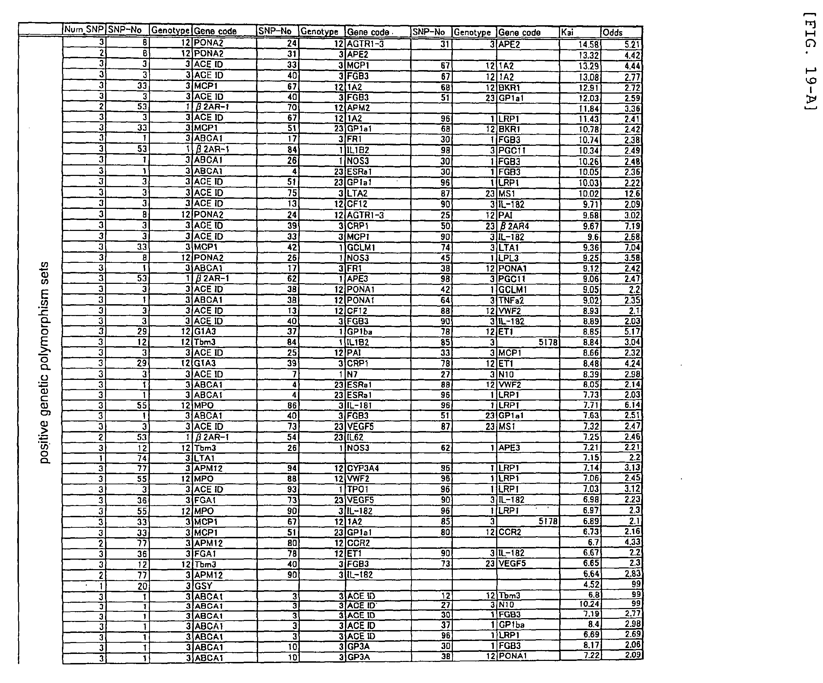

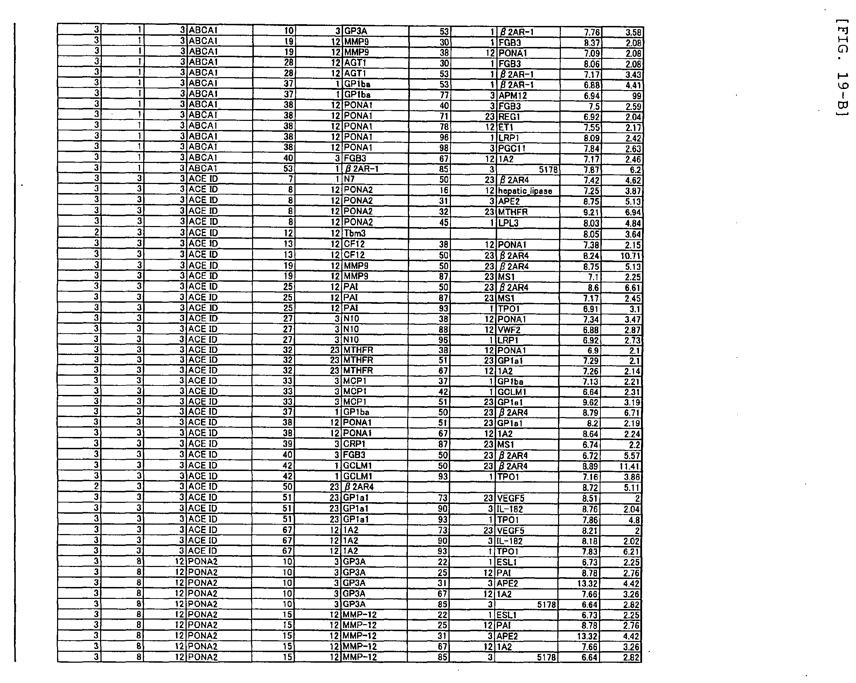

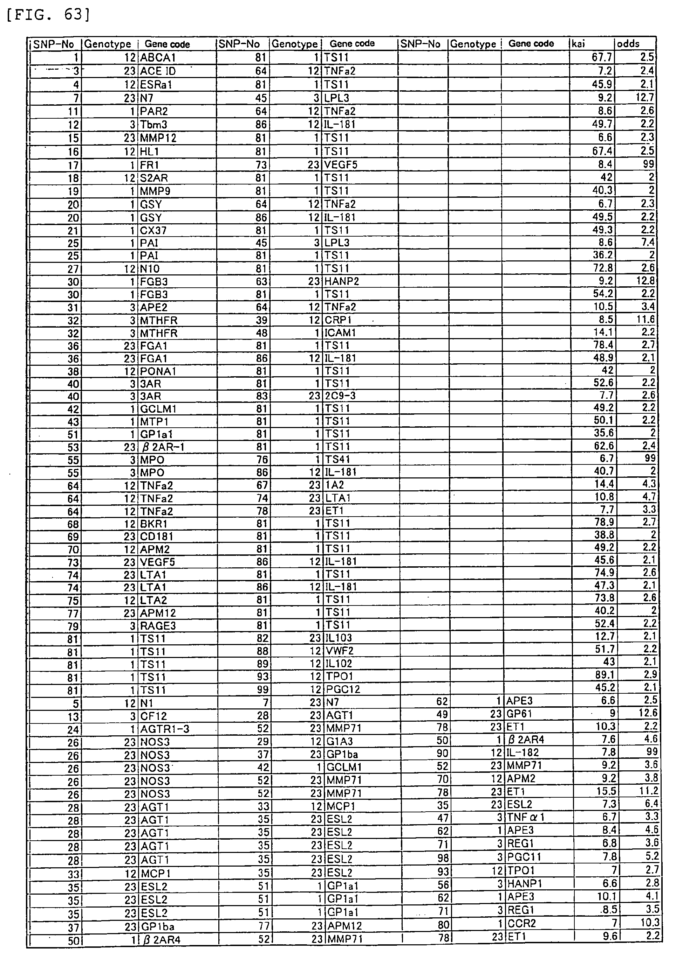

In the present invention, a "polymorphism set" specifically means a "combination of polymorphisms" which shows, as a whole, a negative (resistant) or positive (sensitive) association with a target disease. Examples of such genetic polymorphism sets (combinations) are shown in FIGS. 1 to 9 and 38 to 43 (groups of polymorphism sets negatively associated with arteriosclerosis), FIGS. 19 to 27 and 48 to 51 (groups of polymorphism sets positively associated with arteriosclerosis), FIGS. 56 to 58 (groups of polymorphism sets negatively associated with myocardial infarction), and FIGS. 63 to 69 (groups of polymorphism sets positively associated with myocardial infarction). In every figure, each row (one horizontal row) contains a combination of polymorphisms (SNPs) which as a whole has a negative or positive association with a disease. In FIGS. 19 to 27, as well as cases with two or three polymorphisms being shown in one row, there also are some cases with a single polymorphism being shows in one row. Such a case indicates that each of those polymorphisms by itself shows a positive association with arteriosclerosis. In this specification, such a single polymorphism is also referred to as a "polymorphism set" in order to avoid complexity of explanation.

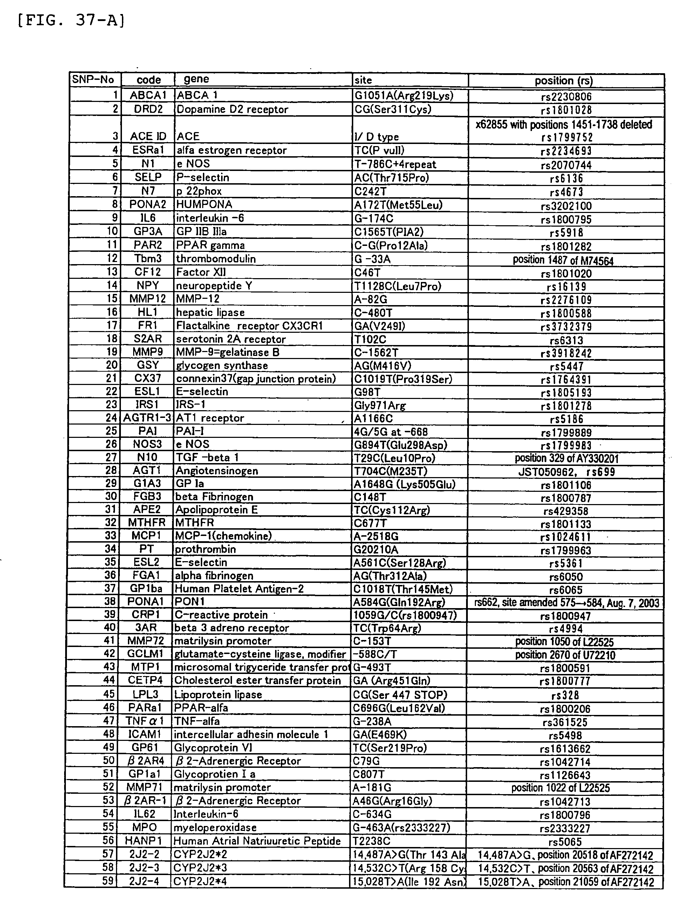

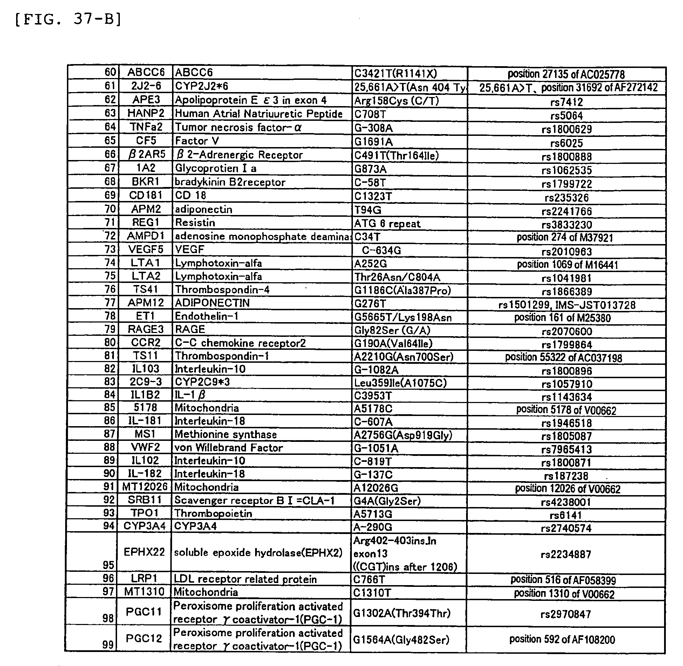

Taking FIG. 1 as an example, each row thereof contains a polymorphism set negatively associated with an arteriosclerosis index (an indicator for arteriosclerosis). More specifically, the first row describes a polymorphism sets consisting of, from the left, "CF12" (Factor XII gene) (genotype: 3), "BRK1" (bradykinin B2 receptor gene) (genotype: 3), and "IL-182" (Interluekin-18 gene) (genotype 12). "CF12" has an allele having G or T at position 46 (see FIG. 37), and therefore, according to the above definition, the left columns indicate a polymorphism of "CF12" with a genotype 3: T/T. Likewise, "BKR1" has an allele having C or T at position -58 (see FIG. 37), and accordingly, indicated in the middle columns is a polymorphism of "BKR1" with a genotype 3:T/T. "IL-182" has an allele having G or C at position -137 (see FIG. 37). Here, as the genotype of "IL-182" is "12", indicated in the right columns is a polymorphism of "IL-182" with a genotype 1:C/C or 2:C/G. Considering the combination of genotypes of these three polymorphisms, it can be seen that two different polymorphism sets are denoted: a combination (set) of T/T (CF12), T/T (BKR1), and C/C (II-182); and a combination (set) of T/T (CF12), T/T (BKR1), and C/G (II-182).

These two polymorphism sets can be individually employed, or alternatively, it is also possible to combine C/C (II-182) and C/G(II-182) as C/?(II-182) [wherein ? signifies a possible candidate, G or C in this case], and employ the two sets as one set consisting of T/T(CF12), C/G(II-182), and C/?(II-182).

"Arteriosclerosis" means ischemic diseases in general, including angina pectoris, myocardial infarction, cerebral infarction, and peripheral arterial occlusion. Types of arteriosclerosis targeted in the present invention are especially those which develop due to diabetes. "Arteriosclerosis risk" indicates the likelihood of onset of such arteriosclerosis and the probability of its progression. "Myocardial infarction" is one form of arteriosclerosis, and "myocardial infarction risk" indicates, focusing on myocardial infarction among various arteriosclerotic diseases, the likelihood of occurrence of myocardial infarction and the probability of its progression.

**(1) Method of determining genetic polymorphisms for disease risk assessment**

The present invention provides a method of determining genetic polymorphisms for disease risk assessment, used in assessing the likelihood that a subject will get a disease or the probability that such a disease will progress (disease risk). The invention is explained below using arteriosclerosis as an example; however, the invention is not limited thereto and is applicable to any gene-related disease. In any case, the below-described correlation can be evaluated using an index suitable for each disease (e.g., carotid artery intima-media thickness for arteriosclerosis; urinary albumin excretion rate for nephropathy; and for myocardial infarction, the presence of abnormal Q waves on an electrocardiogram indicating old myocardial infarction, or the presence of a past history of myocardial infarction) (see, e.g., *"Tounyoubyou chiryou gaido 2004-2005* (Guide to the Treatment of Diabetes Mellitus, 2004-2005)", ed. Japan Diabetes Society, Bunkoudo).

(1-1) Polymorphisms for assessing arteriosclerosis risk

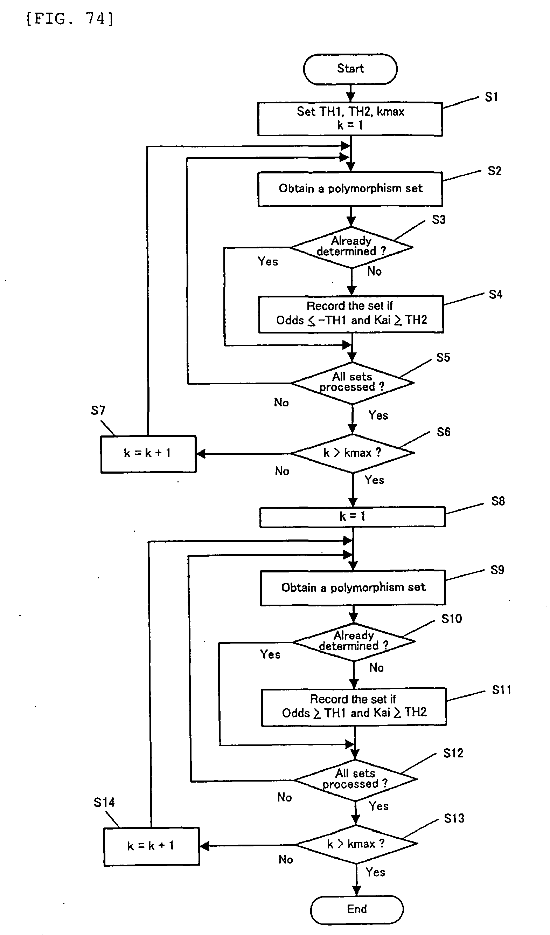

The method of the invention of determining polymorphisms for arteriosclerosis risk assessment is explained hereafter with reference to the flowchart shown in FIG. 74. The processing described herein is performed using a computer equipped with a CPU, a memory, a storage device (e.g., hard disk drive), a controlling device (e.g., keyboard, mouse), a display device (e.g., CRT display), etc. That is, data to be processed are inputted from a controller etc., and recorded in a storage unit. A CPU executes each processing using a memory as a work area. Interim results and final results of the processing are recorded in a suitable area of the storage unit as necessary.

First, information about genes associated with arteriosclerosis, which is the target disease, is collected from references, patient's medical records, and like sources, and polymorphisms used in the determination method are thereby preselected. This preselection can be made based on the experience of skilled persons, and it is also possible to use a computer to search third-party databases by entering the disease name and other relevant search terms, and make a selection according to the number of thereby obtained hits. Examples of such preselected polymorphisms are shown in FIG. 37.

FIG. 37 lists 99 polymorphisms chosen, based on experience, among about 200 polymorphisms obtained from various references (a detailed explanation of FIG. 37 is provided later). A numeral indicating a figure used alone in this specification includes its sub-letters, unless the sub-letters are explicitly mentioned. Thus, "FIG. 37" refers to both "FIG. 37-A" and "FIG. 37-B".

The processing according to the flowchart of FIG. 74 is performed using a predetermined number of polymorphisms chosen in the above preselection.

In the processing, carotid artery intima-media thickness (IMT) is employed as an arteriosclerosis index, and the association between the index and polymorphisms is statistically analyzed. The method of measuring IMT is explained later. For this purpose, in a storage unit, IMTs of individuals in a population are each matched with the corresponding polymorphisms (each having a genotype, same hereafter) of such individuals, and prerecorded as data for subsequent analysis. The data is recorded, for example, together with an individual ID given to each individual, in the form of [individual ID, IMT, polymorphisms].

In step S1, thresholds TH1 and TH2, which are used in and after step S2, and the upper limit kmax of a counter indicating the number of the iterations during the processing are determined, and 1 is set as the initial value of counter k. TH1 and TH2 are the thresholds of the below-described Odds (odds ratio) and Kai (chi-square value), respectively. TH1 is at least 2. TH2 is a threshold for determining statistical significance and is at least 0. For example, when TH2 = 6.63, the probability p that the target phenomenon will happen is $p < 0.01$. The upper limit kmax is preferably 2 to 5, and more preferably 2 or 3. Although it will complicate the analysis, the upper limit kmax may also be 6 or more, i.e., the processing may process polymorphism sets each consisting of 6 or more polymorphisms.

In step S2, k polymorphisms having genotypes are chosen from preselected polymorphisms $G_i$ ($_i$ = 1 to n) and employed as a set.

In step S3, it is assessed whether the set selected in step S2 includes any of the polymorphism sets that have already been assessed as being significant in step S4 of a prior iteration, and if it is assessed that such a set is included, step S5 is performed, and if not included, then step 4 is performed. When k = 1, step S4 is performed.

In step S4, Odds (odds ratio) and Kai (chi-square value) of the set selected in step S2 are calculated, and only when Odds ≤ -TH1 and Kai ≥ TH2, such Odds and Kai are recorded in correspondence with the set. Odds is calculated using IMT corresponding to an individual ID associated with all the constituent polymorphisms $G_j$ of the set. Methods of calculating Odds (odds ratio) are well known in references, etc. (see, e.g., Yamada Y, Izawa H, Ichihara S, Takatsu F, Ishihara H, Hirayama H, Sone T, Tanaka M, Yokota M. "Prediction of the risk of myocardial infarction from polymorphisms in candidate genes", *N. Engl. J. Med.,* 2002, 347(24), pp. 1916-23), and methods of calculating Kai (chi-square value) are also widely known in statistics. The details of these calculation methods are therefore not given herein. An Odds value is normally 0 or more, and in this specification, the raw value is employed when the Odds calculated by such a conventional method is at least 1; however, when such a calculated value is less than 1, -1/Odds is obtained from the calculated value and used as the Odds. A set may comprise a combination of a polymorphism having genotype 1 and genotype 2. However, when a polymorphism with a genotype 1 and a polymorphism combination with genotypes 1 and 2 both fulfill Odds ≤ -TH1 and Kai ≤ TH2, it is preferable to select either of these according to a given criteria. Such a selection can be made, for example, by employing the one having the greater Kai, the one having the greater absolute value of Odds, or the one having a greater below-described third contributory value.

In step S5, it is determined whether all the possible sets each consisting of k polymorphisms have been processed, and steps S2 to S4 are performed again and again until the processing of all the sets is completed.

In step S6, it is determined whether k > kmax, and if it is determined that k is not larger than the upper limit kmax, step S7 is performed to increase the counter k by 1, and the processing returns to step S2. The processing of steps S2 to S7 is thereby reiterated until k = kmax.

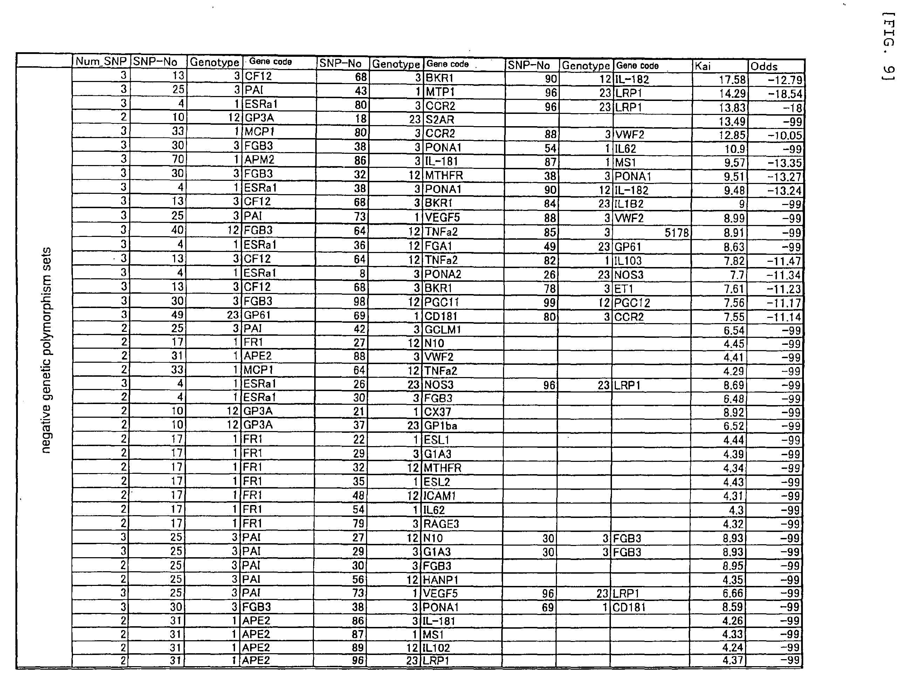

By the above processing of steps S2 to S7, sets negatively associated with a disease index (IMT in this case) (i.e., Odds is negative) are determined. For example, using the polymorphisms shown in FIG. 37, where kmax = 3 and TH2 = 6.63, the sets shown in FIG. 1 are obtained when TH1 = 2, and the sets shown in FIG. 2 are obtained when TH1 = 3. The meaning of each item used in each figure is explained later. Likewise, where kmax = 3 and TH2 = 6.63, the sets shown in FIG. 3, FIG. 4, FIG. 5, FIG. 6, FIG. 7, FIG. 8, and FIG. 9 are respectively obtained when TH1 = 4, TH1 = 5, TH1 = 6, TH1 = 7, TH1 = 8, TH1 = 9, and TH1 = 10.

In step S8, counter k is reset to be k = 1 in order to determine sets having a positive association (i.e., Odds is plus) in the same manner as in steps S2 to S7.

In steps S9 to S14, the same processing as in steps S2 to S7 is performed. In step S10, it is assessed whether the set includes polymorphisms that form any of the sets which have already been assessed as being significant in step S11 of a prior iteration. In step S11, only when Odds ≥ TH1 and Kai ≥ TH2 are such Odds and Kai recorded in correspondence with the set.

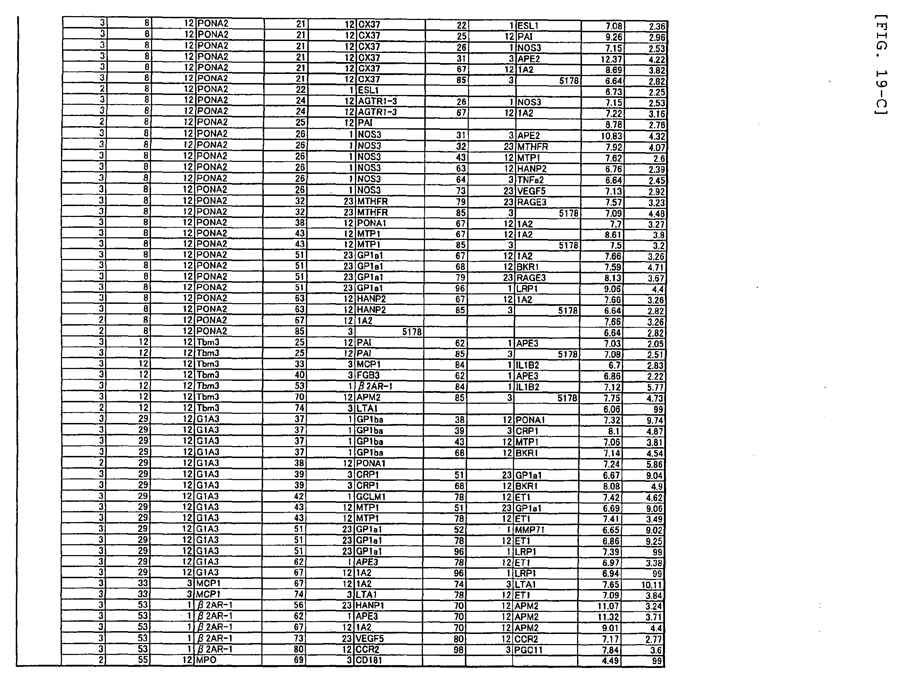

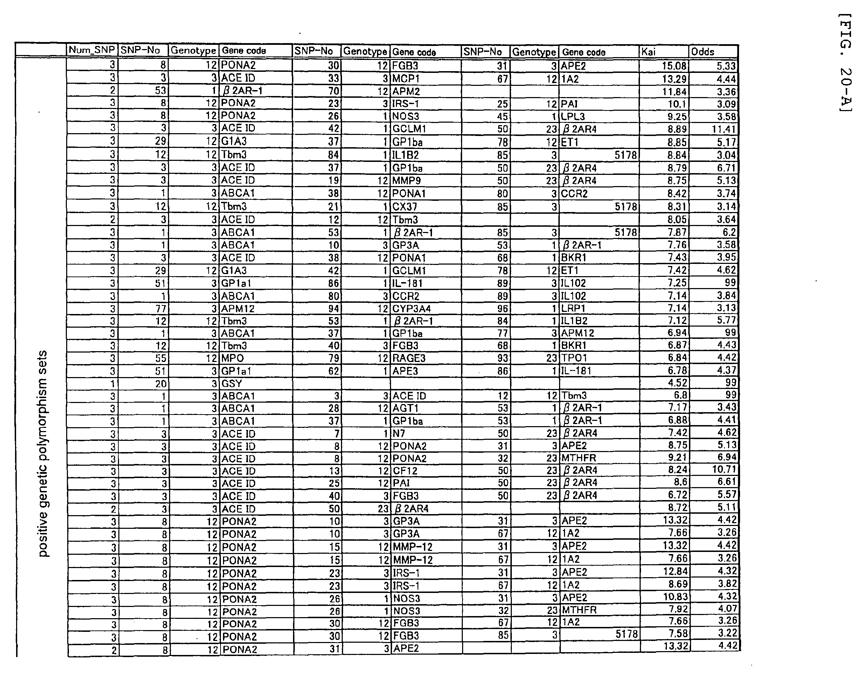

By such processing of steps S9 to S14, sets positively associated with a disease index (IMT in this case) (i.e., Odds is positive) are determined. For example, using the polymorphisms shown in FIG. 37, where kmax = 3 and TH2 = 6.63, the sets shown in FIG. 19 are obtained when TH1 = 2, and the sets shown in FIG. 20 are obtained when TH1 = 3. Likewise, where kmax = 3 and TH2 = 6.63, the sets shown in FIG. 21, FIG. 22, FIG. 23, FIG. 24, FIG. 25, FIG. 26, and FIG. 27 are respectively obtained when TH1 = 4, TH1 = 5, TH1 = 6, TH1 = 7, TH1 = 8, TH1 = 9, and TH1 = 10.

This completes the determination of polymorphism sets useful in assessing arteriosclerosis risk.

With respect to the flowchart of FIG. 74, the processing shown therein may be modified in various ways as described below, and also, it may further include additional processes.

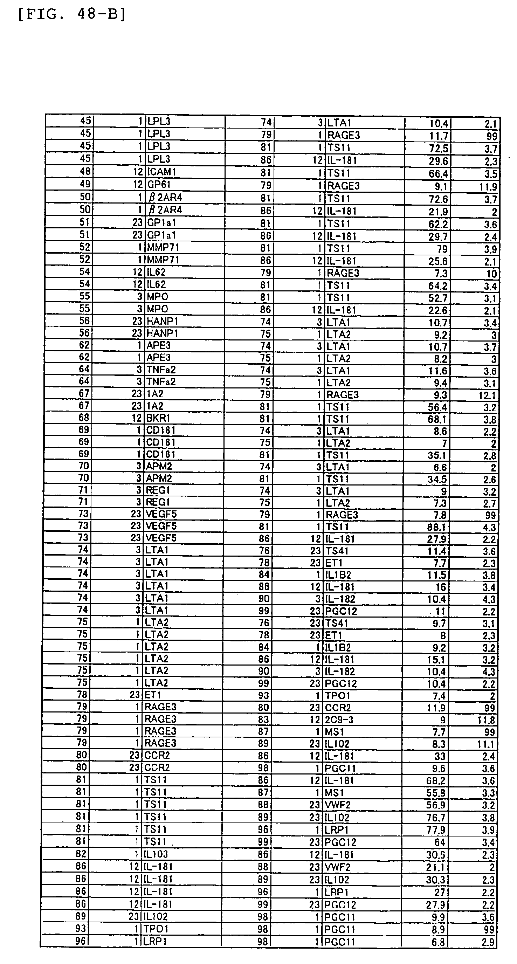

For example, although negative association and positive association with a disease index (IMT in this case) are treated equivalently in the above description, the processing may place a higher premium on either of these. That is, attaching more importance on negative association, it is possible to first determine negative sets, and, in consideration of the determination results, to then determine positive sets. Vice versa, attaching more importance on positive association, it is also possible to first determine positive sets, and, in consideration of the determination results, to then determine negative sets. For example, when negative association is emphasized, step S10 shown in FIG. 74 determines, in addition to whether the set includes polymorphisms that form any of the sets that have been assessed as being significant in step S11 of a prior iteration, whether the set includes polymorphisms that form any of the sets that have been assessed as being significant in step S4 of a prior iteration. When positive association is emphasized, the Odds evaluation formulae of steps S4 and S11 (Odds $\leq$ -TH1 and Odds $\geq$ TH1, respectively) are swapped, and it is determined in step S10 whether the set includes polymorphisms that form any of the sets that have been assessed as being significant in, in addition to step s11, step S4 of a prior iteration. Examples of sets obtained as a result of such processing are shown in FIGS. 38 to 43 and 48 to 51. FIG. 38, FIG. 39, FIG. 40, FIG. 41, FIG. 42, and FIG. 43 show negative sets obtained when TH1 = 2, TH1 = 3, TH1 = 4, TH1 = 5, TH1 = 6, TH1 = 7, respectively. FIG. 48, FIG. 49, FIG. 50, and FIG. 51 show positive sets obtained TH1 = 2, TH1 = 3, TH1 = 4, and TH1 = 5, respectively.

Sets of polymorphisms effective in assessing disease risk are determined as above. When it is necessary to reduce the number of polymorphisms, polymorphisms may be respectively ranked and selected according to the ranking.

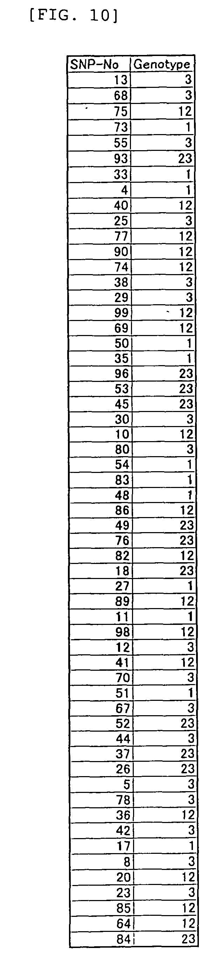

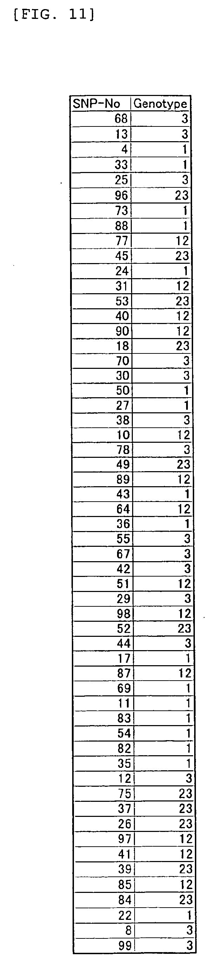

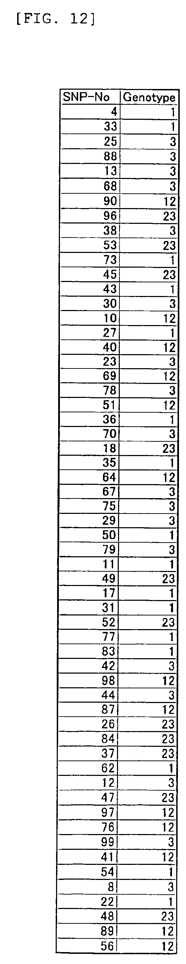

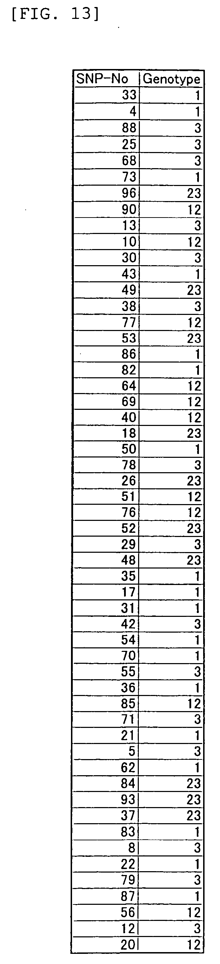

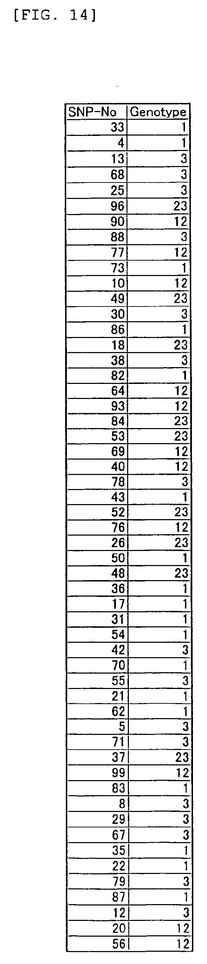

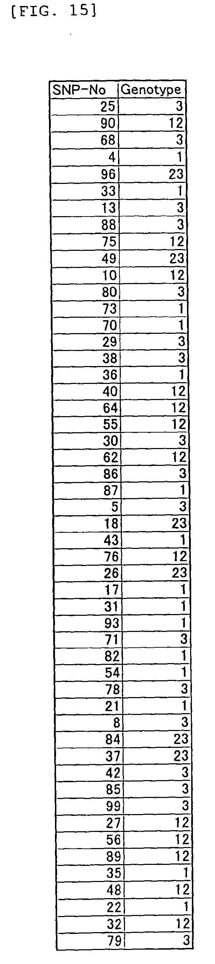

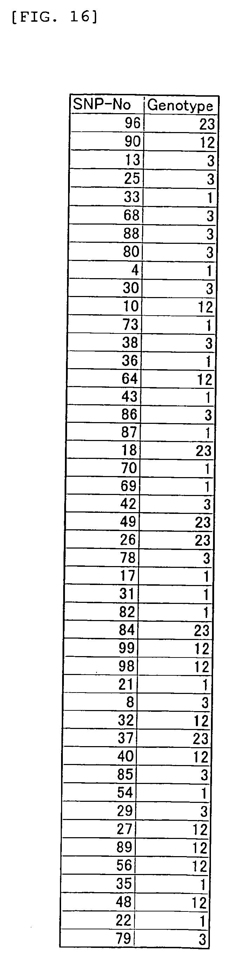

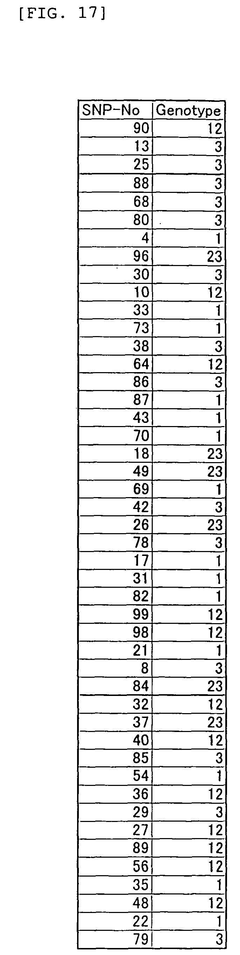

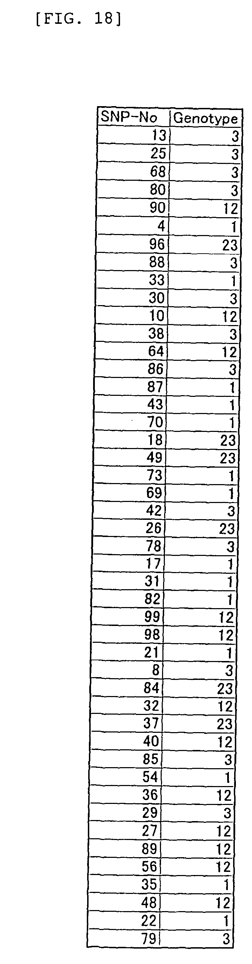

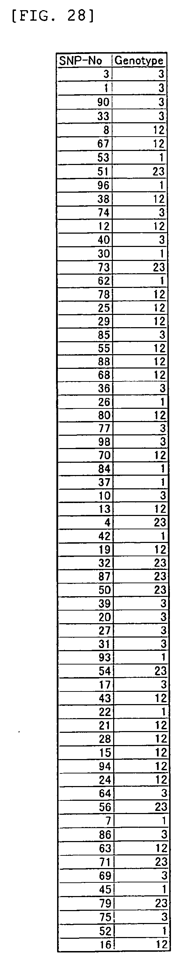

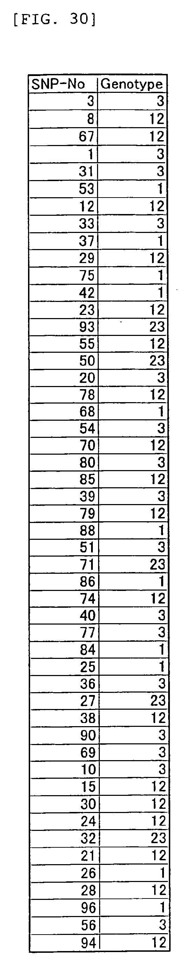

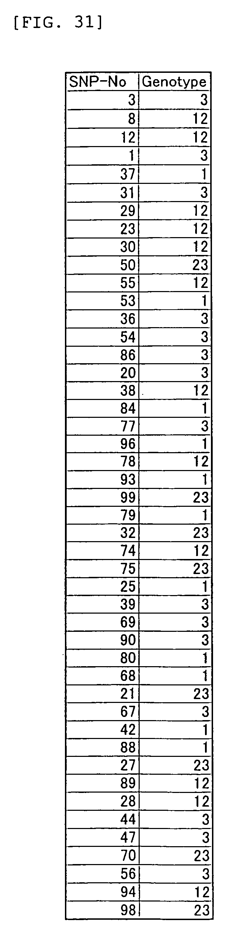

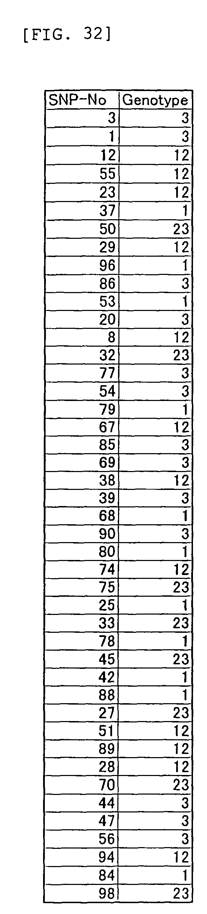

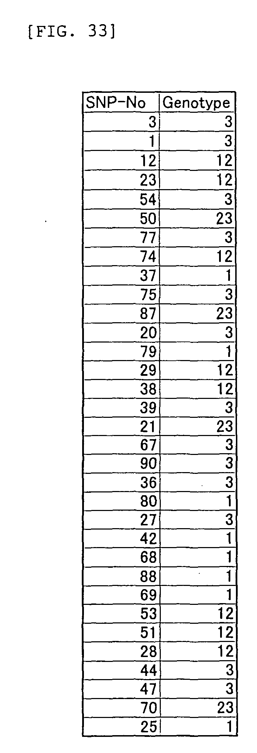

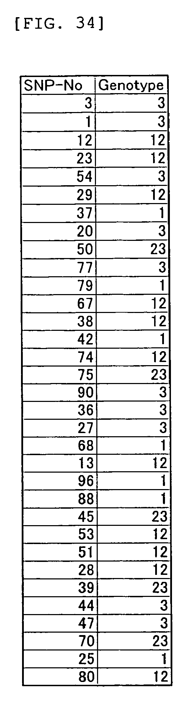

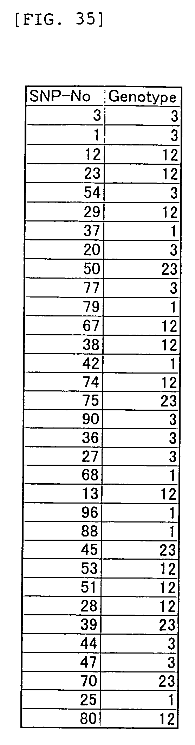

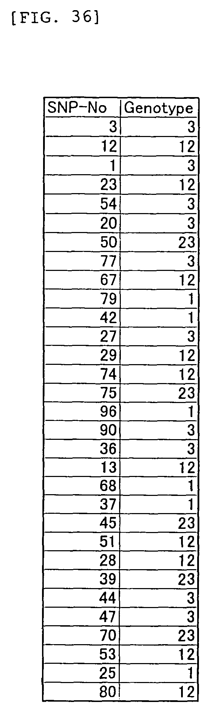

For example, polymorphisms can be selected according to their Odds. As a result, for example, in correspondence with FIG 1 (TH1 = 2), the polymorphisms as shown in FIG. 10 can be determined. Likewise, the polymorphisms as shown in FIGS. 11 to 18 are obtained from FIGS. 2 (TH1 = 3) to 9 (TH1 = 10), respectively, and the polymorphisms of FIGS. 28 to 36 are obtained from FIGS. 19 (TH1 = 2) to 27 (TH1 = 10), respectively.

Further, selection of particularly useful polymorphism sets is possible as follows.

First, with respect to the subjects in a population, a first contributory value of each set of the polymorphisms determined in the processing shown in FIG. 74 is calculated by determining by how many sets each case (patient) can be explained. For example, if a case #210 is explained by five sets (one set thereof is referred to as set 20), the first contributory value (contribution to the case) of each set (e.g., set 20) to the case (i.e., case #210) is 1/5 = 0.2.

Next, the sum of first contributory values to all the cases that a set explains (if set 20 explains 10 cases, the sum of its contributions to these 10 cases) is obtained, and then a second contributory value of each of its constituent polymorphisms according to its genotype is calculated from the sum. For example, when set 20 consists of two polymorphisms SNP 1-1 and SNP 13-23, the sum of first contributory values of set 20 is equally divided into two, and then assigned to each of the polymorphisms with specific genotypes. More specifically, when the sum of first contributory values of set 20 is 1.4, then 1.4/2 = 0.7 is assigned to each of SNP 1-1 and SNP 13-23. The sum of first contributory values of each set is divided for each of the constituent polymorphisms (if a set consists of three polymorphisms each having a genotype, the sum is divided into three), and then a second contributory value of each polymorphism according to its genotype is calculated. In FIGS. 10 to 18 and 28 to 37, polymorphisms are arranged from the top in decreasing order of second contributory values (not shown).

The greatest of the second contributory values of the genotypes corresponding to a polymorphism is defined as a third contributory value of such a polymorphism, and the genotype providing the greatest contributory value is employed as an effective genotype of this polymorphism. For example, if the second contributory value of ACE-DD (1) is 5 and the second contributory value of ACE-DD+D/I (1 + 2) is 2, ACE-DD is defined as an effective polymorphism of ACE gene. The third contributory value of this polymorphism is 5.

Among sets of the polymorphisms determined by the series of processes shown in FIG. 74, those containing above-explained effective polymorphisms are then selectively chosen. For example, when the effective genotype for ACE is DD, then a set containing a polymorphism of ACE with the genotype DD+D/I (1+2) is abandoned.

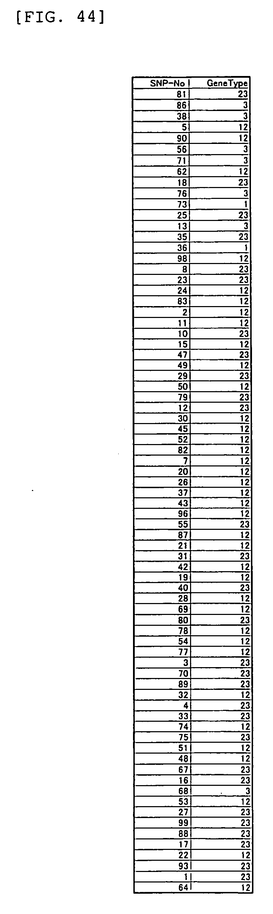

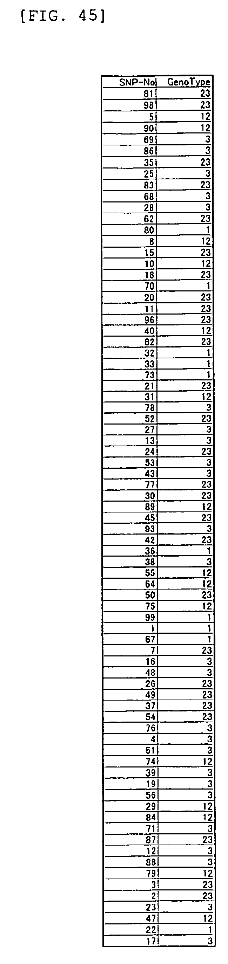

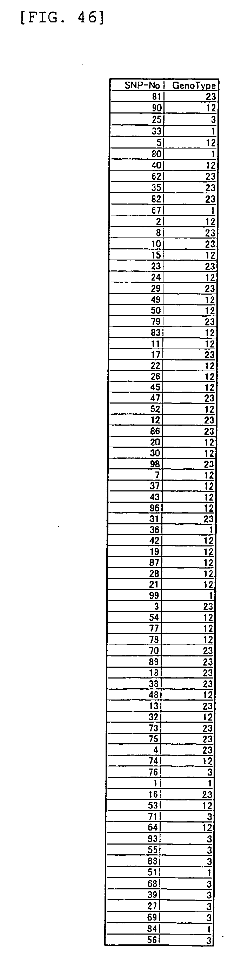

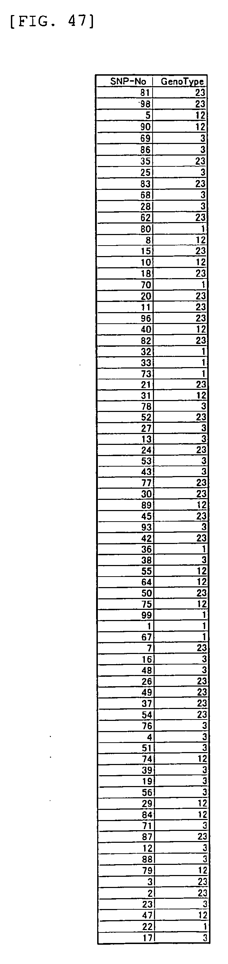

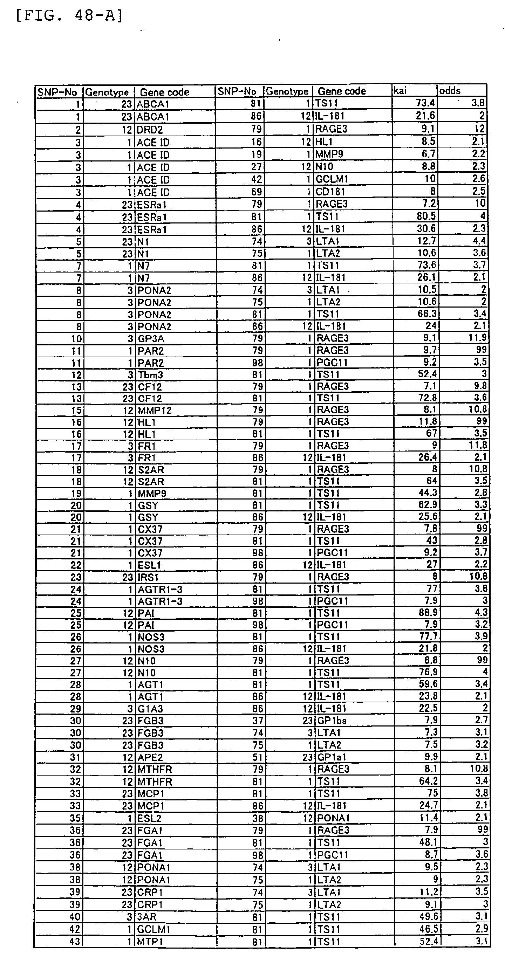

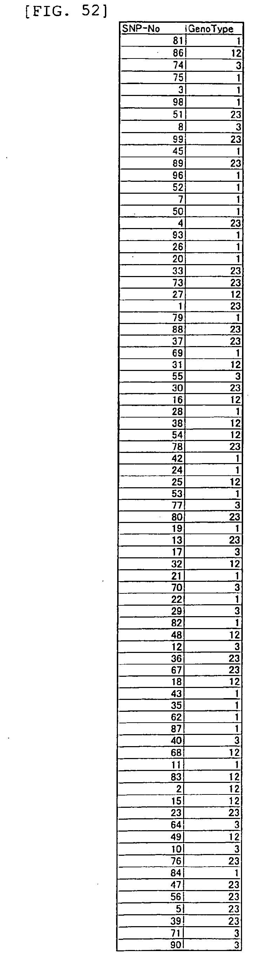

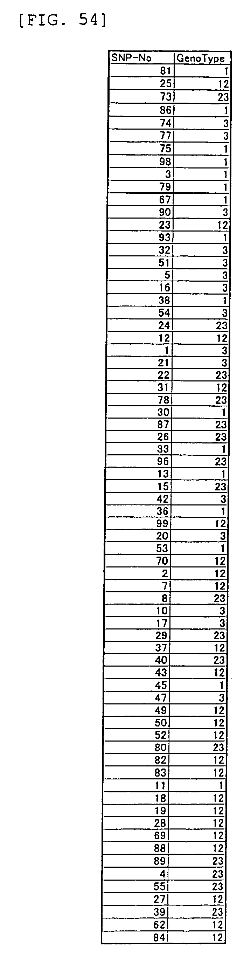

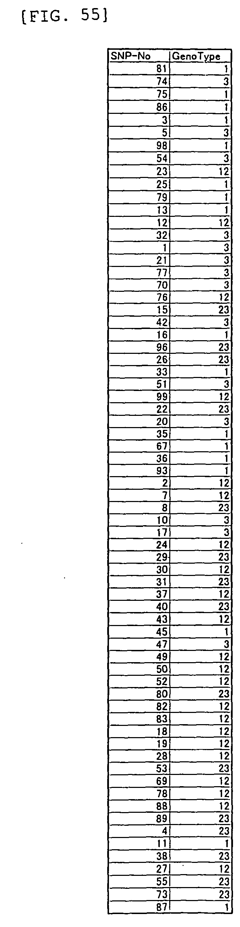

Accordingly, for example, in correspondence with FIG. 38 (TH1 = 2), the polymorphisms as shown in FIG. 44 are determined. Likewise, FIGS. 45 to 47 are obtained from FIGS. 39 (TH1 = 3) to 43 (TH1 = 7), and FIGS. 52 to 55 are obtained based on FIGS. 48 (TH1 = 2) to 51 (TH1 = 5), respectively. In FIGS. 44 to 47, polymorphisms are arranged from the top in decreasing order of third contributory value (not shown).

A contributory value can be an index of association between each polymorphism and IMT, indicating the degree of association between the polymorphism and arteriosclerosis. Therefore, by selecting polymorphisms whose contributory values are at a certain level or higher, the below-described risk assessment can be preformed using a reduced number of polymorphisms. If the threshold for selection is appropriately specified, accuracy is barely degraded.

When additional clinical data are obtained, even more effective polymorphism sets can be determined by applying the above-described method of determining polymorphisms to an updated data group containing such newly obtained clinical data, whereby the accuracy of disease risk assessment can be further enhanced.

The information about an individual's polymorphisms itself can be used as an ID. When, for example, data are recorded in the above form of [individual ID, disease index value, polymorphisms], if individual IDs are given and administered by each hospital, the individual ID will be changed when the subject changes hospitals. Accordingly, it becomes impossible to track one's past history, and the data cannot be put to effective use. In contrast, because polymorphism information is unchanging information unique to an individual, by using such information itself as an ID, it is possible to obtain the past data to be analyzed, in particular, past disease index values (e.g., IMT measurement values in case of arteriosclerosis), without referring to an individual ID. Analyses in consideration of personal history are accordingly achievable, which makes it possible to determine polymorphisms even more effective in disease risk assessment. The form of data is not limited to [individual ID, disease index value, polymorphisms], and it may also include, for example, various clinical data other than disease index values.

(1-2) Determination of polymorphisms for assessing myocardial infarction risk

Polymorphisms and polymorphism sets useful in assessing the risk of myocardial infarction can be determined in the same manner as above using, as a disease index, the presence of abnormal Q waves on an electrocardiogram indicating old myocardial infarction, or the presence of a past history of myocardial infarction.

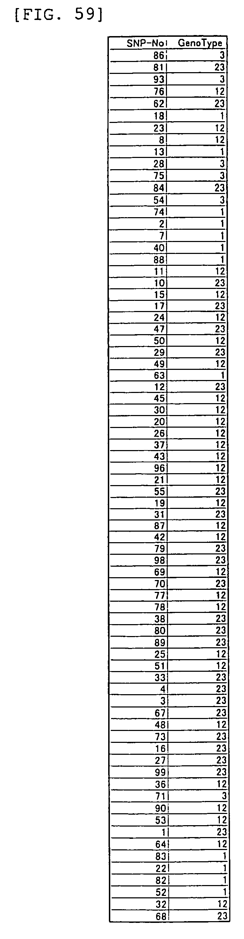

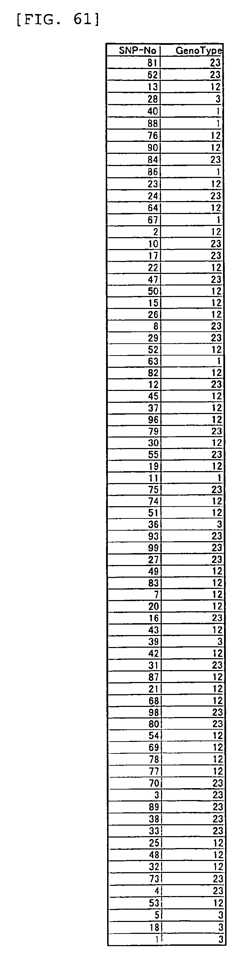

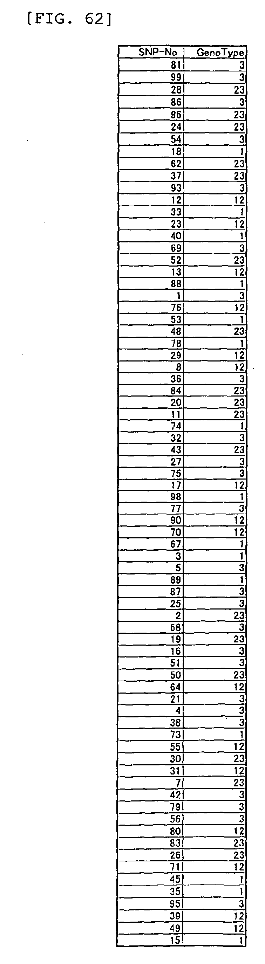

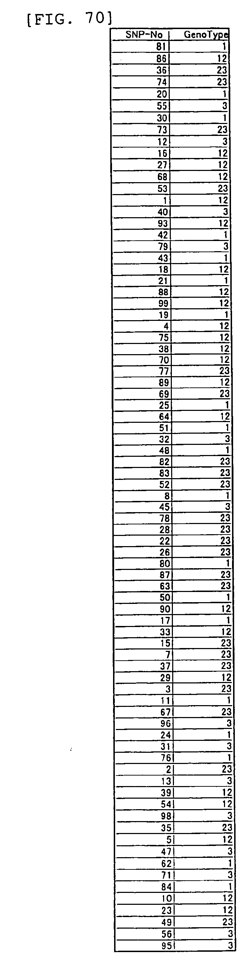

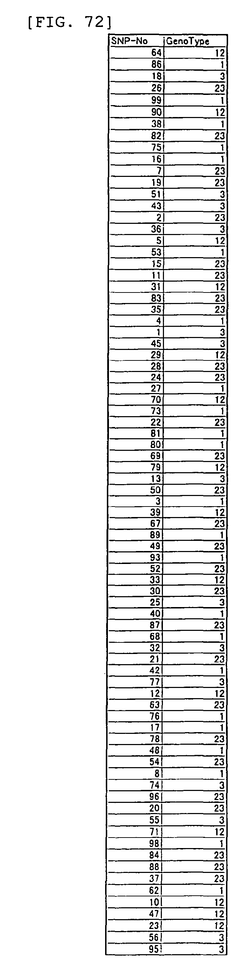

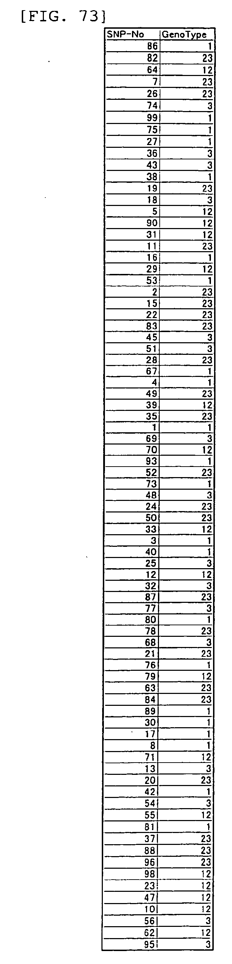

For example, FIG. 56, FIG. 57, and FIG. 58 show a list of polymorphism sets negatively associated with myocardial infarction, which are determined when TH1 = 2, TH1 = 3, and TH1 = 4, respectively. FIG. 63, FIG. 64, FIG. 65, FIG. 66, FIG. 67, FIG. 68, and FIG. 69 show polymorphism sets positively associated with myocardial infarction, which are determined when TH1 = 2, TH1 = 3, TH1 = 4, TH1 = 5, TH1 = 6, TH1 = 7, and TH1 = 8, respectively. FIGS. 59 to 62 show polymorphisms determined using FIGS. 56 to 58 (TH1 = 2 to 3) respectively, and FIGS. 70 to 73 show polymorphisms determined using FIGS. 63 to 69 (TH1 = 2 to 8) respectively.

The polymorphisms listed in FIG. 37 can be categorized as follows:

a) lipid-related polymorphisms,
b) blood pressure-related polymorphisms,
c) metabolism-related polymorphisms,
d) insulin resistance-related polymorphisms,
e) adhesion molecule-related polymorphisms,
f) oxidative stress-related polymorphisms,
g) inflammatory response-related polymorphisms,
h) coagulation and fibrinolytic system-related polymorphisms,
i) obesity-related polymorphisms, and
j) cell proliferation- or vascular proliferation-related polymorphisms.

Polymorphisms related to these factors herein means not only those present in exons and/or introns of such factor-related genes, but also include polymorphisms present in promoter regions, 3' noncoding regions, 5' noncoding regions, and the like. Polymorphisms in coding regions occasionally cause changes in amino acid sequences and mRNA expression, and even polymorphisms in regulatory regions may induce changes in mRNA expression and splicing. That is, there is always a possibility that the expression and characteristics of proteins might be altered.

Specific examples of polymorphisms of each category are as follows.

a) Examples of lipid-related polymorphisms include ABCA1, HUMPONA, PPAR gamma, hepatic lipase (C-480T), Apo E (Cys112Arg), PON1 (Gly192Arg), microsomal triglyceride transfer protein (G-493T), CETP (Arg451Glu), lipoprotein lipase (Ser447STOP), PPARgamma (Leu162Val), ABCC6 (C3421T), apolipoproteinE (E3 inexon 4 (Arg 158Cys), adiponectin (T94G), Adiponectin (G276T), Scavenger receptor BI=CLA-1 (G4A(Gly2SEr)), LDL receptor related protein (C766T), adiponectin (Arg112Cys), and Scavenger receptor BI=CLA-1 (G403A(Va1135Ile));
b) Examples of blood pressure-related polymorphisms include Dopamine-D2receptor (Ser311Cys), ACE (I/D), AT2-receptor (A1166C), angiotensinogen (t704c), HANP (T2238C), HANP (C708T), bradykinin B2 receptor (C-58T), and endothelin-1 (G5665T);
c) Examples of metabolism-related polymorphisms include Alfa#estrogen#receptor (P vuII), MTHFR (C677T), CYP2J2*2 (A14487G), CYP2J2*3 (C14532T), CYP2J2*4 (15028T), CYP2J2-6 (A25661T), CYP2C9*3 (Leu359Ile), and CYP3A4 (A-290G).
d) Examples of insulin resistance-related polymorphisms include Enos (T-786C), glycogen#synthase ((M416V), IRS-1 (G3494A(Gly972Arg)), Enos (Glu298Asp(G894T)), TGF beta (T29C(Leu10Pro)), resistin (ATG repeat)(1:6/6, 2:6/7, 3:7/7, 4:7/8, 5:8/8), RAGE (Gly82Ser), PGC-1 (G1302A(Thr394Thr)), and PGC-1 (G1564A(Gly482Ser)).
e) Examples of adhesion molecule-related polymorphismsinclude P-selectin (A76666C(Thr715Pro)), fractalkine#receptor (G84635A(Val249Ile)), connexin37 (C1019T(Pro319Ser)), E-selectin (G98T), E-selectin (Ser128Arg), ICAM1 (E469K), GlycoproteinVI (Ser219Pro), and glycoproteinIa (C807T).
f) Examples of oxidative stress-related polymorphisms include p22phox (C242T(His72Tyr)), Mitochondria (A5178C), Mitochondria (A12026G), EPHX2 (Arg402-403ins.inExon13), Mitochondria (C1310T), and Mitochondria (T14577C).

g) Examples of inflammatory response-related polymorphisms include IL-6 (G-174C), CRP (G1059C), TNFalfa (G-238A), interleukin6 (C-634G), MPO (G-463A), TNF-alfa (G-308A), CD18 (C1323T), LTA (A252G), LTA (C804A (Thr26Asn)), C-C chemokine receptor 2 (G190A), Interleukin10 (G-1082A), interleukin 1 beta (C3953T), IL-18 (C-607A), IL-10 (C-819T), IL-18 (G-137C), and interleukin 1 receptor antagonist (tandem repeat (2 repeat) in intron 2).
h) Examples of coagulation and fibrinolytic system-related polymorphisms include PIIbIIIa (C1565T(PIA2)), Thrombomodulin (G-33A), FactorXII, serotonin#2A#receptor (T102C), PAI-1 (4G-668/5G), GPIa (A1648G), beta Fib (C148T), prothrombin (G20210), alfa-Fib (Thr312Ala), FactorV (G1691A), GP Ia (G873A), Thrombospondin4 (G1186C(Ala387Pro)), Thrombospondin-1 (A2210G), von Willebrand Factor (G-1051A), and Thrombopoietin (A5713G).
i) Examples of obesity-related polymorphisms include beta3 adrenoceptor (Trp64Arg), beta2 Adrenoreceptor (C79T), beta-adrenergic receptor (A46G), and beta2 adrenoceptor (C491T).
j) Examples of cell proliferation- or vascular proliferation-related polymorphisms include VEGF (C-634G) and Glutamate-cystein ligase (C-588T).

Polymorphisms usable in the present invention further include neuropepyideY (T1128C(Leu7Pro)), MMP-12 (A-82G), mmp-9=Gelatinase B (C-1562T), MCP-1 (A-2518G), HPA-2 (Thr145Met), MMP7 (C-153T), matrilyn promoter (A-181G), AMPD (C34T), Methionine synthase (A2756G(Asp919gly)), matrix Gla protein (G-7A), etc.

**(2) Array for assessing disease risk**

The present invention provides an array for assessing the likelihood that a subject will get a disease and the probability that such a disease will progress (disease risk), based on the genetic polymorphisms that the subject possesses. The array of the invention comprises a probe or probes for detecting one or more target polymorphisms, the probe or probes being arranged and fixed at a high density on a support medium such as a silicon wafer or a glass slide. Usable probes are any of those capable of specifically recognizing and trapping certain polymorphisms. A concrete example thereof is a probe having a base sequence comprising all or part of a base sequence corresponding to a certain polymorphism or of a complementary sequence thereto.

In particular, the present invention provides an array for assessing arteriosclerosis risk and an array for assessing myocardial infarction risk.

(2-1) Array for assessing arteriosclerosis risk

The array of the invention for assessing arteriosclerosis risk can be used to assess the likelihood of arteriosclerosis (the likelihood of onset) and of its progression. It can be advantageously used to assess arteriosclerosis risk of diabetics and patients prone to diabetes. Such an array of the invention is characterized by comprising a probe or probes for detecting one or more polymorphisms that form a "negative (resistant) polymorphism set", which is significantly negatively associated with "carotid artery intima-media thickness (IMT)" that is an arteriosclerosis index. The presence of a "negative association" between a polymorphism set and IMT can be determined from an odds ratio (Odds) obtained by a method described in (1) above. That is, a "negative association" is indicated when the Odds value is negative (minus), while a "positive association" is indicated when the Odds value is positive (plus). Whether such a negative or positive association is significant can be evaluated using a qui-square value (Kai).

Specific examples of such "negative (resistant) polymorphism sets" are shown in FIGS. 1 to 9 and 38 to 43. More specifically, in FIGS. 1 to 9 and 38 to 43, a combination of polymorphisms (SNPs) arranged in a single horizontal row is one "polymorphism set negatively associated with (resistant to) arteriosclerosis". Note that, as is explained above, although FIG. 1, for example, consists of two figures indicated by the sub-letters "FIG. 1-A" and "FIG. 1-B", "FIG. 1" used alone refers to both "FIG. 9-A" and "FIG. 9-B" (and likewise for any figures having sub-letters hereafter). "Polymorphism" as used herein encompasses genotype as described above, and accordingly, this term means a polymorphism having a specific genotype. In these figures, each polymorphism has an indicated "gene code". Detailed information about each polymorphism is as given in FIG. 37.

The array of the invention for assessing arteriosclerosis risk comprises a probe or probes for detecting one or more polymorphisms of at least one polymorphism set selected from the negative polymorphism sets shown in any one of FIGS. 1 to 9, preferably FIGS. 5 to 9, more preferably FIGS. 6 to 7, still more preferably FIGS. 7 to 9, and yet more preferably FIGS. 8 and 9. The selection of polymorphism sets is not limited and can be made in any manner. For the selection, the Odds and Kai that are given for every polymorphism set in each figure can be used as indices. The array of the invention preferably comprises a probe or probes for detecting one or more constituent polymorphisms of a polymorphism set that is evaluated to have a high negative association with IMT, based on the Odds and Kai.

Such a probe or probes for detecting one or more polymorphisms of at least one negative set preferably detect at least half of, preferably at least 60%, 70%, 80%, or 90% of, and more preferably all of the polymorphisms shown in one of FIGS. 10 to 18.

FIGS. 10 to 18 categorize the polymorphism sets negatively associated with IMT (resistant) shown in FIGS. 1 to 9 into their constituent polymorphisms according to the Odds of the sets indicated therein. FIG. 10 is a list of polymorphisms that form the negative polymorphism sets with Odds of -2 or less (i.e., Odds is 1/2 or less) shown in FIG. 1. Likewise, FIG. 11, FIG. 12, FIG. 13, FIG. 14, FIG. 15, FIG. 16, FIG. 17, and FIG. 10 respectively list polymorphisms that make up the negative polymorphism sets with Odds of -3 or less (i.e., Odds is 1/3 or less) shown in FIG. 2, Odds of -4 or less (i.e., Odds is 1/4 or less) shown in FIG. 3, Odds of -5 or less (i.e., Odds is 1/5 or less) shown in FIG. 4, Odds of -6 or less (i.e., Odds is 1/6 or less) shown in FIG. 5, Odds of -7 or less (i.e., Odds is 1/7 or less) shown in FIG. 6, Odds of -8 or less (i.e., Odds is 1/8 or less) shown in FIG. 7, Odds of -9 or less (i.e., Odds is 1/9 or less) shown in FIG. 8, and Odds of -10 or less (i.e., Odds is 1/10 or less) shown in FIG. 9.

When selecting at least half, 60%, 70%, 80%, or 90% of the polymorphisms shown in one of the above figures, although not limited thereto, it is preferable that those closer to the top of the columns are selectively chosen.

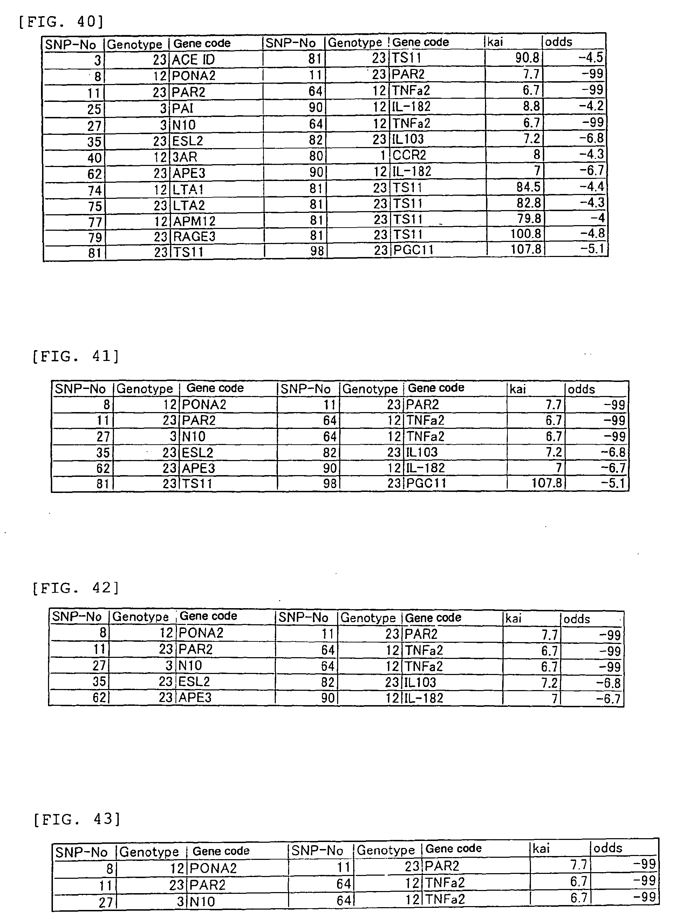

The array of the invention for assessing arteriosclerosis risk may instead comprise a probe or probes for detecting one or more polymorphisms of at least one polymorphism set selected from the negative polymorphism sets shown in any one of FIGS. 38 to 43, preferably FIGS. 39 to 43, more preferably FIGS. 40 to 43, still more preferably FIGS. 41 to 43, and yet more preferably FIGS. 42 and 43. As aforementioned, the array of the invention preferably comprises a probe or probes for detecting one or more constituent polymorphisms of a polymorphism set that is evaluated to have a high negative association with IMT, based on Odds and Kai.

Such a probe or probes for detecting one or more polymorphisms of at least one negative set preferably detect at least half of, preferably at least 60%, 70%, 80%, or 90% of, and more preferably all of the polymorphisms shown in one of FIG. 44 to 47. FIGS. 44 to 47 categorize the polymorphism sets negatively associated with IMT (resistant) shown in FIGS. 38 to 43 into their constituent polymorphisms according to the Odds of the sets indicated therein. FIG. 44 is a list of polymorphisms that form the negative polymorphism sets with Odds of -2 or less (i.e., Odds is 1/2 or less) shown in FIG 38. Likewise, FIG. 45, FIG. 46, and FIG. 47 respectively list the polymorphisms the make up the negative polymorphism sets with Odds of -3 or less (i.e., Odds is 1/3 or less) shown in FIG. 39, Odds of -4 or less (i.e., Odds is 1/4 or less) shown in FIG. 40, and Odds of -5 or less (i.e., Odds is 1/5 or less) shown in FIG. 41. As with selecting from FIGS. 10 to 18, when selecting at least half, 60%, 70%, 80%, or 90% of the polymorphisms shown in one of the above figures, it is preferable that those closer to the top of the columns are selectively chosen.

The array of the invention for assessing arteriosclerosis risk preferably further comprises, in addition to the above-described probe or probes for detecting one or more polymorphisms of at least one negative (resistant) polymorphism set, a probe or probes for detecting one or more positive (sensitive) polymorphisms, or a probe or probes for detecting one or more polymorphisms of at least one positive (sensitive) polymorphism set.

A "positive (sensitive) polymorphism" means a polymorphism that is significantly positively associated with "carotid artery intima-media thickness (IMT)", which is an arteriosclerosis index. A "positive (sensitive) polymorphism set" means a "combination of polymorphisms" that is significantly positively associated with IMT. The presence of a "positive association" between a polymorphism set and IMT can be determined from Odds obtained by a method described in (1) above. That is, a "positive association" is indicated when the Odds value is positive (plus). Whether such a positive association is significant can be evaluated using Kai.

Specific examples of such "positive (sensitive) polymorphism sets" are shown in FIGS. 19 to 27 and 48 to 51. More specifically, in FIGS. 19 to 27 and 48 to 51, a combination of polymorphisms (SNPs) arranged in a single horizontal row is one "positive (sensitive) polymorphism set" positively associated with IMT. When a row contains a single polymorphism, such a polymorphism by itself constitutes a "positive (sensitive) polymorphism set" having a positive association with IMT (e.g., "GSY" shown in FIG. 19).

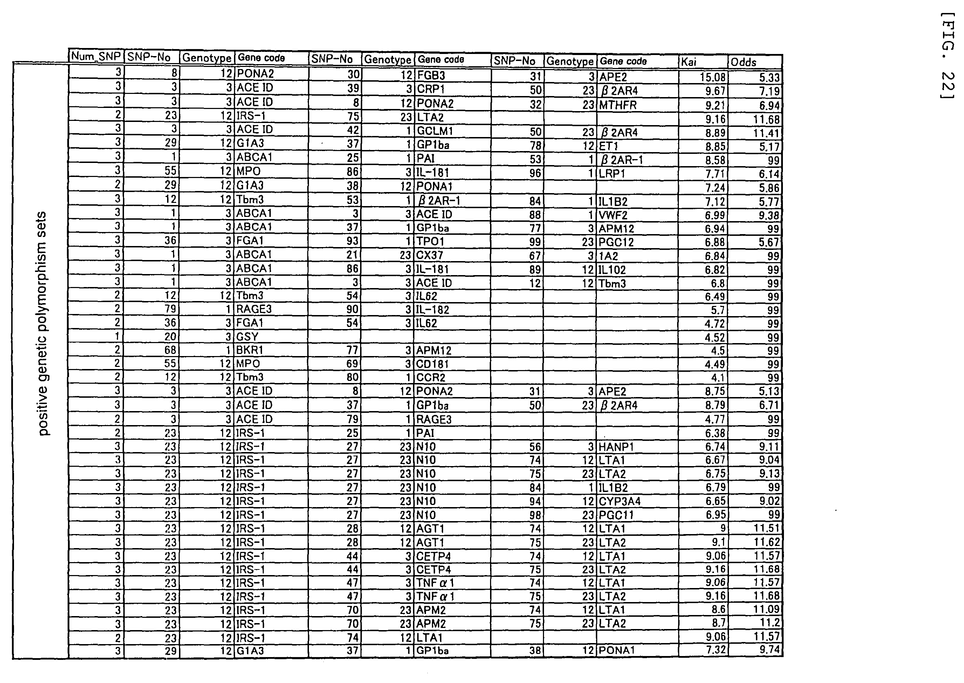

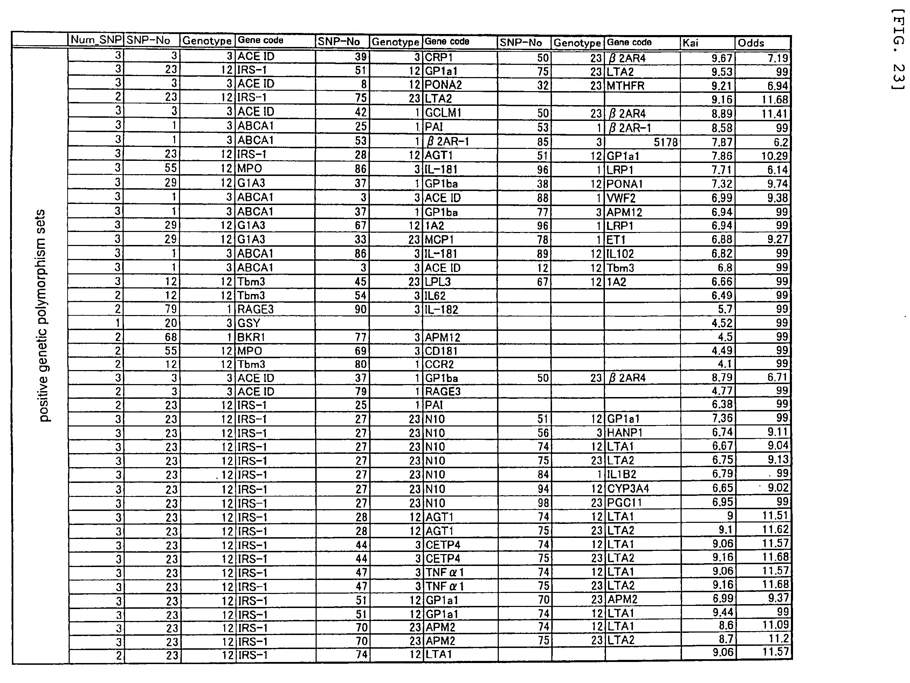

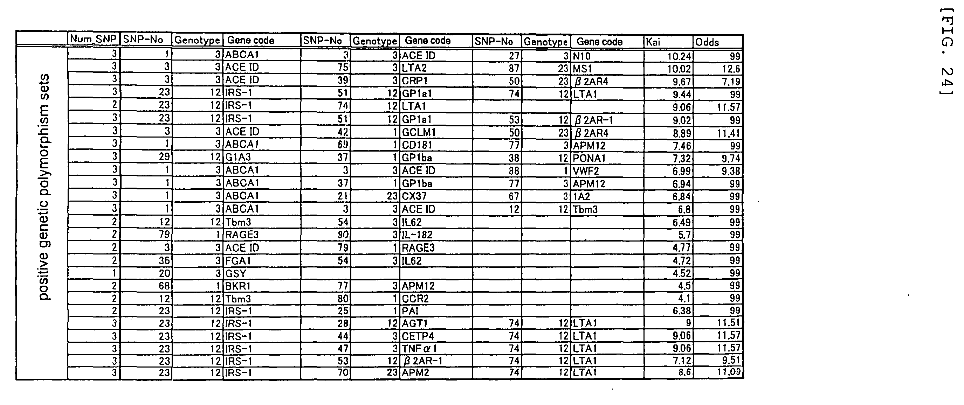

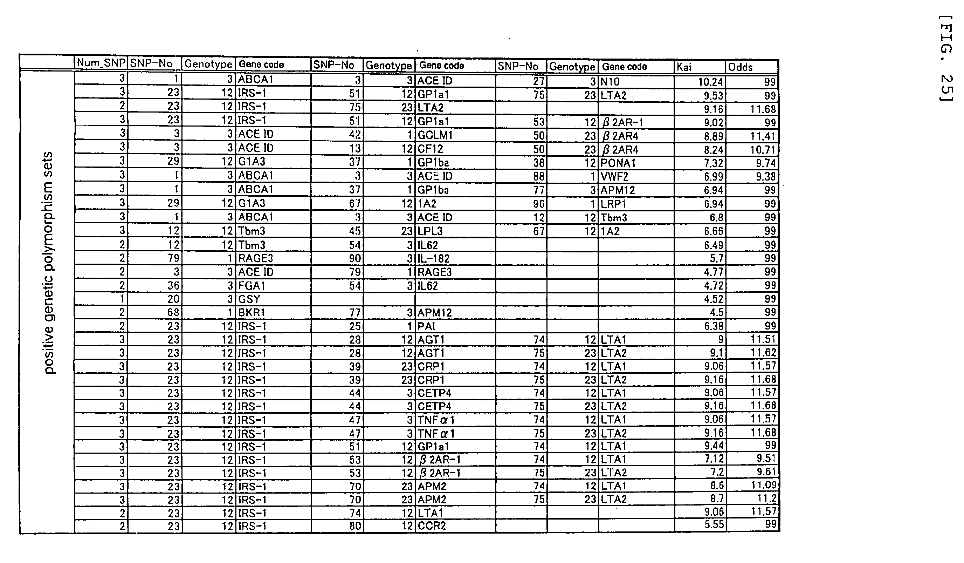

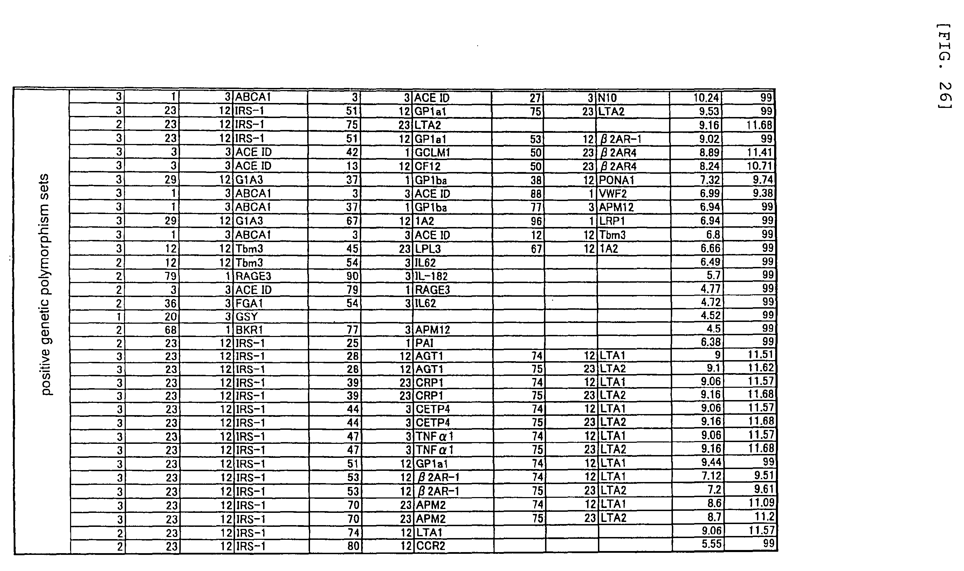

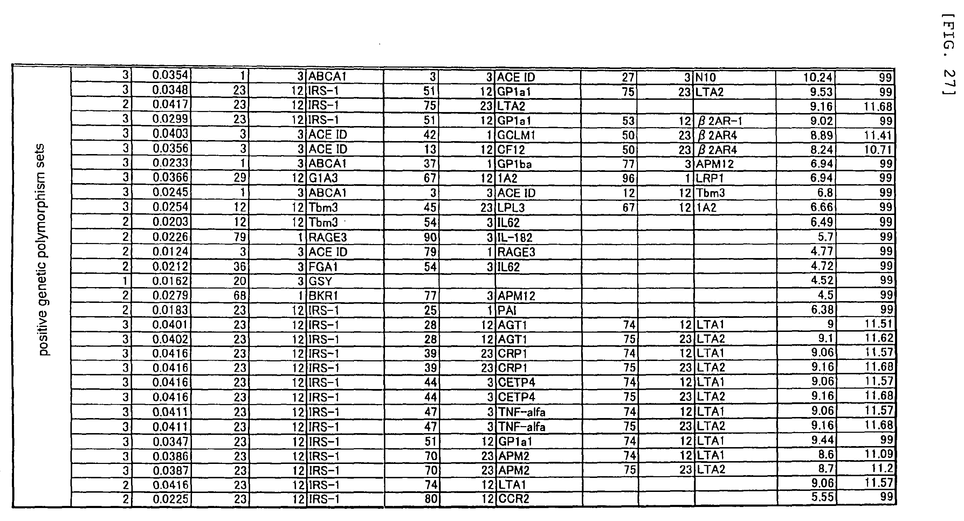

Specifically, the array of the invention may comprises, in addition to the probe or probes for detecting one or more polymorphisms of at least one polymorphism set selected from the negative polymorphism sets shown in any one of FIGS. 1 to 9, a probe or probes for detecting one or more polymorphisms of at least one polymorphism set selected from the positive polymorphism sets shown in any one of FIGS. 19 to 27, preferably FIGS. 23 to 27, more preferably FIGS. 24 to 27, still more preferably FIGS. 25 to 27, and yet more preferably FIGS. 26 and 27.

Such a probe or probes for detecting one or more polymorphisms of at least one positive polymorphism set preferably detect at least half of, more preferably at least 60%, 70%, 80%, or 90% of, and still more preferably all of the polymorphisms shown in one of FIGS. 28 to 36.

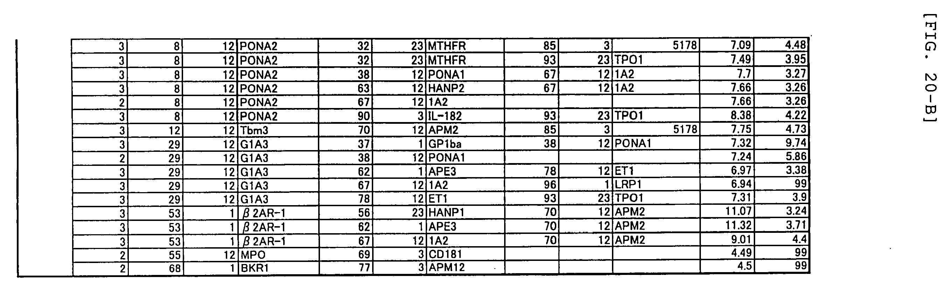

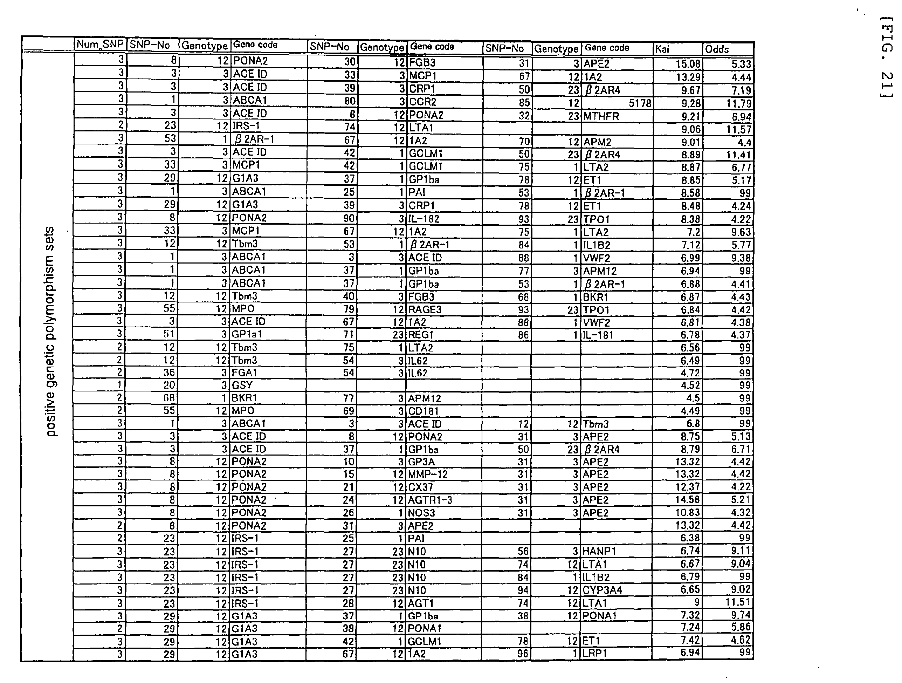

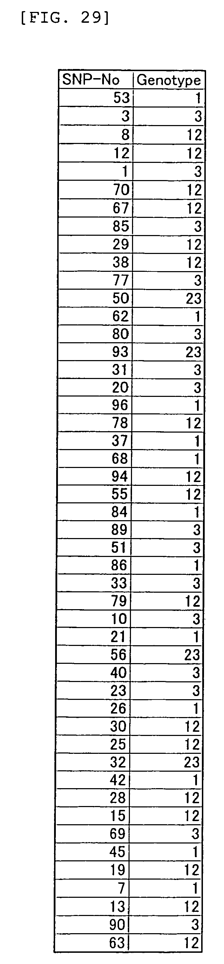

FIGS. 28 to 36 categorize the polymorphism sets positively associated with IMT (sensitive) shown in FIGS. 19 to 27 into their constituent polymorphisms according to the Odds of the sets indicated therein. FIG. 28 is a list of polymorphisms that form the positive polymorphism sets with Odds of 2 or more shown in FIG 19. Likewise, FIG. 29, FIG. 30, FIG. 31, FIG. 32, FIG. 33, FIG. 34, FIG. 35, and FIG. 36 respectively list polymorphisms that make up the positive polymorphism sets with Odds of 3 or more shown in FIG. 20, Odds of 4 or more shown in FIG. 21, Odds of 5 or more shown in FIG. 22, Odds of 6 or more shown in FIG. 23, Odds of 7 or more shown in FIG. 24, Odds of 8 or more shown in FIG. 25, Odds of 9 or more shown in FIG. 26, and Odds of 10 or more shown in FIG. 27. When selecting at least half, 60%, 70%, 80%, or 90% of the polymorphisms shown in one of the above figures, it is preferable that those closer to the top of the columns are selectively chosen.

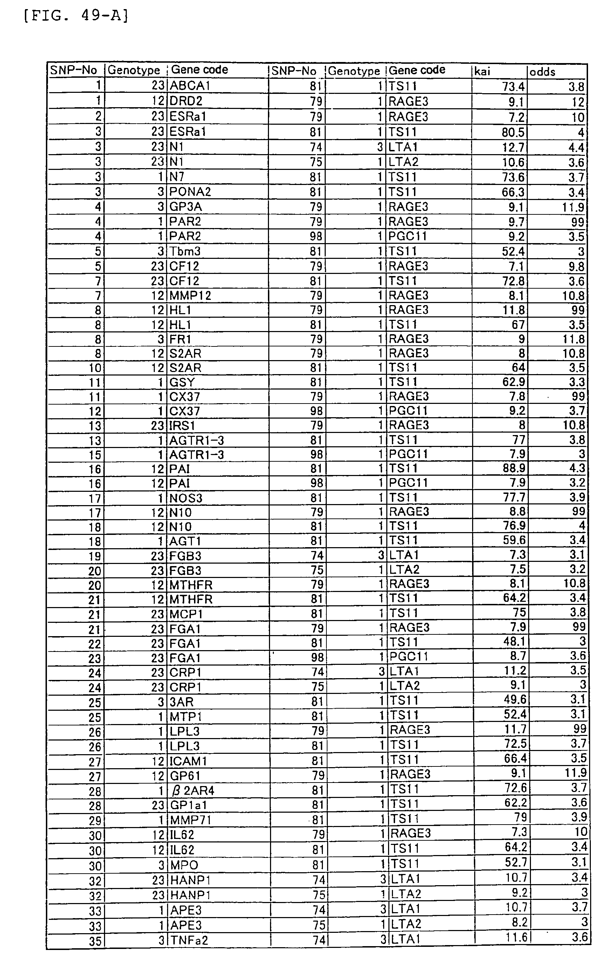

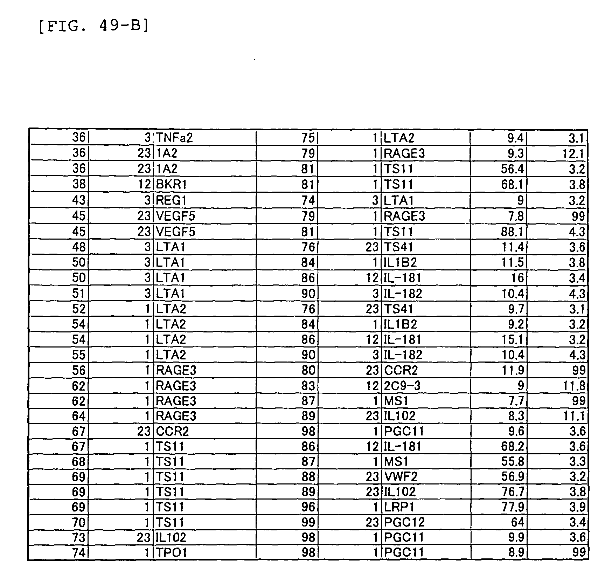

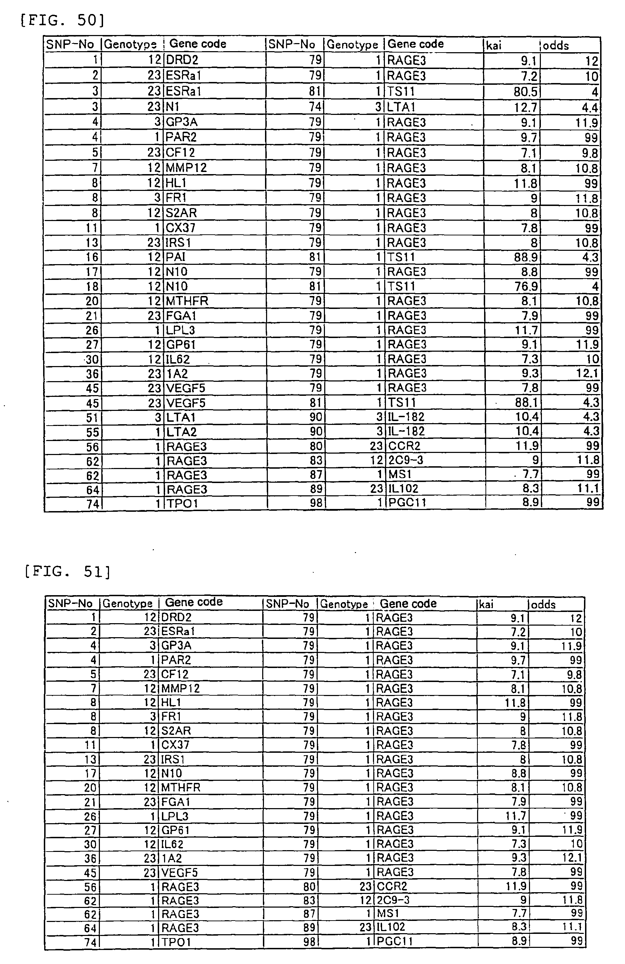

The array of the invention may instead comprise, in addition to the probe or probes for detecting one or more polymorphisms of at least one polymorphism set selected from the negative polymorphism sets shown in any one of FIGS. 38 to 43, a probe or probes for detecting one or more polymorphisms of at least one polymorphism set selected from the positive polymorphism sets shown in any one of FIGS. 48 to 51, preferably FIGS 49 to 51, and more preferably FIGS. 50 and 51.

Such a probe or probes for detecting one or more polymorphisms of at least one positive polymorphism set preferably detect at least half of, preferably at least 60%, 70%, 80%, or 90% of, and more preferably all of the polymorphisms shown in one of FIGS. 52 to 55.

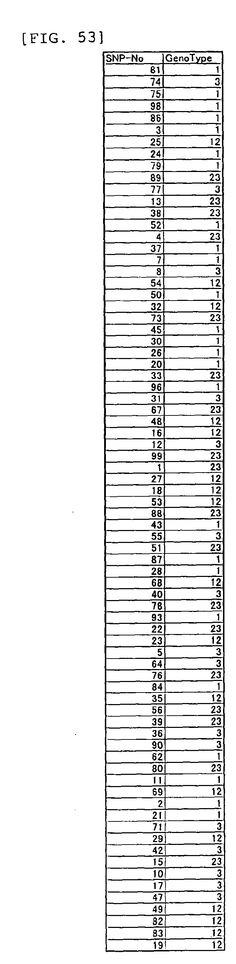

FIGS. 52 to 55 categorize the polymorphism sets positively associated with IMT (sensitive) shown in FIGS. 48 to 51 into their constituent polymorphisms according to the Odds of the sets indicated therein. FIG. 52 is a list of polymorphisms that form the positive polymorphism sets with Odds of 2 or more shown in FIG. 48. Likewise, FIG. 53, FIG. 54, and FIG. 55 respectively list polymorphisms that make up the positive polymorphism sets with Odds of 3 or more shown in FIG. 49, Odds of 4 or more shown in FIG. 50, and Odds of 5 or more shown in FIG. 51. When selecting at least half, 60%, 70%, 80%, or 90% of the polymorphisms shown in one of the above figures, it is preferable that those closer to the top of the columns are selectively chosen.

After examining the above requirements, the present inventors confirmed their original prediction that the polymorphisms shown in FIG. 37 are useful for assessing the risk of various types of arteriosclerosis, especially diabetic arteriosclerosis, and that the risk can be assessed with high accuracy by using these polymorphisms individually or in combination. Accordingly, the array of the present invention for assessing arteriosclerosis risk may instead comprise a probe or probes for detecting any of the 99 genetic polymorphisms described in FIG. 37.

The array of the invention can be employed to evaluate the resistance of a subject to arteriosclerosis (the likelihood of not developing the disease). This can be performed, for example, by hybridizing the probe or probes on the array with a probe or probes prepared from a specimen, and checking a polymorphism detected in a subject against polymorphisms negatively associated with carotid artery intima-media thickness (IMT) that is an arteriosclerosis index. The thereby obtained information about whether the subject has any negatively-IMT-associated polymorphism sets can be efficiently used to assess whether the subject is resistant to arteriosclerosis (if the subject is prone to arteriosclerosis or not).

Further, the array of the invention can be used to evaluate the resistance and sensitivity of a subject to arteriosclerosis. Specifically, such assessment can be performed, for example, by hybridizing the probe or probes on the array with probes prepared from a specimen, and checking a polymorphism detected in a subject against one or more polymorphism sets negatively associated with carotid artery intima-media thickness (IMT), and also against one or more polymorphism sets positively associated with IMT. The thereby obtained information (information about whether the subject has any negatively-IMT-associated polymorphism sets and any positively-IMT-associated polymorphism sets can be efficiently used to evaluate resistance and sensitivity of a subject to arteriosclerosis.

The array of the invention can also be used to evaluate the presence and degree of arteriosclerosis risk of a subject (probabilities that the disease will occur and progress in the subject). Specifically, such an assessment can be performed, for example, by hybridizing the probe or probes on the array with probes prepared form a specimen, and checking a polymorphism detected in a subject against one or more polymorphism sets negatively associated with carotid artery intima-media thickness (IMT), and also against one or more polymorphism sets positively associated with IMT to thereby obtain the balance of detected polymorphisms toward positive or negative association. The thereby obtained information (information about the balance between negative and positive associations of the polymorphisms of the subjects) can be efficiently used to evaluate arteriosclerosis risk of a subject.

For the above checking, one or more sets selected from the negative polymorphism sets shown in any one of FIGS. 1 to 9 and 38 to 43 are preferable used as said one or more one or more polymorphism sets negatively associated with IMT. When one or more negative sets are selected from those shown in FIGS. 1 to 9, one or more sets selected from the positive polymorphism sets shown in any one of FIGS. 19 to 27 are preferably used as said one or more polymorphism sets positively associated with IMT. When one or more negative sets are selected from those show in FIGS. 38 to 43, one or more sets selected from the positive polymorphism sets shown in any one of FIGS. 48 to 51 are preferably used.

Assessment of arteriosclerosis risk can be performed, for example, as follows.

[Table 1]

| | Number of positive polymorphism sets | | Number of negative polymorphism sets | | |
|---|---|---|---|---|---|
| Case 1 | +++ | > | - | → | High risk |
| Case 2 | +++ | > | 0 | → | High risk |
| Case 3 | + | < | - - - | → | Low risk |
| Case 4 | 0 | < | - - - | → | Low risk |

A polymorphism detected in a specimen is checked against one or more polymorphism sets negatively associated with IMT (e.g., those shown in FIGS. 1 to 9 or 38 to 43) and also against one or more polymorphism sets positively associated with IMT (e.g., those shown in FIGS. 19 to 27 or 48 to 51). A subject whose total positive polymorphism sets outnumber total negative polymorphism sets is assessed as being a high-risk case of arteriosclerosis (Case 1). A subject with at least one positive polymorphism set present while negative polymorphisms are absent is also assessed as being a high-risk case of arteriosclerosis (Case 2). Vice versa, a subject whose total negative polymorphism sets outnumbers total positive polymorphism sets is assessed as being a low-risk case of arteriosclerosis (Case 3). A subject with at least one negative polymorphism set present while positive polymorphisms are absent is also assessed as being a low-risk case of arteriosclerosis (Case 4).

That is to say, after obtaining the balance between positive association (sensitively) and negative association (resistance) of polymorphisms detected in a subject, when positive association is significant, the arteriosclerosis risk of such a subject is assessed as being high (or the existence of arteriosclerosis is assessed), and when negative association is significant, the arteriosclerosis risk is assessed as being low (or non-existence of arteriosclerosis is assessed).

Assessment results obtained using the array of the invention are preferably such that when compared with the results of clinical examinations (e.g., IMT), the percentage of accuracy (i.e., the percentage that the assessment results match the examination results) is at least 60%, preferably at least 65%, and more preferably at least 70%, while the percentage of inaccuracy (i.e., the percentage that the assessment results do not match the examination results) is 45% or less, preferably 40% or less, and more preferably 35% or less. An example of criteria to make clinical examinations applicable in this case is defining a non-arteriosclerosis case as one where carotid artery intima-media thickness (IMT) is less than 1.1 mm, while defining an arteriosclerosis case as having an IMT of 1.1 mm or more. Another example is defining an arteriosclerosis case as having an increase in average carotid artery intima-media thickness (ΔIMT) in a multiple regression analysis of 0.2 mm or more, or an increase in maximum carotid artery intima-media thickness (ΔPIMT) in a multiple regression analysis of 0.3 mm or more, with a non-arteriosclerosis case being defined otherwise.

(2-2) Array for assessing myocardial infarction risk

The array of the invention for assessing arteriosclerosis risk can be used to assess the likelihood of myocardial infarction (the likelihood of occurrence) and the probability of its progression. It can be advantageously used to assess myocardial infarction risk of diabetics and borderline diabetics. The array of the invention for assessing myocardial infarction risk is characterized by comprising a probe or probes for detecting one or more polymorphisms of a "negative (resistant) polymorphism set" that is significantly negatively associated with a myocardial infarction index. The myocardial infarction index is not limited and may be any of those commonly used in the art. Preferably used as an index is the presence of abnormal Q waves on an electrocardiogram indicating old myocardial infarction, or the presence of a past history of myocardial infarction.

Specific examples of "negative (resistant) polymorphism sets" are shown in FIGS. 56 to 58. More specifically, in FIGS. 56 to 58, a combination of polymorphisms (SNPs) arranged in a single horizontal row is one "polymorphism set negatively associated with (resistant to) myocardial infarction".

The array of the invention for assessing myocardial infarction risk comprises a probe or probes for detecting one or more polymorphisms of at least one polymorphism set selected from the negative genetic polymorphism sets shown in any one of FIGS. 56 to 58, and preferably FIG. 57 and 58. For the selection, the Odds and Kai that are given for every polymorphism set in each figure can be used as indices. The array of the invention preferably comprises a probe or probes for detecting constituent polymorphisms of a polymorphism set that is evaluated to have a high negative association with a myocardial infarction index, based on Odds and Kai.

Such a probe or probes for detecting one or more polymorphisms of at least one negative genetic polymorphism set preferably detect at least half of, preferably at least 60%, 70%, 80%, or 90% of, and more preferably all of the polymorphisms shown in one of FIG. 59, FIG. 60, FIG. 61, and FIG. 62.

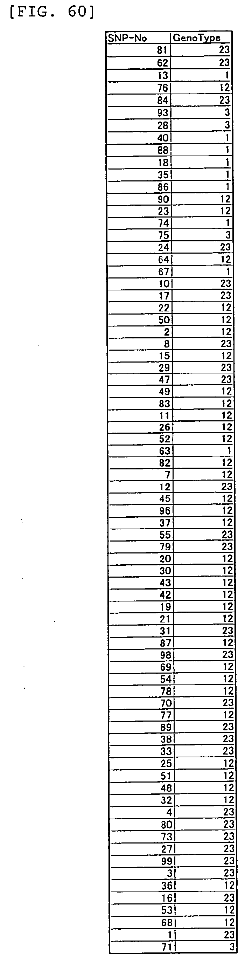

FIGS. 59 to 62 categorize the polymorphism sets negatively associated with a myocardial infarction index (resistant) shown in FIGS. 56 to 68 into their constituent polymorphisms according to the Odds of the sets indicated therein. FIG. 59 is a list of polymorphisms that form the negative polymorphism sets with Odds of -2 or less (i.e., Odds is 1/2 or less) shown in FIG 56. Likewise, FIG. 60, FIG. 61, and FIG. 64 respectively list polymorphisms that make up the negative polymorphism sets with Odds of -3 or less (i.e., Odds is 1/3 or less) shown in FIG. 57, Odds of -4 or less (i.e., Odds is 1/4 or less) shown in FIG. 58, and Odds of -5 or less (i.e., Odds is 1/5 or less) shown in FIG. 58.

When selecting at least half, 60%, 70%, 80%, or 90% of the polymorphisms shown in one of the above figures, it is preferable that those closer to the top of the columns are selectively chosen.

The array of the invention for assessing myocardial infarction risk preferably comprises, in addition to the above-described a probe or probes for detecting one or more polymorphisms of at least one negative (resistant) polymorphism set, a probe or probes for detecting one or more polymorphisms of at least one positive polymorphism set.

A "positive (sensitive) polymorphism" means a polymorphism that is significantly positively associated with a myocardial infarction index. A "positive (sensitive) polymorphism set" means a 'combination of polymorphisms' that is significantly positively associated with a myocardial infarction index.

Specific examples of such "positive (sensitive) polymorphism sets" are shown in FIGS. 63 to 69. More specifically, in FIGS. 63 to 69, a combination of polymorphisms (SNPs) arranged in each row is one "positive (sensitive) polymorphism set" that is positively associated with (sensitive to) myocardial infarction.

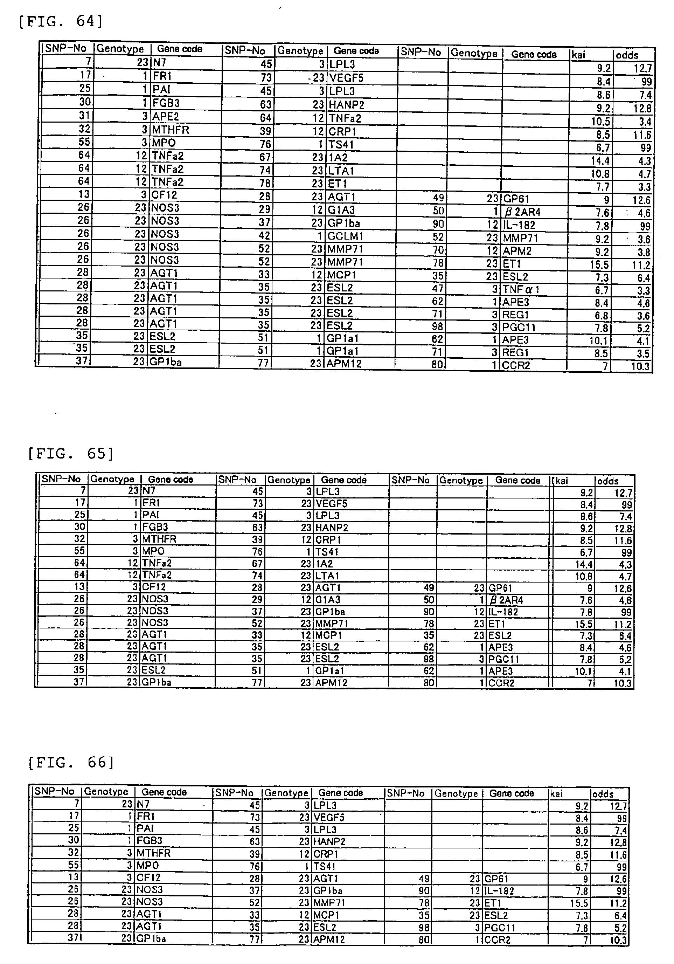

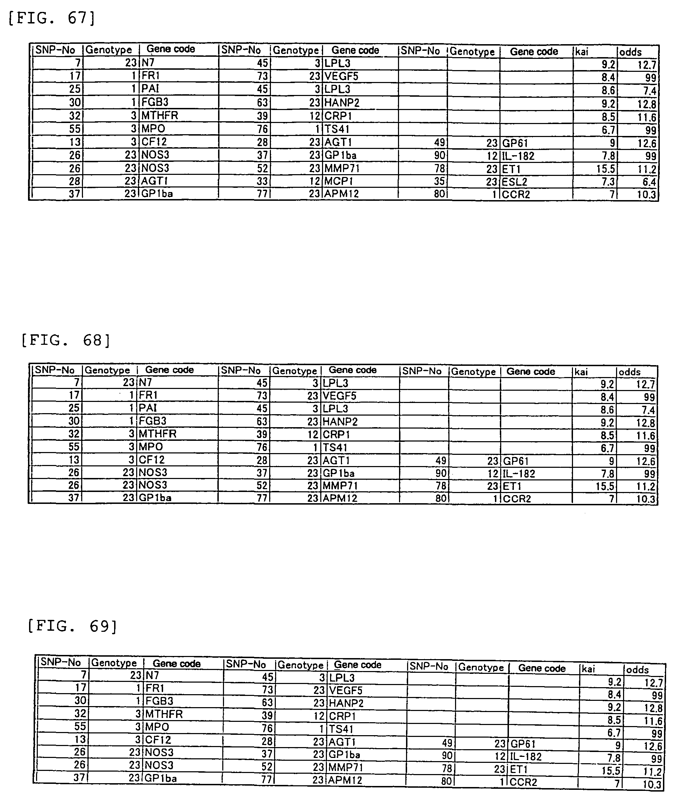

Specifically, the array of the invention for assessing myocardial infarction risk may comprise, in addition to the probe or probes for detecting one or more polymorphisms of at least one polymorphism set selected from the negative (resistant) polymorphism sets shown in any one of 56 to 58, a probe or probes for detecting one or more polymorphisms of at least one polymorphism set selected from the positive polymorphism sets shown in any one of FIGS. 63 to 69, preferably FIGS. 64 to 69, and more preferably FIGS. 65 to 69.

Such a probe or probes for detecting one or more polymorphisms of at least one positive polymorphism set preferably detect at least half of, preferably at least 60%, 70%, 80%, or 90% of, and more preferably all of the polymorphisms shown in any one of FIGS. 70 to 73.

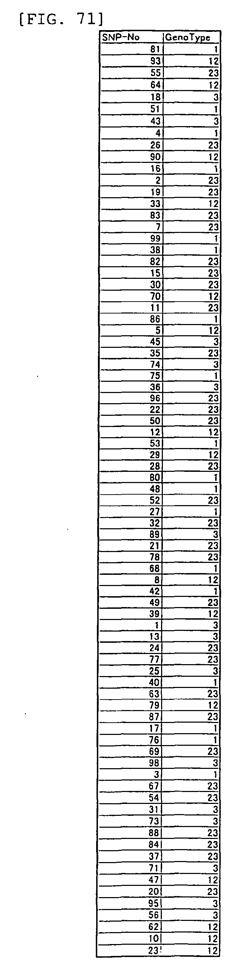

FIGS. 70 to 73 categorize the polymorphism sets positively associated with a myocardial infarction index (sensitive) shown in FIGS. 63 to 69 into their constituent polymorphisms according to the Odds of the sets indicated therein. To be more specific, FIG. 70 is a list of polymorphisms that form the positive polymorphism sets with Odds of 2 or more shown in FIG. 63. Likewise, FIG. 71, FIG. 72, and FIG. 73 respectively list polymorphisms that make up the polymorphism sets with Odds of 3 or more shown in FIG. 64, Odds of 4 or more shown in FIG. 65, and Odds of 5 or more shown in FIG. 66. When selecting at least half, 60%, 70%, 80%, or 90% of the polymorphisms shown in one of the above figures, it is preferable that those closer to the top of the columns are selectively chosen.

The present inventors confirmed that the polymorphisms shown in FIG. 37 are also useful in assessing the risk of myocardial infarction, especially diabetic myocardial infarction, and that myocardial infarction risk can be assessed with high accuracy by using such polymorphisms individually or in combination. Accordingly, the array of the present invention for assessing myocardial infarction risk may instead comprise probes for detecting any of the 99 polymorphisms of FIG. 37.

The array of the invention for assessing myocardial infarction risk may be, as with the above described array for assessing arteriosclerosis risk, employed to evaluate the resistance of a subject to myocardial infarction (the likelihood of not getting the condition), and also to assess the resistance and sensitivity of a subject to myocardial infarction. It can also be employed to determine the presence and degree of myocardial infarction risk of a subject (probabilities that myocardial infarction will occur and progress in the subject).

In such myocardial infarction risk assessment, a polymorphism detected in a subject is checked against one or more polymorphism sets negatively/positively associated with a myocardial infarction index. One or more sets selected from the negative polymorphism sets shown in any one of FIGS. 56 to 58 are preferably used in the checking as such one or more negative polymorphism sets. One or more sets selected from the positive polymorphisms or polymorphism sets shown in any one of FIGS. 63 to 69 are preferably used as such one or more positive polymorphism sets.

Myocardial infarction risk assessment can be made in the same manner as in the above arteriosclerosis risk assessment.

Within the range in which the objects of the present invention can be achieved, and as long as a probe for detecting a target condition-associated polymorphism is included therein, the array of the invention may further comprise a known probe and other additional probes. The probe for detecting a polymorphism may be suitably labeled.

The array of the invention can be prepared by, in addition to a method of fixing a pre-prepared probe on a substrate, other methods such as the method of Affimetrix Inc. of synthesizing a probe on a substrate. There also are no limitations on substrates on which to fix a probe, and known ones such as glass plates and filters can be used. As long as polymorphisms can be detected, the length of the probes to be fixed and the kinds of nucleic acids to be used are not limited either. It is preferable that regions containing polymorphisms be amplified in advance by PCR.

Especially, methods of amplifying polymorphism-containing regions using labeled primers are desirable in terms of sensitivity and ease of operation. For example, in the Hybrigene method, regions containing polymorphisms are amplified using biotin-labeled primers and placed on an array. After hybridization on the array, nucleic acids that have not been hybridized are washed away. Hybridized probes are then detected by avidin-labeled fluorescent dyes. Probes obtained by such a method can detect polymorphisms with high sensitivity.

The array of the present invention for assessing disease risk includes the following modes.

(A) An array for assessing arteriosclerosis risk, comprising:

a probe or probes for detecting one or more genetic polymorphisms of at least one genetic polymorphism set selected from the negative (resistant) genetic polymorphism sets shown in any one of FIGS. 1 to 9; or
a probe or probes for detecting one or more genetic polymorphisms of at least one genetic polymorphism set selected from the negative (resistant) genetic polymorphism sets shown in any one of FIGS. 38 to 43.

(B) An array for assessing arteriosclerosis risk according to (A), comprising:

a probe or probes for detecting at least half of the genetic polymorphisms shown in any one of FIGS. 10 to 18; or
a probe or probes for detecting at least half of the genetic polymorphisms shown in any one of FIGS. 44 to 47.

(C) An array for assessing arteriosclerosis risk according to (A) or (B), further comprising:

a probe or probes for detecting one or more genetic polymorphisms of at least one genetic polymorphism set selected from the positive (sensitive) genetic polymorphism sets shown in any one of FIGS. 19 to 27; or
a probe or probes for detecting one or more genetic polymorphisms of at least one genetic polymorphism set selected from the positive (sensitive) genetic polymorphism sets shown in any one of FIGS. 63 to 69.

(D) An array for assessing arteriosclerosis risk according to (A) or (B), comprising:

a probe or probes for detecting at least half of the genetic polymorphisms shown in any one of FIGS. 28 to 37; or
a probe or probes for detecting at least half of the genetic polymorphisms shown in any one of FIGS. 52 to 55.

(E) An array for assessing myocardial infarction risk, comprising a probe or probes for detecting one or more genetic polymorphisms of at least one genetic polymorphism set selected from the negative (resistant) genetic polymorphism sets shown in any one of FIGS. 56 to 58.
(F) An array for assessing myocardial infarction risk according to (E), comprising a probe or probes for detecting at least half of the genetic polymorphisms shown in any one of FIGS. 59 to 62.
(G) An array for assessing myocardial infarction risk according to (E) or (F), further comprising a probe or probes for detecting one or more genetic polymorphisms of at least one genetic polymorphism set selected from of the positive (sensitive) genetic polymorphism sets shown in any one of FIGS. 63 to 69.
(H) An array according to (E) or (F), further comprising a probe or probes for detecting at least half of the genetic polymorphisms shown in any one of FIGS. 70 to 73.
(I) An array for assessing arteriosclerosis risk according to any one of (A) to (H), for use in an assessment wherein:

said array is hybridized with a probe or probes prepared from a specimen to detect genetic polymorphisms in a subject,
a genetic polymorphism detected in a subject is checked against one or more genetic polymorphism sets negatively associated with arteriosclerosis, and
resistance of the detected genetic polymorphisms to the disease is thereby evaluated.

(J) An array for assessing arteriosclerosis risk according to (I), wherein said one or more genetic polymorphism sets negatively associated with arteriosclerosis are:

one or more sets selected from the negative genetic polymorphism sets shown in one figure selected from FIGS. 1 to 9, or

one or more sets selected from the negative genetic polymorphism sets shown in any one of FIGS. 38 to 43.

(K) An array for assessing arteriosclerosis risk according to any one of (A) to (H), which is used in an assessment wherein:

said array is hybridized with a probe or probes prepared from a specimen to detect genetic polymorphisms in a subject,
a genetic polymorphism detected in a subject is checked against one or more genetic polymorphism sets negatively associated with arteriosclerosis and one or more genetic polymorphism sets positively associated with arteriosclerosis, and
resistance and sensitivity of the detected genetic polymorphisms to arteriosclerosis is thereby evaluated.

(L) An array for assessing arteriosclerosis risk according to any one of (A) to (H), which is used in an assessment wherein:

said array is hybridized with a probe or probes prepared from a specimen to detect genetic polymorphisms in a subject,
a genetic polymorphism detected in a specimen is checked against one or more genetic polymorphism sets negatively associated with arteriosclerosis and one or more genetic polymorphism sets positively associated with arteriosclerosis to thereby obtain the balance of the genetic polymorphisms toward negative association or positive association, and
the degree of arteriosclerosis risk of the subject is evaluated based on the obtained balance.

(M) An array for assessing arteriosclerosis risk according to (L), wherein said one or more genetic polymorphism sets negatively associated with arteriosclerosis are:

one or more sets selected from the negative genetic polymorphism sets shown in any one of FIGS. 1 to 9, or one or more sets selected from the negative genetic polymorphism sets shown in anyone of FIGS. 38 to 43, and wherein said one or more genetic polymorphism sets positively associated with arteriosclerosis are:

one or more sets selected from the positive genetic polymorphism sets shown in any one of FIGS. 19 to 27, or one or more sets selected from the positive genetic polymorphism sets shown in any one of FIGS. 48 to 51.

**(3) Apparatus, method, and program for assessing disease risk**

The apparatus, method, and program of the present invention for assessing disease risk make it possible to assess the likelihood that a subject will get a disease and the probability that such a disease will progress (disease risk). Although the invention is explained below using arteriosclerosis as an example, it is not limited thereto and is applicable to any gene-related disease. In any case, an association, which is to be described below, may be evaluated using an index suitable for each disease (e.g., carotid artery intima-media thickness for arteriosclerosis; urinary albumin concentration for nephropathy; and for myocardial infarction, the presence of abnormal Q waves on an electrocardiogram indicating old myocardial infarction, and the presence of a past history of myocardial infarction).

(3-1) Apparatus, method, and program for assessing arteriosclerosis risk

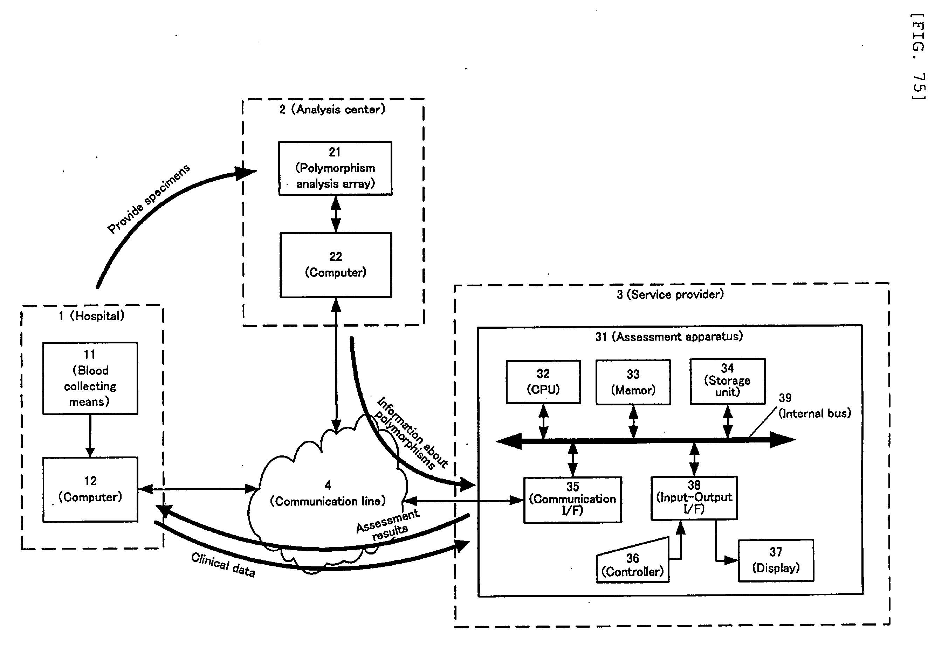

FIG. 75 is a block diagram showing an entire system containing the apparatus of the invention for assessing arteriosclerosis risk (hereinafter referred to as an assessment apparatus). As shown in FIG. 75, the system comprises a blood collecting means 11 and a computer 12 located in a hospital 1, a polymorphism analysis array 21 and a computer 22 located in an analysis center 2, and a assessment apparatus 31 located in a service provider 3. Here, the computers 12 and 22 and the assessment apparatus 31 are connected to a communication line 4 such as the Internet.
The assessment apparatus 31 is provided with a CPU 32, a memory 33, a storage unit 34 such as a hard disk drive, a communication interface (hereinafter referred to as an I/F) 35 for external communication, a controller 36 such as a keyboard, a display 37 such as a CRT display, an input-and-output I/F 38, and an internal bus 39 for data exchange between these. In the storage unit 34 is recorded, as a reference table, information about arteriosclerosis-associated genetic polymorphisms (e.g., those listed in FIG. 10) or genetic polymorphism sets (e.g., those listed in FIG. 1).
A detailed description of the risk assessment processing performed by the assessment apparatus 31 is given later. An overview of the operation of the entire system is explained hereafter. First, blood (hereinafter referred to as a specimen) is collected from a subject in the hospital 1. At this time, clinical data (subject ID, examination data, anamnestic information, blood collection information, etc.) are recorded in a storage unit of the computer 12. The specimen is given to the analysis center 2 and analyzed using the polymorphism analysis array 21, and polymorphisms with genotypes are then detected. Here, the above-described array for assessing arteriosclerosis risk can be employed as the polymorphism analysis array 21. Information about such detected polymorphisms is temporarily recorded in a storage unit of the computer 22, and subsequently transmitted to the assessment apparatus 31 of the service provider 3. The assessment apparatus 31 receives the information about the polymorphisms through the communication I/F 35 and temporarily records it in the storage unit 34. Thereafter, the assessment apparatus 31 conducts a search to determine whether the polymorphisms of the received information are included in a reference table, which is pre-recorded in the storage unit 34, and determines arteriosclerosis risk in accordance with the search results. The assessment apparatus 31 transmits the assessment results to the computer 12 located in the hospital 1 via the communication line 4. The results that the computer 12 receives are recorded in its storage unit in correspondence with clinical data (at least an subject ID), and recalled and used as required (e.g., to be shown to the subject). Information identifying the computer 12 of the hospital 1, to which assessment results are transmitted, may be, for example, included in clinical data transmitted from the computer 12 of the hospital 1.

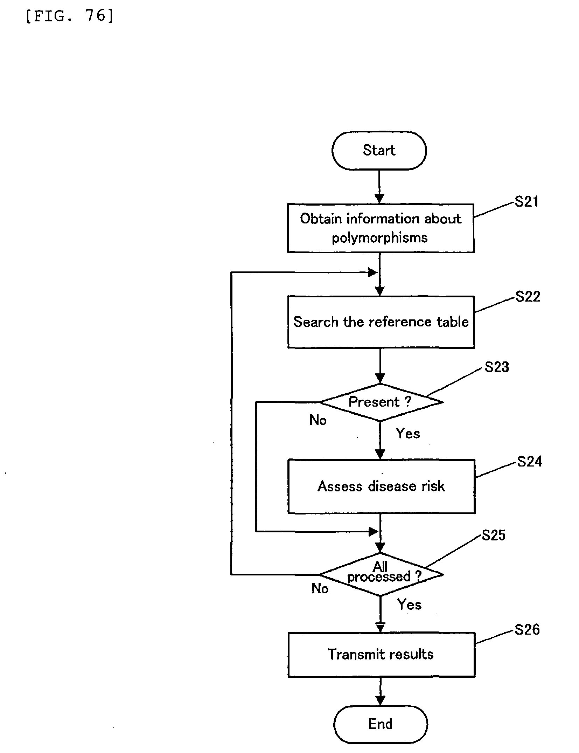

FIG. 76 is a flowchart illustrating the assessment processing that the assessment apparatus 31 performs. The risk assessment processing by the assessment apparatus 31 is described in detail hereafter in reference to the flowchart in FIG. 76. Unless otherwise noted, it is the CPU 32 that performs the processing described hereafter. The CPU 32 uses the memory 33 as a work area and/or as an area to temporarily store data that are being processed, and, as necessary, records data during or after processing in the storage unit 34.

In step S21, information about polymorphisms is obtained from the analysis center 2 via the communication line 4, and recorded in the storage unit 34. Here, the information about polymorphisms is transmitted as polymorphism codes given to every polymorphism having a genotype. The data format is, for example, such that a plurality of polymorphism codes $G_i$ (i = 1 to n) are in correspondence with a hospital code given to each client hospital and a subject ID given to each subject.

In step S22, a plurality of polymorphism codes $G_i$ (i = 1 to n) corresponding to a set of [hospital code, subject ID] are read out from the storage unit 34, and assessment is made on whether such polymorphism codes $G_i$ are included in a reference table recorded in the storage unit 34. For example, provided that sets of polymorphism codes corresponding to FIG. 1 are recorded as a reference table, two or three polymorphism codes are selected from the polymorphism codes $G_i$ (i = 1 to n) to obtain a set, and it is assessed whether the obtained set is included in the reference table corresponding to FIG. 1. If included, a first flag corresponding to the set is set to "1" (flags are preset to 0). Likewise, if the set is included in the reference table corresponding to FIG. 19, then a second flag corresponding to such a set is set to "1".

In step S23, assessment is made on whether both the first and second flags are 0. If both flags are 0, i.e., if the set of polymorphism codes is not included in the reference tables, then the processing moves to step S25. If a value other than 0 is set in any flag, then it moves to step S24.

In step S24, the risk is determined according to the results obtained in step S22. The determined risk is recorded in the storage unit 34 in correspondence with the [hospital code, subject ID]. This determination is made such that, for example, the number $n_1$ of first flags having "1" and the number $n_2$ of second flags having "1" are obtained, and the risk is then determined in accordance with Table 1 above.

That is, a subject with a number $n_2$ of positive polymorphism sets larger than the number $n_1$ of negative polymorphism sets ($n_2 > n_1$) is assessed as being at high risk of arteriosclerosis (Case 1 of Table 1). A subject who has at least one polymorphism set ($n_2 > 0$) but does not have any negative polymorphism set ($n_1 = 0$) is also assessed as being at high risk (Case 2). Vice versa, a subject with a number $n_1$ of negative polymorphism sets larger than the number $n_2$ of positive polymorphism sets ($n_1 > n_2$) is assessed as being at a low risk (Case 3). A subject who has at least one negative polymorphism set ($n_1 > 0$) but does not have any positive polymorphism set ($n_2 = 0$) is also assessed as being at low risk (Case 4).

In step S25, steps S22 to S24 are repeated until it is determined that all sets of [hospital code, subject ID] have been processed.

After processing is completed for all sets of [hospital code, subject ID], then in step S26, the determined risk code (in the above example, the code indicating a high or low risk) and the corresponding subject ID are transmitted to the computer 12 corresponding to the hospital code via the communication line 4.

This completes the series of risk-assessment processes performed by the assessment apparatus 31. The above processing may also be conducted by using a general-purpose computer, i.e., reading out a computer program recorded on a computer-readable recording medium, such as a hard disk drive, CD-ROM, etc., or by obtaining a computer program via a communication line, and then making a CPU execute the computer program.

If necessary, as long as the apparatus is capable of executing the above assessment functions, other means can be suitably added to the apparatus of the present invention.

The criteria for assessing risk in step S24 are not limited to those shown in Table 1. Assessment may be made such that, after obtaining the balance in a subject between positive association and negative association, when positive association is significant, arteriosclerosis risk is assessed as being high (or the existence of arteriosclerosis is assessed), and when negative association is significant, arteriosclerosis risk is assessed as being low (or the non-existence of arteriosclerosis is assessed). The balance is not limited to the one obtained based on the difference between the number of sets included in a positive reference table and the number of sets included in a negative reference table. Various other values may also be employed in obtaining the balance, such as values calculated by weighting according to each of the included sets or individual polymorphisms, and those calculated in consideration of clinical data.

Usable as reference tables for use in assessing arteriosclerosis risk are tables corresponding to any of FIGS. 1 to 36 and 38 to 55, and, as mentioned above, the application of the apparatus, method, and program of the present invention for assessing disease risk is not limited to arteriosclerosis. For assessing myocardial infarction risk, reference tables corresponding to any of FIGS. 56 to 73 can be used.

Although the above describes an example wherein the target of assessment is information about the polymorphisms of a subject, which the assessment apparatus of a service provider obtains from an analysis center, the target is not limited thereto. It is also possible to read out previously analyzed information about an individual's polymorphisms recorded in some sort of recording means (e.g., a portable recording means such as an individual IC card or a memory card), and to then perform the processing of disease risk assessment. Because the genetic information of an organism does not change, once polymorphism information is analyzed and recorded, there is no need to collect blood again for a polymorphism analysis even when reference tables and/or criteria for risk assessment have been changed and assessment accuracy has been accordingly improved, thereby reducing the burden on the subject.

If information about an individual's polymorphisms obtained from an analysis center is recorded in a database of a service provider in correspondence with an individual ID, and the individual has been informed of such an ID, it is possible to make a risk assessment once again using the corresponding polymorphisms recorded in the database, simply by obtaining the individual ID.

Further, as mentioned above in the description of the method for determining polymorphisms for disease risk assessment, if the polymorphism information itself is utilized as an ID, it is possible to use, in addition to newly obtained data (e.g., in the case of arteriosclerosis, IMT measurement values), data obtained in the past, without referring to an individual ID. It accordingly becomes possible to track the history of accuracy of risk assessment and to make a new risk assessment in consideration of this history. Here, the data for analysis may include various clinical data as well as disease index values.

(3-2) Method of assessing arteriosclerosis risk

The method of assessing arteriosclerosis risk is explained hereafter in detail.

The method of the present invention of assessing arteriosclerosis risk can be used to assess the likelihood of onset of arteriosclerosis and the probability of its progression. The method is advantageously applied to diabetics and patients prone to diabetes (borderline diabetics) so as to assess their risk of arteriosclerosis (the likelihood of getting the disease, the probability of its progression, etc.).

The method of the invention for assessing arteriosclerosis risk is characterized by comprising a step (b) of checking a genetic polymorphism detected in a specimen against one or more polymorphisms sets negatively associated with "carotid artery intima-media thickness (IMT)", which is an arteriosclerosis index. One or more sets selected from the negative polymorphism sets shown in any one of FIGS. 1 to 9 or one or more sets selected from the negative polymorphism sets shown in any one of FIGS. 38 to 43 are preferably used as such one or more polymorphisms sets negatively associated with IMT.

Such an assessment method of the invention optionally comprises checking a polymorphism detected in a specimen against one or more polymorphism sets negatively associated with an arteriosclerosis index, and also against one or more polymorphism sets positively associated with an arteriosclerosis index. That is, the assessment method may comprise, in addition to the above step (b), a step (b') of checking a polymorphism detected in a specimen against one or more polymorphism sets positively associated with IMT, and a step (c) of, based on the results of steps (b) and (b'), comparing negative association with positive association of detected polymorphisms to thereby calculate the balance. One or more polymorphism sets selected from the positive polymorphism sets shown in any one of FIGS 19 to 27 or one or more polymorphism sets selected from the positive polymorphism sets shown in any one of FIGS. 48 to 51 are preferably used as such one or more polymorphism sets positively associated with IMT.

Such an assessment method of the invention may further comprise a step (a) of, prior to step (b) and/or (b'), detecting polymorphisms in a specimen. The detection step (a) may be directed at two or more polymorphisms selected from the 99 polymorphisms shown in FIG. 37.

Step (a) may be a step of detecting the presence of one or more polymorphisms shown in any one of FIGS. 10 to 18, which form negative polymorphism sets, and the presence of one or more polymorphisms shown in any one of FIGS. 28 to 37, which form positive polymorphism sets. Specifically, it may be a step of detecting the presence of one or more polymorphisms shown in FIGS. 10 and 19, 11 and 20, 12 and 21, 13 and 22, 14 and 23, 15 and 24, 16 and 25, 17 and 26, or 18 and 27.

Step (a) may alternatively be a step of detecting the presence of one or more polymorphisms shown in any one of FIGS. 38 to 43, which form negative polymorphism sets, and the presence of one or more polymorphisms shown in any one of FIGS. 48 to 51, which form positive polymorphism sets. Specifically, it may be a step of detecting the presence of one or more polymorphisms shown in FIGS 38 and 48, 39 and 49, 40 and 50, 41 and 51, 42 and 51, or 43 and 51.

When the array of the invention for assessing arteriosclerosis risk is employed in step (a), the detection can be performed with high accuracy. That is, step (a) may be a step of detecting polymorphisms by hybridizing, on the array of the present invention, a probe or probes prepared from a specimen with the probe or probes on said array for detecting one or more polymorphisms.

In such a step (a), any method of detecting a subject's genotypes can be employed. Conventional methods employ, as specimens, materials containing DNA, such as blood, sputum, skin, bronchoalveolar lavage fluid, other body fluids, and tissues. A large number of analytical techniques are known, and examples thereof include DNA sequencing, PCR, ASP-PCR, TaqMan, invader assay, MALDI-TOF/MS, molecular beacon assay, ligation, etc. (Clin. *Chem.* 43: pp. 1114-1120, 1997). Sequencing methods directly sequence DNA regions containing polymorphisms. In PCR methods, a certain polymorphism is selectively amplified using a polymorphism-specific primer. It is usual in PCR methods that polymorphic nucleic acids are positioned at the 3'-end. However, insofar as polymorphisms can be identified, there are no particular limitations on primer designs, such as on the primer region in which to locate polymorphisms and the kinds of nucleotide sequences to be inserted other than the target gene. For example, in the Allele Specific Primer (ASP)-PCR method, primers having a polymorphism next to the 3'-end are employed. In a TaqMan method, an allele-specific probe labeled at each end with a fluorescent dye and a quencher respectively is hybridized with target site, and a PCR is conducted using a primer so designed as to amplify the region containing the target site. When the PCR from the primer reaches the region hybridized with the allele-specific probe, the fluorescent dye at the 5'-end of the hybridized probe is cleaved by the 5' nuclease activity of Taq polymerase, and leaves the quencher dye to thereby emit fluorescence. This technique reveals how much the allele-specific probe has been hybridized. The invader assay method makes it possible, on the same principle as in TaqMan methods, to know which allele probe has been hybridized, using three kinds of oligonucleotides: an allele probe, an invader probe, and a FRET probe. An allele probe has a specific sequence on the 5' side and a flap sequence on the 3' side of a template polymorphic site. An invader probe has a specific sequence at the 3' side of the template polymorphism site. A FRET probe includes a sequence complementary to the flap sequence. In MALDI-TOF/MS methods, a primer adjacent to a polymorphic site is prepared to amplify this region, and the single base of the polymorphic site is then amplified using ddNTP. The type of added ddNTP is then identified using MALDI-TOF/MS, to thereby determine the polymorphism. In methods collectively called DNA chip methods, such as the Hybrigene method, oligonugleotide probes containing polymorphisms are arranged on the array, and hybridization with PRC-amplified sample DNA is detected.

According to the present invention, assessment of arteriosclerosis risk can be made, for example, as follows:

[Table 2]

| | Number of positive polymorphism sets | | Number of negative polymorphism sets | | |
|---|---|---|---|---|---|
| Case 1 | +++ | > | - | → | High risk |
| Case 2 | +++ | > | 0 | → | High risk |
| Case 3 | + | < | - - - | → | Low risk |
| Case 4 | 0 | < | - - - | → | Low risk |

A polymorphism detected in a specimen is checked against one or more polymorphism sets negatively associated with IMT that is an arteriosclerosis index, and also against one or more sets positively associated with IMT (e.g., those shown in FIGS. 19 to 27 and 48 to 51). A subject whose total positive polymorphism sets outnumber total negative polymorphism sets is assessed as being a high-risk case of arteriosclerosis (Case 1). A subject with at least one positive polymorphism set present while negative polymorphisms are absent is also assessed as being a high-risk case of arteriosclerosis (Case 2). Vice versa, a subject whose total negative polymorphism sets outnumber total positive polymorphism sets is assessed as being a low-risk of arteriosclerosis (Case 3). A subject with at least one negative polymorphism set present while positive polymorphisms are absent is also assessed as being a low-risk case of arteriosclerosis (Case 4).

That is to say, after obtaining the balance between positive association and negative association of polymorphisms detected in a subject, when positive association is significant, arteriosclerosis risk is assessed as being high (or the existence of arteriosclerosis is assessed), and when negative association is significant, arteriosclerosis risk is assessed as being low (or non-existence of arteriosclerosis is assessed).

By using the method of the invention, it is possible to obtain highly accurate results in assessing arteriosclerosis risk.

For example, when subjects are assessed as being a low-risk case (or high-risk case) of arteriosclerosis based on their detected polymorphisms, the assessment results can be evaluated as being highly accurate if the percentage of these subjects assessed as being non-arteriosclerosis cases (or arteriosclerosis cases) by clinical examinations (i.e., the percentage that the assessment results match the examination results) is at least 60%, preferably at least 65%, and more preferably at least 70%, while the percentage of disagreement is 45% or less, preferably 40% or less, and more preferably 35% or less. An example of criteria to make clinical examinations applicable in this example is defining a non-arteriosclerosis case as one where carotid artery intima-media thickness (IMT) is less than 1.1 mm, while defining an arteriosclerosis case as having an IMT of 1.1 mm or more. Another example is defining an arteriosclerosis case as having an increase in average carotid artery intima-media thickness (ΔIMT) in a multiple regression analysis of 0.2 mm or more, or an increase in maximum carotid artery intima-media thickness (ΔPIMT) in a multiple regression analysis of 0.3 mm or more, with a non-arteriosclerosis case being defined otherwise.

The arteriosclerosis cases and non-arteriosclerosis cases are preferably set to be a group of cases having diabetes but not having a history of myocardial infarction. It is preferable in terms of efficiency that the detected polymorphisms of a subject comprise combinations of two or three different polymorphisms.

The method of the invention of assessing arteriosclerosis risk may further comprise a step (d) of weighting the balance calculated in step (c), and thereby determining the degree of balance (i.e., the degree of disease risk).

Factors usable for weighting are, for example, results of clinical examinations (usable as disease indices) closely related to the disease (or the extent of disease). When weighting, it is preferable that the association between polymorphisms and such a factor be analyzed and the relevancy be determined in advance. According to a preferable method for such a procedure, for example, the frequency of the presence of a significant positive/negative association between a polymorphism or a polymorphism set and a clinical examination result closely related to the disease (or to the extent of disease) is expressed as an odds ratio. Alternatively, the presence of a significant positive/negative association may be expressed as by either 1 or 0.

For arteriosclerosis, for example, a measurement value of carotid artery stiffness can be mentioned as one example of usable closely-disease-related clinical examination results. As an example of an index that indicates carotid artery stiffness, carotid artery thickness can be mentioned. Methods for measuring the thickness of carotid artery are not limited, and a common method measures carotid artery intima-media thickness (IMT) using an ultrasonic tomograph. Such a method is noninvasive and quantitative, and measures the thickness of carotid artery that is ultrasonically accessible. It is desirable that the ultrasonic tomograph has a linear pulse-echo probe with a center frequency of 7.5 MHz or more. Because the cranial external carotid artery is present in a superficial layer under the skin, a center frequency of 7.5 MHz or more is applicable, and a high resolution (distance resolution of 0.1 mm) can be thereby achieved. Note that this is just one example.

A vascular wall is analyzed echographically as a two-layer structure: a low echogenicity layer in the vascular lumen and an external high echogenicity layer. Through the observation of 104 healthy individuals, the present inventors have confirmed that, for a person in his/her 10's to 70's, common carotid artery IMT increases almost linearly with increasing age, but the thickness does not exceed 1.0 mm. Common carotid artery IMT of healthy persons can be obtained from their age as follows:

$$\mathrm{IMT} = 0.06 \times \mathrm{Age} + 0.3 \qquad (3 < \mathrm{Age} < 80\ \mathrm{yr}).$$

Examples of indices for indicating the stiffness of carotid artery other than the above carotid artery intima-media thickness (IMT) are: maximum IMT (Max-IMT) indicating the maximum thickness; average IMT (AvgIMT) indicating the average thickness; Plaque Score (PS); carotid artery stiffness; etc. Various measurement methods for obtaining these indices have been established. According to one example of such measurement methods, Max-IMT is defined as the maximum intima-media thickness of longitudinal anterior oblique, lateral, and posterior oblique projections, and AvgIMT is defined as the average of the thickness at three sites, i.e., the site of Max-IMT, and sites 1 cm toward the center and 1 cm toward the far wall therefrom. AvgIMT may also be defined as the mean maximum thickness of 12 carotid sites, i.e., common carotid (CC), bifurcation, internal carotid (IC), at the near and far walls, on left and right sides; or defined as the average of the thickness of the left and right sides. It is also possible to define a mean thickness of a certain section of the far wall as a mean IMT. The thickness of the far wall at the point 10 mm toward the center from the bifurcation of one side may also be employed as an index.

Plaque Score (PS) is obtained as follows: the carotid artery is divided on both left and right sides into four parts each having a length of 15 mm starting from the bifurcation, and the sum of plaque thickness of 1.1 mm or more on each side is added up, to define the obtained value as PS. It is also possible to add up the total number of plaques (IMT ≥ 1.1 cm) in each of the above three or four parts, define it as Plaque Number (PN), and employ the same as an index.

Numerical values for carotid artery stiffness are calculated from the change in carotid diameter during systole and diastole. A method that employs, as an index, the thickness of the carotid far wall 10 mm toward the center from the carotid bifurcation of one side is simple and easy, and further, such a method is said to cause few measurement errors because there are small numbers of lesions in the common carotid. IMT is the most important indicator of carotid lesions. A suitable index varies according to each target case. For example, although PS is capable of providing an overview of carotid arteries wherein arteriosclerosis has already developed, it is disadvantageous in non-disease cases (i.e., the thickness is less than 1.1 mm) in that the value is 0. Carotid arteries often thicken relatively uniformly when arteriosclerosis is complicated by diabetes and/or hyperlipidemia, and AvgIMT and a mean IMT are important indices in such a case. When complicated by hypertonia, plaques are often seen, and PS, PN, and MaxIMT are accordingly effective indices.

As an example of clinical examination results closely related to arteriosclerosis, an increase in carotid artery intima-media thickness can also be mentioned. Increases in the average IMT (ΔIMT) and in the maximum IMT (ΔPIMT) etc. can be used as indices of the increase in carotid artery intima-media thickness. ΔIMT is especially desirable as this comprehensively indicates arteriosclerosis risk. A number of reports have been made about the relationship between an increase in carotid artery intima-media thickness and arteriosclerosis, and in particular, it is known that the odds of myocardial infarction increase 4.9 times for every increase of 0.339 mm in ΔIMT (Yamasaki, *Diabetes Care,* 2000 (9)). ΔIMT can thus be categorized as one clinical examination result closely relevant to arteriosclerosis, and a method using this can assess arteriosclerosis risk extremely effectively. Although the raw increase in carotid artery intima-media thickness can be used for evaluation of arteriosclerosis risk, it is also possible to suitably obtain a function from an increase in carotid artery intima-media thickness and use such a function for the above evaluation.

An increase in carotid artery intima-media thickness (ΔIMT, ΔPIMT, etc.) can be expressed in terms of a partial regression coefficient calculated, using a multiple regression analytical method, from IMT or PIMT values obtained from a group.

The method of the present invention of assessing arteriosclerosis risk can further includes a step of determining arteriosclerosis risk based on a clinical examination result and environmental factors closely related to the disease.

Clinical examination results from subject by themselves can reveal the state of arteriosclerosis of the subjects at the time of measurement, and it is also possible to determine the risk of onset of the disease based on environmental factors and such clinical examination results. However, by further combining the above-described method of the invention, it becomes possible to, while checking the present status of the subject, predict the future risk of arteriosclerosis and its progression, based on the disease risk inherent to the subject. If a future risk could be assessed in a young subject wherein thickening has not yet progressed, it should then be possible to avoid the onset of the disease by taking some prophylactic measures, such as lifestyle improvement, for a person assessed to be at high risk.

Reported arteriosclerosis-related environmental factors are age, gender, hypertension, obesity, smoking history, hemoglobin Alc, history of diabetes or hyperlipidemia, periods thereof, etc.

Vitelli et al. made a comparison, in the Atherosclerosis Risk in Communities (ARIC) study, between 208 cases of non-diabetics with thickened carotid arteries (average IMT: 1.21 mm) and 208 cases of non-diabetics not having their carotid artery thickened (average IMT: 0.63 mm), and estimated that an increase of 1% in hemoglobin Alc would increase the risk of arteriosclerosis 1.77 times (Vitelli LL., *Diabetes Care,* 1997; 20: pp. 1454-1458). Smoking is said to be a risk factor for arteriosclerosis, and the ARIC study targeted at community members has revealed a strong correlation between smoking history and IMT, showing that smoking can be an even stronger promotion factor in diabetics and hypertensives (Howard G, *JAMA,* 1998; 279: pp. 119-124). Sutton-Tyrrell et al. evaluated IMT values and plaque lesions in perimenopausal and postmenopausal women of similar ages, and, based on the fact that the average IMT and the proportion of women with plaques after menopause changed from 0.69 to 0.77 mm and from 25 to 54%, respectively, reported that menopause promotes arteriosclerosis in women (Sutton-Tyrrell K, *Stroke,* 1998; 29: pp. 1116-1121).

The possibility of involvement of various infections has also been noted as a cause of arteriosclerosis. Neito et al. extracted IMT progression groups and non-progression groups in the ARIC study and evaluated antibody titers to cytomegalovirus. The odds ratio of cases with an antibody titer of 20 or more relative to cases with an antibody titer of less than 4 was 5.3, which is significantly high, suggesting the possibility that cytmegalovirus may function as a promotion factor for arteriosclerosis (Nieto Fj, *Circulation,* 1996; 94: 955-7).

The present inventors have previously performed multiple regression analyses using, as dependent variables, IMTs of type I diabetics, type II diabetics, and borderline diabetics. They accordingly reported that independent risk factors in type I diabetics (younger than the age of 30) are age, period of diabetes, and hemoglobin Alc; independent risk factors in type II diabetics (age of 30 or older) are age, hemoglobin Alc, non-HDL cholesterol, blood pressure during systole, and smoking history; and risk factors in borderline diabetes are blood pressure during systole and smoking, as well as age (Yamasaki Y., *Diabetes,* 1994; 43: pp. 634-639).

Age, gender, period of diabetes, and hemoglobin Alc are especially important among the above-mentioned environmental factors.

The method of the present invention for assessing arteriosclerosis risk has been accomplished based on the findings that even if polymorphisms that increase disease risk are present, the risk can be cancelled out by the presence of polymorphisms that confer resistance to the disease; and that even if individual polymorphism by itself does not confer resistance to the disease, a plurality of polymorphisms in combination can confer resistance to the disease. The method of the invention defines as a combination (set) of polymorphisms as a factor that provides resistance to arteriosclerosis risk, and uses such a resistance factor together with a sensitivity factor to thereby assess the risk of disease onset. This makes highly accurate assessment possible. Furthermore, one method of the invention employs, as a disease index, carotid artery intima-media thickness that can be quantitatively measured both in healthy subjects and in patients. This method carefully examines the association between the index and polymorphisms, and suitably indexes positive and negative associations between the thickness and polymorphism sets, thereby achieving an even higher measurement accuracy.

(3-3) Method of assessing myocardial infarction risk

The method of the present invention of assessing myocardial infarction risk can be used to assess the likelihood of occurrence of myocardial infarction and the probability of its progression. The method is advantageously applied to diabetics and patients prone to diabetes (borderline diabetics) for assessing their risk of myocardial infarction (the likelihood of getting myocardial infarction, the probability of it progression, etc.).

The method of the invention of assessing myocardial infarction risk is characterized by comprising a step (b) of checking a genetic polymorphism detected in a specimen against one or more polymorphisms sets negatively associated with a myocardial infarction index. The myocardial infarction index may be, as described above, the presence of abnormal Q waves on an electrocardiogram indicating old myocardial infarction, and the presence of a past history of myocardial infarction, or the like. One or more sets selected from the negative polymorphism sets shown in any one of FIGS. 56 to 58 are preferably used as such one or more polymorphism sets negatively associated with a myocardial infarction index.

Such an assessment method of the invention optionally comprises checking a polymorphism detected in a specimen against one or more polymorphism sets negatively associated with a myocardial infarction index, and also against one or more polymorphism sets positively associated with a myocardial infarction index. That is, the assessment method for assessing myocardial infarction risk may comprise, in addition to the above step (b), a step (b') of checking a polymorphism detected in a specimen against one or more polymorphism sets positively associated with a myocardial infarction index, and a step (c) of, based on the results of steps (b) and (b'), comparing negative association with positive association of the detected polymorphism or polymorphisms to thereby calculate the balance. One or more polymorphism sets selected from the polymorphism sets shown in any one of FIGS 63 to 69 are preferably used as such one or more polymorphism sets positively associated with a myocardial infarction index.

The assessment method of the invention of assessing myocardial infarction risk may further comprise, like the method of assessing arteriosclerosis risk, a step (a) of, prior to step (b) and/or (b'), detecting polymorphisms in a specimen. The detection step (a) may target two or more polymorphisms selected from the 99 polymorphisms shown in FIG. 37. Step (a) may alternatively be a step of detecting the presence of one or more polymorphisms shown in any one of FIGS. 58 to 62, which form negative polymorphism sets, and the presence of one or more polymorphisms shown in any one of FIGS. 70 to 73, which form positive polymorphism sets. Specifically, it may be a step of detecting the presence of one or more polymorphisms shown in FIGS 59 and 70, 60 and 71, 61 and 72, or 62 and 73.

When the above-described array of the present invention for assessing myocardial infarction risk is employed in step (a), the detection can be performed with high accuracy. That is, step (a) may be a step of detecting polymorphisms by hybridizing, on the array of the invention, a probe or probes prepared from a specimen with the probe or probes provided on said array for detecting one or more polymorphisms.

The method of the invention of assessing myocardial infarction risk may further comprise, as with the method of assessing arteriosclerosis risk, a step (d) of weighing the balance calculated in step (c), and thereby determining the degree of balance (i.e., the degree of disease risk). Further, the method may also include a step of determining myocardial infarction risk based on a clinical examination result from a subject and/or environmental factors closely related to the disease.

The method of the invention may be performed in the same manner as the above-described method of assessing arteriosclerosis risk, and this makes it possible to obtain highly accurate results in assessing myocardial infarction risk.

The assessment method of the invention include the following modes:

(A) method of assessing disease risk, comprising:

a step (b) of checking a genetic polymorphism detected in a specimen against one or more genetic polymorphisms negatively associated with a disease index, or against one or more genetic polymorphism sets negatively associated with a disease index.

(B) A method of assessing disease risk according to (A), further comprising:

a step (b') of checking a genetic polymorphism detected in the specimen against one or more genetic polymorphisms positively associated with a disease index, or against one or more genetic polymorphism sets positively associated with a disease index; and

a step (c) of, based on the result of step (b'), comparing the negative association with the positive association of a set of detected genetic polymorphisms to thereby calculate the balance.

(C) A method of assessing disease risk according to (B), further comprising:

a step (d) of evaluating disease risk from the calculated balance.

(D) A method of assessing disease risk according to any one of (A) to (D), wherein the target disease is arteriosclerosis, and said one or more genetic polymorphism sets negatively associated with a disease index are:

one or more sets selected from the negative genetic polymorphism sets shown in any one of FIGS. 1 to 9; or one or more sets selected from the negative genetic polymorphism sets shown in any one of FIGS. 38 to 43.

(E) A method of assessing disease risk according to any one of (B) to (D), wherein the target disease is arteriosclerosis, and

(1) said one or more genetic polymorphism sets negatively associated with a disease index are one or more sets selected from the negative genetic polymorphism sets shown in any one of FIGS. 1 to 9, and
said one or more genetic polymorphism sets positively associated with a disease index are one or more sets selected from the positive genetic polymorphism sets shown in any one of FIGS 19 to 27; or
(2) said one or more genetic polymorphism sets negatively associated with a disease index are one or more sets selected from the negative genetic polymorphism sets shown in any one of FIGS. 38 to 43, and
said one or more genetic polymorphism sets positively associated with a disease index are one or more sets selected from the positive genetic polymorphism sets shown in any one of FIGS. 48 to 51.

(F) A method of assessing disease risk according to any one of (A) to (E), comprising:

a step (a) of, prior to step (b) and/or (b'), detecting genetic polymorphisms in a specimen.

(G) A method of assessing disease risk according to (F), wherein step (a) is a step of detecting two or more genetic polymorphisms selected from the 99 genetic polymorphisms shown in FIG. 37.
(H) A method of assessing disease risk according to (F) or (G), wherein the target disease is arteriosclerosis, and wherein step (a) is a step of detecting genetic polymorphisms by hybridizing, on the array of the present invention for assessing arteriosclerosis risk, a probe or probes prepared from a specimen with the probe or probes of said array for detecting one or more genetic polymorphisms.
(I) A method of assessing disease risk according to (F), wherein the target disease is myocardial infarction, and step (a) is a step of detecting genetic polymorphisms by hybridizing, on the array of the present invention for assessing myocardial infarction risk, a probe or probes prepared from prepared from a specimen with the probe or probes of said array for detecting one or more genetic polymorphisms.
(J) A method of assessing disease risk according to any one of (A) to (I), wherein a group of arteriosclerosis cases and non-arteriosclerosis cases is a group of cases having diabetes but not having a history of myocardial infarction.

**(4) Method of selecting active drugs**

The present invention also provides a method of selecting active drugs according to characteristics of a subject, based on a categorization of genetic polymorphisms detected in a specimen. This method comprises selecting drugs that are considered to be applicable based on a categorization of polymorphisms detected in a specimen. Polymorphisms may be categorized according to items a) to j) given above.
Specific examples of active drugs usable for each polymorphism category are, although not limited thereto, as follows:

a) stains for lipid-related polymorphisms,
b) ACE inhibitors and angiotensin II receptor blockers for blood pressure-related polymorphisms,
d) insulin sensitivity enhancers for insulin resistance-related polymorphisms,

f) antioxidants for oxidation stress-related polymorphisms,
g) immunosuppression agents and statins for inflammatory response-related polymorphisms, and
h) antiplatelet agents for coagulation and fibrinolytic system-related polymorphisms.

Antiproteases can be selected as active drugs when MMP-12 (A-82G), mmp-9=Gelatinase B (C-1562T), MMP7 (C-153T), etc. are detected. It is also possible to employ drugs from two or more of the above categories together, when two or more categories of polymorphisms have been detected in a specimen.
By selecting suitable drugs depending upon the polymorphisms of each subject, it becomes possible to provide subjects with suitable preventive measures, therapeutic measures, and prognostic measures against a disease, according to their physical predisposition and characteristics.

**(5) Method of eliciting disease-resistance factors/ disease risk**

From another point of view, the present invention provides a method of eliciting the disease-resistance factors that a subject possesses and a method of eliciting the disease risk that a subject possesses.
The method of the invention of eliciting disease-resistance factors comprises:

a step (i) of checking a polymorphism detected in a specimen against polymorphisms negatively associated with a disease index, or against one or more polymorphism sets negatively associated with a disease index, and
a step (ii) of, based on the results of step (i), eliciting disease-resistance factors in the specimen. As long as these steps are included, any other additional steps can be included in the method of the invention.

The elicitation step (ii) is performed by revealing whether any of the polymorphisms detected in a specimen match one or more polymorphisms or one or more polymorphism sets negatively associated with a disease index. For example, when the target disease is arteriosclerosis (preferably, diabetic arteriosclerosis), step (i) is performed by checking a polymorphism detected in a specimen against one or more polymorphisms or one or more polymorphism sets negatively associated with carotid artery intima-media thickness (IMT). One or more sets selected from the negative polymorphism sets shown in any one of FIGS. 1 to 9 or one or more sets selected from the negative polymorphism sets shown in any one of FIGS 38 to 43 can be used as such one or more polymorphisms sets.
As another example, when the target disease is myocardial infarction (preferably, diabetic myocardial infarction), step (i) is performed by checking a polymorphism detected in a specimen against one or more polymorphisms or one or more polymorphism sets negatively associated with a myocardial infarction index. One or more sets selected from the negative polymorphism sets shown in any one of FIGS. 56 to 58 can be used as such one or more polymorphism sets.
The elicitation method may further comprise, prior to step (i), a step (0) of detecting polymorphisms in a specimen.
Step (0) may be a step of detecting at least two polymorphisms selected from the 99 polymorphisms shown in FIG. 37. When the target disease is arteriosclerosis (preferably, diabetic arteriosclerosis), the target of detection may be one or more polymorphisms shown in any one of FIGS. 10 to 18, or one or more polymorphisms shown in any one of FIGS. 44 to 47. When the target disease is myocardial infarction (preferably, diabetic myocardial infarction), the target of detection may be one or more polymorphisms shown in any one of FIGS. 59 to 62.
The detection step (0) may be performed by hybridizing, on the array of the present invention, a probe or probes prepared from a specimen with the probe or probes provided on said array for detecting one or more polymorphisms. Although the specimen is not limited, the present invention is more beneficial when it is a biological specimen taken from a subject who is a diabetic not having a history of arteriosclerosis or myocardial infarction.
A considerable number of polymorphisms are present in the human genome, and it is impossible to assess the risk from only a single example thereof, because single polymorphisms have low odds and their frequencies are limited. Therefore, if polymorphisms are analyzed one by one, disease-related factors, which each consist of a combination of polymorphisms inherent in an individual, cannot be found. The present inventors revealed from analyses of a large number of populations that there exist a plurality of polymorphism sets significantly negatively associated with disease indices, and defined such polymorphism sets as disease-resistance factors. The present invention has been accomplished based on the findings that disease-resistance factors can be elicited when the presence of polymorphisms forming such specific polymorphism sets has been selectively revealed in a specimen. Such elicited disease-resistance factors are extremely valuable as information for disease risk determination. To "selectively reveal" herein means to select and reveal specific polymorphisms out of a multiplicity of combinations of polymorphisms.
Disease-resistance factors can be elicited simply by combining combinations (sets) of such polymorphisms. Each factor may also be elicited as an odds ratio for the frequency of the presence of a significant negative association between the corresponding polymorphism set and a suitable disease index, depending on whether genotypes of the selectively revealed polymorphisms match any disease-resistance factor (disease-resistant polymorphism set) (e.g., whether 0 or 1), or when genotypes of the selectively revealed polymorphisms match any disease-resistant factor (disease-resistant polymorphism set). Further, every disease-resistant polymorphism set suppresses an increase in a disease index value, and therefore, when genotypes of the selectively revealed polymorphisms match any disease-resistant polymorphism sets, it is also possible to elicit the factor as a suppression peculiar to such a disease-resistant polymorphism set. That is, the method is not limited as long as it is capable of eliciting at least one disease-resistant polymorphism set present in the targeted polymorphisms.

The method of the present invention of eliciting disease risk comprises:

a step (i) of checking a polymorphism detected in a specimen against one or more polymorphism negatively associated with a disease index or one or more polymorphism sets negatively associated with a disease index, and also against one or more polymorphisms positively associated with a disease index or one or more polymorphism sets positively associated with a disease index; and

a step (ii) of, based on the results of step (i), eliciting disease-resistance factors and disease-sensitivity factors in the specimen.

As long as these steps are included, any other additional steps may be included in the method of the invention.

Such a risk elicitation method may further comprise a step (iii') of calculating the balance between disease-resistance factors and disease-sensitivity factors in the specimen.

For example, when the target disease is arteriosclerosis (preferably, diabetic arteriosclerosis), step (i') is performed by checking a polymorphism detected in a specimen with one or more polymorphisms or one or more polymorphism sets negatively associated with "carotid artery intima-media thickness" (IMT), which is an arteriosclerosis index, and also against one or more polymorphisms or one or more polymorphism sets positively associated with IMT. One or more negative polymorphism sets shown in any one of FIGS. 1 to 9 or one or more negative polymorphism sets shown in any one of FIGS. 38 to 43 can be used as such one or more polymorphism sets having a negative association. One or more positive polymorphism sets shown in any one of FIGS. 19 to 27 or one or more positive polymorphism sets shown in any one of FIGS. 48 to 51 can be used as such one or more polymorphism sets having a positive association.

For another example, when the target disease is myocardial infarction (preferably, diabetic myocardial infarction), step (i') is performed by checking a polymorphism detected in a specimen against one or more polymorphisms or one or more polymorphism sets negatively associated with a myocardial infarction index, and also against one or more polymorphisms or one or more polymorphism sets positively associated with a myocardial infarction index. One or more negative polymorphism sets shown any one of in FIGS. 38 to 43 can be used as such one or more polymorphism sets having a negative association. One or more positive polymorphism sets shown in any one of FIGS. 50 to 53 can be used such one or more polymorphism sets having a positive association.

The method of the present invention may comprise a step (0') of, prior to step (1'), detecting polymorphisms in a specimen. The detection step (0') may be a step of detecting at least two polymorphisms selected from the 99 polymorphisms shown in FIG. 38. When the target disease is arteriosclerosis (preferably, diabetic arteriosclerosis), the detection step (0') may be a step of detecting the presence of one or more polymorphisms shown in, for example, FIGS. 10 and 28, FIGS. 11 and 29, FIGS. 12 and 30, FIGS. 13 and 31, FIGS. 14 and 32, FIGS. 15 and 33, FIGS. 16 and 34, FIGS. 17 and 35, or FIGS. 18 and 36. The step may alternatively be a step of detecting the presence of one or more polymorphisms shown in FIGS. 38 and 48, FIGS. 39 and 49, FIGS. 40 and 50, FIGS. 41 and 51, FIGS. 42 and 51, or FIGS. 43 and 51.

When the target disease is myocardial infarction (preferably, diabetic myocardial infarction), the detection step (0') may be a step of, for example, detecting the presence of one or more polymorphisms shown in FIGS. 59 and 70, FIGS. 60 and 71, FIGS. 61 and 72, or FIG. 62 and 73.

Step (0') may also be a step of detecting polymorphisms by hybridizing, on the array of the present invention, a probe or probes prepared from a specimen with the probe or probes provided on the array for detecting one or more polymorphisms.

The present invention is more beneficial when the specimen is a biological specimen taken from a subject who is a diabetic not having a history of arteriosclerosis or myocardial infarction.

The present inventors revealed from analyses of a large number of populations that there exist a plurality of polymorphism sets significantly negatively or positively associated with disease indices, and defined such polymorphism sets as disease-resistance or disease-sensitivity factors. The present invention has been accomplished based on the findings that the disease risk of a subject can be elicited when a risk evaluation is made using polymorphisms that form resistance factor polymorphism sets together with those forming sensitivity factor polymorphism sets.

The information about elicited disease-resistance factors (disease-resistant polymorphism sets) combined with elicited disease-sensitivity factors (disease-sensitive polymorphism sets) is highly valuable information for disease risk assessment.

Disease risk can be elicited by simply combining combinations (sets) of such polymorphisms. The risk may also be elicited as an odds ratio for the frequency of presence of a significant negative or positive association between a disease-associated polymorphism set and a suitable disease index, depending on whether genotypes of selectively revealed polymorphisms match any disease-associated polymorphism set, or when genotypes of the selectively revealed polymorphisms match any disease-associated polymorphism set. Further, every disease-associated polymorphism set either increases a disease index value or suppresses an increase in a disease index value, and therefore, when genotypes of the selectively revealed polymorphisms match a disease-associated polymorphism sets (positive or negative), it is possible to elicit the risk as an increase or a suppression unique to such a disease-associated polymorphism set. That is, the method is not limited as long as it is capable of assessing and eliciting resistance and sensitivity of disease-associated polymorphism sets of the targeted polymorphisms.

## (6) Genetic marker

(6-1) Genetic marker exhibiting resistance or sensitivity to arteriosclerosis

The present invention provides a genetic marker exhibiting resistance to arteriosclerosis. Such a genetic marker can be advantageously used in detecting and selecting arteriosclerosis-resistant polymorphisms in specimens. This genetic marker comprises at least one polymorphism selected from negative (resistant) polymorphisms or polymorphism sets. Specifically, it comprises one or more polymorphisms of at least one polymorphism set selected from the negative polymorphism sets shown in any one of FIGS. 1 to 9, or one or more polymorphisms of at least one set selected from the negative polymorphism sets shown in any one of FIGS. 38 to 43.

The present invention also provides a genetic marker exhibiting sensitivity to arteriosclerosis. Such a genetic marker can be advantageously used in detecting an arteriosclerosis-sensitive polymorphism in a specimen. This genetic marker comprises at least one polymorphism selected from positive (sensitive) polymorphisms or polymorphism sets. Specifically, it comprises one or more polymorphisms of at least one polymorphism set selected from the positive polymorphism sets shown in any one of FIGS. 19 to 27, or one or more polymorphisms of at least one polymorphism set selected from the positive polymorphism sets shown in any one of FIGS. 48 to 51. The genetic marker sensitive to arteriosclerosis is preferably used in combination with the above genetic marker resistant to arteriosclerosis.

These genetic markers exhibiting resistance/sensitivity to arteriosclerosis can be used to detect and select arteriosclerosis-resistant polymorphisms and arteriosclerosis-sensitive polymorphisms, respectively, and also to determine the existence of arteriosclerosis and measure its degree.

(6-2) Genetic marker exhibiting resistance or sensitivity to myocardial infarction

The present invention further provides a genetic marker exhibiting resistance to myocardial infarction. Such a genetic marker can be advantageously used in detecting and selecting myocardial infarction-resistant polymorphisms in specimens. This genetic marker comprises at least one polymorphism selected from negative (resistant) polymorphisms or polymorphism sets. Specifically, it comprises one or more polymorphisms of at least one polymorphism set selected from the negative polymorphism sets shown in any one of FIGS. 56 to 58.

The present invention also provides a genetic marker exhibiting sensitivity to myocardial infarction. Such a genetic marker can be suitably used in detecting a myocardial infarction-sensitive polymorphism in a specimen. This genetic marker comprises at least one polymorphism selected from positive (sensitive) polymorphisms or polymorphism sets. Specifically, it comprises one or more polymorphisms of at least one polymorphism set selected from the positive polymorphism sets shown in any one of FIGS. 63 to 68. The genetic marker sensitive to myocardial infarction is preferably used in combination with the above genetic marker resistant to myocardial infarction.

These genetic markers exhibiting resistance/sensitivity to myocardial infarction can be used to detect and select myocardial infarction-resistant polymorphisms and myocardial infarction-sensitive polymorphisms, respectively, and also to determine the existence of myocardial infarction and measure its degree.

The marker of the present invention includes the following modes:

(A) A genetic marker resistant to arteriosclerosis comprising:

one or more genetic polymorphisms of at least one genetic polymorphism set selected from the negative genetic polymorphism sets shown in any one of FIGS. 1 to 9; or

one or more genetic polymorphisms of at least one genetic polymorphism set selected from the negative genetic polymorphism sets shown in any one of FIGS. 38 to 43.

(B) (1) A genetic marker resistant to arteriosclerosis comprising one or more genetic polymorphisms of at least one genetic polymorphism set selected from the negative genetic polymorphism sets shown in any one of FIGS. 1 to 9, and a genetic marker sensitive to arteriosclerosis comprising one or more genetic polymorphisms of at least one genetic polymorphism set selected from the positive genetic polymorphism sets shown in any one of FIGS. 19 to 27; or
(2) a genetic marker resistant to arteriosclerosis comprising one or more genetic polymorphisms of at least one genetic polymorphism set selected from the negative genetic polymorphism sets shown in any one of FIGS. 38 to 43; and
a genetic marker sensitive to arteriosclerosis comprising one or more genetic polymorphisms of at least one genetic polymorphism set selected from the positive genetic polymorphism sets shown in any one of FIGS. 48 to 55.
(C) A genetic marker resistant to myocardial infarction comprising:

one or more genetic polymorphisms of at least one genetic polymorphism set selected from the negative genetic polymorphism sets shown in any one of FIGS. 56 to 58.

(D) A genetic marker resistant to myocardial infarction comprising one or more genetic polymorphisms of at least one genetic polymorphism set selected from the negative genetic polymorphism sets shown in any one of FIGS. 56 to 58; and
a genetic marker sensitive to myocardial infarction comprising one or more genetic polymorphisms of at least one genetic polymorphism set selected from the positive genetic polymorphism sets shown in any one of FIGS. 63 to 69.

**(7) Kit for analyzing genetic polymorphisms**

The kit for analyzing genetic polymorphisms of the invention comprises at least one primer pair capable of specifically amplifying a gene or genes that contain one or more negative polymorphisms or one or more polymorphisms of at least one negative polymorphism set, or instead comprises at least one nucleic acid probe capable of specifically hybridizing with such a gene of genes. The analysis kit can be advantageously used as a kit for detecting a disease-resistant polymorphism. Further, the analysis kit may comprise at least one primer pair capable of specifically amplifying a gene or genes that contain one or more positive polymorphisms or one or more polymorphisms of at least one positive polymorphism set, or instead comprise at least one nucleic acid probe capable of specifically hybridizing with such a gene or genes. Such an analysis kit can be advantageously used as a kit for detecting a disease-resistant polymorphism and a disease-sensitive polymorphism.
Examples of said at least one negative polymorphism set include one or more polymorphism sets selected from the negative polymorphism sets shown in any one of FIGS. 1 to 9, and one or more polymorphism sets selected from the negative polymorphism sets shown in any one of FIGS. 38 to 43. An analysis kit comprising at least one primer pair capable of specifically amplifying gene(s) that contain polymorphism(s) of a polymorphism set or at least one nucleic acid probe capable of specifically hybridizing with such gene(s) can be advantageously used as an analysis kit for detecting a polymorphism resistant to arteriosclerosis. In this case, the analysis kit may additionally comprise at least one primer pair capable of specifically amplifying a gene or genes that contain one or more polymorphisms of at least one polymorphism set selected from the positive polymorphism sets shown in any one of FIGS. 19 to 27 and FIGS. 50 to 53, or instead comprise at least one nucleic acid probe capable of specifically hybridizing with such a gene or genes. Such an analysis kit can be advantageously used as a kit for detecting a polymorphism resistant to arteriosclerosis and a polymorphism sensitive to arteriosclerosis.
Further examples of said at least one negative polymorphism set include one or more polymorphism sets selected from the negative polymorphism sets shown in any one of FIGS. 56 and 58. An analysis kit comprising at least one primer pair capable of specifically amplifying gene(s) that contain polymorphism(s) of such a polymorphism set or at least one nucleic acid probe capable of specifically hybridizing with such gene(s) can be advantageously used as an analysis kit for detecting a polymorphism resistant to myocardial infarction. In this case, the analysis kit may further comprise at least one prime pair capable of specifically amplifying a gene or genes that contain one or more polymorphisms of at least one polymorphism set selected from the positive polymorphism sets shown in any one of FIGS. 63 to 68, or instead comprise at least one nucleic acid probe capable of specifically hybridizing with such a gene or genes. Such an analysis kit can be advantageously used as a kit for detecting a polymorphism resistant to myocardial infarction and a polymorphism sensitive to myocardial infarction.
The kit for analyzing genetic polymorphisms of the invention comprises at least one primer pair or at least one nucleic acid probe as described above and may further comprise other nucleic acids, reagents, etc. insofar as the object of the invention can be achieved. To detect a negative polymorphism set or a positive polymorphism set, the kit must comprise at least one primer pair or at least one probe capable of detecting the at least two polymorphisms that form such a polymorphism set. A kit comprising a primer pair capable of detecting one polymorphism and a probe for detecting the other polymorphism is encompassed within the scope of such a kit for analyzing genetic polymorphisms of the invention, as long as the kit can analyze polymorphisms.
Genetic polymorphisms can be detected by any of the methods described in the genetic polymorphism detection process above. Especially, Hybrigene, TaqMan, and invader assays using PCR; ASP-PCR using nucleic acid probes capable of specifically hybridizing with genes having polymorphisms; and like methods are preferably used.

Therefore, the kit for analyzing polymorphisms must contain at least either a primer pair or a probe used in such a polymorphism detection process. In PCR methods for detecting polymorphisms, a polymorphic nucleic acid is usually positioned at the 3' end. However, primer designs as to in which region polymorphisms are to be located and which nucleotide sequences other than the target gene are to be inserted are not particularly limited, insofar as polymorphisms can be identified. For example, in allele specific primer (ASP)-PCR methods, primers having a polymorphism next to the 3' end can be employed.

The analysis kit of the invention encompasses the following embodiments:

(A) A kit for analyzing genetic polymorphisms resistant to arteriosclerosis, comprising:

(1) at least one primer pair capable of specifically amplifying a gene or genes that contain one or more genetic polymorphisms of at least one genetic polymorphism set selected from the negative genetic polymorphism sets shown in any one of FIGS. 1 to 9, or at least one nucleic acid probe capable of specifically hybridizing with said gene or genes; or

(2) at least one primer pair capable of specifically amplifying a gene or genes that contain one or more genetic polymorphisms of at least one genetic polymorphism set selected from the negative genetic polymorphism sets shown in any one of FIGS. 38 to 43, or

at least one nucleic acid probe capable of specifically hybridizing with said gene or genes.

(B) A kit for analyzing genetic polymorphisms resistant or sensitive to arteriosclerosis, comprising:

(1) at least one primer pair capable of specifically amplifying a gene or genes that contain one or more genetic polymorphisms of at least one genetic polymorphism set selected from the negative genetic polymorphism sets shown in any one of FIGS. 1 to 9, or

at least one nucleic acid probe capable of specifically hybridizing with said gene or genes; and

at least one primer pair capable of specifically amplifying a gene or genes that contain one or more genetic polymorphisms of at least one genetic polymorphism set selected the positive genetic polymorphism sets shown in any one of FIGS. 19 to 27, or at least one nucleic acid probe capable of specifically hybridizing with said gene or genes; or

(2) at least one primer pair capable of specifically amplifying a gene or genes that contain one or more genetic polymorphisms of at least one genetic polymorphism set selected from the negative genetic polymorphism sets shown in any one of FIGS. 38 to 43, or

at least one nucleic acid probe capable of specifically hybridizing with said gene or genes; and

at least one primer pair capable of specifically amplifying a gene or genes that contain one or more genetic polymorphisms of at least one genetic polymorphism set selected from the positive genetic polymorphism sets shown in any one of FIGS. 48 to 51, or

at least one nucleic acid probe capable of specifically hybridizing with said gene or genes.

(C) A kit for analyzing genetic polymorphisms resistant to myocardial infarction, comprising:

at least one primer pair capable of specifically amplifying a gene or genes that contain one or more genetic polymorphisms of at least one genetic polymorphism set selected from the negative genetic polymorphism sets shown in any one of FIGS. 56 to 58;

or at least one nucleic acid probe capable of specifically hybridizing with said gene or genes.

(D) A kit for analyzing genetic polymorphisms resistant to or sensitive to myocardial infarction, comprising:

at least one primer pair capable of specifically amplifying a gene or genes that contain one or more genetic polymorphisms of at least one genetic polymorphism set selected from the negative genetic polymorphism sets shown in any one of FIGS. 56 to 58, or

at least one nucleic acid probe capable of specifically hybridizing with said gene or genes; and

at least one primer pair capable of specifically amplifying a gene or genes that contain one or more genetic polymorphisms of at least one genetic polymorphism set selected from the positive genetic polymorphism sets shown in any one of FIGS. 63 to 69, or

at least one nucleic acid probe capable of specifically hybridizing with said gene or genes.

EXAMPLES

The following examples illustrate the present invention in more detail, without limiting the invention thereto. Example 1 Assessment of arteriosclerosis risk

1. Determination of genetic polymorphisms positively or negatively associated with arteriosclerosis

<Analytical procedure>

Among diabetic cases, those with a carotid artery intima-media thickness at least 0.2 mm greater than a healthy group in the same age bracket (i.e., 20s, 30s, etc.) were defined as Cases (disease cases), and the others as Controls (non-disease cases). In the Case group (disease group) and the Control group (non-disease group), polymorphisms were detected by the following procedures.

For ease of explanation, insertional polymorphisms of ACE gene (gene code: ACE ID) are described below, but polymorphisms of other genes were also subjected to similar procedures.

(1) Synthesis of primers for amplifying ACE gene fragment

Primer 1 (CTGGAGACCA CTCCCATCCT TTCT) having a sequence homologous to a part of the sequence of human ACE gene and primer 2 (GATGTGGCCATCACATTCGT CAGAT) having a sequence complementary to a part of the sequence of human ACE gene and having biotin linked to the 5' terminus were synthesized according to the phosphoamidite method using a Perkin-Elmer DNA synthesizer, model 392. Synthesis was carried out according to the manufacturer's manual. The oligonucleotides were deprotected at 55°C using aqueous ammonia overnight. The oligonucleotides were purified using a Perkin-Elmer OPC column.

(2) Synthesis of oligonucleotides having a linker arm

An oligonucleotide (probe I: TTACAGGCGT GATACAGTCA C) having a sequence homologous to a part of the sequence of a human ACE gene and having a linker arm at the 5' terminus, and an oligonucleotide (probe D: GCCTATACAG TCACTTTTAT GTG) having a sequence complementary to a part of the sequence of human ACE gene and having a linker arm at the 5' terminus were synthesized according to the phosphoamidite method using a Perkin-Elmer DNA synthesizer, model 392.

Uridine having a linker arm at position 5, prepared from deoxyuridine by chemical synthesis according to the synthetic method described in Japanese Unexamined Patent Publication No. 1985-500717, was introduced into the oligonucleotides. The obtained linker oligonucleotides were deprotected at 50°C using aqueous ammonia overnight, and then purified using a Perkin-Elmer OPC column.

(3) Immobilization of probe oligonucleotide to microtiter plate

The probe oligonucleotides obtained in (2) were attached via their linker arms to the interior surface of a microtiter plate. More specifically, each oligonucleotide was diluted in a solution of 50mM boric acid buffer (pH 10) and 100mM $MgCl_2$ to a concentration of 0.05 pmol/μl, and 100 μl thereof was pipetted into each well of the microtiter plate (MicroFLUOR B; product of Dynatech, Inc.) and allowed to stand at room temperature for about 15 hours to immobilize the linker oligonucleotide to the interior surface of the microtiter plate. The solution was replaced with a solution of 0.1 pmol dNTP, 0.5%PVP and 5 x SSC, and blocking was performed to prevent nonspecific reactions at room temperature for about 2 hours, followed by washing with 1 x SSC and drying.

(4) Amplification of human ACE gene fragment by PCR

A DNA solution extracted from human leukocyte was used as a sample, and the following reagent was added to amplify the human ACE gene fragment under the conditions given below.
<Reagent> A solution (25 μl) containing the following components was prepared.

| | |
|---|---|
| Primer 1 | 10 pmol |
| Primer 2 | 10 pmol |
| x 10 buffer solution | 2.5 μl |
| 2 mM dNTP | 2.5 μl |
| Tth DNA polymerase | 1U |

Extracted DNA solution 100 ng

<Amplification conditions>

94°C for 2 minutes
94°C for 1 minute, 65°C for 2 minutes, 75°C for 1.5 minutes (35 cycles).

(5) Hybridization in the microtiter plate

The amplification reaction mixture obtained in (4) was diluted 10-fold, and the amplified DNA in the amplification reaction mixture was denatured in 0.3N NaOH. 20 μl of the amplification reaction mixture of each sample was added to 100 μl of a solution of 200mM citric acid-phosphate buffer (pH 6.0), 2%SDS, 750mM NaCl, and 0.1% $NaN_3$. The resulting mixture was placed in the microtitler plate obtained in (3) with a capture probe immobilized thereto. Liquid paraffin was overlaid to prevent evaporation, and such a microtiter was shaken at 55°C for 30 minutes, whereby the amplified human ACE gene fragment was specifically captured onto the microtiter plate by the immobilized probe.
The solution was then replaced with a solution of 2 x SSC (pH 7.0) and 1% SDS, and liquid paraffin was overlaid to prevent evaporation. The microtiter was shaken at 55°C for 20 minutes. The solution was then replaced with 100 μl of a dilute solution of alkaline phosphatase-labeled streptavidin (D0396, product of DAKO) 2000-fold diluted in a solution of 50 mM Tris-hydrochloric acid buffer (pH 7.5) and 1% BSA, followed by shaking at 37°C for 15 minutes, whereby the alkaline phosphatase-labeled streptavidin was specifically bound to the biotin of the captured DNA. After washing three times with 250 μl of a solution of 50 mM Tris-hydrochloric acid buffer (pH 7.5) and 0.025% Tween 20, 50 μl of a dioxycetane compound (trade name: Lumiphos 480; product of Lumigen, Inc.), that is a luminescent substrate for alkaline phosphatase, was injected. After maintaining at 37°C for 15 minutes, the amount of light emission was measured in a dark room using a photon counter (product of Hamamatsu Photonics K.K.).
The entire process was performed automatically by a DNA probe automatic measurement system (see Journal of the Japan Society for Clinical Laboratory Automation, vol.20, page 728 (1995)), and took about 2.5 hours.

(6) Results

ACE gene polymorphisms are studied based on the results of amplification and detection made in (4) and (5) above. Signal I is defined as the signal detected from an amplified nucleic acid fragment reacting with probe I, and signal D as the signal detected from an amplified nucleic acid fragment reacting with probe D. Base polymorphisms can be identified by calculating the logarithm of the ratio of the signals obtained using the probes. More specifically, when the logarithm of the ratio of the signals is 0.0 or more, the genotype can be identified as a I/I (insertion/insertion) homozygous genotype. When the logarithm of the ratio of the signals is -1.0 or less, the genotype can be identified as a D/D (deletion/deletion) homozygous genotype. When the logarithm of the ratio of the signals is between -1.0 and 0.0, the genotype can be identified as an I/D heterozygous genotype.

2. Analysis of SNP

2-1. Determination of Odds and Kai

Odds (odds ratio) and Kai (chi-square value) of a genotype of each SNP determined.
"Genotypes" are indicated by the numerals 1, 2 and 3 according to the classification described above. For example, genotypes of polymorphisms of the ACE gene having I/D are expressed as,

1: DD (D/D homozygote)
2: I/D (heterozygote)
3: II (I/I homozygote).

I/D + DD (having D alleles) is expressed as "12", and II + I/D (having I alleles) as "23".
"Odds" indicates the balance of a SNP genotype toward Case or Control. When Odds is 2, it means that the frequency of appearance of the genotype in the Case is twice that in the Control.
"Kai (chi-square value)" indicates statistical significance of the balance of a SNP genotype toward Case or Control. When Kai is at least 3.8, $P < 0.05$.

$$Kai \geq 5.024 \rightarrow p<0.025$$

$$Kai \geq 6.635 \rightarrow p<0.01$$

$$Kai \geq 10.827 \rightarrow p<0.001.$$

The difference between Kai and Odds is that while Odds does not depend on the frequency of appearance, Kai may decrease when the number of subjects increases. For example, when polymorphism A appears in 1% of the Case group among 500 subjects and polymorphism B appears in 0.5% of the Case group among 5000 subjects, Kai of polymorphism B may be higher than that of polymorphism A even if they have the same Odds.

2-2 Selection of disease-sensitive genetic polymorphisms and disease-resistant genetic polymorphisms

Based on Odds and Kai of each SNP genotype obtained above, and using a selection condition of Odds > 2.0 and Kai > 3.8, disease-sensitive SNPs (SNPs from the Case group, having an Odds of more than 2.0) and disease-resistant SNPs (SNPs from the Control group, having an Odds of more than 2.0).

2-3 Multiplex analysis of SNPs

Two hundred SNPs were first chosen from scientific papers by keyword search using search terms related to arteriosclerotic, etc., and 99 SNPs were selected therefrom.Subsequently, a few SNPs were arbitrarily picked from the 99 SNPs to make all possible combinations of 2 SNPs. With respect to both Case group and Control group, Odds and Kai were determined for each combination of 2 SNPs each having a specific genotype (1, 2, 3) (4 x 4 = 16 combinations for one SNP pair; 99 x 98 x 16/2 combinations for all SNP pairs). Such a specific SNP combination is herein referred to as an SSNP (Synergetic SNP).

2-4 Selection of significant SSNPs

(1) The SSNPs obtained above are ranked in decreasing order of Kai (for example, when 10 SSNPs are sorted, SSNPs with the highest, second highest, third highest, ... Kai values were ranked from the top).
(2) SSNPs explicative of the Case group and the Control group are determined.

Specifically, such determination is made, for example, as follows. Provided that:

a first SSNP is explicative of 5 subjects, case Nos. 5, 10, 15, 20 and 28;
a second SSNP is explicative of 3 subjects, case Nos. 5, 6 and 30; and
a fifth SSNP is explicative of 3 subjects, case Nos. 5, 10 and 15, the first SSNP is used as an "explicative SSNP"; the second SNP is used as an "explicative SSNP" because case Nos. 6 and 30, which are not explained by the first SSNP, can be explained thereby; and the fifth SSNP is not used because all the cases explicable thereby can be explained by the first "explicative SSNP". In this way, "SSNPs explicative of" the Case and Control groups were selected from the SSNPs obtained in 2-3.

2-5 Abandonment of insignificant SNP polymorphisms

The frequency (%) of SNP polymorphisms included in the "explicative SSNP" group obtained in 2-4 was calculated, and those with low frequency were discarded.
For example, when

a first SSNP is [ACE-II and MTHFR TT],
a second SSNP is [ACE-II and eNos (abbreviation: N1) C allele],
a third SSNP is [ACE-Iallele and eNos (abbreviation: N1) C allele],
the frequency of ACE-II is 3.5% and that of ACE-I allele is 0.5%. Therefore, only ACE-II is used, and ACE-I allele is discarded (either ACE-II or I allele, whichever has a higher frequency, is considered to be the more important).

2-6 Re-evaluation of SSNPs

SSNPs including any of the SNP polymorphisms discarded in 2-5 were selected from the SSNP group obtained in 2-3, and those SSNPs were discarded. The remaining SSNP group was subjected to the procedures of 2-4 and 2-5 once more to select explicative SSNPs.

FIG. 19 is a list of the thus obtained SSNPs explicative of the Case group (disease group) (polymorphisms positively associated with arteriosclerosis). FIG. 1 is a list of the thus obtained SSNPs explicative of the Control group (non-disease group)(polymorphisms negatively associated with arteriosclerosis).

3. Assessment of arteriosclerosis using explicative SNPs

With respect to diabetic patients (subjects) without a history of myocardial infarction, arteriosclerosis-sensitive genetic polymorphisms ("explicative SSNPs") and arteriosclerosis-resistant genetic polymorphisms ("explicative SSNPs") were selected as described above. The number of arteriosclerosis-sensitive polymorphisms ("explicative SSNPs") and arteriosclerosis-resistant polymorphisms ("explicative SSNPs") in each subject was determined with reference to FIGS. 1 and 19. When the number of sensitive polymorphisms was greater than that of resistant polymorphisms, the subject was classified as "a high-risk case of arteriosclerosis". When the number of resistant polymorphisms was greater than that of sensitive polymorphisms, the subject was classified as "a low-risk case of arteriosclerosis".

The carotid artery intima-media thickness of these subjects was also determined. When the carotid artery intima-media thickness was at least 0.2 mm greater than the normal average thickness, the subject was classified as an "arteriosclerosis case". When the thickness was otherwise, the subject was classified as a "non-arteriosclerosis case".

With respect to each subject, the degree of agreement between an arteriosclerosis case and a high-risk case of arteriosclerosis and also between a non-arteriosclerosis case and a low-risk case of arteriosclerosis was determined as "sensitivity (accuracy)". The degree of disagreement between an arteriosclerosis case and a high-risk case of arteriosclerosis and also between a non-arteriosclerosis case and a low-risk case of arteriosclerosis was determined as "false-positive (inaccuracy)"

<Analysis results>

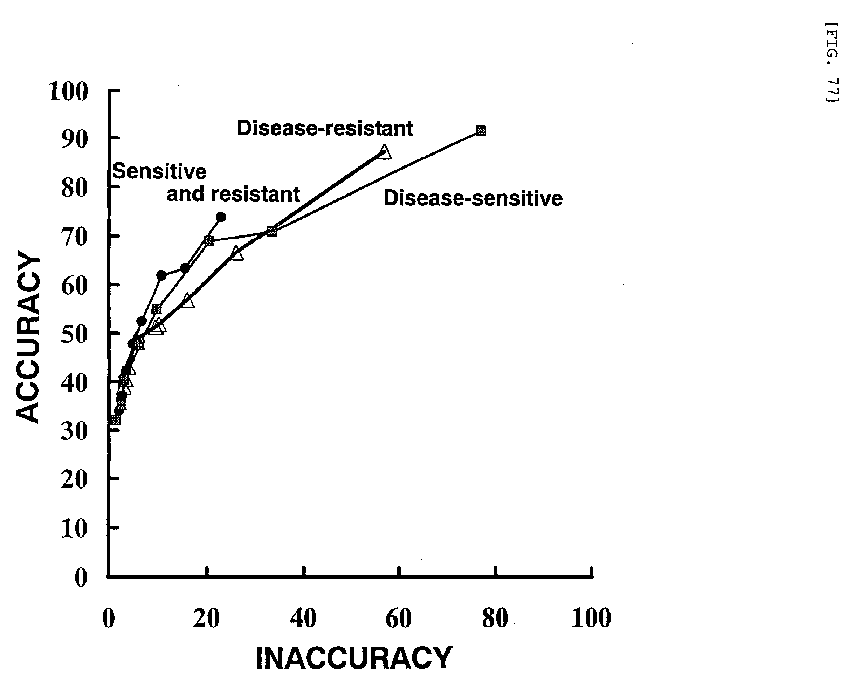

Fig. 77 and Table 3 show the analysis results.

[Table 3]

| | Sensitive SSNPs and resistant SSNPs | | Sensitive SSNPs | | Resistant SSNPs | |
|---|---|---|---|---|---|---|
| | Inaccuracy (%) | Accuracy(%) | Inaccuracy (%) | Accuracy (%) | Inaccuracy (%) | Accuracy (%) |
| Odds 2 | 22.8 | 73.9 | 76.9 | 91.8 | 56.8 | 87.2 |
| Odds 3 | 15.5 | 63.9 | 33.3 | 70.9 | 25.9 | 66.7 |
| Odds 4 | 10.6 | 61.9 | 20.5 | 68.9 | 15.8 | 56.9 |
| Odds 5 | 6.6 | 52.4 | 9.7 | 54.9 | 10.1 | 51.8 |
| Odds 6 | 4.9 | 47.8 | 6.2 | 47.6 | 9.4 | 51.3 |
| Odds 7 | 3.5 | 42.4 | 3.1 | 40.3 | 5.5 | 48.7 |
| Odds 8 | 2.8 | 37.2 | 2.6 | 35.2 | 3.9 | 43.1 |
| Odds 9 | 2.5 | 36.4 | 2.6 | 35.2 | 3.4 | 40.5 |
| Odds 10 | 2.1 | 34 | 1.5 | 32 | 3 | 39 |

In FIG. 77, □ represents the predicted sensitivity (accuracy), and false-positive (inaccuracy) calculated using only sensitive "explicative SSNPs"; Δ represents those calculated using only resistant "explicative SSNP"; and • represents those calculated using both sensitive "explicative SSNPs" and resistant "explicative SSNPs". Each plot represents a value calculated based on a set of polymorphisms with an Odds value of 2 to 10.

As shown in FIG. 77 and Table 3, the values calculated using both the sensitive polymorphisms (explicative SSNPs) and resistant polymorphisms (explicative SSNPs) exhibited a remarkably low inaccuracy. This clearly shows that assessment of arteriosclerosis by the combined use of sensitive "explicative SSNPs" and resistant "explicative SSNPs" enables highly accurate assessment.

INDUSTRIAL APPLICABILITY

The present invention provides a method of assessing disease risk, an apparatus for assessing disease risk, and a program for assessing disease risk, which enable accurate assessment of disease risk (i.e., the likelihood of onset, the probability of progression, etc.) and are available for the prevention and treatment of disease. According to a method of the invention, the likelihood of occurrence of arteriosclerosis or myocardial infarction in a diabetic or a patient prone to diabetes (a borderline diabetic) and the probability of its progression can be determined with high accuracy. The method of the invention can thus be suitably employed in preventing and treating such conditions.

In contrast to conventional assessment methods for disease risk that consider only sensitivity to a disease (positive association) as an index to determine the existence and/or the risk of the disease, the method of the present invention additionally uses resistance to the disease (negative association). Accordingly, it is possible with the invention to determine disease risk based not only on sensitivity but also on resistance, thereby achieving comprehensive risk assessment while improving precision and accuracy of the assessment results.

Further, by considering genetic polymorphisms, the present invention elicits a disease-resistance factor and a disease-sensitivity factor that are useful in assessing the risk of arteriosclerosis, especially diabetic arteriosclerosis, as well as a disease-resistance factor and a disease-sensitivity factor that are useful in assessing the risk of myocardial infarction, especially diabetic myocardial infarction. The invention defines these factors, respectively, as an arteriosclerosis-resistant polymorphism set and an arteriosclerosis-sensitive polymorphism set, and a myocardial infarction-resistant polymorphism set and a myocardial infarction-sensitive polymorphism set. It is accordingly possible to assess the risk of arteriosclerosis and myocardial infarction in, in particular, diabetics and patients prone to diabetes (borderline diabetics), and to provide preventive measures and therapeutic measures in a more suitable manner to each of the subjects depending on their characteristics. A method of assessing disease risk, an array for disease risk assessment, a disease-resistant genetic marker and a disease-sensitive genetic marker, and a kit for analyzing disease-resistant polymorphisms or disease-sensitive polymorphisms provided by the present invention are useful in assessing the risk of arteriosclerosis or myocardial infarction.

Such techniques of the invention are also applicable to various diseases, other than arteriosclerosis and myocardial infarction that are described by way of example in this specification. In particular, the invention can be applied to cerebral infarction due to diabetes, diabetic nephropathy, diabetic neuropathy, and the like.

## Claims

**1.** A method of determining genetic polymorphisms for assessing disease risk, comprising:

a first step of selecting, from preselected genetic polymorphisms, a predetermined number of genetic polymorphisms each having a specified genotype to thereby obtain a genetic polymorphism set;
a second step of using a group comprising elements, each element being a combination of a disease index value with genetic polymorphisms each having a genotype, to calculate an association between a disease index and the obtained genetic polymorphism set and a statistical significance of the association; and,
if the calculated association is negative and significant, a third step of employing the genetic polymorphism or genetic polymorphisms that form the genetic polymorphism set as a genetic polymorphism or genetic polymorphisms for assessing disease risk.

**2.** A method of determining genetic polymorphisms for assessing disease risk according to claim 1, further comprising:

if the calculated association is positive and significant, a fourth step of employing the genetic polymorphism or genetic polymorphisms that form the genetic polymorphism set as a genetic polymorphism or genetic polymorphisms for assessing disease risk.

3. A method of determining genetic polymorphisms for assessing disease risk according to claim 2, wherein:

the association is expressed as an odds ratio;
the significance is expressed as a chi-square value;
the target disease is arteriosclerosis; and
the disease index is carotid artery intima-media thickness.

4. A method of determining genetic polymorphisms for assessing disease risk according to claim 2, wherein:

the association is expressed as an odds ratio;
the significance is expressed as a chi-square value;
the target disease is myocardial infarction; and
the disease index is the presence of abnormal Q waves in an electrocardiogram indicating old myocardial infarction, or the presence of a past history of myocardial infarction.

5. A method of determining genetic polymorphisms for assessing disease risk according to claim 3, wherein:

the first through third steps are repeated each time a genetic polymorphism set that is not identical to any genetic polymorphism set that has been obtained in a previous first step is found;
the first step is followed by a fifth step of determining whether a genetic polymorphism set consisting of some but not all of the genetic polymorphisms of the genetic polymorphism set obtained in the first step has been employed in a previous third step as a genetic polymorphism set for assessing disease risk; and,
if it is assessed that such a genetic polymorphism set has been employed, the third step is not performed.

6. A method of determining genetic polymorphisms for assessing disease risk according to claim 4, wherein:

the first through fourth steps are repeated each time a genetic polymorphism set that is not identical to any genetic polymorphism set that has been obtained in a previous first step is found;
the first step is followed by a sixth step of determining whether a genetic polymorphism set consisting of some but not all the genetic polymorphisms of the genetic polymorphism set obtained has been employed in a previous third step as a genetic polymorphism set for assessing disease risk; and,
if it is assessed that such a genetic polymorphism set has been employed, the fourth step is not performed.

7. A method of determining genetic polymorphisms for assessing disease risk according to claim 3, comprising:

a seventh step of calculating a contributory value for each of the genetic polymorphisms employed in the third step, and selecting genetic polymorphisms for assessing disease risk based on the calculated contributory values.

8. A method of determining genetic polymorphisms for assessing disease risk to claim 4, comprising:

an eighth step of calculating a contributory value for each of the genetic polymorphisms employed in the fifth step, and selecting genetic polymorphisms for assessing disease risk based on the calculated contributory values.

9. A method of determining genetic polymorphisms for assessing disease risk according to claim 7, comprising:

a ninth step of selecting a genetic polymorphism having a genotype whose contributory value is the greatest.

10. A method of determining genetic polymorphisms for assessing disease risk according to claim 8, comprising:

a tenth step of selecting a genetic polymorphism having a genotype whose contributory value is the greatest.

11. A method of determining genetic polymorphisms for assessing disease risk according to claim 1, comprising, if a new disease index value and new genetic polymorphisms each having a genotype have been obtained:

searching the group to obtain a disease index value corresponding to the new genetic polymorphisms each having a genotype; and

determining genetic polymorphisms for assessing disease risk by using the corresponding disease index value and the new disease index value.

**12.** An array for assessing arteriosclerosis risk, comprising:

a probe or probes for detecting one or more genetic polymorphisms of at least one genetic polymorphism set selected from the negative (resistant) genetic polymorphism sets shown in any one of FIGS. 1 to 9; or
a probe or probes for detecting one or more genetic polymorphisms of at least one genetic polymorphism set selected from the negative (resistant) genetic polymorphism sets shown in any one of FIGS. 38 to 43.

**13.** An array for assessing arteriosclerosis risk according to claim 12, comprising:

a probe or probes for detecting at least half of the genetic polymorphisms shown in any one of FIGS. 10 to 18; or
a probe or probes for detecting at least half of the genetic polymorphisms shown in any one of FIGS. 44 to 47.

**14.** An array for assessing arteriosclerosis risk according to claim 12, further comprising:

a probe or probes for detecting one or more genetic polymorphisms of at least one genetic polymorphism set selected from the positive (sensitive) genetic polymorphism sets shown in any one of FIGS. 19 to 27; or
a probe or probes for detecting one or more genetic polymorphisms of at least one genetic polymorphism set selected from the positive (sensitive) genetic polymorphism sets shown in any one of FIGS. 63 to 69.

**15.** An array for assessing arteriosclerosis risk according to claim 12, comprising:

a probe or probes for detecting at least half of the genetic polymorphisms shown in any one of FIGS. 28 to 37; or
a probe or probes for detecting at least half of the genetic polymorphisms shown in any one of FIGS. 52 to 55.

**16.** An array for assessing myocardial infarction risk, comprising a probe or probes for detecting one or more genetic polymorphisms of at least one genetic polymorphism set selected from the negative (resistant) genetic polymorphism sets shown in any one of FIGS. 56 to 58.

**17.** An array for assessing myocardial infarction risk according to claim 16, comprising a probe or probes for detecting at least half of the genetic polymorphisms shown in any one of FIGS. 59 to 62.

**18.** An array for assessing myocardial infarction risk according to claim 16, further comprising a probe or probes for detecting one or more genetic polymorphisms of at least one genetic polymorphism set selected from of the positive (sensitive) genetic polymorphism sets shown in any one of FIGS. 63 to 69.

**19.** An array according to claim 16, further comprising a probe or probes for detecting at least half of the genetic polymorphisms shown in any one of FIGS. 70 to 73.

**20.** A method of assessing disease risk, comprising:

a step (b) of checking a genetic polymorphism detected in a specimen against one or more genetic polymorphisms negatively associated with a disease index, or against one or more genetic polymorphism sets negatively associated with a disease index.

**21.** A method of assessing disease risk according to claim 20, further comprising:

a step (b') of checking a genetic polymorphism detected in the specimen against one or more genetic polymorphisms positively associated with a disease index, or against one or more genetic polymorphism sets positively associated with a disease index; and
a step (c) of, based on the result of step (b'), comparing the negative association with the positive association of a set of detected genetic polymorphisms to thereby calculate the balance.

**22.** A method of assessing disease risk according to claim 21, further comprising:

a step (d) of evaluating disease risk from the calculated balance.

**23.** A method of assessing disease risk according to claim 20, wherein the target disease is arteriosclerosis, and said one or more genetic polymorphism sets negatively associated with a disease index are:

one or more sets selected from the negative genetic polymorphism sets shown in any one of FIGS. 1 to 9; or one or more sets selected from the negative genetic polymorphism sets shown in any one of FIGS. 38 to 43.

**24.** A method of assessing disease risk according to claim 21, wherein the target disease is arteriosclerosis, and

(1) said one or more genetic polymorphism sets negatively associated with a disease index are one or more sets selected from the negative genetic polymorphism sets shown in any one of FIGS. 1 to 9, and
said one or more genetic polymorphism sets positively associated with a disease index are one or more sets selected from the positive genetic polymorphism sets shown in any one of FIGS 19 to 27; or
(2) said one or more genetic polymorphism sets negatively associated with a disease index are one or more sets selected from the negative genetic polymorphism sets shown in any one of FIGS. 38 to 43, and
said one or more genetic polymorphism sets positively associated with a disease index are one or more sets selected from the positive genetic polymorphism sets shown in any one of FIGS. 48 to 51.

**25.** A method of assessing disease risk according to claim 20 or 21, comprising:

a step (a) of, prior to step (b) and/or (b'), detecting polymorphisms in a specimen.

**26.** A method of assessing disease risk according to claim 25, wherein step (a) is a step of detecting two or more genetic polymorphisms selected from the 99 genetic polymorphisms shown in FIG. 37.

**27.** A method of assessing disease risk according to claim 25, wherein the target disease is arteriosclerosis, and wherein step (a) is a step of detecting genetic polymorphisms by hybridizing, on the array of any one of claims 12 to 15 for assessing arteriosclerosis risk, a probe or probes prepared from a specimen with the probe or probes of said array for detecting one or more genetic polymorphisms.

**28.** A method of assessing disease risk according to claim 25, wherein the target disease is myocardial infarction, and step (a) is a step of detecting genetic polymorphisms by hybridizing, on the array of any one of claims 16 to 19 for assessing myocardial infarction risk, a probe or probes prepared from a specimen with the probe or probes of said array for detecting one or more genetic polymorphisms.

**29.** An apparatus for assessing disease risk, comprising:

a storage unit for recording a reference table in which first genetic polymorphism sets, each consisting of one or more genetic polymorphisms each having a genotype, are each related to a positive or negative association between the set and a disease index;
an interface for obtaining from a specimen genetic polymorphisms having genotypes; and
a processor for checking a second genetic polymorphism set comprising a predetermined number of genetic polymorphisms obtained from a specimen against said first genetic polymorphism sets in the reference table, wherein:

the processor, if the checking finds an agreement between the second genetic polymorphism set and any first genetic polymorphism set, determines the balance of the specimen toward positive or negative association based on the association of such a first genetic polymorphism set.

**30.** An apparatus for assessing disease risk according to claim 29, wherein:

the target disease is arteriosclerosis; and
the reference table is a table in which genetic polymorphism sets positively associated with carotid artery intima-media thickness are each matched with a corresponding odds ratio that indicates the frequency with which carotid artery intima-media thickness exceeds a normal range, the odds ratio being an index of sensitivity to arteriosclerosis.

**31.** An apparatus for assessing disease risk according to claim 29, wherein:

the target disease of arteriosclerosis; and
the reference table is a table in which genetic polymorphism sets positively associated with carotid artery intima-media thickness are each matched with a corresponding increase in carotid artery intima-media thickness, the increase being an index of sensitivity to arteriosclerosis.

**32.** An apparatus for assessing disease risk according to claim 29, wherein:

the target disease is arteriosclerosis; and
each said first genetic polymorphism set positively associated with carotid artery intima-media thickness is a genetic polymorphism set whose corresponding odds ratio that indicates the frequency with which carotid artery intima-media thickness exceeds a normal range is higher than a predetermined level, and/or whose corresponding average carotid artery intima-media thickness has a significant difference.

**33.** An apparatus for assessing disease risk according to claim 29, wherein:

the target disease is arteriosclerosis; and
the reference table is a table in which genetic polymorphism sets negatively associated with carotid artery intima-media thickness are each matched with a corresponding odds ratio that indicates the frequency with which carotid artery intima-media thickness does not exceed a normal range, the odds ratio being an index of resistance to arteriosclerosis.

**34.** An apparatus for assessing disease risk according to claim 29, wherein:

the target disease is arteriosclerosis; and
the reference table is a table in which genetic polymorphisms negatively associated with carotid artery intima-media thickness or polymorphism sets negatively associated with carotid artery intima-media thickness are each matched with a corresponding suppression of an increase in carotid artery intima-media thickness, the suppression of an increase being an index of resistance to arteriosclerosis.

**35.** An apparatus for assessing disease risk according to claim 29, further comprising a detector for detecting arteriosclerosis risk, wherein:

the target disease is arteriosclerosis;
the reference table is a table in which information indicating presence of environmental factors or numerical values thereof are each related to a corresponding sensitivity or resistance to arteriosclerosis; and
the detector detects arteriosclerosis risk by:

checking inputted information indicating the presence of one or more environmental factors of a subject or numerical value(s) thereof against information indicating the presence of environmental factors or numerical values thereof in the reference table, and
detecting arteriosclerosis risk based on the sensitivity or resistance to arteriosclerosis corresponding to said inputted information indicating presence of one or more environmental factors or numerical value(s) thereof.

**36.** An apparatus for assessing disease risk according to claim 29, further comprising a detector for detecting arteriosclerosis risk, wherein:

the target disease is arteriosclerosis;
the reference table is a table in which carotid artery intima-media thicknesses are each related to a corresponding sensitivity or resistance to arteriosclerosis; and
the detector detects arteriosclerosis risk by:

checking inputted caroid artery intima-media thickness of a subject against carotid artery intima-media thicknesses in the reference table, and
assessing arteriosclerosis risk based on the sensitivity or resistance to arteriosclerosis corresponding to the inputted carotid artery intima-media thickness.

**37.** An apparatus for assessing disease risk according to claim 29, further comprises a device for measuring vascular wall thickness, wherein:

the target disease is arteriosclerosis, and
the device for measuring vascular wall thickness measures the carotid artery intima-media thickness of a subject and provides the measurement result to the interface.

**38.** A program for assessing disease risk, the program enabling a computer to perform the functions of:

receiving an input concerning genetic polymorphisms having genotypes obtained from a specimen;
recording, in a storage unit, a reference table in which first genetic polymorphism sets, each consisting of one or more genetic polymorphisms each having a genotype, are each related to a positive or negative association between a set and a disease index;
checking a second genetic polymorphism set consisting of a predetermined number of the inputted genetic polymorphisms obtained from the specimen against said one or more first genetic polymorphism sets in the reference table; and
if the checking finds an agreement between the second genetic polymorphism set and any first genetic polymorphism set, determining the balance of the specimen toward positive or negative association based on the association of such a first genetic polymorphism set.

**39.** A program for assessing disease risk according to claim 38, wherein:

the target disease is arteriosclerosis; and
the reference table is a table in which genetic polymorphism sets positively associated with carotid artery intima-media thickness are each matched with a corresponding odds ratio that indicates frequency with which carotid artery intima-media thickness exceeds a normal range, the odds ratio being an index of sensitivity to polymorphism.

**40.** A program for assessing disease risk according to claim 29, wherein:

the target disease is arteriosclerosis; and
the reference table is a table in which genetic polymorphisms significantly positively associated with carotid artery intima-media thickness or one or more genetic polymorphism sets significantly positively associated with carotid artery intima-media thickness are each matched with a corresponding increase in carotid artery intima-media thickness, the thickness being an index of sensitivity to arteriosclerosis.

**41.** A program for assessing disease risk according to claim 38, wherein:

the target disease is arteriosclerosis; and
each said genetic polymorphism set positively associated with carotid artery intima-media thickness is a genetic polymorphism or genetic polymorphism set whose corresponding odds ratio that indicates the frequency with which carotid artery intima-media thickness exceeds a normal range is higher than a predetermined level, and/or whose corresponding average carotid artery intima-media thickness has a significant difference.

**42.** A program for assessing disease risk according to claim 38, wherein:

the target disease is arteriosclerosis; and
the reference table is a table in which genetic polymorphisms negatively associated with carotid artery intima-media thickness or genetic polymorphism sets negatively associated with carotid artery intima-media thickness are each matched with a corresponding odds ratio that indicates the frequency with which carotid artery intima-media thickness does not exceed a normal range, the odds ratio being an index of resistance to arteriosclerosis.

**43.** A program for assessing disease risk according to claim 38, wherein
the target disease is arteriosclerosis, and
the reference table is a table in which genetic polymorphisms negatively associated with carotid artery intima-media thickness or polymorphism sets negatively associated with carotid artery intima-media thickness are each matched with a corresponding suppression of an increase in carotid artery intima-media thickness, the suppression of an increase being an index of resistance to arteriosclerosis.

**44.** A program for assessing disease risk according to claim 38, wherein:

the target disease is arteriosclerosis; and
the degree of agreement between an arteriosclerosis case and a high-risk case of arteriosclerosis and also between a non-arteriosclerosis case and a low-risk case of arteriosclerosis is 30 % or more, and the degree of disagreement therebetween is 30% or less, provided that:

an arteriosclerosis case is defined as having a carotid artery intima-media thickness at least 0.2 mm greater than the normal average carotid artery intima-media thickness, and a non-arteriosclerosis case is defined otherwise; and
with respect to detected genetic polymorphisms, a low-risk case of arteriosclerosis is defined as the number of negative genetic polymorphisms or negative genetic polymorphism sets being greater than the number of positive genetic polymorphisms or positive genetic polymorphism sets, and a high-risk case of arteriosclerosis is defined as the number of positive genetic polymorphisms or positive genetic polymorphism sets being greater than the number of negative genetic polymorphisms or negative genetic polymorphism sets.

**45.** A computer-readable recording medium having stored thereon a program for assessing disease risk, the program enabling a computer to perform the functions of:

receiving an input concerning genetic polymorphisms each having a genotype obtained from a specimen;
recording, in a storage unit, a reference table in which first genetic polymorphism sets, each consisting of one or more genetic polymorphisms each having a genotype, are each related to a positive or negative association between a set and a disease index;
checking a second genetic polymorphism set consisting of a predetermined number of the inputted genetic polymorphisms obtained from the specimen against said one or more first genetic polymorphism sets in the reference table; and,
if the checking finds an agreement between the second genetic polymorphism set and any first genetic polymorphism set, determining the balance of the specimen toward positive or negative association based on the association of such a first genetic polymorphism set.

**46.** A genetic marker resistant to arteriosclerosis comprising:

one or more genetic polymorphisms of at least one genetic polymorphism set selected from the negative genetic polymorphism sets shown in any one of FIGS. 1 to 9; or
one or more genetic polymorphisms of at least one genetic polymorphism set selected from the negative genetic polymorphism sets shown in any one of FIGS. 38 to 43.

**47.** (1) A genetic marker resistant to arteriosclerosis comprising one or more genetic polymorphisms of at least one genetic polymorphism set selected from the negative genetic polymorphism sets shown in any one of FIGS. 1 to 9, and a genetic marker sensitive to arteriosclerosis comprising one or more genetic polymorphisms of at least one genetic polymorphism set selected from the positive genetic polymorphism sets shown in any one of FIGS. 19 to 27; or
(2) a genetic marker resistant to arteriosclerosis comprising one or more genetic polymorphisms of at least one genetic polymorphism set selected from the negative genetic polymorphism sets shown in any one of FIGS. 38 to 43; and
a genetic marker sensitive to arteriosclerosis comprising one or more genetic polymorphisms of at least one genetic polymorphism set selected from the positive genetic polymorphism sets shown in any one of FIGS. 48 to 55.

**48.** A genetic marker resistant to myocardial infarction comprising:

one or more genetic polymorphisms of at least one genetic polymorphism set selected from the negative genetic polymorphism sets shown in any one of FIGS. 56 to 58.

**49.** A genetic marker resistant to myocardial infarction comprising one or more genetic polymorphisms of at least one genetic polymorphism set selected from the negative genetic polymorphism sets shown in any one of FIGS. 56 to 58; and
a genetic marker sensitive to myocardial infarction comprising one or more genetic polymorphisms of at least one genetic polymorphism set selected from the positive genetic polymorphism sets shown in any one of FIGS. 63 to 69.

**50.** A kit for analyzing genetic polymorphisms resistant to arteriosclerosis, comprising:

(1) at least one primer pair capable of specifically amplifying a gene or genes that contain one or more polymorphisms of at least one genetic polymorphism set selected from the negative genetic polymorphism sets shown in any one of FIGS. 1 to 9, or
at least one nucleic acid probe capable of specifically hybridizing with said gene or genes; or
(2) at least one primer pair capable of specifically amplifying a gene or genes that contain one or more polymorphisms of at least one genetic polymorphism set selected from the negative genetic polymorphism sets shown in any one of FIGS. 38 to 43, or
at least one nucleic acid probe capable of specifically hybridizing with said gene or genes.

**51.** A kit for analyzing genetic polymorphisms resistant or sensitive to arteriosclerosis, comprising:

(1) at least one primer pair capable of specifically amplifying a gene or genes that contain one or more polymorphisms of at least one genetic polymorphism set selected from the negative genetic polymorphism sets shown in any one of FIGS. 1 to 9, or
at least one nucleic acid probe capable of specifically hybridizing with said gene or genes; and
at least one primer pair capable of specifically amplifying a gene or genes that contain one or more polymorphisms of at least one genetic polymorphism set selected the positive genetic polymorphism sets shown in any one of FIGS. 19 to 27, or
at least one nucleic acid probe capable of specifically hybridizing with said gene or genes; or
(2) at least one primer pair capable of specifically amplifying a gene or genes that contain one or more polymorphisms of at least one genetic polymorphism set selected from the negative genetic polymorphism sets shown in any one of FIGS. 38 to 43, or
at least one nucleic acid probe capable of specifically hybridizing with said gene or genes; and
at least one primer pair capable of specifically amplifying a gene or genes that contain one or more polymorphisms of at least one genetic polymorphism set selected from the positive genetic polymorphism sets shown in any one of FIGS. 48 to 51, or
at least one nucleic acid probe capable of specifically hybridizing with said gene or genes.

**52.** A kit for analyzing genetic polymorphisms resistant to myocardial infarction, comprising:

at least one primer pair capable of specifically amplifying a gene or genes that contain one or more polymorphisms of at least one genetic polymorphism set selected from the negative genetic polymorphism sets shown in any one of FIGS. 56 to 58;
or at least one nucleic acid probe capable of specifically hybridizing with said gene or genes.

**53.** A kit for analyzing genetic polymorphisms resistant to or sensitive to myocardial infarction, comprising:

at least one primer pair capable of specifically amplifying a gene or genes that contain one or more polymorphisms of at least one genetic polymorphism set selected from the negative genetic polymorphism sets shown in any one of FIGS. 56 to 58, or
at least one nucleic acid probe capable of specifically hybridizing with said gene or genes; and
at least one primer pair capable of specifically amplifying a gene or genes that contain one or more polymorphisms of at least one genetic polymorphism set selected from the positive genetic polymorphism sets shown in any one of FIGS. 63 to 69, or
at least one nucleic acid probe capable of specifically hybridizing with said gene or genes.

[FIG. 1-A]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| negative genetic polymorphism sets | 3 | 13 | 3 | CF12 | 68 | 3 | BKR1 | 90 | 12 | IL-182 | 17.58 | -12.79 |
| | 3 | 49 | 23 | GP61 | 55 | 3 | MPO | 75 | 12 | LTA2 | 14.41 | -8.27 |
| | 3 | 4 | 1 | ESRa1 | 23 | 3 | IRS-1 | 40 | 12 | FGB3 | 14.26 | -4.38 |
| | 3 | 4 | 1 | ESRa1 | 80 | 3 | CCR2 | 96 | 23 | LRP1 | 13.83 | -18 |
| | 2 | 10 | 12 | GP3A | 18 | 23 | S2AR | | | | 13.49 | -99 |
| | 3 | 33 | 1 | MCP1 | 35 | 1 | ESL2 | 38 | 3 | PONA1 | 13.35 | -3 |
| | 3 | 4 | 1 | ESRa1 | 75 | 12 | LTA2 | 86 | 12 | IL-181 | 13.31 | -2.99 |
| | 3 | 13 | 3 | CF12 | 55 | 3 | MPO | 99 | 12 | PGC12 | 13.03 | -2.23 |
| | 3 | 73 | 1 | VEGF5 | 74 | 12 | LTA1 | 93 | 23 | TPO1 | 13.03 | -2.45 |
| | 3 | 25 | 3 | PAI | 86 | 12 | IL-181 | 96 | 23 | LRP1 | 12.82 | -6.42 |
| | 3 | 13 | 3 | CF12 | 55 | 3 | MPO | 77 | 12 | APM12 | 12.72 | -2.04 |
| | 3 | 68 | 3 | BKR1 | 69 | 12 | CD181 | 78 | 3 | ET1 | 12.38 | -9.76 |
| | 3 | 33 | 1 | MCP1 | 35 | 1 | ESL2 | 54 | 1 | IL62 | 11.85 | -2.72 |
| | 3 | 33 | 1 | MCP1 | 50 | 1 | β2AR4 | 55 | 3 | MPO | 11.54 | -2.57 |
| | 3 | 13 | 3 | CF12 | 53 | 23 | β2AR-1 | 90 | 12 | IL-182 | 11.51 | -2.88 |
| | 3 | 30 | 3 | FGB3 | 38 | 3 | PONA1 | 54 | 1 | IL62 | 10.9 | -99 |
| | 3 | 25 | 3 | PAI | 38 | 3 | PONA1 | 75 | 12 | LTA2 | 10.8 | -4 |
| | 3 | 13 | 3 | CF12 | 48 | 1 | ICAM1 | 80 | 3 | CCR2 | 10.67 | -2.69 |
| | 2 | 68 | 3 | BKR1 | 75 | 12 | LTA2 | | | | 10.46 | -2.12 |
| | 3 | 13 | 3 | CF12 | 29 | 3 | G1A3 | 40 | 12 | FGB3 | 10.24 | -2.21 |
| | 2 | 73 | 1 | VEGF5 | 93 | 23 | TPO1 | | | | 10.1 | -2.12 |
| | 3 | 30 | 3 | FGB3 | 93 | 23 | TPO1 | 98 | 12 | PGC11 | 9.69 | -13.5 |
| | 3 | 68 | 3 | BKR1 | 77 | 12 | APM12 | 90 | 12 | IL-182 | 9.12 | -2.71 |
| | 3 | 73 | 1 | VEGF5 | 74 | 12 | LTA1 | 75 | 12 | LTA2 | 8.92 | -2.32 |
| | 2 | 68 | 3 | BKR1 | 90 | 12 | IL-182 | | | | 8.82 | -2.53 |
| | 3 | 68 | 3 | BKR1 | 69 | 12 | CD181 | 96 | 23 | LRP1 | 8.79 | -3.42 |
| | 3 | 25 | 3 | PAI | 29 | 3 | G1A3 | 74 | 12 | LTA1 | 8.67 | -2.18 |
| | 3 | 4 | 1 | ESRa1 | 5 | 3 | N1 | 40 | 12 | FGB3 | 10.92 | -3.81 |
| | 3 | 4 | 1 | ESRa1 | 5 | 3 | N1 | 90 | 12 | IL-182 | 12.59 | -7.54 |
| | 3 | 4 | 1 | ESRa1 | 8 | 3 | PONA2 | 40 | 12 | FGB3 | 11.38 | -3.55 |
| | 3 | 4 | 1 | ESRa1 | 11 | 1 | PAR2 | 40 | 12 | FGB3 | 12.72 | -3.92 |
| | 3 | 4 | 1 | ESRa1 | 12 | 3 | Tbm3 | 37 | 23 | GP1ba | 7.09 | -3.7 |
| | 3 | 4 | 1 | ESRa1 | 12 | 3 | Tbm3 | 40 | 12 | FGB3 | 9.37 | -3.53 |
| | 3 | 4 | 1 | ESRa1 | 18 | 23 | S2AR | 40 | 12 | FGB3 | 14.13 | -4.62 |
| | 3 | 4 | 1 | ESRa1 | 18 | 23 | S2AR | 90 | 12 | IL-182 | 10.67 | -6.77 |
| | 3 | 4 | 1 | ESRa1 | 20 | 12 | GSY | 40 | 12 | FGB3 | 12.72 | -3.92 |
| | 3 | 4 | 1 | ESRa1 | 26 | 23 | NOS3 | 55 | 3 | MPO | 6.8 | -10.23 |
| | 3 | 4 | 1 | ESRa1 | 26 | 23 | NOS3 | 83 | 1 | 2C9-3 | 7.47 | -11.05 |
| | 3 | 4 | 1 | ESRa1 | 26 | 23 | NOS3 | 96 | 23 | LRP1 | 8.69 | -99 |
| | 3 | 4 | 1 | ESRa1 | 29 | 3 | G1A3 | 40 | 12 | FGB3 | 12.72 | -3.92 |
| | 2 | 4 | 1 | ESRa1 | 30 | 3 | FGB3 | | | | 6.48 | -99 |
| | 3 | 4 | 1 | ESRa1 | 36 | 12 | FGA1 | 40 | 12 | FGB3 | 10.87 | -3.8 |
| | 3 | 4 | 1 | ESRa1 | 36 | 12 | FGA1 | 49 | 23 | GP61 | 8.63 | -99 |
| | 3 | 4 | 1 | ESRa1 | 38 | 3 | PONA1 | 40 | 12 | FGB3 | 6.92 | -3.98 |
| | 2 | 4 | 1 | ESRa1 | 40 | 12 | FGB3 | | | | 11.43 | -3.56 |
| | 3 | 4 | 1 | ESRa1 | 49 | 23 | GP61 | 75 | 12 | LTA2 | 10.68 | -99 |
| | 3 | 4 | 1 | ESRa1 | 70 | 3 | APM2 | 75 | 12 | LTA2 | 9.71 | -3.29 |
| | 3 | 4 | 1 | ESRa1 | 75 | 12 | LTA2 | 90 | 12 | IL-182 | 10.53 | -6.7 |
| | 2 | 10 | 12 | GP3A | 11 | 1 | PAR2 | | | | 9.82 | -13.65 |
| | 2 | 10 | 12 | GP3A | 37 | 23 | GP1ba | | | | 6.52 | -99 |
| | 3 | 10 | 12 | GP3A | 38 | 3 | PONA1 | 74 | 12 | LTA1 | 8.63 | -99 |
| | 3 | 10 | 12 | GP3A | 41 | 12 | MMP72 | 51 | 1 | GP1a1 | 7.71 | -11.34 |
| | 3 | 10 | 12 | GP3A | 41 | 12 | MMP72 | 67 | 3 | 1A2 | 7.3 | -10.85 |
| | 2 | 10 | 12 | GP3A | 51 | 1 | GP1a1 | | | | 7.71 | -11.34 |
| | 2 | 10 | 12 | GP3A | 67 | 3 | 1A2 | | | | 7.3 | -10.85 |
| | 3 | 13 | 3 | CF12 | 27 | 1 | N10 | 40 | 12 | FGB3 | 7.59 | -3.85 |
| | 3 | 13 | 3 | CF12 | 27 | 1 | N10 | 90 | 12 | IL-182 | 7.2 | -4.57 |
| | 3 | 13 | 3 | CF12 | 49 | 23 | GP61 | 53 | 23 | β2AR-1 | 7.55 | -11.15 |
| | 3 | 13 | 3 | CF12 | 49 | 23 | GP61 | 77 | 12 | APM12 | 7.81 | -11.47 |

[FIG. 1-B]

| | | | | | | | | | | | | |
|---|---|---|---|---|---|---|---|---|---|---|---|---|
| | 3 | 13 | 3 | CF12 | 55 | 3 | MPO | 68 | 3 | BKR1 | 11.86 | -3.48 |
| | 3 | 13 | 3 | CF12 | 68 | 3 | BKR1 | 75 | 12 | LTA2 | 8 | -2.77 |
| | 3 | 13 | 3 | CF12 | 68 | 3 | BKR1 | 84 | 23 | IL1B2 | 9 | -99 |
| | 2 | 17 | 1 | FR1 | 29 | 3 | G1A3 | | | | 4.39 | -99 |
| | 2 | 17 | 1 | FR1 | 35 | 1 | ESL2 | | | | 4.43 | -99 |
| | 2 | 17 | 1 | FR1 | 54 | 1 | IL62 | | | | 4.3 | -99 |
| | 2 | 17 | 1 | FR1 | 70 | 3 | APM2 | | | | 4.37 | -99 |
| | 3 | 25 | 3 | PAI | 29 | 3 | G1A3 | 30 | 3 | FGB3 | 8.93 | -99 |
| | 3 | 25 | 3 | PAI | 29 | 3 | G1A3 | 45 | 23 | LPL3 | 7.46 | -4.17 |
| | 3 | 25 | 3 | PAI | 29 | 3 | G1A3 | 96 | 23 | LRP1 | 10.83 | -5.11 |
| | 2 | 25 | 3 | PAI | 30 | 3 | FGB3 | | | | 8.95 | -99 |
| | 3 | 25 | 3 | PAI | 38 | 3 | PONA1 | 99 | 12 | PGC12 | 7.41 | -2.94 |
| | 3 | 25 | 3 | PAI | 41 | 12 | MMP72 | 45 | 23 | LPL3 | 7.46 | -4.17 |
| | 2 | 25 | 3 | PAI | 42 | 3 | GCLM1 | | | | 6.54 | -99 |
| | 3 | 25 | 3 | PAI | 44 | 3 | CETP4 | 45 | 23 | LPL3 | 7.43 | -4.16 |
| | 2 | 25 | 3 | PAI | 45 | 23 | LPL3 | | | | 7.46 | -4.17 |
| | 3 | 25 | 3 | PAI | 50 | 1 | β2AR4 | 74 | 12 | LTA1 | 8.55 | -2.23 |
| | 3 | 25 | 3 | PAI | 50 | 1 | β2AR4 | 96 | 23 | LRP1 | 12.76 | -6.4 |
| | 3 | 25 | 3 | PAI | 51 | 1 | GP1a1 | 96 | 23 | LRP1 | 7.69 | -11.33 |
| | 3 | 25 | 3 | PAI | 67 | 3 | 1A2 | 96 | 23 | LRP1 | 7.74 | -11.39 |
| | 3 | 25 | 3 | PAI | 73 | 1 | VEGF5 | 74 | 12 | LTA1 | 7.22 | -4.09 |
| | 3 | 25 | 3 | PAI | 73 | 1 | VEGF5 | 96 | 23 | LRP1 | 6.66 | -99 |
| | 3 | 30 | 3 | FGB3 | 36 | 12 | FGA1 | 85 | 12 | 5178 | 6.64 | -99 |
| | 3 | 30 | 3 | FGB3 | 38 | 3 | PONA1 | 50 | 1 | β2AR4 | 7.55 | -11.15 |
| | 3 | 30 | 3 | FGB3 | 38 | 3 | PONA1 | 75 | 12 | LTA2 | 7.38 | -10.94 |
| | 3 | 30 | 3 | FGB3 | 38 | 3 | PONA1 | 83 | 1 | 2C9-3 | 7.22 | -6.78 |
| | 3 | 30 | 3 | FGB3 | 38 | 3 | PONA1 | 86 | 12 | IL-181 | 7.44 | -11.01 |
| | 3 | 30 | 3 | FGB3 | 38 | 3 | PONA1 | 93 | 23 | TPO1 | 9.26 | -12.99 |
| | 3 | 30 | 3 | FGB3 | 38 | 3 | PONA1 | 99 | 12 | PGC12 | 7.56 | -11.17 |
| | 3 | 30 | 3 | FGB3 | 54 | 1 | IL62 | 98 | 12 | PGC11 | 7.15 | -6.73 |
| | 3 | 30 | 3 | FGB3 | 74 | 12 | LTA1 | 99 | 12 | PGC12 | 6.96 | -5.22 |
| | 3 | 30 | 3 | FGB3 | 83 | 1 | 2C9-3 | 85 | 12 | 5178 | 6.64 | -99 |
| | 3 | 30 | 3 | FGB3 | 83 | 1 | 2C9-3 | 98 | 12 | PGC11 | 7.03 | -5.25 |
| | 2 | 30 | 3 | FGB3 | 85 | 12 | 5178 | | | | 6.64 | -99 |
| | 3 | 30 | 3 | FGB3 | 98 | 12 | PGC11 | 99 | 12 | PGC12 | 7.56 | -11.17 |
| | 3 | 33 | 1 | MCP1 | 35 | 1 | ESL2 | 45 | 23 | LPL3 | 8.41 | -4.95 |
| | 2 | 33 | 1 | MCP1 | 38 | 3 | PONA1 | | | | 12.34 | -2.85 |
| | 3 | 33 | 1 | MCP1 | 44 | 3 | CETP4 | 45 | 23 | LPL3 | 6.81 | -3.95 |
| | 2 | 33 | 1 | MCP1 | 45 | 23 | LPL3 | | | | 6.81 | -3.95 |
| | 3 | 33 | 1 | MCP1 | 50 | 1 | β2AR4 | 73 | 1 | VEGF5 | 7.17 | -3.47 |
| | 3 | 33 | 1 | MCP1 | 52 | 23 | MMP71 | 53 | 23 | β2AR-1 | 6.7 | -6.42 |
| | 3 | 33 | 1 | MCP1 | 52 | 23 | MMP71 | 99 | 12 | PGC12 | 6.7 | -6.42 |
| | 3 | 33 | 1 | MCP1 | 53 | 23 | β2AR-1 | 68 | 3 | BKR1 | 9.46 | -4.68 |
| | 2 | 33 | 1 | MCP1 | 64 | 12 | TNFa2 | | | | 4.29 | -99 |
| | 3 | 33 | 1 | MCP1 | 73 | 1 | VEGF5 | 85 | 12 | 5178 | 6.85 | -4.43 |
| | 3 | 40 | 12 | FGB3 | 49 | 23 | GP61 | 75 | 12 | LTA2 | 9.48 | -13.25 |
| | 3 | 49 | 23 | GP61 | 68 | 3 | BKR1 | 75 | 12 | LTA2 | 8.68 | -99 |
| | 3 | 68 | 3 | BKR1 | 69 | 12 | CD181 | 75 | 12 | LTA2 | 10.44 | -2.17 |
| | 3 | 68 | 3 | BKR1 | 69 | 12 | CD181 | 90 | 12 | IL-182 | 7.62 | -2.44 |
| | 2 | 68 | 3 | BKR1 | 96 | 23 | LRP1 | | | | 7.43 | -3.03 |
| | 3 | 73 | 1 | VEGF5 | 75 | 12 | LTA2 | 93 | 23 | TPO1 | 8.71 | -2.38 |
| | 3 | 73 | 1 | VEGF5 | 76 | 23 | TS41 | 93 | 23 | TPO1 | 10.02 | -2.11 |
| | 3 | 73 | 1 | VEGF5 | 82 | 12 | IL103 | 93 | 23 | TPO1 | 9.23 | -2.06 |
| | 3 | 73 | 1 | VEGF5 | 83 | 1 | 2C9-3 | 93 | 23 | TPO1 | 9.6 | -2.13 |
| | 3 | 73 | 1 | VEGF5 | 89 | 12 | IL102 | 93 | 23 | TPO1 | 7.61 | -2.27 |

[FIG. 2-A]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| negative genetic polymorphism sets | 3 | 13 | 3 | CF12 | 68 | 3 | BKR1 | 90 | 12 | IL-182 | 17.58 | -12.79 |
| | 3 | 25 | 3 | PAI | 43 | 1 | MTP1 | 96 | 23 | LRP1 | 14.29 | -18.54 |
| | 3 | 4 | 1 | ESRa1 | 43 | 1 | MTP1 | 70 | 3 | APM2 | 14.15 | -4.36 |
| | 3 | 4 | 1 | ESRa1 | 18 | 23 | S2AR | 40 | 12 | FGB3 | 14.13 | -4.62 |
| | 2 | 10 | 12 | GP3A | 18 | 23 | S2AR | | | | 13.49 | -99 |
| | 3 | 25 | 3 | PAI | 50 | 1 | β2AR4 | 96 | 23 | LRP1 | 12.76 | -6.4 |
| | 3 | 4 | 1 | ESRa1 | 70 | 3 | APM2 | 77 | 12 | APM12 | 12.24 | -3.44 |
| | 3 | 13 | 3 | CF12 | 55 | 3 | MPO | 68 | 3 | BKR1 | 11.86 | -3.48 |
| | 3 | 13 | 3 | CF12 | 53 | 23 | β2AR-1 | 68 | 3 | BKR1 | 11.76 | -3.09 |
| | 3 | 13 | 3 | CF12 | 24 | 1 | AGTR1-3 | 31 | 12 | APE2 | 11.19 | -3.2 |
| | 3 | 30 | 3 | FGB3 | 38 | 3 | PONA1 | 54 | 1 | IL62 | 10.9 | -99 |
| | 3 | 33 | 1 | MCP1 | 36 | 1 | FGA1 | 96 | 23 | LRP1 | 10.9 | -8.93 |
| | 2 | 68 | 3 | BKR1 | 88 | 1 | VWF2 | | | | 10.62 | -3.44 |
| | 3 | 4 | 1 | ESRa1 | 11 | 1 | PAR2 | 27 | 1 | N10 | 10.6 | -4.57 |
| | 3 | 4 | 1 | ESRa1 | 12 | 3 | Tbm3 | 96 | 23 | LRP1 | 10.45 | -4.98 |
| | 3 | 33 | 1 | MCP1 | 36 | 1 | FGA1 | 38 | 3 | PONA1 | 10.14 | -4.88 |
| | 2 | 68 | 3 | BKR1 | 78 | 3 | ET1 | | | | 9.91 | -6.43 |
| | 3 | 33 | 1 | MCP1 | 53 | 23 | β2AR-1 | 68 | 3 | BKR1 | 9.46 | -4.68 |
| | 3 | 73 | 1 | VEGF5 | 89 | 12 | IL102 | 90 | 12 | IL-182 | 9.18 | -3.93 |
| | 3 | 30 | 3 | FGB3 | 38 | 3 | PONA1 | 69 | 1 | CD181 | 8.59 | -99 |
| | 3 | 33 | 1 | MCP1 | 35 | 1 | ESL2 | 45 | 23 | LPL3 | 8.41 | -4.95 |
| | 3 | 13 | 3 | CF12 | 64 | 12 | TNFa2 | 82 | 1 | IL103 | 7.82 | -11.47 |
| | 3 | 13 | 3 | CF12 | 49 | 23 | GP61 | 77 | 12 | APM12 | 7.81 | -11.47 |
| | 3 | 13 | 3 | CF12 | 27 | 1 | N10 | 40 | 12 | FGB3 | 7.59 | -3.85 |
| | 2 | 25 | 3 | PAI | 45 | 23 | LPL3 | | | | 7.46 | -4.17 |
| | 2 | 68 | 3 | BKR1 | 96 | 23 | LRP1 | | | | 7.43 | -3.03 |
| | 3 | 40 | 12 | FGB3 | 49 | 23 | GP61 | 77 | 12 | APM12 | 7.31 | -6.85 |
| | 3 | 25 | 3 | PAI | 73 | 1 | VEGF5 | 87 | 12 | MS1 | 7.27 | -4.11 |
| | 3 | 33 | 1 | MCP1 | 50 | 1 | β2AR4 | 73 | 1 | VEGF5 | 7.17 | -3.47 |
| | 3 | 30 | 3 | FGB3 | 54 | 1 | IL62 | 98 | 12 | PGC11 | 7.15 | -6.73 |
| | 3 | 30 | 3 | FGB3 | 83 | 1 | 2C9-3 | 98 | 12 | PGC11 | 7.03 | -5.25 |
| | 3 | 4 | 1 | ESRa1 | 67 | 3 | 1A2 | 69 | 1 | CD181 | 6.81 | -4.42 |
| | 3 | 33 | 1 | MCP1 | 52 | 23 | MMP71 | 53 | 23 | β2AR-1 | 6.7 | -6.42 |
| | 2 | 25 | 3 | PAI | 42 | 3 | GCLM1 | | | | 6.54 | -99 |
| | 2 | 17 | 1 | FR1 | 88 | 1 | VWF2 | | | | 4.47 | -99 |
| | 2 | 33 | 1 | MCP1 | 64 | 12 | TNFa2 | | | | 4.29 | -99 |
| | 3 | 4 | 1 | ESRa1 | 8 | 3 | PONA2 | 49 | 23 | GP61 | 7.43 | -11 |
| | 3 | 4 | 1 | ESRa1 | 12 | 3 | Tbm3 | 49 | 23 | GP61 | 7.43 | -11 |
| | 3 | 4 | 1 | ESRa1 | 18 | 23 | S2AR | 90 | 12 | IL-182 | 10.67 | -6.77 |
| | 3 | 4 | 1 | ESRa1 | 26 | 23 | NOS3 | 83 | 1 | 2C9-3 | 7.47 | -11.05 |
| | 3 | 4 | 1 | ESRa1 | 26 | 23 | NOS3 | 96 | 23 | LRP1 | 8.69 | -99 |
| | 2 | 4 | 1 | ESRa1 | 30 | 3 | FGB3 | | | | 6.48 | -99 |
| | 3 | 4 | 1 | ESRa1 | 38 | 3 | PONA1 | 96 | 23 | LRP1 | 8.92 | -7.77 |
| | 3 | 4 | 1 | ESRa1 | 49 | 23 | GP61 | 50 | 1 | β2AR4 | 7.37 | -10.92 |
| | 3 | 4 | 1 | ESRa1 | 49 | 23 | GP61 | 83 | 1 | 2C9-3 | 7.49 | -11.07 |
| | 3 | 4 | 1 | ESRa1 | 51 | 12 | GP1a1 | 90 | 12 | IL-182 | 10.56 | -5.67 |
| | 3 | 4 | 1 | ESRa1 | 67 | 3 | 1A2 | 90 | 12 | IL-182 | 7 | -6.63 |

[FIG. 2-B]

| | | | | | | | | | | | |
|---|---|---|---|---|---|---|---|---|---|---|---|
| 3 | 4 | 1 | ESRa1 | 67 | 3 | 1A2 | 96 | 23 | LRP1 | 7.46 | -11.04 |
| 3 | 4 | 1 | ESRa1 | 96 | 23 | LRP1 | 99 | 3 | PGC12 | 8.55 | -99 |
| 2 | 10 | 12 | GP3A | 37 | 23 | GP1ba | | | | 6.52 | -99 |
| 3 | 10 | 12 | GP3A | 38 | 3 | PONA1 | 51 | 12 | GP1a1 | 8.91 | -99 |
| 3 | 10 | 12 | GP3A | 38 | 3 | PONA1 | 75 | 23 | LTA2 | 8.6 | -99 |
| 3 | 13 | 3 | CF12 | 49 | 23 | GP61 | 53 | 23 | β2AR-1 | 7.55 | -11.15 |
| 3 | 13 | 3 | CF12 | 68 | 3 | BKR1 | 78 | 3 | ET1 | 7.61 | -11.23 |
| 3 | 13 | 3 | CF12 | 68 | 3 | BKR1 | 84 | 23 | IL1B2 | 9 | -99 |
| 3 | 13 | 3 | CF12 | 68 | 3 | BKR1 | 88 | 1 | VWF2 | 9.23 | -7.98 |
| 2 | 17 | 1 | FR1 | 22 | 1 | ESL1 | | | | 4.44 | -99 |
| 2 | 17 | 1 | FR1 | 29 | 3 | G1A3 | | | | 4.39 | -99 |
| 2 | 17 | 1 | FR1 | 35 | 1 | ESL2 | | | | 4.43 | -99 |
| 2 | 17 | 1 | FR1 | 54 | 1 | IL62 | | | | 4.3 | -99 |
| 2 | 17 | 1 | FR1 | 70 | 3 | APM2 | | | | 4.37 | -99 |
| 3 | 25 | 3 | PAI | 29 | 3 | G1A3 | 30 | 3 | FGB3 | 8.93 | -99 |
| 3 | 25 | 3 | PAI | 29 | 3 | G1A3 | 45 | 23 | LPL3 | 7.46 | -4.17 |
| 3 | 25 | 3 | PAI | 29 | 3 | G1A3 | 96 | 23 | LRP1 | 10.83 | -5.11 |
| 2 | 25 | 3 | PAI | 30 | 3 | FGB3 | | | | 8.95 | -99 |
| 3 | 25 | 3 | PAI | 39 | 23 | CRP1 | 45 | 23 | LPL3 | 7.43 | -4.16 |
| 3 | 25 | 3 | PAI | 41 | 12 | MMP72 | 45 | 23 | LPL3 | 7.46 | -4.17 |
| 3 | 25 | 3 | PAI | 43 | 1 | MTP1 | 45 | 23 | LPL3 | 7.32 | -5.4 |
| 3 | 25 | 3 | PAI | 44 | 3 | CETP4 | 45 | 23 | LPL3 | 7.43 | -4.16 |
| 3 | 25 | 3 | PAI | 67 | 3 | 1A2 | 96 | 23 | LRP1 | 7.74 | -11.39 |
| 3 | 25 | 3 | PAI | 73 | 1 | VEGF5 | 89 | 12 | IL102 | 6.86 | -5.17 |
| 3 | 25 | 3 | PAI | 73 | 1 | VEGF5 | 96 | 23 | LRP1 | 6.66 | -99 |
| 3 | 30 | 3 | FGB3 | 38 | 3 | PONA1 | 50 | 1 | β2AR4 | 7.55 | -11.15 |
| 3 | 30 | 3 | FGB3 | 44 | 3 | CETP4 | 88 | 1 | VWF2 | 6.72 | -99 |
| 3 | 30 | 3 | FGB3 | 51 | 12 | GP1a1 | 54 | 1 | IL62 | 7.11 | -6.71 |
| 3 | 30 | 3 | FGB3 | 51 | 12 | GP1a1 | 85 | 12 | 5178 | 6.67 | -99 |
| 3 | 30 | 3 | FGB3 | 75 | 23 | LTA2 | 88 | 1 | VWF2 | 6.7 | -99 |
| 3 | 30 | 3 | FGB3 | 82 | 1 | IL103 | 85 | 12 | 5178 | 6.64 | -99 |
| 3 | 30 | 3 | FGB3 | 83 | 1 | 2C9-3 | 85 | 12 | 5178 | 6.64 | -99 |
| 2 | 30 | 3 | FGB3 | 85 | 12 | 5178 | | | | 6.64 | -99 |
| 3 | 30 | 3 | FGB3 | 97 | 12 | MT1310 | 98 | 12 | PGC11 | 7 | -5.24 |
| 3 | 33 | 1 | MCP1 | 44 | 3 | CETP4 | 45 | 23 | LPL3 | 6.81 | -3.95 |
| 2 | 33 | 1 | MCP1 | 45 | 23 | LPL3 | | | | 6.81 | -3.95 |
| 3 | 68 | 3 | BKR1 | 77 | 12 | APM12 | 78 | 3 | ET1 | 8.12 | -5.68 |
| 3 | 68 | 3 | BKR1 | 77 | 12 | APM12 | 88 | 1 | VWF2 | 8.96 | -3.3 |

[FIG. 3-A]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| negative genetic polymorphism sets | 3 | 13 | 3 | CF12 | 68 | 3 | BKR1 | 90 | 12 | IL-182 | 17.58 | -12.79 |
| | 3 | 25 | 3 | PAI | 43 | 1 | MTP1 | 96 | 23 | LRP1 | 14.29 | -18.54 |
| | 3 | 4 | 1 | ESRa1 | 23 | 3 | IRS-1 | 40 | 12 | FGB3 | 14.26 | -4.38 |
| | 3 | 4 | 1 | ESRa1 | 43 | 1 | MTP1 | 70 | 3 | APM2 | 14.15 | -4.36 |
| | 3 | 33 | 1 | MCP1 | 51 | 12 | GP1a1 | 88 | 3 | VWF2 | 14.1 | -5.96 |
| | 3 | 33 | 1 | MCP1 | 35 | 1 | ESL2 | 88 | 3 | VWF2 | 14.04 | -5.36 |
| | 2 | 10 | 12 | GP3A | 18 | 23 | S2AR | | | | 13.49 | -99 |
| | 3 | 25 | 3 | PAI | 50 | 1 | β2AR4 | 96 | 23 | LRP1 | 12.76 | -6.4 |
| | 3 | 68 | 3 | BKR1 | 69 | 12 | CD181 | 78 | 3 | ET1 | 12.38 | -9.76 |
| | 3 | 73 | 1 | VEGF5 | 88 | 3 | VWF2 | 90 | 12 | IL-182 | 11.24 | -9.14 |
| | 3 | 30 | 3 | FGB3 | 38 | 3 | PONA1 | 54 | 1 | IL62 | 10.9 | -99 |
| | 3 | 33 | 1 | MCP1 | 36 | 1 | FGA1 | 96 | 23 | LRP1 | 10.9 | -8.93 |
| | 3 | 4 | 1 | ESRa1 | 11 | 1 | PAR2 | 27 | 1 | N10 | 10.6 | -4.57 |
| | 3 | 4 | 1 | ESRa1 | 12 | 3 | Tbm3 | 96 | 23 | LRP1 | 10.45 | -4.98 |
| | 3 | 33 | 1 | MCP1 | 36 | 1 | FGA1 | 38 | 3 | PONA1 | 10.14 | -4.88 |
| | 3 | 33 | 1 | MCP1 | 53 | 23 | β2AR-1 | 68 | 3 | BKR1 | 9.46 | -4.68 |
| | 3 | 13 | 3 | CF12 | 68 | 3 | BKR1 | 84 | 23 | IL1B2 | 9 | -99 |
| | 3 | 25 | 3 | PAI | 73 | 1 | VEGF5 | 88 | 3 | VWF2 | 8.99 | -99 |
| | 3 | 33 | 1 | MCP1 | 35 | 1 | ESL2 | 45 | 23 | LPL3 | 8.41 | -4.95 |
| | 3 | 73 | 1 | VEGF5 | 77 | 1 | APM12 | 88 | 3 | VWF2 | 7.63 | -4.23 |
| | 3 | 13 | 3 | CF12 | 64 | 12 | TNFa2 | 75 | 3 | LTA2 | 7.56 | -11.16 |
| | 3 | 13 | 3 | CF12 | 49 | 23 | GP61 | 53 | 23 | β2AR-1 | 7.55 | -11.15 |
| | 2 | 25 | 3 | PAI | 45 | 23 | LPL3 | | | | 7.46 | -4.17 |
| | 3 | 25 | 3 | PAI | 73 | 1 | VEGF5 | 87 | 12 | MS1 | 7.27 | -4.11 |
| | 3 | 13 | 3 | CF12 | 27 | 1 | N10 | 90 | 12 | IL-182 | 7.2 | -4.57 |
| | 3 | 33 | 1 | MCP1 | 52 | 23 | MMP71 | 62 | 1 | APE3 | 6.92 | -6.58 |
| | 3 | 4 | 1 | ESRa1 | 67 | 3 | 1A2 | 79 | 3 | RAGE3 | 6.82 | -4.42 |
| | 3 | 25 | 3 | PAI | 38 | 3 | PONA1 | 76 | 12 | TS41 | 6.67 | -99 |
| | 2 | 25 | 3 | PAI | 42 | 3 | GCLM1 | | | | 6.54 | -99 |
| | 2 | 17 | 1 | FR1 | 22 | 1 | ESL1 | | | | 4.44 | -99 |
| | 2 | 31 | 1 | APE2 | 88 | 3 | VWF2 | | | | 4.41 | -99 |
| | 3 | 4 | 1 | ESRa1 | 8 | 3 | PONA2 | 26 | 23 | NOS3 | 7.7 | -11.34 |
| | 3 | 4 | 1 | ESRa1 | 18 | 23 | S2AR | 90 | 12 | IL-182 | 10.67 | -6.77 |
| | 3 | 4 | 1 | ESRa1 | 26 | 23 | NOS3 | 83 | 1 | 2C9-3 | 7.47 | -11.05 |
| | 3 | 4 | 1 | ESRa1 | 26 | 23 | NOS3 | 96 | 23 | LRP1 | 8.69 | -99 |
| | 2 | 4 | 1 | ESRa1 | 30 | 3 | FGB3 | | | | 6.48 | -99 |
| | 3 | 4 | 1 | ESRa1 | 38 | 3 | PONA1 | 96 | 23 | LRP1 | 8.92 | -7.77 |
| | 3 | 4 | 1 | ESRa1 | 51 | 12 | GP1a1 | 90 | 12 | IL-182 | 10.56 | -5.67 |
| | 3 | 4 | 1 | ESRa1 | 67 | 3 | 1A2 | 90 | 12 | IL-182 | 7 | -6.63 |
| | 3 | 4 | 1 | ESRa1 | 67 | 3 | 1A2 | 96 | 23 | LRP1 | 7.46 | -11.04 |
| | 3 | 4 | 1 | ESRa1 | 96 | 23 | LRP1 | 99 | 3 | PGC12 | 8.55 | -99 |
| | 2 | 10 | 12 | GP3A | 37 | 23 | GP1ba | | | | 6.52 | -99 |
| | 3 | 10 | 12 | GP3A | 38 | 3 | PONA1 | 51 | 12 | GP1a1 | 8.91 | -99 |

[FIG. 3-B]

| | | | | | | | | | | | |
|---|---|---|---|---|---|---|---|---|---|---|---|
| 2 | 17 | 1 | FR1 | 29 | 3 | G1A3 | | | | 4.39 | -99 |
| 2 | 17 | 1 | FR1 | 35 | 1 | ESL2 | | | | 4.43 | -99 |
| 2 | 17 | 1 | FR1 | 54 | 1 | IL62 | | | | 4.3 | -99 |
| 2 | 17 | 1 | FR1 | 70 | 3 | APM2 | | | | 4.37 | -99 |
| 2 | 17 | 1 | FR1 | 79 | 3 | RAGE3 | | | | 4.32 | -99 |
| 3 | 25 | 3 | PAI | 29 | 3 | G1A3 | 30 | 3 | FGB3 | 8.93 | -99 |
| 3 | 25 | 3 | PAI | 29 | 3 | G1A3 | 45 | 23 | LPL3 | 7.46 | -4.17 |
| 3 | 25 | 3 | PAI | 29 | 3 | G1A3 | 96 | 23 | LRP1 | 10.83 | -5.11 |
| 2 | 25 | 3 | PAI | 30 | 3 | FGB3 | | | | 8.95 | -99 |
| 3 | 25 | 3 | PAI | 41 | 12 | MMP72 | 45 | 23 | LPL3 | 7.46 | -4.17 |
| 3 | 25 | 3 | PAI | 43 | 1 | MTP1 | 45 | 23 | LPL3 | 7.32 | -5.4 |
| 3 | 25 | 3 | PAI | 44 | 3 | CETP4 | 45 | 23 | LPL3 | 7.43 | -4.16 |
| 2 | 25 | 3 | PAI | 56 | 12 | HANP1 | | | | 4.35 | -99 |
| 3 | 25 | 3 | PAI | 67 | 3 | 1A2 | 96 | 23 | LRP1 | 7.74 | -11.39 |
| 3 | 25 | 3 | PAI | 73 | 1 | VEGF5 | 96 | 23 | LRP1 | 6.66 | -99 |
| 3 | 30 | 3 | FGB3 | 38 | 3 | PONA1 | 50 | 1 | β2AR4 | 7.55 | -11.15 |
| 3 | 30 | 3 | FGB3 | 38 | 3 | PONA1 | 83 | 1 | 2C9-3 | 7.22 | -6.78 |
| 3 | 30 | 3 | FGB3 | 97 | 12 | MT1310 | 98 | 12 | PGC11 | 7 | -5.24 |
| 2 | 31 | 1 | APE2 | 48 | 23 | ICAM1 | | | | 4.22 | -99 |
| 2 | 31 | 1 | APE2 | 53 | 23 | β2AR-1 | | | | 4.32 | -99 |
| 2 | 31 | 1 | APE2 | 89 | 12 | IL102 | | | | 4.24 | -99 |
| 2 | 31 | 1 | APE2 | 96 | 23 | LRP1 | | | | 4.37 | -99 |
| 2 | 31 | 1 | APE2 | 99 | 3 | PGC12 | | | | 4.33 | -99 |
| 3 | 33 | 1 | MCP1 | 44 | 3 | CETP4 | 88 | 3 | VWF2 | 12.26 | -4.57 |
| 3 | 33 | 1 | MCP1 | 47 | 23 | TNFα1 | 88 | 3 | VWF2 | 12.32 | -4.59 |
| 3 | 33 | 1 | MCP1 | 50 | 1 | β2AR4 | 88 | 3 | VWF2 | 12.19 | -5.45 |
| 3 | 33 | 1 | MCP1 | 52 | 23 | MMP71 | 53 | 23 | β2AR-1 | 6.7 | -6.42 |
| 3 | 33 | 1 | MCP1 | 53 | 23 | β2AR-1 | 88 | 3 | VWF2 | 12.1 | -5.42 |
| 2 | 33 | 1 | MCP1 | 64 | 12 | TNFa2 | | | | 4.29 | -99 |
| 3 | 33 | 1 | MCP1 | 83 | 1 | 2C9-3 | 88 | 3 | VWF2 | 12.31 | -4.59 |
| 2 | 33 | 1 | MCP1 | 88 | 3 | VWF2 | | | | 12.31 | -4.59 |

[FIG. 4-A]

negative genetic polymorphism sets

| Num_SNP | SNP-No | Genotype | Gene code | SNP-No | Genotype | Gene code | SNP-No | Genotype | Gene code | Kai | Odds |
|---|---|---|---|---|---|---|---|---|---|---|---|
| 3 | 13 | 3 | CF12 | 68 | 3 | BKR1 | 90 | 12 | IL-182 | 17.58 | -12.79 |
| 3 | 25 | 3 | PAI | 43 | 1 | MTP1 | 96 | 23 | LRP1 | 14.29 | -18.54 |
| 3 | 33 | 1 | MCP1 | 77 | 12 | APM12 | 88 | 3 | VWF2 | 14.27 | -6.01 |
| 3 | 33 | 1 | MCP1 | 51 | 12 | GP1a1 | 88 | 3 | VWF2 | 14.1 | -5.96 |
| 2 | 10 | 12 | GP3A | 18 | 23 | S2AR | | | | 13.49 | -99 |
| 3 | 25 | 3 | PAI | 50 | 1 | β2AR4 | 96 | 23 | LRP1 | 12.76 | -6.4 |
| 3 | 4 | 1 | ESRa1 | 5 | 3 | N1 | 90 | 12 | IL-182 | 12.59 | -7.54 |
| 3 | 68 | 3 | BKR1 | 69 | 12 | CD181 | 78 | 3 | ET1 | 12.38 | -9.76 |
| 3 | 73 | 1 | VEGF5 | 88 | 3 | VWF2 | 90 | 12 | IL-182 | 11.24 | -9.14 |
| 3 | 30 | 3 | FGB3 | 38 | 3 | PONA1 | 54 | 1 | IL62 | 10.9 | -99 |
| 3 | 33 | 1 | MCP1 | 36 | 1 | FGA1 | 96 | 23 | LRP1 | 10.9 | -8.93 |
| 3 | 30 | 3 | FGB3 | 54 | 1 | IL62 | 86 | 1 | IL-181 | 10.84 | -99 |
| 3 | 4 | 1 | ESRa1 | 55 | 3 | MPO | 68 | 3 | BKR1 | 10 | -6.47 |
| 3 | 4 | 1 | ESRa1 | 43 | 1 | MTP1 | 68 | 3 | BKR1 | 9.84 | -6.39 |
| 3 | 30 | 3 | FGB3 | 38 | 3 | PONA1 | 93 | 23 | TPO1 | 9.26 | -12.99 |
| 3 | 13 | 3 | CF12 | 68 | 3 | BKR1 | 84 | 23 | IL1B2 | 9 | -99 |
| 3 | 25 | 3 | PAI | 73 | 1 | VEGF5 | 88 | 3 | VWF2 | 8.99 | -99 |
| 3 | 4 | 1 | ESRa1 | 38 | 3 | PONA1 | 96 | 23 | LRP1 | 8.92 | -7.77 |
| 3 | 4 | 1 | ESRa1 | 26 | 23 | NOS3 | 96 | 23 | LRP1 | 8.69 | -99 |
| 3 | 13 | 3 | CF12 | 64 | 12 | TNFa2 | 82 | 1 | IL103 | 7.82 | -11.47 |
| 3 | 13 | 3 | CF12 | 49 | 23 | GP61 | 77 | 12 | APM12 | 7.81 | -11.47 |
| 3 | 25 | 3 | PAI | 71 | 3 | REG1 | 76 | 12 | TS41 | 7.76 | -11.41 |
| 3 | 4 | 1 | ESRa1 | 8 | 3 | PONA2 | 26 | 23 | NOS3 | 7.7 | -11.34 |
| 3 | 40 | 12 | FGB3 | 53 | 23 | β2AR-1 | 70 | 1 | APM2 | 7.67 | -11.31 |
| 3 | 4 | 1 | ESRa1 | 49 | 23 | GP61 | 83 | 1 | 2C9-3 | 7.49 | -11.07 |
| 3 | 40 | 12 | FGB3 | 49 | 23 | GP61 | 77 | 12 | APM12 | 7.31 | -6.85 |
| 3 | 33 | 1 | MCP1 | 52 | 23 | MMP71 | 62 | 1 | APE3 | 6.92 | -6.58 |
| 3 | 33 | 1 | MCP1 | 48 | 23 | ICAM1 | 96 | 23 | LRP1 | 6.88 | -6.55 |
| 3 | 4 | 1 | ESRa1 | 43 | 1 | MTP1 | 73 | 1 | VEGF5 | 6.87 | -5.17 |
| 3 | 33 | 1 | MCP1 | 73 | 1 | VEGF5 | 86 | 1 | IL-181 | 6.7 | -6.42 |
| 2 | 25 | 3 | PAI | 42 | 3 | GCLM1 | | | | 6.54 | -99 |
| 2 | 17 | 1 | FR1 | 22 | 1 | ESL1 | | | | 4.44 | -99 |
| 2 | 31 | 1 | APE2 | 77 | 12 | APM12 | | | | 4.42 | -99 |
| 3 | 4 | 1 | ESRa1 | 8 | 3 | PONA2 | 49 | 23 | GP61 | 7.43 | -11 |
| 3 | 4 | 1 | ESRa1 | 12 | 3 | Tbm3 | 49 | 23 | GP61 | 7.43 | -11 |
| 3 | 4 | 1 | ESRa1 | 18 | 23 | S2AR | 90 | 12 | IL-182 | 10.67 | -6.77 |
| 3 | 4 | 1 | ESRa1 | 20 | 12 | GSY | 49 | 23 | GP61 | 7.43 | -11 |
| 3 | 4 | 1 | ESRa1 | 26 | 23 | NOS3 | 55 | 3 | MPO | 6.8 | -10.23 |
| 3 | 4 | 1 | ESRa1 | 26 | 23 | NOS3 | 83 | 1 | 2C9-3 | 7.47 | -11.05 |
| 3 | 4 | 1 | ESRa1 | 26 | 23 | NOS3 | 86 | 1 | IL-181 | 7.24 | -10.76 |
| 2 | 4 | 1 | ESRa1 | 30 | 3 | FGB3 | | | | 6.48 | -99 |
| 3 | 4 | 1 | ESRa1 | 38 | 3 | PONA1 | 68 | 3 | BKR1 | 8.3 | -7.36 |
| 3 | 4 | 1 | ESRa1 | 49 | 23 | GP61 | 50 | 1 | β2AR4 | 7.37 | -10.92 |
| 3 | 4 | 1 | ESRa1 | 49 | 23 | GP61 | 55 | 3 | MPO | 6.85 | -10.29 |
| 3 | 4 | 1 | ESRa1 | 49 | 23 | GP61 | 69 | 12 | CD181 | 7.35 | -10.9 |

[FIG. 4-B]

| | | | | | | | | | | | | |
|---|---|---|---|---|---|---|---|---|---|---|---|---|
| | 3 | 4 | 1 | ESRa1 | 49 | 23 | GP61 | 71 | 3 | REG1 | 7.07 | -10.56 |
| | 3 | 4 | 1 | ESRa1 | 49 | 23 | GP61 | 93 | 23 | TPO1 | 7.12 | -10.62 |
| | 2 | 10 | 12 | GP3A | 21 | 1 | CX37 | | | | 8.92 | -99 |
| | 2 | 10 | 12 | GP3A | 37 | 23 | GP1ba | | | | 6.52 | -99 |
| | 3 | 10 | 12 | GP3A | 38 | 3 | PONA1 | 51 | 12 | GP1a1 | 8.91 | -99 |
| | 2 | 17 | 1 | FR1 | 29 | 3 | G1A3 | | | | 4.39 | -99 |
| | 2 | 17 | 1 | FR1 | 35 | 1 | ESL2 | | | | 4.43 | -99 |
| | 2 | 17 | 1 | FR1 | 54 | 1 | IL62 | | | | 4.3 | -99 |
| | 2 | 17 | 1 | FR1 | 79 | 3 | RAGE3 | | | | 4.32 | -99 |
| | 3 | 25 | 3 | PAI | 29 | 3 | G1A3 | 30 | 3 | FGB3 | 8.93 | -99 |
| | 3 | 25 | 3 | PAI | 29 | 3 | G1A3 | 96 | 23 | LRP1 | 10.83 | -5.11 |
| | 2 | 25 | 3 | PAI | 30 | 3 | FGB3 | | | | 8.95 | -99 |
| | 3 | 25 | 3 | PAI | 38 | 3 | PONA1 | 76 | 12 | TS41 | 6.67 | -99 |
| | 2 | 25 | 3 | PAI | 56 | 12 | HANP1 | | | | 4.35 | -99 |
| | 3 | 25 | 3 | PAI | 73 | 1 | VEGF5 | 96 | 23 | LRP1 | 6.66 | -99 |
| | 3 | 25 | 3 | PAI | 76 | 12 | TS41 | 85 | 12 | 5178 | 6.7 | -99 |
| | 3 | 30 | 3 | FGB3 | 51 | 12 | GP1a1 | 85 | 12 | 5178 | 6.67 | -99 |
| | 3 | 30 | 3 | FGB3 | 82 | 1 | IL103 | 85 | 12 | 5178 | 6.64 | -99 |
| | 3 | 30 | 3 | FGB3 | 82 | 1 | IL103 | 86 | 1 | IL-181 | 7.44 | -11.01 |
| | 3 | 30 | 3 | FGB3 | 83 | 1 | 2C9-3 | 85 | 12 | 5178 | 6.64 | -99 |
| | 2 | 30 | 3 | FGB3 | 85 | 12 | 5178 | | | | 6.64 | -99 |
| | 2 | 31 | 1 | APE2 | 48 | 23 | ICAM1 | | | | 4.22 | -99 |
| | 2 | 31 | 1 | APE2 | 53 | 23 | β2AR-1 | | | | 4.32 | -99 |
| | 2 | 31 | 1 | APE2 | 87 | 1 | MS1 | | | | 4.33 | -99 |
| | 2 | 31 | 1 | APE2 | 88 | 3 | VWF2 | | | | 4.41 | -99 |
| | 2 | 31 | 1 | APE2 | 96 | 23 | LRP1 | | | | 4.37 | -99 |
| | 3 | 33 | 1 | MCP1 | 35 | 1 | ESL2 | 88 | 3 | VWF2 | 14.04 | -5.36 |
| | 3 | 33 | 1 | MCP1 | 50 | 1 | β2AR4 | 88 | 3 | VWF2 | 12.19 | -5.45 |
| | 3 | 33 | 1 | MCP1 | 52 | 23 | MMP71 | 53 | 23 | β2AR-1 | 6.7 | -6.42 |
| | 3 | 33 | 1 | MCP1 | 53 | 23 | β2AR-1 | 88 | 3 | VWF2 | 12.1 | -5.42 |
| | 2 | 33 | 1 | MCP1 | 64 | 12 | TNFa2 | | | | 4.29 | -99 |

[FIG. 5-A]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| negative genetic polymorphism sets | 3 | 13 | 3 | CF12 | 68 | 3 | BKR1 | 90 | 12 | IL-182 | 17.58 | -12.79 |
| | 3 | 25 | 3 | PAI | 43 | 1 | MTP1 | 96 | 23 | LRP1 | 14.29 | -18.54 |
| | 3 | 33 | 1 | MCP1 | 77 | 12 | APM12 | 88 | 3 | VWF2 | 14.27 | -6.01 |
| | 2 | 10 | 12 | GP3A | 18 | 23 | S2AR | | | | 13.49 | -99 |
| | 3 | 25 | 3 | PAI | 50 | 1 | β2AR4 | 96 | 23 | LRP1 | 12.76 | -6.4 |
| | 3 | 4 | 1 | ESRa1 | 5 | 3 | N1 | 90 | 12 | IL-182 | 12.59 | -7.54 |
| | 3 | 68 | 3 | BKR1 | 69 | 12 | CD181 | 78 | 3 | ET1 | 12.38 | -9.76 |
| | 3 | 73 | 1 | VEGF5 | 88 | 3 | VWF2 | 90 | 12 | IL-182 | 11.24 | -9.14 |
| | 3 | 30 | 3 | FGB3 | 38 | 3 | PONA1 | 54 | 1 | IL62 | 10.9 | -99 |
| | 3 | 33 | 1 | MCP1 | 36 | 1 | FGA1 | 96 | 23 | LRP1 | 10.9 | -8.93 |
| | 3 | 30 | 3 | FGB3 | 54 | 1 | IL62 | 86 | 1 | IL-181 | 10.84 | -99 |
| | 3 | 4 | 1 | ESRa1 | 55 | 3 | MPO | 68 | 3 | BKR1 | 10 | -6.47 |
| | 3 | 4 | 1 | ESRa1 | 43 | 1 | MTP1 | 68 | 3 | BKR1 | 9.84 | -6.39 |
| | 3 | 13 | 3 | CF12 | 68 | 3 | BKR1 | 84 | 23 | IL1B2 | 9 | -99 |
| | 3 | 25 | 3 | PAI | 73 | 1 | VEGF5 | 88 | 3 | VWF2 | 8.99 | -99 |
| | 3 | 4 | 1 | ESRa1 | 38 | 3 | PONA1 | 96 | 23 | LRP1 | 8.92 | -7.77 |
| | 3 | 4 | 1 | ESRa1 | 26 | 23 | NOS3 | 96 | 23 | LRP1 | 8.69 | -99 |
| | 3 | 13 | 3 | CF12 | 64 | 12 | TNFa2 | 82 | 1 | IL103 | 7.82 | -11.47 |
| | 3 | 13 | 3 | CF12 | 49 | 23 | GP61 | 77 | 12 | APM12 | 7.81 | -11.47 |
| | 3 | 25 | 3 | PAI | 71 | 3 | REG1 | 76 | 12 | TS41 | 7.76 | -11.41 |
| | 3 | 4 | 1 | ESRa1 | 8 | 3 | PONA2 | 26 | 23 | NOS3 | 7.7 | -11.34 |
| | 3 | 40 | 12 | FGB3 | 53 | 23 | β2AR-1 | 70 | 1 | APM2 | 7.67 | -11.31 |
| | 3 | 4 | 1 | ESRa1 | 49 | 23 | GP61 | 83 | 1 | 2C9-3 | 7.49 | -11.07 |
| | 3 | 40 | 12 | FGB3 | 49 | 23 | GP61 | 77 | 12 | APM12 | 7.31 | -6.85 |
| | 3 | 13 | 3 | CF12 | 84 | 23 | IL1B2 | 93 | 12 | TPO1 | 7.01 | -6.64 |
| | 3 | 33 | 1 | MCP1 | 52 | 23 | MMP71 | 62 | 1 | APE3 | 6.92 | -6.58 |
| | 3 | 33 | 1 | MCP1 | 48 | 23 | ICAM1 | 96 | 23 | LRP1 | 6.88 | -6.55 |
| | 3 | 33 | 1 | MCP1 | 73 | 1 | VEGF5 | 86 | 1 | IL-181 | 6.7 | -6.42 |
| | 2 | 25 | 3 | PAI | 42 | 3 | GCLM1 | | | | 6.54 | -99 |
| | 2 | 17 | 1 | FR1 | 22 | 1 | ESL1 | | | | 4.44 | -99 |
| | 2 | 31 | 1 | APE2 | 77 | 12 | APM12 | | | | 4.42 | -99 |
| | 3 | 4 | 1 | ESRa1 | 8 | 3 | PONA2 | 49 | 23 | GP61 | 7.43 | -11 |
| | 3 | 4 | 1 | ESRa1 | 12 | 3 | Tbm3 | 49 | 23 | GP61 | 7.43 | -11 |
| | 3 | 4 | 1 | ESRa1 | 18 | 23 | S2AR | 90 | 12 | IL-182 | 10.67 | -6.77 |
| | 3 | 4 | 1 | ESRa1 | 20 | 12 | GSY | 49 | 23 | GP61 | 7.43 | -11 |
| | 3 | 4 | 1 | ESRa1 | 26 | 23 | NOS3 | 55 | 3 | MPO | 6.8 | -10.23 |
| | 3 | 4 | 1 | ESRa1 | 26 | 23 | NOS3 | 83 | 1 | 2C9-3 | 7.47 | -11.05 |
| | 3 | 4 | 1 | ESRa1 | 26 | 23 | NOS3 | 86 | 1 | IL-181 | 7.24 | -10.76 |
| | 2 | 4 | 1 | ESRa1 | 30 | 3 | FGB3 | | | | 6.48 | -99 |
| | 3 | 4 | 1 | ESRa1 | 38 | 3 | PONA1 | 68 | 3 | BKR1 | 8.3 | -7.36 |
| | 3 | 4 | 1 | ESRa1 | 49 | 23 | GP61 | 50 | 1 | β2AR4 | 7.37 | -10.92 |
| | 3 | 4 | 1 | ESRa1 | 49 | 23 | GP61 | 55 | 3 | MPO | 6.85 | -10.29 |
| | 3 | 4 | 1 | ESRa1 | 49 | 23 | GP61 | 69 | 12 | CD181 | 7.35 | -10.9 |
| | 3 | 4 | 1 | ESRa1 | 49 | 23 | GP61 | 71 | 3 | REG1 | 7.07 | -10.56 |
| | 2 | 10 | 12 | GP3A | 21 | 1 | CX37 | | | | 8.92 | -99 |

[FIG. 5-B]

| | | | | | | | | | | | |
|---|---|---|---|---|---|---|---|---|---|---|---|
| 2 | 10 | 12 | GP3A | 37 | 23 | GP1ba | | | | 6.52 | -99 |
| 2 | 17 | 1 | FR1 | 29 | 3 | G1A3 | | | | 4.39 | -99 |
| 2 | 17 | 1 | FR1 | 35 | 1 | ESL2 | | | | 4.43 | -99 |
| 2 | 17 | 1 | FR1 | 54 | 1 | IL62 | | | | 4.3 | -99 |
| 2 | 17 | 1 | FR1 | 79 | 3 | RAGE3 | | | | 4.32 | -99 |
| 3 | 25 | 3 | PAI | 29 | 3 | G1A3 | 30 | 3 | FGB3 | 8.93 | -99 |
| 2 | 25 | 3 | PAI | 30 | 3 | FGB3 | | | | 8.95 | -99 |
| 3 | 25 | 3 | PAI | 38 | 3 | PONA1 | 76 | 12 | TS41 | 6.67 | -99 |
| 2 | 25 | 3 | PAI | 56 | 12 | HANP1 | | | | 4.35 | -99 |
| 3 | 25 | 3 | PAI | 67 | 3 | 1A2 | 96 | 23 | LRP1 | 7.74 | -11.39 |
| 3 | 25 | 3 | PAI | 73 | 1 | VEGF5 | 96 | 23 | LRP1 | 6.66 | -99 |
| 3 | 25 | 3 | PAI | 76 | 12 | TS41 | 93 | 12 | TPO1 | 7.21 | -10.74 |
| 3 | 30 | 3 | FGB3 | 38 | 3 | PONA1 | 99 | 12 | PGC12 | 7.56 | -11.17 |
| 3 | 30 | 3 | FGB3 | 82 | 1 | IL103 | 86 | 1 | IL-181 | 7.44 | -11.01 |
| 2 | 31 | 1 | APE2 | 48 | 23 | ICAM1 | | | | 4.22 | -99 |
| 2 | 31 | 1 | APE2 | 53 | 23 | β2AR-1 | | | | 4.32 | -99 |
| 2 | 31 | 1 | APE2 | 87 | 1 | MS1 | | | | 4.33 | -99 |
| 2 | 31 | 1 | APE2 | 88 | 3 | VWF2 | | | | 4.41 | -99 |
| 2 | 31 | 1 | APE2 | 96 | 23 | LRP1 | | | | 4.37 | -99 |
| 3 | 33 | 1 | MCP1 | 52 | 23 | MMP71 | 53 | 23 | β2AR-1 | 6.7 | -6.42 |
| 2 | 33 | 1 | MCP1 | 64 | 12 | TNFa2 | | | | 4.29 | -99 |

[FIG. 6]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| negative genetic polymorphism sets | 3 | 13 | 3 | CF12 | 68 | 3 | BKR1 | 90 | 12 | IL-182 | 17.58 | -12.79 |
| | 3 | 49 | 23 | GP61 | 62 | 12 | APE3 | 75 | 12 | LTA2 | 14.36 | -8.24 |
| | 3 | 25 | 3 | PAI | 43 | 1 | MTP1 | 96 | 23 | LRP1 | 14.29 | -18.54 |
| | 3 | 4 | 1 | ESRa1 | 80 | 3 | CCR2 | 96 | 23 | LRP1 | 13.83 | -18 |
| | 2 | 10 | 12 | GP3A | 18 | 23 | S2AR | | | | 13.49 | -99 |
| | 3 | 33 | 1 | MCP1 | 80 | 3 | CCR2 | 88 | 3 | VWF2 | 12.85 | -10.05 |
| | 3 | 4 | 1 | ESRa1 | 5 | 3 | N1 | 90 | 12 | IL-182 | 12.59 | -7.54 |
| | 3 | 25 | 3 | PAI | 29 | 3 | G1A3 | 55 | 12 | MPO | 11.73 | -7.16 |
| | 3 | 73 | 1 | VEGF5 | 88 | 3 | VWF2 | 90 | 12 | IL-182 | 11.24 | -9.14 |
| | 3 | 30 | 3 | FGB3 | 38 | 3 | PONA1 | 54 | 1 | IL62 | 10.9 | -99 |
| | 3 | 33 | 1 | MCP1 | 36 | 1 | FGA1 | 96 | 23 | LRP1 | 10.9 | -8.93 |
| | 3 | 70 | 1 | APM2 | 86 | 3 | IL-181 | 87 | 1 | MS1 | 9.57 | -13.35 |
| | 3 | 13 | 3 | CF12 | 68 | 3 | BKR1 | 84 | 23 | IL1B2 | 9 | -99 |
| | 3 | 25 | 3 | PAI | 73 | 1 | VEGF5 | 88 | 3 | VWF2 | 8.99 | -99 |
| | 3 | 40 | 12 | FGB3 | 64 | 12 | TNFa2 | 85 | 3 | 5178 | 8.91 | -99 |
| | 3 | 40 | 12 | FGB3 | 70 | 1 | APM2 | 93 | 1 | TPO1 | 8.55 | -99 |
| | 3 | 4 | 1 | ESRa1 | 38 | 3 | PONA1 | 68 | 3 | BKR1 | 8.3 | -7.36 |
| | 3 | 13 | 3 | CF12 | 64 | 12 | TNFa2 | 82 | 1 | IL103 | 7.82 | -11.47 |
| | 3 | 25 | 3 | PAI | 71 | 3 | REG1 | 76 | 12 | TS41 | 7.76 | -11.41 |
| | 3 | 4 | 1 | ESRa1 | 8 | 3 | PONA2 | 26 | 23 | NOS3 | 7.7 | -11.34 |
| | 3 | 13 | 3 | CF12 | 68 | 3 | BKR1 | 78 | 3 | ET1 | 7.61 | -11.23 |
| | 2 | 17 | 1 | FR1 | 27 | 12 | N10 | | | | 4.45 | -99 |
| | 2 | 31 | 1 | APE2 | 88 | 3 | VWF2 | | | | 4.41 | -99 |
| | 2 | 33 | 1 | MCP1 | 64 | 12 | TNFa2 | | | | 4.29 | -99 |
| | 3 | 4 | 1 | ESRa1 | 26 | 23 | NOS3 | 96 | 23 | LRP1 | 8.69 | -99 |
| | 2 | 4 | 1 | ESRa1 | 30 | 3 | FGB3 | | | | 6.48 | -99 |
| | 3 | 4 | 1 | ESRa1 | 49 | 23 | GP61 | 75 | 12 | LTA2 | 10.68 | -99 |
| | 3 | 4 | 1 | ESRa1 | 96 | 23 | LRP1 | 99 | 3 | PGC12 | 8.55 | -99 |
| | 2 | 10 | 12 | GP3A | 21 | 1 | CX37 | | | | 8.92 | -99 |
| | 2 | 10 | 12 | GP3A | 37 | 23 | GP1ba | | | | 6.52 | -99 |
| | 2 | 17 | 1 | FR1 | 22 | 1 | ESL1 | | | | 4.44 | -99 |
| | 2 | 17 | 1 | FR1 | 29 | 3 | G1A3 | | | | 4.39 | -99 |
| | 2 | 17 | 1 | FR1 | 32 | 12 | MTHFR | | | | 4.34 | -99 |
| | 2 | 17 | 1 | FR1 | 35 | 1 | ESL2 | | | | 4.43 | -99 |
| | 2 | 17 | 1 | FR1 | 48 | 12 | ICAM1 | | | | 4.31 | -99 |
| | 2 | 17 | 1 | FR1 | 54 | 1 | IL62 | | | | 4.3 | -99 |
| | 2 | 17 | 1 | FR1 | 79 | 3 | RAGE3 | | | | 4.32 | -99 |
| | 3 | 25 | 3 | PAI | 27 | 12 | N10 | 30 | 3 | FGB3 | 8.93 | -99 |
| | 3 | 25 | 3 | PAI | 29 | 3 | G1A3 | 30 | 3 | FGB3 | 8.93 | -99 |
| | 2 | 25 | 3 | PAI | 30 | 3 | FGB3 | | | | 8.95 | -99 |
| | 3 | 25 | 3 | PAI | 38 | 3 | PONA1 | 76 | 12 | TS41 | 6.67 | -99 |
| | 2 | 25 | 3 | PAI | 42 | 3 | GCLM1 | | | | 6.54 | -99 |
| | 2 | 25 | 3 | PAI | 56 | 12 | HANP1 | | | | 4.35 | -99 |
| | 3 | 25 | 3 | PAI | 73 | 1 | VEGF5 | 96 | 23 | LRP1 | 6.66 | -99 |
| | 2 | 31 | 1 | APE2 | 86 | 3 | IL-181 | | | | 4.26 | -99 |
| | 2 | 31 | 1 | APE2 | 87 | 1 | MS1 | | | | 4.33 | -99 |
| | 2 | 31 | 1 | APE2 | 89 | 12 | IL102 | | | | 4.24 | -99 |
| | 2 | 31 | 1 | APE2 | 96 | 23 | LRP1 | | | | 4.37 | -99 |
| | 2 | 31 | 1 | APE2 | 99 | 3 | PGC12 | | | | 4.33 | -99 |
| | 3 | 40 | 12 | FGB3 | 49 | 23 | GP61 | 75 | 12 | LTA2 | 9.48 | -13.25 |
| | 3 | 49 | 23 | GP61 | 68 | 3 | BKR1 | 75 | 12 | LTA2 | 8.68 | -99 |

[FIG. 7]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| negative genetic polymorphism sets | 3 | 13 | 3 | CF12 | 68 | 3 | BKR1 | 90 | 12 | IL-182 | 17.58 | -12.79 |
| | 3 | 25 | 3 | PAI | 43 | 1 | MTP1 | 96 | 23 | LRP1 | 14.29 | -18.54 |
| | 3 | 4 | 1 | ESRa1 | 80 | 3 | CCR2 | 96 | 23 | LRP1 | 13.83 | -18 |
| | 2 | 10 | 12 | GP3A | 18 | 23 | S2AR | | | | 13.49 | -99 |
| | 3 | 33 | 1 | MCP1 | 80 | 3 | CCR2 | 88 | 3 | VWF2 | 12.85 | -10.05 |
| | 3 | 73 | 1 | VEGF5 | 88 | 3 | VWF2 | 90 | 12 | IL-182 | 11.24 | -9.14 |
| | 3 | 30 | 3 | FGB3 | 38 | 3 | PONA1 | 54 | 1 | IL62 | 10.9 | -99 |
| | 3 | 33 | 1 | MCP1 | 36 | 1 | FGA1 | 96 | 23 | LRP1 | 10.9 | -8.93 |
| | 3 | 70 | 1 | APM2 | 86 | 3 | IL-181 | 87 | 1 | MS1 | 9.57 | -13.35 |
| | 3 | 30 | 3 | FGB3 | 32 | 12 | MTHFR | 38 | 3 | PONA1 | 9.51 | -13.27 |
| | 3 | 4 | 1 | ESRa1 | 38 | 3 | PONA1 | 90 | 12 | IL-182 | 9.48 | -13.24 |
| | 3 | 13 | 3 | CF12 | 68 | 3 | BKR1 | 84 | 23 | IL1B2 | 9 | -99 |
| | 3 | 25 | 3 | PAI | 73 | 1 | VEGF5 | 88 | 3 | VWF2 | 8.99 | -99 |
| | 3 | 40 | 12 | FGB3 | 64 | 12 | TNFa2 | 85 | 3 | 5178 | 8.91 | -99 |
| | 3 | 13 | 3 | CF12 | 64 | 12 | TNFa2 | 82 | 1 | IL103 | 7.82 | -11.47 |
| | 3 | 4 | 1 | ESRa1 | 8 | 3 | PONA2 | 26 | 23 | NOS3 | 7.7 | -11.34 |
| | 3 | 13 | 3 | CF12 | 68 | 3 | BKR1 | 78 | 3 | ET1 | 7.61 | -11.23 |
| | 3 | 30 | 3 | FGB3 | 98 | 12 | PGC11 | 99 | 12 | PGC12 | 7.56 | -11.17 |
| | 3 | 49 | 23 | GP61 | 69 | 1 | CD181 | 80 | 3 | CCR2 | 7.55 | -11.14 |
| | 2 | 25 | 3 | PAI | 42 | 3 | GCLM1 | | | | 6.54 | -99 |
| | 2 | 17 | 1 | FR1 | 27 | 12 | N10 | | | | 4.45 | -99 |
| | 2 | 31 | 1 | APE2 | 88 | 3 | VWF2 | | | | 4.41 | -99 |
| | 2 | 33 | 1 | MCP1 | 64 | 12 | TNFa2 | | | | 4.29 | -99 |
| | 3 | 4 | 1 | ESRa1 | 26 | 23 | NOS3 | 96 | 23 | LRP1 | 8.69 | -99 |
| | 2 | 4 | 1 | ESRa1 | 30 | 3 | FGB3 | | | | 6.48 | -99 |
| | 2 | 10 | 12 | GP3A | 21 | 1 | CX37 | | | | 8.92 | -99 |
| | 2 | 10 | 12 | GP3A | 37 | 23 | GP1ba | | | | 6.52 | -99 |
| | 2 | 17 | 1 | FR1 | 22 | 1 | ESL1 | | | | 4.44 | -99 |
| | 2 | 17 | 1 | FR1 | 29 | 3 | G1A3 | | | | 4.39 | -99 |
| | 2 | 17 | 1 | FR1 | 32 | 12 | MTHFR | | | | 4.34 | -99 |
| | 2 | 17 | 1 | FR1 | 35 | 1 | ESL2 | | | | 4.43 | -99 |
| | 2 | 17 | 1 | FR1 | 48 | 12 | ICAM1 | | | | 4.31 | -99 |
| | 2 | 17 | 1 | FR1 | 54 | 1 | IL62 | | | | 4.3 | -99 |
| | 2 | 17 | 1 | FR1 | 79 | 3 | RAGE3 | | | | 4.32 | -99 |
| | 3 | 25 | 3 | PAI | 27 | 12 | N10 | 30 | 3 | FGB3 | 8.93 | -99 |
| | 3 | 25 | 3 | PAI | 29 | 3 | G1A3 | 30 | 3 | FGB3 | 8.93 | -99 |
| | 2 | 25 | 3 | PAI | 30 | 3 | FGB3 | | | | 8.95 | -99 |
| | 2 | 25 | 3 | PAI | 56 | 12 | HANP1 | | | | 4.35 | -99 |
| | 3 | 25 | 3 | PAI | 73 | 1 | VEGF5 | 96 | 23 | LRP1 | 6.66 | -99 |
| | 3 | 30 | 3 | FGB3 | 38 | 3 | PONA1 | 69 | 1 | CD181 | 8.59 | -99 |
| | 2 | 31 | 1 | APE2 | 86 | 3 | IL-181 | | | | 4.26 | -99 |
| | 2 | 31 | 1 | APE2 | 87 | 1 | MS1 | | | | 4.33 | -99 |
| | 2 | 31 | 1 | APE2 | 89 | 12 | IL102 | | | | 4.24 | -99 |
| | 2 | 31 | 1 | APE2 | 96 | 23 | LRP1 | | | | 4.37 | -99 |

[FIG. 8]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| negative genetic polymorphism sets | 3 | 13 | 3 | CF12 | 68 | 3 | BKR1 | 90 | 12 | IL-182 | 17.58 | -12.79 |
| | 3 | 25 | 3 | PAI | 43 | 1 | MTP1 | 96 | 23 | LRP1 | 14.29 | -18.54 |
| | 3 | 4 | 1 | ESRa1 | 80 | 3 | CCR2 | 96 | 23 | LRP1 | 13.83 | -18 |
| | 2 | 10 | 12 | GP3A | 18 | 23 | S2AR | | | | 13.49 | -99 |
| | 3 | 33 | 1 | MCP1 | 80 | 3 | CCR2 | 88 | 3 | VWF2 | 12.85 | -10.05 |
| | 3 | 73 | 1 | VEGF5 | 88 | 3 | VWF2 | 90 | 12 | IL-182 | 11.24 | -9.14 |
| | 3 | 30 | 3 | FGB3 | 38 | 3 | PONA1 | 54 | 1 | IL62 | 10.9 | -99 |
| | 3 | 70 | 1 | APM2 | 86 | 3 | IL-181 | 87 | 1 | MS1 | 9.57 | -13.35 |
| | 3 | 30 | 3 | FGB3 | 32 | 12 | MTHFR | 38 | 3 | PONA1 | 9.51 | -13.27 |
| | 3 | 4 | 1 | ESRa1 | 38 | 3 | PONA1 | 90 | 12 | IL-182 | 9.48 | -13.24 |
| | 3 | 13 | 3 | CF12 | 68 | 3 | BKR1 | 84 | 23 | IL1B2 | 9 | -99 |
| | 3 | 25 | 3 | PAI | 73 | 1 | VEGF5 | 88 | 3 | VWF2 | 8.99 | -99 |
| | 3 | 40 | 12 | FGB3 | 64 | 12 | TNFa2 | 85 | 3 | 5178 | 8.91 | -99 |
| | 3 | 4 | 1 | ESRa1 | 36 | 12 | FGA1 | 49 | 23 | GP61 | 8.63 | -99 |
| | 3 | 13 | 3 | CF12 | 64 | 12 | TNFa2 | 82 | 1 | IL103 | 7.82 | -11.47 |
| | 3 | 4 | 1 | ESRa1 | 8 | 3 | PONA2 | 26 | 23 | NOS3 | 7.7 | -11.34 |
| | 3 | 13 | 3 | CF12 | 68 | 3 | BKR1 | 78 | 3 | ET1 | 7.61 | -11.23 |
| | 3 | 30 | 3 | FGB3 | 98 | 12 | PGC11 | 99 | 12 | PGC12 | 7.56 | -11.17 |
| | 3 | 49 | 23 | GP61 | 69 | 1 | CD181 | 80 | 3 | CCR2 | 7.55 | -11.14 |
| | 2 | 25 | 3 | PAI | 42 | 3 | GCLM1 | | | | 6.54 | -99 |
| | 2 | 17 | 1 | FR1 | 27 | 12 | N10 | | | | 4.45 | -99 |
| | 2 | 31 | 1 | APE2 | 88 | 3 | VWF2 | | | | 4.41 | -99 |
| | 2 | 33 | 1 | MCP1 | 64 | 12 | TNFa2 | | | | 4.29 | -99 |
| | 3 | 4 | 1 | ESRa1 | 26 | 23 | NOS3 | 96 | 23 | LRP1 | 8.69 | -99 |
| | 2 | 4 | 1 | ESRa1 | 30 | 3 | FGB3 | | | | 6.48 | -99 |
| | 2 | 10 | 12 | GP3A | 21 | 1 | CX37 | | | | 8.92 | -99 |
| | 2 | 10 | 12 | GP3A | 37 | 23 | GP1ba | | | | 6.52 | -99 |
| | 2 | 17 | 1 | FR1 | 22 | 1 | ESL1 | | | | 4.44 | -99 |
| | 2 | 17 | 1 | FR1 | 29 | 3 | G1A3 | | | | 4.39 | -99 |
| | 2 | 17 | 1 | FR1 | 32 | 12 | MTHFR | | | | 4.34 | -99 |
| | 2 | 17 | 1 | FR1 | 35 | 1 | ESL2 | | | | 4.43 | -99 |
| | 2 | 17 | 1 | FR1 | 48 | 12 | ICAM1 | | | | 4.31 | -99 |
| | 2 | 17 | 1 | FR1 | 54 | 1 | IL62 | | | | 4.3 | -99 |
| | 2 | 17 | 1 | FR1 | 79 | 3 | RAGE3 | | | | 4.32 | -99 |
| | 3 | 25 | 3 | PAI | 27 | 12 | N10 | 30 | 3 | FGB3 | 8.93 | -99 |
| | 3 | 25 | 3 | PAI | 29 | 3 | G1A3 | 30 | 3 | FGB3 | 8.93 | -99 |
| | 2 | 25 | 3 | PAI | 30 | 3 | FGB3 | | | | 8.95 | -99 |
| | 2 | 25 | 3 | PAI | 56 | 12 | HANP1 | | | | 4.35 | -99 |
| | 3 | 25 | 3 | PAI | 73 | 1 | VEGF5 | 96 | 23 | LRP1 | 6.66 | -99 |
| | 3 | 30 | 3 | FGB3 | 38 | 3 | PONA1 | 69 | 1 | CD181 | 8.59 | -99 |
| | 2 | 31 | 1 | APE2 | 86 | 3 | IL-181 | | | | 4.26 | -99 |
| | 2 | 31 | 1 | APE2 | 87 | 1 | MS1 | | | | 4.33 | -99 |
| | 2 | 31 | 1 | APE2 | 89 | 12 | IL102 | | | | 4.24 | -99 |
| | 2 | 31 | 1 | APE2 | 96 | 23 | LRP1 | | | | 4.37 | -99 |

[FIG. 9]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| negative genetic polymorphism sets | 3 | 13 | 3 | CF12 | 68 | 3 | BKR1 | 90 | 12 | IL-182 | 17.58 | -12.79 |
| | 3 | 25 | 3 | PAI | 43 | 1 | MTP1 | 96 | 23 | LRP1 | 14.29 | -18.54 |
| | 3 | 4 | 1 | ESRa1 | 80 | 3 | CCR2 | 96 | 23 | LRP1 | 13.83 | -18 |
| | 2 | 10 | 12 | GP3A | 18 | 23 | S2AR | | | | 13.49 | -99 |
| | 3 | 33 | 1 | MCP1 | 80 | 3 | CCR2 | 88 | 3 | VWF2 | 12.85 | -10.05 |
| | 3 | 30 | 3 | FGB3 | 38 | 3 | PONA1 | 54 | 1 | IL62 | 10.9 | -99 |
| | 3 | 70 | 1 | APM2 | 86 | 3 | IL-181 | 87 | 1 | MS1 | 9.57 | -13.35 |
| | 3 | 30 | 3 | FGB3 | 32 | 12 | MTHFR | 38 | 3 | PONA1 | 9.51 | -13.27 |
| | 3 | 4 | 1 | ESRa1 | 38 | 3 | PONA1 | 90 | 12 | IL-182 | 9.48 | -13.24 |
| | 3 | 13 | 3 | CF12 | 68 | 3 | BKR1 | 84 | 23 | IL1B2 | 9 | -99 |
| | 3 | 25 | 3 | PAI | 73 | 1 | VEGF5 | 88 | 3 | VWF2 | 8.99 | -99 |
| | 3 | 40 | 12 | FGB3 | 64 | 12 | TNFa2 | 85 | 3 | 5178 | 8.91 | -99 |
| | 3 | 4 | 1 | ESRa1 | 36 | 12 | FGA1 | 49 | 23 | GP61 | 8.63 | -99 |
| | 3 | 13 | 3 | CF12 | 64 | 12 | TNFa2 | 82 | 1 | IL103 | 7.82 | -11.47 |
| | 3 | 4 | 1 | ESRa1 | 8 | 3 | PONA2 | 26 | 23 | NOS3 | 7.7 | -11.34 |
| | 3 | 13 | 3 | CF12 | 68 | 3 | BKR1 | 78 | 3 | ET1 | 7.61 | -11.23 |
| | 3 | 30 | 3 | FGB3 | 98 | 12 | PGC11 | 99 | 12 | PGC12 | 7.56 | -11.17 |
| | 3 | 49 | 23 | GP61 | 69 | 1 | CD181 | 80 | 3 | CCR2 | 7.55 | -11.14 |
| | 2 | 25 | 3 | PAI | 42 | 3 | GCLM1 | | | | 6.54 | -99 |
| | 2 | 17 | 1 | FR1 | 27 | 12 | N10 | | | | 4.45 | -99 |
| | 2 | 31 | 1 | APE2 | 88 | 3 | VWF2 | | | | 4.41 | -99 |
| | 2 | 33 | 1 | MCP1 | 64 | 12 | TNFa2 | | | | 4.29 | -99 |
| | 3 | 4 | 1 | ESRa1 | 26 | 23 | NOS3 | 96 | 23 | LRP1 | 8.69 | -99 |
| | 2 | 4 | 1 | ESRa1 | 30 | 3 | FGB3 | | | | 6.48 | -99 |
| | 2 | 10 | 12 | GP3A | 21 | 1 | CX37 | | | | 8.92 | -99 |
| | 2 | 10 | 12 | GP3A | 37 | 23 | GP1ba | | | | 6.52 | -99 |
| | 2 | 17 | 1 | FR1 | 22 | 1 | ESL1 | | | | 4.44 | -99 |
| | 2 | 17 | 1 | FR1 | 29 | 3 | G1A3 | | | | 4.39 | -99 |
| | 2 | 17 | 1 | FR1 | 32 | 12 | MTHFR | | | | 4.34 | -99 |
| | 2 | 17 | 1 | FR1 | 35 | 1 | ESL2 | | | | 4.43 | -99 |
| | 2 | 17 | 1 | FR1 | 48 | 12 | ICAM1 | | | | 4.31 | -99 |
| | 2 | 17 | 1 | FR1 | 54 | 1 | IL62 | | | | 4.3 | -99 |
| | 2 | 17 | 1 | FR1 | 79 | 3 | RAGE3 | | | | 4.32 | -99 |
| | 3 | 25 | 3 | PAI | 27 | 12 | N10 | 30 | 3 | FGB3 | 8.93 | -99 |
| | 3 | 25 | 3 | PAI | 29 | 3 | G1A3 | 30 | 3 | FGB3 | 8.93 | -99 |
| | 2 | 25 | 3 | PAI | 30 | 3 | FGB3 | | | | 8.95 | -99 |
| | 2 | 25 | 3 | PAI | 56 | 12 | HANP1 | | | | 4.35 | -99 |
| | 3 | 25 | 3 | PAI | 73 | 1 | VEGF5 | 96 | 23 | LRP1 | 6.66 | -99 |
| | 3 | 30 | 3 | FGB3 | 38 | 3 | PONA1 | 69 | 1 | CD181 | 8.59 | -99 |
| | 2 | 31 | 1 | APE2 | 86 | 3 | IL-181 | | | | 4.26 | -99 |
| | 2 | 31 | 1 | APE2 | 87 | 1 | MS1 | | | | 4.33 | -99 |
| | 2 | 31 | 1 | APE2 | 89 | 12 | IL102 | | | | 4.24 | -99 |
| | 2 | 31 | 1 | APE2 | 96 | 23 | LRP1 | | | | 4.37 | -99 |

[FIG. 10]

| SNP-No | Genotype |
|---|---|
| 13 | 3 |
| 68 | 3 |
| 75 | 12 |
| 73 | 1 |
| 55 | 3 |
| 93 | 23 |
| 33 | 1 |
| 4 | 1 |
| 40 | 12 |
| 25 | 3 |
| 77 | 12 |
| 90 | 12 |
| 74 | 12 |
| 38 | 3 |
| 29 | 3 |
| 99 | 12 |
| 69 | 12 |
| 50 | 1 |
| 35 | 1 |
| 96 | 23 |
| 53 | 23 |
| 45 | 23 |
| 30 | 3 |
| 10 | 12 |
| 80 | 3 |
| 54 | 1 |
| 83 | 1 |
| 48 | 1 |
| 86 | 12 |
| 49 | 23 |
| 76 | 23 |
| 82 | 12 |
| 18 | 23 |
| 27 | 1 |
| 89 | 12 |
| 11 | 1 |
| 98 | 12 |
| 12 | 3 |
| 41 | 12 |
| 70 | 3 |
| 51 | 1 |
| 67 | 3 |
| 52 | 23 |
| 44 | 3 |
| 37 | 23 |
| 26 | 23 |
| 5 | 3 |
| 78 | 3 |
| 36 | 12 |
| 42 | 3 |
| 17 | 1 |
| 8 | 3 |
| 20 | 12 |
| 23 | 3 |
| 85 | 12 |
| 64 | 12 |
| 84 | 23 |

[FIG. 11]

| SNP-No | Genotype |
|---|---|
| 68 | 3 |
| 13 | 3 |
| 4 | 1 |
| 33 | 1 |
| 25 | 3 |
| 96 | 23 |
| 73 | 1 |
| 88 | 1 |
| 77 | 12 |
| 45 | 23 |
| 24 | 1 |
| 31 | 12 |
| 53 | 23 |
| 40 | 12 |
| 90 | 12 |
| 18 | 23 |
| 70 | 3 |
| 30 | 3 |
| 50 | 1 |
| 27 | 1 |
| 38 | 3 |
| 10 | 12 |
| 78 | 3 |
| 49 | 23 |
| 89 | 12 |
| 43 | 1 |
| 64 | 12 |
| 36 | 1 |
| 55 | 3 |
| 67 | 3 |
| 42 | 3 |
| 51 | 12 |
| 29 | 3 |
| 98 | 12 |
| 52 | 23 |
| 44 | 3 |
| 17 | 1 |
| 87 | 12 |
| 69 | 1 |
| 11 | 1 |
| 83 | 1 |
| 54 | 1 |
| 82 | 1 |
| 35 | 1 |
| 12 | 3 |
| 75 | 23 |
| 37 | 23 |
| 26 | 23 |
| 97 | 12 |
| 41 | 12 |
| 39 | 23 |
| 85 | 12 |
| 84 | 23 |
| 22 | 1 |
| 8 | 3 |
| 99 | 3 |

[FIG. 12]

| SNP-No | Genotype |
|---|---|
| 4 | 1 |
| 33 | 1 |
| 25 | 3 |
| 88 | 3 |
| 13 | 3 |
| 68 | 3 |
| 90 | 12 |
| 96 | 23 |
| 38 | 3 |
| 53 | 23 |
| 73 | 1 |
| 45 | 23 |
| 43 | 1 |
| 30 | 3 |
| 10 | 12 |
| 27 | 1 |
| 40 | 12 |
| 23 | 3 |
| 69 | 12 |
| 78 | 3 |
| 51 | 12 |
| 36 | 1 |
| 70 | 3 |
| 18 | 23 |
| 35 | 1 |
| 64 | 12 |
| 67 | 3 |
| 75 | 3 |
| 29 | 3 |
| 50 | 1 |
| 79 | 3 |
| 11 | 1 |
| 49 | 23 |
| 17 | 1 |
| 31 | 1 |
| 52 | 23 |
| 77 | 1 |
| 83 | 1 |
| 42 | 3 |
| 98 | 12 |
| 44 | 3 |
| 87 | 12 |
| 26 | 23 |
| 84 | 23 |
| 37 | 23 |
| 62 | 1 |
| 12 | 3 |
| 47 | 23 |
| 97 | 12 |
| 76 | 12 |
| 99 | 3 |
| 41 | 12 |
| 54 | 1 |
| 8 | 3 |
| 22 | 1 |
| 48 | 23 |
| 89 | 12 |
| 56 | 12 |

[FIG. 13]

| SNP-No | Genotype |
|---|---|
| 33 | 1 |
| 4 | 1 |
| 88 | 3 |
| 25 | 3 |
| 68 | 3 |
| 73 | 1 |
| 96 | 23 |
| 90 | 12 |
| 13 | 3 |
| 10 | 12 |
| 30 | 3 |
| 43 | 1 |
| 49 | 23 |
| 38 | 3 |
| 77 | 12 |
| 53 | 23 |
| 86 | 1 |
| 82 | 1 |
| 64 | 12 |
| 69 | 12 |
| 40 | 12 |
| 18 | 23 |
| 50 | 1 |
| 78 | 3 |
| 26 | 23 |
| 51 | 12 |
| 76 | 12 |
| 52 | 23 |
| 29 | 3 |
| 48 | 23 |
| 35 | 1 |
| 17 | 1 |
| 31 | 1 |
| 42 | 3 |
| 54 | 1 |
| 70 | 1 |
| 55 | 3 |
| 36 | 1 |
| 85 | 12 |
| 71 | 3 |
| 21 | 1 |
| 5 | 3 |
| 62 | 1 |
| 84 | 23 |
| 93 | 23 |
| 37 | 23 |
| 83 | 1 |
| 8 | 3 |
| 22 | 1 |
| 79 | 3 |
| 87 | 1 |
| 56 | 12 |
| 12 | 3 |
| 20 | 12 |

[FIG. 14]

| SNP-No | Genotype |
|---|---|
| 33 | 1 |
| 4 | 1 |
| 13 | 3 |
| 68 | 3 |
| 25 | 3 |
| 96 | 23 |
| 90 | 12 |
| 88 | 3 |
| 77 | 12 |
| 73 | 1 |
| 10 | 12 |
| 49 | 23 |
| 30 | 3 |
| 86 | 1 |
| 18 | 23 |
| 38 | 3 |
| 82 | 1 |
| 64 | 12 |
| 93 | 12 |
| 84 | 23 |
| 53 | 23 |
| 69 | 12 |
| 40 | 12 |
| 78 | 3 |
| 43 | 1 |
| 52 | 23 |
| 76 | 12 |
| 26 | 23 |
| 50 | 1 |
| 48 | 23 |
| 36 | 1 |
| 17 | 1 |
| 31 | 1 |
| 54 | 1 |
| 42 | 3 |
| 70 | 1 |
| 55 | 3 |
| 21 | 1 |
| 62 | 1 |
| 5 | 3 |
| 71 | 3 |
| 37 | 23 |
| 99 | 12 |
| 83 | 1 |
| 8 | 3 |
| 29 | 3 |
| 67 | 3 |
| 35 | 1 |
| 22 | 1 |
| 79 | 3 |
| 87 | 1 |
| 12 | 3 |
| 20 | 12 |
| 56 | 12 |

[FIG. 15]

| SNP-No | Genotype |
|---|---|
| 25 | 3 |
| 90 | 12 |
| 68 | 3 |
| 4 | 1 |
| 96 | 23 |
| 33 | 1 |
| 13 | 3 |
| 88 | 3 |
| 75 | 12 |
| 49 | 23 |
| 10 | 12 |
| 80 | 3 |
| 73 | 1 |
| 70 | 1 |
| 29 | 3 |
| 38 | 3 |
| 36 | 1 |
| 40 | 12 |
| 64 | 12 |
| 55 | 12 |
| 30 | 3 |
| 62 | 12 |
| 86 | 3 |
| 87 | 1 |
| 5 | 3 |
| 18 | 23 |
| 43 | 1 |
| 76 | 12 |
| 26 | 23 |
| 17 | 1 |
| 31 | 1 |
| 93 | 1 |
| 71 | 3 |
| 82 | 1 |
| 54 | 1 |
| 78 | 3 |
| 21 | 1 |
| 8 | 3 |
| 84 | 23 |
| 37 | 23 |
| 42 | 3 |
| 85 | 3 |
| 99 | 3 |
| 27 | 12 |
| 56 | 12 |
| 89 | 12 |
| 35 | 1 |
| 48 | 12 |
| 22 | 1 |
| 32 | 12 |
| 79 | 3 |

[FIG. 16]

| SNP-No | Genotype |
|---|---|
| 96 | 23 |
| 90 | 12 |
| 13 | 3 |
| 25 | 3 |
| 33 | 1 |
| 68 | 3 |
| 88 | 3 |
| 80 | 3 |
| 4 | 1 |
| 30 | 3 |
| 10 | 12 |
| 73 | 1 |
| 38 | 3 |
| 36 | 1 |
| 64 | 12 |
| 43 | 1 |
| 86 | 3 |
| 87 | 1 |
| 18 | 23 |
| 70 | 1 |
| 69 | 1 |
| 42 | 3 |
| 49 | 23 |
| 26 | 23 |
| 78 | 3 |
| 17 | 1 |
| 31 | 1 |
| 82 | 1 |
| 84 | 23 |
| 99 | 12 |
| 98 | 12 |
| 21 | 1 |
| 8 | 3 |
| 32 | 12 |
| 37 | 23 |
| 40 | 12 |
| 85 | 3 |
| 54 | 1 |
| 29 | 3 |
| 27 | 12 |
| 89 | 12 |
| 56 | 12 |
| 35 | 1 |
| 48 | 12 |
| 22 | 1 |
| 79 | 3 |

[FIG. 17]

| SNP-No | Genotype |
|---|---|
| 90 | 12 |
| 13 | 3 |
| 25 | 3 |
| 88 | 3 |
| 68 | 3 |
| 80 | 3 |
| 4 | 1 |
| 96 | 23 |
| 30 | 3 |
| 10 | 12 |
| 33 | 1 |
| 73 | 1 |
| 38 | 3 |
| 64 | 12 |
| 86 | 3 |
| 87 | 1 |
| 43 | 1 |
| 70 | 1 |
| 18 | 23 |
| 49 | 23 |
| 69 | 1 |
| 42 | 3 |
| 26 | 23 |
| 78 | 3 |
| 17 | 1 |
| 31 | 1 |
| 82 | 1 |
| 99 | 12 |
| 98 | 12 |
| 21 | 1 |
| 8 | 3 |
| 84 | 23 |
| 32 | 12 |
| 37 | 23 |
| 40 | 12 |
| 85 | 3 |
| 54 | 1 |
| 36 | 12 |
| 29 | 3 |
| 27 | 12 |
| 89 | 12 |
| 56 | 12 |
| 35 | 1 |
| 48 | 12 |
| 22 | 1 |
| 79 | 3 |

[FIG. 18]

| SNP-No | Genotype |
|---|---|
| 13 | 3 |
| 25 | 3 |
| 68 | 3 |
| 80 | 3 |
| 90 | 12 |
| 4 | 1 |
| 96 | 23 |
| 88 | 3 |
| 33 | 1 |
| 30 | 3 |
| 10 | 12 |
| 38 | 3 |
| 64 | 12 |
| 86 | 3 |
| 87 | 1 |
| 43 | 1 |
| 70 | 1 |
| 18 | 23 |
| 49 | 23 |
| 73 | 1 |
| 69 | 1 |
| 42 | 3 |
| 26 | 23 |
| 78 | 3 |
| 17 | 1 |
| 31 | 1 |
| 82 | 1 |
| 99 | 12 |
| 98 | 12 |
| 21 | 1 |
| 8 | 3 |
| 84 | 23 |
| 32 | 12 |
| 37 | 23 |
| 40 | 12 |
| 85 | 3 |
| 54 | 1 |
| 36 | 12 |
| 29 | 3 |
| 27 | 12 |
| 89 | 12 |
| 56 | 12 |
| 35 | 1 |
| 48 | 12 |
| 22 | 1 |
| 79 | 3 |

[FIG. 19-A]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| positive genetic polymorphism sets | 3 | 8 | 12 | PONA2 | 24 | 12 | AGTR1-3 | 31 | 3 | APE2 | 14.58 | 5.21 |
| | 2 | 8 | 12 | PONA2 | 31 | 3 | APE2 | | | | 13.32 | 4.42 |
| | 3 | 3 | 3 | ACE ID | 33 | 3 | MCP1 | 67 | 12 | 1A2 | 13.29 | 4.44 |
| | 3 | 3 | 3 | ACE ID | 40 | 3 | FGB3 | 67 | 12 | 1A2 | 13.08 | 2.77 |
| | 3 | 33 | 3 | MCP1 | 67 | 12 | 1A2 | 68 | 12 | BKR1 | 12.91 | 2.72 |
| | 3 | 3 | 3 | ACE ID | 40 | 3 | FGB3 | 51 | 23 | GP1a1 | 12.03 | 2.59 |
| | 2 | 53 | 1 | β2AR-1 | 70 | 12 | APM2 | | | | 11.84 | 3.36 |
| | 3 | 3 | 3 | ACE ID | 67 | 12 | 1A2 | 96 | 1 | LRP1 | 11.43 | 2.41 |
| | 3 | 33 | 3 | MCP1 | 51 | 23 | GP1a1 | 68 | 12 | BKR1 | 10.78 | 2.42 |
| | 3 | 1 | 3 | ABCA1 | 17 | 3 | FR1 | 30 | 1 | FGB3 | 10.74 | 2.38 |
| | 3 | 53 | 1 | β2AR-1 | 84 | 1 | IL1B2 | 98 | 3 | PGC11 | 10.34 | 2.49 |
| | 3 | 1 | 3 | ABCA1 | 26 | 1 | NOS3 | 30 | 1 | FGB3 | 10.26 | 2.48 |
| | 3 | 1 | 3 | ABCA1 | 4 | 23 | ESRa1 | 30 | 1 | FGB3 | 10.05 | 2.36 |
| | 3 | 3 | 3 | ACE ID | 51 | 23 | GP1a1 | 96 | 1 | LRP1 | 10.03 | 2.22 |
| | 3 | 3 | 3 | ACE ID | 75 | 3 | LTA2 | 87 | 23 | MS1 | 10.02 | 12.6 |
| | 3 | 3 | 3 | ACE ID | 13 | 12 | CF12 | 90 | 3 | IL-182 | 9.71 | 2.09 |
| | 3 | 8 | 12 | PONA2 | 24 | 12 | AGTR1-3 | 25 | 12 | PAI | 9.68 | 3.02 |
| | 3 | 3 | 3 | ACE ID | 39 | 3 | CRP1 | 50 | 23 | β2AR4 | 9.67 | 7.19 |
| | 3 | 3 | 3 | ACE ID | 33 | 3 | MCP1 | 90 | 3 | IL-182 | 9.6 | 2.68 |
| | 3 | 33 | 3 | MCP1 | 42 | 1 | GCLM1 | 74 | 3 | LTA1 | 9.36 | 7.04 |
| | 3 | 8 | 12 | PONA2 | 26 | 1 | NOS3 | 45 | 1 | LPL3 | 9.25 | 3.58 |
| | 3 | 1 | 3 | ABCA1 | 17 | 3 | FR1 | 38 | 12 | PONA1 | 9.12 | 2.42 |
| | 3 | 53 | 1 | β2AR-1 | 62 | 1 | APE3 | 98 | 3 | PGC11 | 9.06 | 2.47 |
| | 3 | 3 | 3 | ACE ID | 38 | 12 | PONA1 | 42 | 1 | GCLM1 | 9.05 | 2.2 |
| | 3 | 1 | 3 | ABCA1 | 38 | 12 | PONA1 | 64 | 3 | TNFa2 | 9.02 | 2.35 |
| | 3 | 3 | 3 | ACE ID | 13 | 12 | CF12 | 88 | 12 | VWF2 | 8.93 | 2.1 |
| | 3 | 3 | 3 | ACE ID | 40 | 3 | FGB3 | 90 | 3 | IL-182 | 8.89 | 2.03 |
| | 3 | 29 | 12 | G1A3 | 37 | 1 | GP1ba | 78 | 12 | ET1 | 8.85 | 5.17 |
| | 3 | 12 | 12 | Tbm3 | 84 | 1 | IL1B2 | 85 | 3 | 5178 | 8.84 | 3.04 |
| | 3 | 3 | 3 | ACE ID | 25 | 12 | PAI | 33 | 3 | MCP1 | 8.66 | 2.32 |
| | 3 | 29 | 12 | G1A3 | 39 | 3 | CRP1 | 78 | 12 | ET1 | 8.48 | 4.24 |
| | 3 | 3 | 3 | ACE ID | 7 | 1 | N7 | 27 | 3 | N10 | 8.39 | 2.98 |
| | 3 | 1 | 3 | ABCA1 | 4 | 23 | ESRa1 | 88 | 12 | VWF2 | 8.05 | 2.14 |
| | 3 | 1 | 3 | ABCA1 | 4 | 23 | ESRa1 | 96 | 1 | LRP1 | 7.73 | 2.03 |
| | 3 | 55 | 12 | MPO | 86 | 3 | IL-181 | 96 | 1 | LRP1 | 7.71 | 6.14 |
| | 3 | 1 | 3 | ABCA1 | 40 | 3 | FGB3 | 51 | 23 | GP1a1 | 7.63 | 2.51 |
| | 3 | 3 | 3 | ACE ID | 73 | 23 | VEGF5 | 87 | 23 | MS1 | 7.32 | 2.47 |
| | 2 | 53 | 1 | β2AR-1 | 54 | 23 | IL62 | | | | 7.25 | 2.46 |
| | 3 | 12 | 12 | Tbm3 | 26 | 1 | NOS3 | 62 | 1 | APE3 | 7.21 | 2.21 |
| | 1 | 74 | 3 | LTA1 | | | | | | | 7.15 | 2.2 |
| | 3 | 77 | 3 | APM12 | 94 | 12 | CYP3A4 | 96 | 1 | LRP1 | 7.14 | 3.13 |
| | 3 | 55 | 12 | MPO | 88 | 12 | VWF2 | 96 | 1 | LRP1 | 7.06 | 2.45 |
| | 3 | 3 | 3 | ACE ID | 93 | 1 | TPO1 | 96 | 1 | LRP1 | 7.03 | 3.12 |
| | 3 | 36 | 3 | FGA1 | 73 | 23 | VEGF5 | 90 | 3 | IL-182 | 6.98 | 2.23 |
| | 3 | 55 | 12 | MPO | 90 | 3 | IL-182 | 96 | 1 | LRP1 | 6.97 | 2.3 |
| | 3 | 33 | 3 | MCP1 | 67 | 12 | 1A2 | 85 | 3 | 5178 | 6.89 | 2.1 |
| | 3 | 33 | 3 | MCP1 | 51 | 23 | GP1a1 | 80 | 12 | CCR2 | 6.73 | 2.16 |
| | 2 | 77 | 3 | APM12 | 80 | 12 | CCR2 | | | | 6.7 | 4.33 |
| | 3 | 36 | 3 | FGA1 | 78 | 12 | ET1 | 90 | 3 | IL-182 | 6.67 | 2.2 |
| | 3 | 12 | 12 | Tbm3 | 40 | 3 | FGB3 | 73 | 23 | VEGF5 | 6.65 | 2.3 |
| | 2 | 77 | 3 | APM12 | 90 | 3 | IL-182 | | | | 6.64 | 2.83 |
| | 1 | 20 | 3 | GSY | | | | | | | 4.52 | 99 |
| | 3 | 1 | 3 | ABCA1 | 3 | 3 | ACE ID | 12 | 12 | Tbm3 | 6.8 | 99 |
| | 3 | 1 | 3 | ABCA1 | 3 | 3 | ACE ID | 27 | 3 | N10 | 10.24 | 99 |
| | 3 | 1 | 3 | ABCA1 | 3 | 3 | ACE ID | 30 | 1 | FGB3 | 7.19 | 2.77 |
| | 3 | 1 | 3 | ABCA1 | 3 | 3 | ACE ID | 37 | 1 | GP1ba | 8.4 | 2.98 |
| | 3 | 1 | 3 | ABCA1 | 3 | 3 | ACE ID | 96 | 1 | LRP1 | 6.69 | 2.69 |
| | 3 | 1 | 3 | ABCA1 | 10 | 3 | GP3A | 30 | 1 | FGB3 | 8.17 | 2.06 |
| | 3 | 1 | 3 | ABCA1 | 10 | 3 | GP3A | 38 | 12 | PONA1 | 7.22 | 2.09 |

[FIG. 19-B]

| | | | | | | | | | | | |
|---|---|---|---|---|---|---|---|---|---|---|---|
| 3 | 1 | 3 | ABCA1 | 10 | 3 | GP3A | 53 | 1 | β2AR-1 | 7.76 | 3.58 |
| 3 | 1 | 3 | ABCA1 | 19 | 12 | MMP9 | 30 | 1 | FGB3 | 8.37 | 2.08 |
| 3 | 1 | 3 | ABCA1 | 19 | 12 | MMP9 | 38 | 12 | PONA1 | 7.09 | 2.08 |
| 3 | 1 | 3 | ABCA1 | 28 | 12 | AGT1 | 30 | 1 | FGB3 | 8.06 | 2.08 |
| 3 | 1 | 3 | ABCA1 | 28 | 12 | AGT1 | 53 | 1 | β2AR-1 | 7.17 | 3.43 |
| 3 | 1 | 3 | ABCA1 | 37 | 1 | GP1ba | 53 | 1 | β2AR-1 | 6.88 | 4.41 |
| 3 | 1 | 3 | ABCA1 | 37 | 1 | GP1ba | 77 | 3 | APM12 | 6.94 | 99 |
| 3 | 1 | 3 | ABCA1 | 38 | 12 | PONA1 | 40 | 3 | FGB3 | 7.5 | 2.59 |
| 3 | 1 | 3 | ABCA1 | 38 | 12 | PONA1 | 71 | 23 | REG1 | 6.92 | 2.04 |
| 3 | 1 | 3 | ABCA1 | 38 | 12 | PONA1 | 78 | 12 | ET1 | 7.55 | 2.17 |
| 3 | 1 | 3 | ABCA1 | 38 | 12 | PONA1 | 96 | 1 | LRP1 | 8.09 | 2.42 |
| 3 | 1 | 3 | ABCA1 | 38 | 12 | PONA1 | 98 | 3 | PGC11 | 7.84 | 2.63 |
| 3 | 1 | 3 | ABCA1 | 40 | 3 | FGB3 | 67 | 12 | 1A2 | 7.17 | 2.46 |
| 3 | 1 | 3 | ABCA1 | 53 | 1 | β2AR-1 | 85 | 3 | 5178 | 7.87 | 6.2 |
| 3 | 3 | 3 | ACE ID | 7 | 1 | N7 | 50 | 23 | β2AR4 | 7.42 | 4.62 |
| 3 | 3 | 3 | ACE ID | 8 | 12 | PONA2 | 16 | 12 | hepatic_lipase | 7.25 | 3.87 |
| 3 | 3 | 3 | ACE ID | 8 | 12 | PONA2 | 31 | 3 | APE2 | 8.75 | 5.13 |
| 3 | 3 | 3 | ACE ID | 8 | 12 | PONA2 | 32 | 23 | MTHFR | 9.21 | 6.94 |
| 3 | 3 | 3 | ACE ID | 8 | 12 | PONA2 | 45 | 1 | LPL3 | 8.03 | 4.84 |
| 2 | 3 | 3 | ACE ID | 12 | 12 | Tbm3 | | | | 8.05 | 3.64 |
| 3 | 3 | 3 | ACE ID | 13 | 12 | CF12 | 38 | 12 | PONA1 | 7.38 | 2.15 |
| 3 | 3 | 3 | ACE ID | 13 | 12 | CF12 | 50 | 23 | β2AR4 | 8.24 | 10.71 |
| 3 | 3 | 3 | ACE ID | 19 | 12 | MMP9 | 50 | 23 | β2AR4 | 8.75 | 5.13 |
| 3 | 3 | 3 | ACE ID | 19 | 12 | MMP9 | 87 | 23 | MS1 | 7.1 | 2.25 |
| 3 | 3 | 3 | ACE ID | 25 | 12 | PAI | 50 | 23 | β2AR4 | 8.6 | 6.61 |
| 3 | 3 | 3 | ACE ID | 25 | 12 | PAI | 87 | 23 | MS1 | 7.17 | 2.45 |
| 3 | 3 | 3 | ACE ID | 25 | 12 | PAI | 93 | 1 | TPO1 | 6.91 | 3.1 |
| 3 | 3 | 3 | ACE ID | 27 | 3 | N10 | 38 | 12 | PONA1 | 7.34 | 3.47 |
| 3 | 3 | 3 | ACE ID | 27 | 3 | N10 | 88 | 12 | VWF2 | 6.88 | 2.87 |
| 3 | 3 | 3 | ACE ID | 27 | 3 | N10 | 96 | 1 | LRP1 | 6.92 | 2.73 |
| 3 | 3 | 3 | ACE ID | 32 | 23 | MTHFR | 38 | 12 | PONA1 | 6.9 | 2.1 |
| 3 | 3 | 3 | ACE ID | 32 | 23 | MTHFR | 51 | 23 | GP1a1 | 7.29 | 2.1 |
| 3 | 3 | 3 | ACE ID | 32 | 23 | MTHFR | 67 | 12 | 1A2 | 7.26 | 2.14 |
| 3 | 3 | 3 | ACE ID | 33 | 3 | MCP1 | 37 | 1 | GP1ba | 7.13 | 2.21 |
| 3 | 3 | 3 | ACE ID | 33 | 3 | MCP1 | 42 | 1 | GCLM1 | 6.64 | 2.31 |
| 3 | 3 | 3 | ACE ID | 33 | 3 | MCP1 | 51 | 23 | GP1a1 | 9.62 | 3.19 |
| 3 | 3 | 3 | ACE ID | 37 | 1 | GP1ba | 50 | 23 | β2AR4 | 8.79 | 6.71 |
| 3 | 3 | 3 | ACE ID | 38 | 12 | PONA1 | 51 | 23 | GP1a1 | 8.2 | 2.19 |
| 3 | 3 | 3 | ACE ID | 38 | 12 | PONA1 | 67 | 12 | 1A2 | 8.64 | 2.24 |
| 3 | 3 | 3 | ACE ID | 39 | 3 | CRP1 | 87 | 23 | MS1 | 6.74 | 2.2 |
| 3 | 3 | 3 | ACE ID | 40 | 3 | FGB3 | 50 | 23 | β2AR4 | 6.72 | 5.57 |
| 3 | 3 | 3 | ACE ID | 42 | 1 | GCLM1 | 50 | 23 | β2AR4 | 8.89 | 11.41 |
| 3 | 3 | 3 | ACE ID | 42 | 1 | GCLM1 | 93 | 1 | TPO1 | 7.16 | 3.86 |
| 2 | 3 | 3 | ACE ID | 50 | 23 | β2AR4 | | | | 8.72 | 5.11 |
| 3 | 3 | 3 | ACE ID | 51 | 23 | GP1a1 | 73 | 23 | VEGF5 | 8.51 | 2 |
| 3 | 3 | 3 | ACE ID | 51 | 23 | GP1a1 | 90 | 3 | IL-182 | 8.76 | 2.04 |
| 3 | 3 | 3 | ACE ID | 51 | 23 | GP1a1 | 93 | 1 | TPO1 | 7.86 | 4.8 |
| 3 | 3 | 3 | ACE ID | 67 | 12 | 1A2 | 73 | 23 | VEGF5 | 8.21 | 2 |
| 3 | 3 | 3 | ACE ID | 67 | 12 | 1A2 | 90 | 3 | IL-182 | 8.18 | 2.02 |
| 3 | 3 | 3 | ACE ID | 67 | 12 | 1A2 | 93 | 1 | TPO1 | 7.83 | 6.21 |
| 3 | 8 | 12 | PONA2 | 10 | 3 | GP3A | 22 | 1 | ESL1 | 6.73 | 2.25 |
| 3 | 8 | 12 | PONA2 | 10 | 3 | GP3A | 25 | 12 | PAI | 8.78 | 2.76 |
| 3 | 8 | 12 | PONA2 | 10 | 3 | GP3A | 31 | 3 | APE2 | 13.32 | 4.42 |
| 3 | 8 | 12 | PONA2 | 10 | 3 | GP3A | 67 | 12 | 1A2 | 7.66 | 3.26 |
| 3 | 8 | 12 | PONA2 | 10 | 3 | GP3A | 85 | 3 | 5178 | 6.64 | 2.82 |
| 3 | 8 | 12 | PONA2 | 15 | 12 | MMP-12 | 22 | 1 | ESL1 | 6.73 | 2.25 |
| 3 | 8 | 12 | PONA2 | 15 | 12 | MMP-12 | 25 | 12 | PAI | 8.78 | 2.76 |
| 3 | 8 | 12 | PONA2 | 15 | 12 | MMP-12 | 31 | 3 | APE2 | 13.32 | 4.42 |
| 3 | 8 | 12 | PONA2 | 15 | 12 | MMP-12 | 67 | 12 | 1A2 | 7.66 | 3.26 |
| 3 | 8 | 12 | PONA2 | 15 | 12 | MMP-12 | 85 | 3 | 5178 | 6.64 | 2.82 |

[FIG. 19-C]

| | | | | | | | | | | | |
|---|---|---|---|---|---|---|---|---|---|---|---|
| | 3 | 8 | 12 | PONA2 | 21 | 12 | CX37 | 22 | 1 | ESL1 | 7.08 | 2.36 |
| | 3 | 8 | 12 | PONA2 | 21 | 12 | CX37 | 25 | 12 | PAI | 9.26 | 2.96 |
| | 3 | 8 | 12 | PONA2 | 21 | 12 | CX37 | 26 | 1 | NOS3 | 7.15 | 2.53 |
| | 3 | 8 | 12 | PONA2 | 21 | 12 | CX37 | 31 | 3 | APE2 | 12.37 | 4.22 |
| | 3 | 8 | 12 | PONA2 | 21 | 12 | CX37 | 67 | 12 | 1A2 | 8.69 | 3.82 |
| | 3 | 8 | 12 | PONA2 | 21 | 12 | CX37 | 85 | 3 | 5178 | 6.64 | 2.82 |
| | 2 | 8 | 12 | PONA2 | 22 | 1 | ESL1 | | | | 6.73 | 2.25 |
| | 3 | 8 | 12 | PONA2 | 24 | 12 | AGTR1-3 | 26 | 1 | NOS3 | 7.15 | 2.53 |
| | 3 | 8 | 12 | PONA2 | 24 | 12 | AGTR1-3 | 67 | 12 | 1A2 | 7.22 | 3.16 |
| | 2 | 8 | 12 | PONA2 | 25 | 12 | PAI | | | | 8.78 | 2.76 |
| | 3 | 8 | 12 | PONA2 | 26 | 1 | NOS3 | 31 | 3 | APE2 | 10.83 | 4.32 |
| | 3 | 8 | 12 | PONA2 | 26 | 1 | NOS3 | 32 | 23 | MTHFR | 7.92 | 4.07 |
| | 3 | 8 | 12 | PONA2 | 26 | 1 | NOS3 | 43 | 12 | MTP1 | 7.62 | 2.6 |
| | 3 | 8 | 12 | PONA2 | 26 | 1 | NOS3 | 63 | 12 | HANP2 | 6.76 | 2.39 |
| | 3 | 8 | 12 | PONA2 | 26 | 1 | NOS3 | 64 | 3 | TNFa2 | 6.64 | 2.45 |
| | 3 | 8 | 12 | PONA2 | 26 | 1 | NOS3 | 73 | 23 | VEGF5 | 7.13 | 2.92 |
| | 3 | 8 | 12 | PONA2 | 32 | 23 | MTHFR | 79 | 23 | RAGE3 | 7.57 | 3.23 |
| | 3 | 8 | 12 | PONA2 | 32 | 23 | MTHFR | 85 | 3 | 5178 | 7.09 | 4.48 |
| | 3 | 8 | 12 | PONA2 | 38 | 12 | PONA1 | 67 | 12 | 1A2 | 7.7 | 3.27 |
| | 3 | 8 | 12 | PONA2 | 43 | 12 | MTP1 | 67 | 12 | 1A2 | 8.61 | 3.8 |
| | 3 | 8 | 12 | PONA2 | 43 | 12 | MTP1 | 85 | 3 | 5178 | 7.5 | 3.2 |
| | 3 | 8 | 12 | PONA2 | 51 | 23 | GP1a1 | 67 | 12 | 1A2 | 7.66 | 3.26 |
| | 3 | 8 | 12 | PONA2 | 51 | 23 | GP1a1 | 68 | 12 | BKR1 | 7.59 | 4.71 |
| | 3 | 8 | 12 | PONA2 | 51 | 23 | GP1a1 | 79 | 23 | RAGE3 | 8.13 | 3.67 |
| | 3 | 8 | 12 | PONA2 | 51 | 23 | GP1a1 | 96 | 1 | LRP1 | 9.06 | 4.4 |
| | 3 | 8 | 12 | PONA2 | 63 | 12 | HANP2 | 67 | 12 | 1A2 | 7.66 | 3.26 |
| | 3 | 8 | 12 | PONA2 | 63 | 12 | HANP2 | 85 | 3 | 5178 | 6.64 | 2.82 |
| | 2 | 8 | 12 | PONA2 | 67 | 12 | 1A2 | | | | 7.66 | 3.26 |
| | 2 | 8 | 12 | PONA2 | 85 | 3 | 5178 | | | | 6.64 | 2.82 |
| | 3 | 12 | 12 | Tbm3 | 25 | 12 | PAI | 62 | 1 | APE3 | 7.03 | 2.05 |
| | 3 | 12 | 12 | Tbm3 | 25 | 12 | PAI | 85 | 3 | 5178 | 7.08 | 2.51 |
| | 3 | 12 | 12 | Tbm3 | 33 | 3 | MCP1 | 84 | 1 | IL1B2 | 6.7 | 2.83 |
| | 3 | 12 | 12 | Tbm3 | 40 | 3 | FGB3 | 62 | 1 | APE3 | 6.86 | 2.22 |
| | 3 | 12 | 12 | Tbm3 | 53 | 1 | β2AR-1 | 84 | 1 | IL1B2 | 7.12 | 5.77 |
| | 3 | 12 | 12 | Tbm3 | 70 | 12 | APM2 | 85 | 3 | 5178 | 7.75 | 4.73 |
| | 2 | 12 | 12 | Tbm3 | 74 | 3 | LTA1 | | | | 6.06 | 99 |
| | 3 | 29 | 12 | G1A3 | 37 | 1 | GP1ba | 38 | 12 | PONA1 | 7.32 | 9.74 |
| | 3 | 29 | 12 | G1A3 | 37 | 1 | GP1ba | 39 | 3 | CRP1 | 8.1 | 4.87 |
| | 3 | 29 | 12 | G1A3 | 37 | 1 | GP1ba | 43 | 12 | MTP1 | 7.06 | 3.81 |
| | 3 | 29 | 12 | G1A3 | 37 | 1 | GP1ba | 68 | 12 | BKR1 | 7.14 | 4.54 |
| | 2 | 29 | 12 | G1A3 | 38 | 12 | PONA1 | | | | 7.24 | 5.86 |
| | 3 | 29 | 12 | G1A3 | 39 | 3 | CRP1 | 51 | 23 | GP1a1 | 6.67 | 9.04 |
| | 3 | 29 | 12 | G1A3 | 39 | 3 | CRP1 | 68 | 12 | BKR1 | 8.08 | 4.9 |
| | 3 | 29 | 12 | G1A3 | 42 | 1 | GCLM1 | 78 | 12 | ET1 | 7.42 | 4.62 |
| | 3 | 29 | 12 | G1A3 | 43 | 12 | MTP1 | 51 | 23 | GP1a1 | 6.69 | 9.06 |
| | 3 | 29 | 12 | G1A3 | 43 | 12 | MTP1 | 78 | 12 | ET1 | 7.41 | 3.49 |
| | 3 | 29 | 12 | G1A3 | 51 | 23 | GP1a1 | 52 | 1 | MMP71 | 6.65 | 9.02 |
| | 3 | 29 | 12 | G1A3 | 51 | 23 | GP1a1 | 78 | 12 | ET1 | 6.86 | 9.25 |
| | 3 | 29 | 12 | G1A3 | 51 | 23 | GP1a1 | 96 | 1 | LRP1 | 7.39 | 99 |
| | 3 | 29 | 12 | G1A3 | 62 | 1 | APE3 | 78 | 12 | ET1 | 6.97 | 3.38 |
| | 3 | 29 | 12 | G1A3 | 67 | 12 | 1A2 | 96 | 1 | LRP1 | 6.94 | 99 |
| | 3 | 33 | 3 | MCP1 | 67 | 12 | 1A2 | 74 | 3 | LTA1 | 7.65 | 10.11 |
| | 3 | 33 | 3 | MCP1 | 74 | 3 | LTA1 | 78 | 12 | ET1 | 7.09 | 3.84 |
| | 3 | 53 | 1 | β2AR-1 | 56 | 23 | HANP1 | 70 | 12 | APM2 | 11.07 | 3.24 |
| | 3 | 53 | 1 | β2AR-1 | 62 | 1 | APE3 | 70 | 12 | APM2 | 11.32 | 3.71 |
| | 3 | 53 | 1 | β2AR-1 | 67 | 12 | 1A2 | 70 | 12 | APM2 | 9.01 | 4.4 |
| | 3 | 53 | 1 | β2AR-1 | 73 | 23 | VEGF5 | 80 | 12 | CCR2 | 7.17 | 2.77 |
| | 3 | 53 | 1 | β2AR-1 | 80 | 12 | CCR2 | 98 | 3 | PGC11 | 7.84 | 3.6 |
| | 2 | 55 | 12 | MPO | 69 | 3 | CD181 | | | | 4.49 | 99 |

[FIG. 20-A]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| positive genetic polymorphism sets | 3 | 8 | 12 | PONA2 | 30 | 12 | FGB3 | 31 | 3 | APE2 | 15.08 | 5.33 |
| | 3 | 3 | 3 | ACE ID | 33 | 3 | MCP1 | 67 | 12 | 1A2 | 13.29 | 4.44 |
| | 2 | 53 | 1 | β2AR-1 | 70 | 12 | APM2 | | | | 11.84 | 3.36 |
| | 3 | 8 | 12 | PONA2 | 23 | 3 | IRS-1 | 25 | 12 | PAI | 10.1 | 3.09 |
| | 3 | 8 | 12 | PONA2 | 26 | 1 | NOS3 | 45 | 1 | LPL3 | 9.25 | 3.58 |
| | 3 | 3 | 3 | ACE ID | 42 | 1 | GCLM1 | 50 | 23 | β2AR4 | 8.89 | 11.41 |
| | 3 | 29 | 12 | G1A3 | 37 | 1 | GP1ba | 78 | 12 | ET1 | 8.85 | 5.17 |
| | 3 | 12 | 12 | Tbm3 | 84 | 1 | IL1B2 | 85 | 3 | 5178 | 8.84 | 3.04 |
| | 3 | 3 | 3 | ACE ID | 37 | 1 | GP1ba | 50 | 23 | β2AR4 | 8.79 | 6.71 |
| | 3 | 3 | 3 | ACE ID | 19 | 12 | MMP9 | 50 | 23 | β2AR4 | 8.75 | 5.13 |
| | 3 | 1 | 3 | ABCA1 | 38 | 12 | PONA1 | 80 | 3 | CCR2 | 8.42 | 3.74 |
| | 3 | 12 | 12 | Tbm3 | 21 | 1 | CX37 | 85 | 3 | 5178 | 8.31 | 3.14 |
| | 2 | 3 | 3 | ACE ID | 12 | 12 | Tbm3 | | | | 8.05 | 3.64 |
| | 3 | 1 | 3 | ABCA1 | 53 | 1 | β2AR-1 | 85 | 3 | 5178 | 7.87 | 6.2 |
| | 3 | 1 | 3 | ABCA1 | 10 | 3 | GP3A | 53 | 1 | β2AR-1 | 7.76 | 3.58 |
| | 3 | 3 | 3 | ACE ID | 38 | 12 | PONA1 | 68 | 1 | BKR1 | 7.43 | 3.95 |
| | 3 | 29 | 12 | G1A3 | 42 | 1 | GCLM1 | 78 | 12 | ET1 | 7.42 | 4.62 |
| | 3 | 51 | 3 | GP1a1 | 86 | 1 | IL-181 | 89 | 3 | IL102 | 7.25 | 99 |
| | 3 | 1 | 3 | ABCA1 | 80 | 3 | CCR2 | 89 | 3 | IL102 | 7.14 | 3.84 |
| | 3 | 77 | 3 | APM12 | 94 | 12 | CYP3A4 | 96 | 1 | LRP1 | 7.14 | 3.13 |
| | 3 | 12 | 12 | Tbm3 | 53 | 1 | β2AR-1 | 84 | 1 | IL1B2 | 7.12 | 5.77 |
| | 3 | 1 | 3 | ABCA1 | 37 | 1 | GP1ba | 77 | 3 | APM12 | 6.94 | 99 |
| | 3 | 12 | 12 | Tbm3 | 40 | 3 | FGB3 | 68 | 1 | BKR1 | 6.87 | 4.43 |
| | 3 | 55 | 12 | MPO | 79 | 12 | RAGE3 | 93 | 23 | TPO1 | 6.84 | 4.42 |
| | 3 | 51 | 3 | GP1a1 | 62 | 1 | APE3 | 86 | 1 | IL-181 | 6.78 | 4.37 |
| | 1 | 20 | 3 | GSY | | | | | | | 4.52 | 99 |
| | 3 | 1 | 3 | ABCA1 | 3 | 3 | ACE ID | 12 | 12 | Tbm3 | 6.8 | 99 |
| | 3 | 1 | 3 | ABCA1 | 28 | 12 | AGT1 | 53 | 1 | β2AR-1 | 7.17 | 3.43 |
| | 3 | 1 | 3 | ABCA1 | 37 | 1 | GP1ba | 53 | 1 | β2AR-1 | 6.88 | 4.41 |
| | 3 | 3 | 3 | ACE ID | 7 | 1 | N7 | 50 | 23 | β2AR4 | 7.42 | 4.62 |
| | 3 | 3 | 3 | ACE ID | 8 | 12 | PONA2 | 31 | 3 | APE2 | 8.75 | 5.13 |
| | 3 | 3 | 3 | ACE ID | 8 | 12 | PONA2 | 32 | 23 | MTHFR | 9.21 | 6.94 |
| | 3 | 3 | 3 | ACE ID | 13 | 12 | CF12 | 50 | 23 | β2AR4 | 8.24 | 10.71 |
| | 3 | 3 | 3 | ACE ID | 25 | 12 | PAI | 50 | 23 | β2AR4 | 8.6 | 6.61 |
| | 3 | 3 | 3 | ACE ID | 40 | 3 | FGB3 | 50 | 23 | β2AR4 | 6.72 | 5.57 |
| | 2 | 3 | 3 | ACE ID | 50 | 23 | β2AR4 | | | | 8.72 | 5.11 |
| | 3 | 8 | 12 | PONA2 | 10 | 3 | GP3A | 31 | 3 | APE2 | 13.32 | 4.42 |
| | 3 | 8 | 12 | PONA2 | 10 | 3 | GP3A | 67 | 12 | 1A2 | 7.66 | 3.26 |
| | 3 | 8 | 12 | PONA2 | 15 | 12 | MMP-12 | 31 | 3 | APE2 | 13.32 | 4.42 |
| | 3 | 8 | 12 | PONA2 | 15 | 12 | MMP-12 | 67 | 12 | 1A2 | 7.66 | 3.26 |
| | 3 | 8 | 12 | PONA2 | 23 | 3 | IRS-1 | 31 | 3 | APE2 | 12.84 | 4.32 |
| | 3 | 8 | 12 | PONA2 | 23 | 3 | IRS-1 | 67 | 12 | 1A2 | 8.69 | 3.82 |
| | 3 | 8 | 12 | PONA2 | 26 | 1 | NOS3 | 31 | 3 | APE2 | 10.83 | 4.32 |
| | 3 | 8 | 12 | PONA2 | 26 | 1 | NOS3 | 32 | 23 | MTHFR | 7.92 | 4.07 |
| | 3 | 8 | 12 | PONA2 | 30 | 12 | FGB3 | 67 | 12 | 1A2 | 7.66 | 3.26 |
| | 3 | 8 | 12 | PONA2 | 30 | 12 | FGB3 | 85 | 3 | 5178 | 7.58 | 3.22 |
| | 2 | 8 | 12 | PONA2 | 31 | 3 | APE2 | | | | 13.32 | 4.42 |

[FIG. 20-B]

| | | | | | | | | | | | | |
|---|---|---|---|---|---|---|---|---|---|---|---|---|
| | 3 | 8 | 12 | PONA2 | 32 | 23 | MTHFR | 85 | 3 | 5178 | 7.09 | 4.48 |
| | 3 | 8 | 12 | PONA2 | 32 | 23 | MTHFR | 93 | 23 | TPO1 | 7.49 | 3.95 |
| | 3 | 8 | 12 | PONA2 | 38 | 12 | PONA1 | 67 | 12 | 1A2 | 7.7 | 3.27 |
| | 3 | 8 | 12 | PONA2 | 63 | 12 | HANP2 | 67 | 12 | 1A2 | 7.66 | 3.26 |
| | 2 | 8 | 12 | PONA2 | 67 | 12 | 1A2 | | | | 7.66 | 3.26 |
| | 3 | 8 | 12 | PONA2 | 90 | 3 | IL-182 | 93 | 23 | TPO1 | 8.38 | 4.22 |
| | 3 | 12 | 12 | Tbm3 | 70 | 12 | APM2 | 85 | 3 | 5178 | 7.75 | 4.73 |
| | 3 | 29 | 12 | G1A3 | 37 | 1 | GP1ba | 38 | 12 | PONA1 | 7.32 | 9.74 |
| | 2 | 29 | 12 | G1A3 | 38 | 12 | PONA1 | | | | 7.24 | 5.86 |
| | 3 | 29 | 12 | G1A3 | 62 | 1 | APE3 | 78 | 12 | ET1 | 6.97 | 3.38 |
| | 3 | 29 | 12 | G1A3 | 67 | 12 | 1A2 | 96 | 1 | LRP1 | 6.94 | 99 |
| | 3 | 29 | 12 | G1A3 | 78 | 12 | ET1 | 93 | 23 | TPO1 | 7.31 | 3.9 |
| | 3 | 53 | 1 | β2AR-1 | 56 | 23 | HANP1 | 70 | 12 | APM2 | 11.07 | 3.24 |
| | 3 | 53 | 1 | β2AR-1 | 62 | 1 | APE3 | 70 | 12 | APM2 | 11.32 | 3.71 |
| | 3 | 53 | 1 | β2AR-1 | 67 | 12 | 1A2 | 70 | 12 | APM2 | 9.01 | 4.4 |
| | 2 | 55 | 12 | MPO | 69 | 3 | CD181 | | | | 4.49 | 99 |
| | 2 | 68 | 1 | BKR1 | 77 | 3 | APM12 | | | | 4.5 | 99 |

[FIG. 21]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| positive genetic polymorphism sets | 3 | 8 | 12 | PONA2 | 30 | 12 | FGB3 | 31 | 3 | APE2 | 15.08 | 5.33 |
| | 3 | 3 | 3 | ACE ID | 33 | 3 | MCP1 | 67 | 12 | 1A2 | 13.29 | 4.44 |
| | 3 | 3 | 3 | ACE ID | 39 | 3 | CRP1 | 50 | 23 | β 2AR4 | 9.67 | 7.19 |
| | 3 | 1 | 3 | ABCA1 | 80 | 3 | CCR2 | 85 | 12 | 5178 | 9.28 | 11.79 |
| | 3 | 3 | 3 | ACE ID | 8 | 12 | PONA2 | 32 | 23 | MTHFR | 9.21 | 6.94 |
| | 2 | 23 | 12 | IRS-1 | 74 | 12 | LTA1 | | | | 9.06 | 11.57 |
| | 3 | 53 | 1 | β 2AR-1 | 67 | 12 | 1A2 | 70 | 12 | APM2 | 9.01 | 4.4 |
| | 3 | 3 | 3 | ACE ID | 42 | 1 | GCLM1 | 50 | 23 | β 2AR4 | 8.89 | 11.41 |
| | 3 | 33 | 3 | MCP1 | 42 | 1 | GCLM1 | 75 | 1 | LTA2 | 8.87 | 6.77 |
| | 3 | 29 | 12 | G1A3 | 37 | 1 | GP1ba | 78 | 12 | ET1 | 8.85 | 5.17 |
| | 3 | 1 | 3 | ABCA1 | 25 | 1 | PAI | 53 | 1 | β 2AR-1 | 8.58 | 99 |
| | 3 | 29 | 12 | G1A3 | 39 | 3 | CRP1 | 78 | 12 | ET1 | 8.48 | 4.24 |
| | 3 | 8 | 12 | PONA2 | 90 | 3 | IL-182 | 93 | 23 | TPO1 | 8.38 | 4.22 |
| | 3 | 33 | 3 | MCP1 | 67 | 12 | 1A2 | 75 | 1 | LTA2 | 7.2 | 9.63 |
| | 3 | 12 | 12 | Tbm3 | 53 | 1 | β 2AR-1 | 84 | 1 | IL1B2 | 7.12 | 5.77 |
| | 3 | 1 | 3 | ABCA1 | 3 | 3 | ACE ID | 88 | 1 | VWF2 | 6.99 | 9.38 |
| | 3 | 1 | 3 | ABCA1 | 37 | 1 | GP1ba | 77 | 3 | APM12 | 6.94 | 99 |
| | 3 | 1 | 3 | ABCA1 | 37 | 1 | GP1ba | 53 | 1 | β 2AR-1 | 6.88 | 4.41 |
| | 3 | 12 | 12 | Tbm3 | 40 | 3 | FGB3 | 68 | 1 | BKR1 | 6.87 | 4.43 |
| | 3 | 55 | 12 | MPO | 79 | 12 | RAGE3 | 93 | 23 | TPO1 | 6.84 | 4.42 |
| | 3 | 3 | 3 | ACE ID | 67 | 12 | 1A2 | 88 | 1 | VWF2 | 6.81 | 4.38 |
| | 3 | 51 | 3 | GP1a1 | 71 | 23 | REG1 | 86 | 1 | IL-181 | 6.78 | 4.37 |
| | 2 | 12 | 12 | Tbm3 | 75 | 1 | LTA2 | | | | 6.56 | 99 |
| | 2 | 12 | 12 | Tbm3 | 54 | 3 | IL62 | | | | 6.49 | 99 |
| | 2 | 36 | 3 | FGA1 | 54 | 3 | IL62 | | | | 4.72 | 99 |
| | 1 | 20 | 3 | GSY | | | | | | | 4.52 | 99 |
| | 2 | 68 | 1 | BKR1 | 77 | 3 | APM12 | | | | 4.5 | 99 |
| | 2 | 55 | 12 | MPO | 69 | 3 | CD181 | | | | 4.49 | 99 |
| | 3 | 1 | 3 | ABCA1 | 3 | 3 | ACE ID | 12 | 12 | Tbm3 | 6.8 | 99 |
| | 3 | 3 | 3 | ACE ID | 8 | 12 | PONA2 | 31 | 3 | APE2 | 8.75 | 5.13 |
| | 3 | 3 | 3 | ACE ID | 37 | 1 | GP1ba | 50 | 23 | β 2AR4 | 8.79 | 6.71 |
| | 3 | 8 | 12 | PONA2 | 10 | 3 | GP3A | 31 | 3 | APE2 | 13.32 | 4.42 |
| | 3 | 8 | 12 | PONA2 | 15 | 12 | MMP-12 | 31 | 3 | APE2 | 13.32 | 4.42 |
| | 3 | 8 | 12 | PONA2 | 21 | 12 | CX37 | 31 | 3 | APE2 | 12.37 | 4.22 |
| | 3 | 8 | 12 | PONA2 | 24 | 12 | AGTR1-3 | 31 | 3 | APE2 | 14.58 | 5.21 |
| | 3 | 8 | 12 | PONA2 | 26 | 1 | NOS3 | 31 | 3 | APE2 | 10.83 | 4.32 |
| | 2 | 8 | 12 | PONA2 | 31 | 3 | APE2 | | | | 13.32 | 4.42 |
| | 2 | 23 | 12 | IRS-1 | 25 | 1 | PAI | | | | 6.38 | 99 |
| | 3 | 23 | 12 | IRS-1 | 27 | 23 | N10 | 56 | 3 | HANP1 | 6.74 | 9.11 |
| | 3 | 23 | 12 | IRS-1 | 27 | 23 | N10 | 74 | 12 | LTA1 | 6.67 | 9.04 |
| | 3 | 23 | 12 | IRS-1 | 27 | 23 | N10 | 84 | 1 | IL1B2 | 6.79 | 99 |
| | 3 | 23 | 12 | IRS-1 | 27 | 23 | N10 | 94 | 12 | CYP3A4 | 6.65 | 9.02 |
| | 3 | 23 | 12 | IRS-1 | 28 | 12 | AGT1 | 74 | 12 | LTA1 | 9 | 11.51 |
| | 3 | 29 | 12 | G1A3 | 37 | 1 | GP1ba | 38 | 12 | PONA1 | 7.32 | 9.74 |
| | 2 | 29 | 12 | G1A3 | 38 | 12 | PONA1 | | | | 7.24 | 5.86 |
| | 3 | 29 | 12 | G1A3 | 42 | 1 | GCLM1 | 78 | 12 | ET1 | 7.42 | 4.62 |
| | 3 | 29 | 12 | G1A3 | 67 | 12 | 1A2 | 96 | 1 | LRP1 | 6.94 | 99 |

[FIG. 22]

positive genetic polymorphism sets

| Num_SNP | SNP-No | Genotype | Gene code | SNP-No | Genotype | Gene code | SNP-No | Genotype | Gene code | Kai | Odds |
|---|---|---|---|---|---|---|---|---|---|---|---|
| 3 | 8 | 12 | PONA2 | 30 | 12 | FGB3 | 31 | 3 | APE2 | 15.08 | 5.33 |
| 3 | 3 | 3 | ACE ID | 39 | 3 | CRP1 | 50 | 23 | β2AR4 | 9.67 | 7.19 |
| 3 | 3 | 3 | ACE ID | 8 | 12 | PONA2 | 32 | 23 | MTHFR | 9.21 | 6.94 |
| 2 | 23 | 12 | IRS-1 | 75 | 23 | LTA2 | | | | 9.16 | 11.68 |
| 3 | 3 | 3 | ACE ID | 42 | 1 | GCLM1 | 50 | 23 | β2AR4 | 8.89 | 11.41 |
| 3 | 29 | 12 | G1A3 | 37 | 1 | GP1ba | 78 | 12 | ET1 | 8.85 | 5.17 |
| 3 | 1 | 3 | ABCA1 | 25 | 1 | PAI | 53 | 1 | β2AR-1 | 8.58 | 99 |
| 3 | 55 | 12 | MPO | 86 | 3 | IL-181 | 96 | 1 | LRP1 | 7.71 | 6.14 |
| 2 | 29 | 12 | G1A3 | 38 | 12 | PONA1 | | | | 7.24 | 5.86 |
| 3 | 12 | 12 | Tbm3 | 53 | 1 | β2AR-1 | 84 | 1 | IL1B2 | 7.12 | 5.77 |
| 3 | 1 | 3 | ABCA1 | 3 | 3 | ACE ID | 88 | 1 | VWF2 | 6.99 | 9.38 |
| 3 | 1 | 3 | ABCA1 | 37 | 1 | GP1ba | 77 | 3 | APM12 | 6.94 | 99 |
| 3 | 36 | 3 | FGA1 | 93 | 1 | TPO1 | 99 | 23 | PGC12 | 6.88 | 5.67 |
| 3 | 1 | 3 | ABCA1 | 21 | 23 | CX37 | 67 | 3 | 1A2 | 6.84 | 99 |
| 3 | 1 | 3 | ABCA1 | 86 | 3 | IL-181 | 89 | 12 | IL102 | 6.82 | 99 |
| 3 | 1 | 3 | ABCA1 | 3 | 3 | ACE ID | 12 | 12 | Tbm3 | 6.8 | 99 |
| 2 | 12 | 12 | Tbm3 | 54 | 3 | IL62 | | | | 6.49 | 99 |
| 2 | 79 | 1 | RAGE3 | 90 | 3 | IL-182 | | | | 5.7 | 99 |
| 2 | 36 | 3 | FGA1 | 54 | 3 | IL62 | | | | 4.72 | 99 |
| 1 | 20 | 3 | GSY | | | | | | | 4.52 | 99 |
| 2 | 68 | 1 | BKR1 | 77 | 3 | APM12 | | | | 4.5 | 99 |
| 2 | 55 | 12 | MPO | 69 | 3 | CD181 | | | | 4.49 | 99 |
| 2 | 12 | 12 | Tbm3 | 80 | 1 | CCR2 | | | | 4.1 | 99 |
| 3 | 3 | 3 | ACE ID | 8 | 12 | PONA2 | 31 | 3 | APE2 | 8.75 | 5.13 |
| 3 | 3 | 3 | ACE ID | 37 | 1 | GP1ba | 50 | 23 | β2AR4 | 8.79 | 6.71 |
| 2 | 3 | 3 | ACE ID | 79 | 1 | RAGE3 | | | | 4.77 | 99 |
| 2 | 23 | 12 | IRS-1 | 25 | 1 | PAI | | | | 6.38 | 99 |
| 3 | 23 | 12 | IRS-1 | 27 | 23 | N10 | 56 | 3 | HANP1 | 6.74 | 9.11 |
| 3 | 23 | 12 | IRS-1 | 27 | 23 | N10 | 74 | 12 | LTA1 | 6.67 | 9.04 |
| 3 | 23 | 12 | IRS-1 | 27 | 23 | N10 | 75 | 23 | LTA2 | 6.75 | 9.13 |
| 3 | 23 | 12 | IRS-1 | 27 | 23 | N10 | 84 | 1 | IL1B2 | 6.79 | 99 |
| 3 | 23 | 12 | IRS-1 | 27 | 23 | N10 | 94 | 12 | CYP3A4 | 6.65 | 9.02 |
| 3 | 23 | 12 | IRS-1 | 27 | 23 | N10 | 98 | 23 | PGC11 | 6.95 | 99 |
| 3 | 23 | 12 | IRS-1 | 28 | 12 | AGT1 | 74 | 12 | LTA1 | 9 | 11.51 |
| 3 | 23 | 12 | IRS-1 | 28 | 12 | AGT1 | 75 | 23 | LTA2 | 9.1 | 11.62 |
| 3 | 23 | 12 | IRS-1 | 44 | 3 | CETP4 | 74 | 12 | LTA1 | 9.06 | 11.57 |
| 3 | 23 | 12 | IRS-1 | 44 | 3 | CETP4 | 75 | 23 | LTA2 | 9.16 | 11.68 |
| 3 | 23 | 12 | IRS-1 | 47 | 3 | TNFα1 | 74 | 12 | LTA1 | 9.06 | 11.57 |
| 3 | 23 | 12 | IRS-1 | 47 | 3 | TNFα1 | 75 | 23 | LTA2 | 9.16 | 11.68 |
| 3 | 23 | 12 | IRS-1 | 70 | 23 | APM2 | 74 | 12 | LTA1 | 8.6 | 11.09 |
| 3 | 23 | 12 | IRS-1 | 70 | 23 | APM2 | 75 | 23 | LTA2 | 8.7 | 11.2 |
| 2 | 23 | 12 | IRS-1 | 74 | 12 | LTA1 | | | | 9.06 | 11.57 |
| 3 | 29 | 12 | G1A3 | 37 | 1 | GP1ba | 38 | 12 | PONA1 | 7.32 | 9.74 |

[FIG. 23]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| positive genetic polymorphism sets | 3 | 3 | 3 | ACE ID | 39 | 3 | CRP1 | 50 | 23 | β 2AR4 | 9.67 | 7.19 |
| | 3 | 23 | 12 | IRS-1 | 51 | 12 | GP1a1 | 75 | 23 | LTA2 | 9.53 | 99 |
| | 3 | 3 | 3 | ACE ID | 8 | 12 | PONA2 | 32 | 23 | MTHFR | 9.21 | 6.94 |
| | 2 | 23 | 12 | IRS-1 | 75 | 23 | LTA2 | | | | 9.16 | 11.68 |
| | 3 | 3 | 3 | ACE ID | 42 | 1 | GCLM1 | 50 | 23 | β 2AR4 | 8.89 | 11.41 |
| | 3 | 1 | 3 | ABCA1 | 25 | 1 | PAI | 53 | 1 | β 2AR-1 | 8.58 | 99 |
| | 3 | 1 | 3 | ABCA1 | 53 | 1 | β 2AR-1 | 85 | 3 | 5178 | 7.87 | 6.2 |
| | 3 | 23 | 12 | IRS-1 | 28 | 12 | AGT1 | 51 | 12 | GP1a1 | 7.86 | 10.29 |
| | 3 | 55 | 12 | MPO | 86 | 3 | IL-181 | 96 | 1 | LRP1 | 7.71 | 6.14 |
| | 3 | 29 | 12 | G1A3 | 37 | 1 | GP1ba | 38 | 12 | PONA1 | 7.32 | 9.74 |
| | 3 | 1 | 3 | ABCA1 | 3 | 3 | ACE ID | 88 | 1 | VWF2 | 6.99 | 9.38 |
| | 3 | 1 | 3 | ABCA1 | 37 | 1 | GP1ba | 77 | 3 | APM12 | 6.94 | 99 |
| | 3 | 29 | 12 | G1A3 | 67 | 12 | 1A2 | 96 | 1 | LRP1 | 6.94 | 99 |
| | 3 | 29 | 12 | G1A3 | 33 | 23 | MCP1 | 78 | 1 | ET1 | 6.88 | 9.27 |
| | 3 | 1 | 3 | ABCA1 | 86 | 3 | IL-181 | 89 | 12 | IL102 | 6.82 | 99 |
| | 3 | 1 | 3 | ABCA1 | 3 | 3 | ACE ID | 12 | 12 | Tbm3 | 6.8 | 99 |
| | 3 | 12 | 12 | Tbm3 | 45 | 23 | LPL3 | 67 | 12 | 1A2 | 6.66 | 99 |
| | 2 | 12 | 12 | Tbm3 | 54 | 3 | IL62 | | | | 6.49 | 99 |
| | 2 | 79 | 1 | RAGE3 | 90 | 3 | IL-182 | | | | 5.7 | 99 |
| | 1 | 20 | 3 | GSY | | | | | | | 4.52 | 99 |
| | 2 | 68 | 1 | BKR1 | 77 | 3 | APM12 | | | | 4.5 | 99 |
| | 2 | 55 | 12 | MPO | 69 | 3 | CD181 | | | | 4.49 | 99 |
| | 2 | 12 | 12 | Tbm3 | 80 | 1 | CCR2 | | | | 4.1 | 99 |
| | 3 | 3 | 3 | ACE ID | 37 | 1 | GP1ba | 50 | 23 | β 2AR4 | 8.79 | 6.71 |
| | 2 | 3 | 3 | ACE ID | 79 | 1 | RAGE3 | | | | 4.77 | 99 |
| | 2 | 23 | 12 | IRS-1 | 25 | 1 | PAI | | | | 6.38 | 99 |
| | 3 | 23 | 12 | IRS-1 | 27 | 23 | N10 | 51 | 12 | GP1a1 | 7.36 | 99 |
| | 3 | 23 | 12 | IRS-1 | 27 | 23 | N10 | 56 | 3 | HANP1 | 6.74 | 9.11 |
| | 3 | 23 | 12 | IRS-1 | 27 | 23 | N10 | 74 | 12 | LTA1 | 6.67 | 9.04 |
| | 3 | 23 | 12 | IRS-1 | 27 | 23 | N10 | 75 | 23 | LTA2 | 6.75 | 9.13 |
| | 3 | 23 | 12 | IRS-1 | 27 | 23 | N10 | 84 | 1 | IL1B2 | 6.79 | 99 |
| | 3 | 23 | 12 | IRS-1 | 27 | 23 | N10 | 94 | 12 | CYP3A4 | 6.65 | 9.02 |
| | 3 | 23 | 12 | IRS-1 | 27 | 23 | N10 | 98 | 23 | PGC11 | 6.95 | 99 |
| | 3 | 23 | 12 | IRS-1 | 28 | 12 | AGT1 | 74 | 12 | LTA1 | 9 | 11.51 |
| | 3 | 23 | 12 | IRS-1 | 28 | 12 | AGT1 | 75 | 23 | LTA2 | 9.1 | 11.62 |
| | 3 | 23 | 12 | IRS-1 | 44 | 3 | CETP4 | 74 | 12 | LTA1 | 9.06 | 11.57 |
| | 3 | 23 | 12 | IRS-1 | 44 | 3 | CETP4 | 75 | 23 | LTA2 | 9.16 | 11.68 |
| | 3 | 23 | 12 | IRS-1 | 47 | 3 | TNF α 1 | 74 | 12 | LTA1 | 9.06 | 11.57 |
| | 3 | 23 | 12 | IRS-1 | 47 | 3 | TNF α 1 | 75 | 23 | LTA2 | 9.16 | 11.68 |
| | 3 | 23 | 12 | IRS-1 | 51 | 12 | GP1a1 | 70 | 23 | APM2 | 6.99 | 9.37 |
| | 3 | 23 | 12 | IRS-1 | 51 | 12 | GP1a1 | 74 | 12 | LTA1 | 9.44 | 99 |
| | 3 | 23 | 12 | IRS-1 | 70 | 23 | APM2 | 74 | 12 | LTA1 | 8.6 | 11.09 |
| | 3 | 23 | 12 | IRS-1 | 70 | 23 | APM2 | 75 | 23 | LTA2 | 8.7 | 11.2 |
| | 2 | 23 | 12 | IRS-1 | 74 | 12 | LTA1 | | | | 9.06 | 11.57 |

[FIG. 24]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| positive genetic polymorphism sets | 3 | 1 | 3 | ABCA1 | 3 | 3 | ACE ID | 27 | 3 | N10 | 10.24 | 99 |
| | 3 | 3 | 3 | ACE ID | 75 | 3 | LTA2 | 87 | 23 | MS1 | 10.02 | 12.6 |
| | 3 | 3 | 3 | ACE ID | 39 | 3 | CRP1 | 50 | 23 | β2AR4 | 9.67 | 7.19 |
| | 3 | 23 | 12 | IRS-1 | 51 | 12 | GP1a1 | 74 | 12 | LTA1 | 9.44 | 99 |
| | 2 | 23 | 12 | IRS-1 | 74 | 12 | LTA1 | | | | 9.06 | 11.57 |
| | 3 | 23 | 12 | IRS-1 | 51 | 12 | GP1a1 | 53 | 12 | β2AR-1 | 9.02 | 99 |
| | 3 | 3 | 3 | ACE ID | 42 | 1 | GCLM1 | 50 | 23 | β2AR4 | 8.89 | 11.41 |
| | 3 | 1 | 3 | ABCA1 | 69 | 1 | CD181 | 77 | 3 | APM12 | 7.46 | 99 |
| | 3 | 29 | 12 | G1A3 | 37 | 1 | GP1ba | 38 | 12 | PONA1 | 7.32 | 9.74 |
| | 3 | 1 | 3 | ABCA1 | 3 | 3 | ACE ID | 88 | 1 | VWF2 | 6.99 | 9.38 |
| | 3 | 1 | 3 | ABCA1 | 37 | 1 | GP1ba | 77 | 3 | APM12 | 6.94 | 99 |
| | 3 | 1 | 3 | ABCA1 | 21 | 23 | CX37 | 67 | 3 | 1A2 | 6.84 | 99 |
| | 3 | 1 | 3 | ABCA1 | 3 | 3 | ACE ID | 12 | 12 | Tbm3 | 6.8 | 99 |
| | 2 | 12 | 12 | Tbm3 | 54 | 3 | IL62 | | | | 6.49 | 99 |
| | 2 | 79 | 1 | RAGE3 | 90 | 3 | IL-182 | | | | 5.7 | 99 |
| | 2 | 3 | 3 | ACE ID | 79 | 1 | RAGE3 | | | | 4.77 | 99 |
| | 2 | 36 | 3 | FGA1 | 54 | 3 | IL62 | | | | 4.72 | 99 |
| | 1 | 20 | 3 | GSY | | | | | | | 4.52 | 99 |
| | 2 | 68 | 1 | BKR1 | 77 | 3 | APM12 | | | | 4.5 | 99 |
| | 2 | 12 | 12 | Tbm3 | 80 | 1 | CCR2 | | | | 4.1 | 99 |
| | 2 | 23 | 12 | IRS-1 | 25 | 1 | PAI | | | | 6.38 | 99 |
| | 3 | 23 | 12 | IRS-1 | 28 | 12 | AGT1 | 74 | 12 | LTA1 | 9 | 11.51 |
| | 3 | 23 | 12 | IRS-1 | 44 | 3 | CETP4 | 74 | 12 | LTA1 | 9.06 | 11.57 |
| | 3 | 23 | 12 | IRS-1 | 47 | 3 | TNFα1 | 74 | 12 | LTA1 | 9.06 | 11.57 |
| | 3 | 23 | 12 | IRS-1 | 53 | 12 | β2AR-1 | 74 | 12 | LTA1 | 7.12 | 9.51 |
| | 3 | 23 | 12 | IRS-1 | 70 | 23 | APM2 | 74 | 12 | LTA1 | 8.6 | 11.09 |

[FIG. 25]

| | Num_SNP | SNP-No | Genotype | Gene code | SNP-No | Genotype | Gene code | SNP-No | Genotype | Gene code | Kai | Odds |
|---|---|---|---|---|---|---|---|---|---|---|---|---|
| positive genetic polymorphism sets | 3 | 1 | 3 | ABCA1 | 3 | 3 | ACE ID | 27 | 3 | N10 | 10.24 | 99 |
| | 3 | 23 | 12 | IRS-1 | 51 | 12 | GP1a1 | 75 | 23 | LTA2 | 9.53 | 99 |
| | 2 | 23 | 12 | IRS-1 | 75 | 23 | LTA2 | | | | 9.16 | 11.68 |
| | 3 | 23 | 12 | IRS-1 | 51 | 12 | GP1a1 | 53 | 12 | β2AR-1 | 9.02 | 99 |
| | 3 | 3 | 3 | ACE ID | 42 | 1 | GCLM1 | 50 | 23 | β2AR4 | 8.89 | 11.41 |
| | 3 | 3 | 3 | ACE ID | 13 | 12 | CF12 | 50 | 23 | β2AR4 | 8.24 | 10.71 |
| | 3 | 29 | 12 | G1A3 | 37 | 1 | GP1ba | 38 | 12 | PONA1 | 7.32 | 9.74 |
| | 3 | 1 | 3 | ABCA1 | 3 | 3 | ACE ID | 88 | 1 | VWF2 | 6.99 | 9.38 |
| | 3 | 1 | 3 | ABCA1 | 37 | 1 | GP1ba | 77 | 3 | APM12 | 6.94 | 99 |
| | 3 | 29 | 12 | G1A3 | 67 | 12 | 1A2 | 96 | 1 | LRP1 | 6.94 | 99 |
| | 3 | 1 | 3 | ABCA1 | 3 | 3 | ACE ID | 12 | 12 | Tbm3 | 6.8 | 99 |
| | 3 | 12 | 12 | Tbm3 | 45 | 23 | LPL3 | 67 | 12 | 1A2 | 6.66 | 99 |
| | 2 | 12 | 12 | Tbm3 | 54 | 3 | IL62 | | | | 6.49 | 99 |
| | 2 | 79 | 1 | RAGE3 | 90 | 3 | IL-182 | | | | 5.7 | 99 |
| | 2 | 3 | 3 | ACE ID | 79 | 1 | RAGE3 | | | | 4.77 | 99 |
| | 2 | 36 | 3 | FGA1 | 54 | 3 | IL62 | | | | 4.72 | 99 |
| | 1 | 20 | 3 | GSY | | | | | | | 4.52 | 99 |
| | 2 | 68 | 1 | BKR1 | 77 | 3 | APM12 | | | | 4.5 | 99 |
| | 2 | 23 | 12 | IRS-1 | 25 | 1 | PAI | | | | 6.38 | 99 |
| | 3 | 23 | 12 | IRS-1 | 28 | 12 | AGT1 | 74 | 12 | LTA1 | 9 | 11.51 |
| | 3 | 23 | 12 | IRS-1 | 28 | 12 | AGT1 | 75 | 23 | LTA2 | 9.1 | 11.62 |
| | 3 | 23 | 12 | IRS-1 | 39 | 23 | CRP1 | 74 | 12 | LTA1 | 9.06 | 11.57 |
| | 3 | 23 | 12 | IRS-1 | 39 | 23 | CRP1 | 75 | 23 | LTA2 | 9.16 | 11.68 |
| | 3 | 23 | 12 | IRS-1 | 44 | 3 | CETP4 | 74 | 12 | LTA1 | 9.06 | 11.57 |
| | 3 | 23 | 12 | IRS-1 | 44 | 3 | CETP4 | 75 | 23 | LTA2 | 9.16 | 11.68 |
| | 3 | 23 | 12 | IRS-1 | 47 | 3 | TNFα1 | 74 | 12 | LTA1 | 9.06 | 11.57 |
| | 3 | 23 | 12 | IRS-1 | 47 | 3 | TNFα1 | 75 | 23 | LTA2 | 9.16 | 11.68 |
| | 3 | 23 | 12 | IRS-1 | 51 | 12 | GP1a1 | 74 | 12 | LTA1 | 9.44 | 99 |
| | 3 | 23 | 12 | IRS-1 | 53 | 12 | β2AR-1 | 74 | 12 | LTA1 | 7.12 | 9.51 |
| | 3 | 23 | 12 | IRS-1 | 53 | 12 | β2AR-1 | 75 | 23 | LTA2 | 7.2 | 9.61 |
| | 3 | 23 | 12 | IRS-1 | 70 | 23 | APM2 | 74 | 12 | LTA1 | 8.6 | 11.09 |
| | 3 | 23 | 12 | IRS-1 | 70 | 23 | APM2 | 75 | 23 | LTA2 | 8.7 | 11.2 |
| | 2 | 23 | 12 | IRS-1 | 74 | 12 | LTA1 | | | | 9.06 | 11.57 |
| | 2 | 23 | 12 | IRS-1 | 80 | 12 | CCR2 | | | | 5.55 | 99 |

[FIG. 26]

| | | | | | | | | | | | | |
|---|---|---|---|---|---|---|---|---|---|---|---|---|
| positive genetic polymorphism sets | 3 | 1 | 3 | ABCA1 | 3 | 3 | ACE ID | 27 | 3 | N10 | 10.24 | 99 |
| | 3 | 23 | 12 | IRS-1 | 51 | 12 | GP1a1 | 75 | 23 | LTA2 | 9.53 | 99 |
| | 2 | 23 | 12 | IRS-1 | 75 | 23 | LTA2 | | | | 9.16 | 11.68 |
| | 3 | 23 | 12 | IRS-1 | 51 | 12 | GP1a1 | 53 | 12 | β2AR-1 | 9.02 | 99 |
| | 3 | 3 | 3 | ACE ID | 42 | 1 | GCLM1 | 50 | 23 | β2AR4 | 8.89 | 11.41 |
| | 3 | 3 | 3 | ACE ID | 13 | 12 | CF12 | 50 | 23 | β2AR4 | 8.24 | 10.71 |
| | 3 | 29 | 12 | G1A3 | 37 | 1 | GP1ba | 38 | 12 | PONA1 | 7.32 | 9.74 |
| | 3 | 1 | 3 | ABCA1 | 3 | 3 | ACE ID | 88 | 1 | VWF2 | 6.99 | 9.38 |
| | 3 | 1 | 3 | ABCA1 | 37 | 1 | GP1ba | 77 | 3 | APM12 | 6.94 | 99 |
| | 3 | 29 | 12 | G1A3 | 67 | 12 | 1A2 | 96 | 1 | LRP1 | 6.94 | 99 |
| | 3 | 1 | 3 | ABCA1 | 3 | 3 | ACE ID | 12 | 12 | Tbm3 | 6.8 | 99 |
| | 3 | 12 | 12 | Tbm3 | 45 | 23 | LPL3 | 67 | 12 | 1A2 | 6.66 | 99 |
| | 2 | 12 | 12 | Tbm3 | 54 | 3 | IL62 | | | | 6.49 | 99 |
| | 2 | 79 | 1 | RAGE3 | 90 | 3 | IL-182 | | | | 5.7 | 99 |
| | 2 | 3 | 3 | ACE ID | 79 | 1 | RAGE3 | | | | 4.77 | 99 |
| | 2 | 36 | 3 | FGA1 | 54 | 3 | IL62 | | | | 4.72 | 99 |
| | 1 | 20 | 3 | GSY | | | | | | | 4.52 | 99 |
| | 2 | 68 | 1 | BKR1 | 77 | 3 | APM12 | | | | 4.5 | 99 |
| | 2 | 23 | 12 | IRS-1 | 25 | 1 | PAI | | | | 6.38 | 99 |
| | 3 | 23 | 12 | IRS-1 | 28 | 12 | AGT1 | 74 | 12 | LTA1 | 9 | 11.51 |
| | 3 | 23 | 12 | IRS-1 | 28 | 12 | AGT1 | 75 | 23 | LTA2 | 9.1 | 11.62 |
| | 3 | 23 | 12 | IRS-1 | 39 | 23 | CRP1 | 74 | 12 | LTA1 | 9.06 | 11.57 |
| | 3 | 23 | 12 | IRS-1 | 39 | 23 | CRP1 | 75 | 23 | LTA2 | 9.16 | 11.68 |
| | 3 | 23 | 12 | IRS-1 | 44 | 3 | CETP4 | 74 | 12 | LTA1 | 9.06 | 11.57 |
| | 3 | 23 | 12 | IRS-1 | 44 | 3 | CETP4 | 75 | 23 | LTA2 | 9.16 | 11.68 |
| | 3 | 23 | 12 | IRS-1 | 47 | 3 | TNFα1 | 74 | 12 | LTA1 | 9.06 | 11.57 |
| | 3 | 23 | 12 | IRS-1 | 47 | 3 | TNFα1 | 75 | 23 | LTA2 | 9.16 | 11.68 |
| | 3 | 23 | 12 | IRS-1 | 51 | 12 | GP1a1 | 74 | 12 | LTA1 | 9.44 | 99 |
| | 3 | 23 | 12 | IRS-1 | 53 | 12 | β2AR-1 | 74 | 12 | LTA1 | 7.12 | 9.51 |
| | 3 | 23 | 12 | IRS-1 | 53 | 12 | β2AR-1 | 75 | 23 | LTA2 | 7.2 | 9.61 |
| | 3 | 23 | 12 | IRS-1 | 70 | 23 | APM2 | 74 | 12 | LTA1 | 8.6 | 11.09 |
| | 3 | 23 | 12 | IRS-1 | 70 | 23 | APM2 | 75 | 23 | LTA2 | 8.7 | 11.2 |
| | 2 | 23 | 12 | IRS-1 | 74 | 12 | LTA1 | | | | 9.06 | 11.57 |
| | 2 | 23 | 12 | IRS-1 | 80 | 12 | CCR2 | | | | 5.55 | 99 |

[FIG. 27]

| | | | | | | | | | | | | | |
|---|---|---|---|---|---|---|---|---|---|---|---|---|---|
| positive genetic polymorphism sets | 3 | 0.0354 | 1 | 3 | ABCA1 | 3 | 3 | ACE ID | 27 | 3 | N10 | 10.24 | 99 |
| | 3 | 0.0348 | 23 | 12 | IRS-1 | 51 | 12 | GP1a1 | 75 | 23 | LTA2 | 9.53 | 99 |
| | 2 | 0.0417 | 23 | 12 | IRS-1 | 75 | 23 | LTA2 | | | | 9.16 | 11.68 |
| | 3 | 0.0299 | 23 | 12 | IRS-1 | 51 | 12 | GP1a1 | 53 | 12 | β2AR-1 | 9.02 | 99 |
| | 3 | 0.0403 | 3 | 3 | ACE ID | 42 | 1 | GCLM1 | 50 | 23 | β2AR4 | 8.89 | 11.41 |
| | 3 | 0.0356 | 3 | 3 | ACE ID | 13 | 12 | CF12 | 50 | 23 | β2AR4 | 8.24 | 10.71 |
| | 3 | 0.0233 | 1 | 3 | ABCA1 | 37 | 1 | GP1ba | 77 | 3 | APM12 | 6.94 | 99 |
| | 3 | 0.0366 | 29 | 12 | G1A3 | 67 | 12 | 1A2 | 96 | 1 | LRP1 | 6.94 | 99 |
| | 3 | 0.0245 | 1 | 3 | ABCA1 | 3 | 3 | ACE ID | 12 | 12 | Tbm3 | 6.8 | 99 |
| | 3 | 0.0254 | 12 | 12 | Tbm3 | 45 | 23 | LPL3 | 67 | 12 | 1A2 | 6.66 | 99 |
| | 2 | 0.0203 | 12 | 12 | Tbm3 | 54 | 3 | IL62 | | | | 6.49 | 99 |
| | 2 | 0.0226 | 79 | 1 | RAGE3 | 90 | 3 | IL-182 | | | | 5.7 | 99 |
| | 2 | 0.0124 | 3 | 3 | ACE ID | 79 | 1 | RAGE3 | | | | 4.77 | 99 |
| | 2 | 0.0212 | 36 | 3 | FGA1 | 54 | 3 | IL62 | | | | 4.72 | 99 |
| | 1 | 0.0162 | 20 | 3 | GSY | | | | | | | 4.52 | 99 |
| | 2 | 0.0279 | 68 | 1 | BKR1 | 77 | 3 | APM12 | | | | 4.5 | 99 |
| | 2 | 0.0183 | 23 | 12 | IRS-1 | 25 | 1 | PAI | | | | 6.38 | 99 |
| | 3 | 0.0401 | 23 | 12 | IRS-1 | 28 | 12 | AGT1 | 74 | 12 | LTA1 | 9 | 11.51 |
| | 3 | 0.0402 | 23 | 12 | IRS-1 | 28 | 12 | AGT1 | 75 | 23 | LTA2 | 9.1 | 11.62 |
| | 3 | 0.0416 | 23 | 12 | IRS-1 | 39 | 23 | CRP1 | 74 | 12 | LTA1 | 9.06 | 11.57 |
| | 3 | 0.0416 | 23 | 12 | IRS-1 | 39 | 23 | CRP1 | 75 | 23 | LTA2 | 9.16 | 11.68 |
| | 3 | 0.0416 | 23 | 12 | IRS-1 | 44 | 3 | CETP4 | 74 | 12 | LTA1 | 9.06 | 11.57 |
| | 3 | 0.0416 | 23 | 12 | IRS-1 | 44 | 3 | CETP4 | 75 | 23 | LTA2 | 9.16 | 11.68 |
| | 3 | 0.0411 | 23 | 12 | IRS-1 | 47 | 3 | TNF-alfa | 74 | 12 | LTA1 | 9.06 | 11.57 |
| | 3 | 0.0411 | 23 | 12 | IRS-1 | 47 | 3 | TNF-alfa | 75 | 23 | LTA2 | 9.16 | 11.68 |
| | 3 | 0.0347 | 23 | 12 | IRS-1 | 51 | 12 | GP1a1 | 74 | 12 | LTA1 | 9.44 | 99 |
| | 3 | 0.0386 | 23 | 12 | IRS-1 | 70 | 23 | APM2 | 74 | 12 | LTA1 | 8.6 | 11.09 |
| | 3 | 0.0387 | 23 | 12 | IRS-1 | 70 | 23 | APM2 | 75 | 23 | LTA2 | 8.7 | 11.2 |
| | 2 | 0.0416 | 23 | 12 | IRS-1 | 74 | 12 | LTA1 | | | | 9.06 | 11.57 |
| | 2 | 0.0225 | 23 | 12 | IRS-1 | 80 | 12 | CCR2 | | | | 5.55 | 99 |

[FIG. 28]

| SNP-No | Genotype |
|---|---|
| 3 | 3 |
| 1 | 3 |
| 90 | 3 |
| 33 | 3 |
| 8 | 12 |
| 67 | 12 |
| 53 | 1 |
| 51 | 23 |
| 96 | 1 |
| 38 | 12 |
| 74 | 3 |
| 12 | 12 |
| 40 | 3 |
| 30 | 1 |
| 73 | 23 |
| 62 | 1 |
| 78 | 12 |
| 25 | 12 |
| 29 | 12 |
| 85 | 3 |
| 55 | 12 |
| 88 | 12 |
| 68 | 12 |
| 36 | 3 |
| 26 | 1 |
| 80 | 12 |
| 77 | 3 |
| 98 | 3 |
| 70 | 12 |
| 84 | 1 |
| 37 | 1 |
| 10 | 3 |
| 13 | 12 |
| 4 | 23 |
| 42 | 1 |
| 19 | 12 |
| 32 | 23 |
| 87 | 23 |
| 50 | 23 |
| 39 | 3 |
| 20 | 3 |
| 27 | 3 |
| 31 | 3 |
| 93 | 1 |
| 54 | 23 |
| 17 | 3 |
| 43 | 12 |
| 22 | 1 |
| 21 | 12 |
| 28 | 12 |
| 15 | 12 |
| 94 | 12 |
| 24 | 12 |
| 64 | 3 |
| 56 | 23 |
| 7 | 1 |
| 86 | 3 |
| 63 | 12 |
| 71 | 23 |
| 69 | 3 |
| 45 | 1 |
| 79 | 23 |
| 75 | 3 |
| 52 | 1 |
| 16 | 12 |

[FIG. 29]

| SNP-No | Genotype |
|---|---|
| 53 | 1 |
| 3 | 3 |
| 8 | 12 |
| 12 | 12 |
| 1 | 3 |
| 70 | 12 |
| 67 | 12 |
| 85 | 3 |
| 29 | 12 |
| 38 | 12 |
| 77 | 3 |
| 50 | 23 |
| 62 | 1 |
| 80 | 3 |
| 93 | 23 |
| 31 | 3 |
| 20 | 3 |
| 96 | 1 |
| 78 | 12 |
| 37 | 1 |
| 68 | 1 |
| 94 | 12 |
| 55 | 12 |
| 84 | 1 |
| 89 | 3 |
| 51 | 3 |
| 86 | 1 |
| 33 | 3 |
| 79 | 12 |
| 10 | 3 |
| 21 | 1 |
| 56 | 23 |
| 40 | 3 |
| 23 | 3 |
| 26 | 1 |
| 30 | 12 |
| 25 | 12 |
| 32 | 23 |
| 42 | 1 |
| 28 | 12 |
| 15 | 12 |
| 69 | 3 |
| 45 | 1 |
| 19 | 12 |
| 7 | 1 |
| 13 | 12 |
| 90 | 3 |
| 63 | 12 |

[FIG. 30]

| SNP-No | Genotype |
|---|---|
| 3 | 3 |
| 8 | 12 |
| 67 | 12 |
| 1 | 3 |
| 31 | 3 |
| 53 | 1 |
| 12 | 12 |
| 33 | 3 |
| 37 | 1 |
| 29 | 12 |
| 75 | 1 |
| 42 | 1 |
| 23 | 12 |
| 93 | 23 |
| 55 | 12 |
| 50 | 23 |
| 20 | 3 |
| 78 | 12 |
| 68 | 1 |
| 54 | 3 |
| 70 | 12 |
| 80 | 3 |
| 85 | 12 |
| 39 | 3 |
| 79 | 12 |
| 88 | 1 |
| 51 | 3 |
| 71 | 23 |
| 86 | 1 |
| 74 | 12 |
| 40 | 3 |
| 77 | 3 |
| 84 | 1 |
| 25 | 1 |
| 36 | 3 |
| 27 | 23 |
| 38 | 12 |
| 90 | 3 |
| 69 | 3 |
| 10 | 3 |
| 15 | 12 |
| 30 | 12 |
| 24 | 12 |
| 32 | 23 |
| 21 | 12 |
| 26 | 1 |
| 28 | 12 |
| 96 | 1 |
| 56 | 3 |
| 94 | 12 |

[FIG. 31]

| SNP-No | Genotype |
|---|---|
| 3 | 3 |
| 8 | 12 |
| 12 | 12 |
| 1 | 3 |
| 37 | 1 |
| 31 | 3 |
| 29 | 12 |
| 23 | 12 |
| 30 | 12 |
| 50 | 23 |
| 55 | 12 |
| 53 | 1 |
| 36 | 3 |
| 54 | 3 |
| 86 | 3 |
| 20 | 3 |
| 38 | 12 |
| 84 | 1 |
| 77 | 3 |
| 96 | 1 |
| 78 | 12 |
| 93 | 1 |
| 99 | 23 |
| 79 | 1 |
| 32 | 23 |
| 74 | 12 |
| 75 | 23 |
| 25 | 1 |
| 39 | 3 |
| 69 | 3 |
| 90 | 3 |
| 80 | 1 |
| 68 | 1 |
| 21 | 23 |
| 67 | 3 |
| 42 | 1 |
| 88 | 1 |
| 27 | 23 |
| 89 | 12 |
| 28 | 12 |
| 44 | 3 |
| 47 | 3 |
| 70 | 23 |
| 56 | 3 |
| 94 | 12 |
| 98 | 23 |

[FIG. 32]

| SNP-No | Genotype |
|---|---|
| 3 | 3 |
| 1 | 3 |
| 12 | 12 |
| 55 | 12 |
| 23 | 12 |
| 37 | 1 |
| 50 | 23 |
| 29 | 12 |
| 96 | 1 |
| 86 | 3 |
| 53 | 1 |
| 20 | 3 |
| 8 | 12 |
| 32 | 23 |
| 77 | 3 |
| 54 | 3 |
| 79 | 1 |
| 67 | 12 |
| 85 | 3 |
| 69 | 3 |
| 38 | 12 |
| 39 | 3 |
| 68 | 1 |
| 90 | 3 |
| 80 | 1 |
| 74 | 12 |
| 75 | 23 |
| 25 | 1 |
| 33 | 23 |
| 78 | 1 |
| 45 | 23 |
| 42 | 1 |
| 88 | 1 |
| 27 | 23 |
| 51 | 12 |
| 89 | 12 |
| 28 | 12 |
| 70 | 23 |
| 44 | 3 |
| 47 | 3 |
| 56 | 3 |
| 94 | 12 |
| 84 | 1 |
| 98 | 23 |

[FIG. 33]

| SNP-No | Genotype |
|---|---|
| 3 | 3 |
| 1 | 3 |
| 12 | 12 |
| 23 | 12 |
| 54 | 3 |
| 50 | 23 |
| 77 | 3 |
| 74 | 12 |
| 37 | 1 |
| 75 | 3 |
| 87 | 23 |
| 20 | 3 |
| 79 | 1 |
| 29 | 12 |
| 38 | 12 |
| 39 | 3 |
| 21 | 23 |
| 67 | 3 |
| 90 | 3 |
| 36 | 3 |
| 80 | 1 |
| 27 | 3 |
| 42 | 1 |
| 68 | 1 |
| 88 | 1 |
| 69 | 1 |
| 53 | 12 |
| 51 | 12 |
| 28 | 12 |
| 44 | 3 |
| 47 | 3 |
| 70 | 23 |
| 25 | 1 |

[FIG. 34]

| SNP-No | Genotype |
|---|---|
| 3 | 3 |
| 1 | 3 |
| 12 | 12 |
| 23 | 12 |
| 54 | 3 |
| 29 | 12 |
| 37 | 1 |
| 20 | 3 |
| 50 | 23 |
| 77 | 3 |
| 79 | 1 |
| 67 | 12 |
| 38 | 12 |
| 42 | 1 |
| 74 | 12 |
| 75 | 23 |
| 90 | 3 |
| 36 | 3 |
| 27 | 3 |
| 68 | 1 |
| 13 | 12 |
| 96 | 1 |
| 88 | 1 |
| 45 | 23 |
| 53 | 12 |
| 51 | 12 |
| 28 | 12 |
| 39 | 23 |
| 44 | 3 |
| 47 | 3 |
| 70 | 23 |
| 25 | 1 |
| 80 | 12 |

[FIG. 35]

| SNP-No | Genotype |
|---|---|
| 3 | 3 |
| 1 | 3 |
| 12 | 12 |
| 23 | 12 |
| 54 | 3 |
| 29 | 12 |
| 37 | 1 |
| 20 | 3 |
| 50 | 23 |
| 77 | 3 |
| 79 | 1 |
| 67 | 12 |
| 38 | 12 |
| 42 | 1 |
| 74 | 12 |
| 75 | 23 |
| 90 | 3 |
| 36 | 3 |
| 27 | 3 |
| 68 | 1 |
| 13 | 12 |
| 96 | 1 |
| 88 | 1 |
| 45 | 23 |
| 53 | 12 |
| 51 | 12 |
| 28 | 12 |
| 39 | 23 |
| 44 | 3 |
| 47 | 3 |
| 70 | 23 |
| 25 | 1 |
| 80 | 12 |

[FIG. 36]

| SNP-No | Genotype |
|---|---|
| 3 | 3 |
| 12 | 12 |
| 1 | 3 |
| 23 | 12 |
| 54 | 3 |
| 20 | 3 |
| 50 | 23 |
| 77 | 3 |
| 67 | 12 |
| 79 | 1 |
| 42 | 1 |
| 27 | 3 |
| 29 | 12 |
| 74 | 12 |
| 75 | 23 |
| 96 | 1 |
| 90 | 3 |
| 36 | 3 |
| 13 | 12 |
| 68 | 1 |
| 37 | 1 |
| 45 | 23 |
| 51 | 12 |
| 28 | 12 |
| 39 | 23 |
| 44 | 3 |
| 47 | 3 |
| 70 | 23 |
| 53 | 12 |
| 25 | 1 |
| 80 | 12 |

[FIG. 37-A]

| SNP-No | code | gene | site | position (rs) |
|---|---|---|---|---|
| 1 | ABCA1 | ABCA 1 | G1051A(Arg219Lys) | rs2230806 |
| 2 | DRD2 | Dopamine D2 receptor | CG(Ser311Cys) | rs1801028 |
| 3 | ACE ID | ACE | I/ D type | x62855 with positions 1451-1738 deleted rs1799752 |
| 4 | ESRa1 | alfa estrogen receptor | TC(P vull) | rs2234693 |
| 5 | N1 | e NOS | T-786C+4repeat | rs2070744 |
| 6 | SELP | P-selectin | AC(Thr715Pro) | rs6136 |
| 7 | N7 | p 22phox | C242T | rs4673 |
| 8 | PONA2 | HUMPONA | A172T(Met55Leu) | rs3202100 |
| 9 | IL6 | interleukin -6 | G-174C | rs1800795 |
| 10 | GP3A | GP IIB IIIa | C1565T(PIA2) | rs5918 |
| 11 | PAR2 | PPAR gamma | C-G(Pro12Ala) | rs1801282 |
| 12 | Tbm3 | thrombomodulin | G -33A | position 1487 of M74564 |
| 13 | CF12 | Factor XII | C46T | rs1801020 |
| 14 | NPY | neuropeptide Y | T1128C(Leu7Pro) | rs16139 |
| 15 | MMP12 | MMP-12 | A-82G | rs2276109 |
| 16 | HL1 | hepatic lipase | C-480T | rs1800588 |
| 17 | FR1 | Flactalkine receptor CX3CR1 | GA(V249I) | rs3732379 |
| 18 | S2AR | serotonin 2A receptor | T102C | rs6313 |
| 19 | MMP9 | MMP-9=gelatinase B | C-1562T | rs3918242 |
| 20 | GSY | glycogen synthase | AG(M416V) | rs5447 |
| 21 | CX37 | connexin37(gap junction protein) | C1019T(Pro319Ser) | rs1764391 |
| 22 | ESL1 | E-selectin | G98T | rs1805193 |
| 23 | IRS1 | IRS-1 | Gly971Arg | rs1801278 |
| 24 | AGTR1-3 | AT1 receptor | A1166C | rs5186 |
| 25 | PAI | PAI-I | 4G/5G at -668 | rs1799889 |
| 26 | NOS3 | e NOS | G894T(Glu298Asp) | rs1799983 |
| 27 | N10 | TGF -beta 1 | T29C(Leu10Pro) | position 329 of AY330201 |
| 28 | AGT1 | Angiotensinogen | T704C(M235T) | JST050962, rs699 |
| 29 | G1A3 | GP Ia | A1648G (Lys505Glu) | rs1801106 |
| 30 | FGB3 | beta Fibrinogen | C148T | rs1800787 |
| 31 | APE2 | Apolipoprotein E | TC(Cys112Arg) | rs429358 |
| 32 | MTHFR | MTHFR | C677T | rs1801133 |
| 33 | MCP1 | MCP-1(chemokine) | A-2518G | rs1024611 |
| 34 | PT | prothrombin | G20210A | rs1799963 |
| 35 | ESL2 | E-selectin | A561C(Ser128Arg) | rs5361 |
| 36 | FGA1 | alpha fibrinogen | AG(Thr312Ala) | rs6050 |
| 37 | GP1ba | Human Platelet Antigen-2 | C1018T(Thr145Met) | rs6065 |
| 38 | PONA1 | PON1 | A584G(Gln192Arg) | rs662, site amended 575→584, Aug. 7, 2003 |
| 39 | CRP1 | C-reactive protein | 1059G/C(rs1800947) | rs1800947 |
| 40 | 3AR | beta 3 adreno receptor | TC(Trp64Arg) | rs4994 |
| 41 | MMP72 | matrilysin promoter | C-153T | position 1050 of L22525 |
| 42 | GCLM1 | glutamate-cysteine ligase, modifier | -588C/T | position 2670 of U72210 |
| 43 | MTP1 | microsomal trigyceride transfer pro | G-493T | rs1800591 |
| 44 | CETP4 | Cholesterol ester transfer protein | GA (Arg451Gln) | rs1800777 |
| 45 | LPL3 | Lipoprotein lipase | CG(Ser 447 STOP) | rs328 |
| 46 | PARa1 | PPAR-alfa | C696G(Leu162Val) | rs1800206 |
| 47 | TNF α 1 | TNF-alfa | G-238A | rs361525 |
| 48 | ICAM1 | intercellular adhesin molecule 1 | GA(E469K) | rs5498 |
| 49 | GP61 | Glycoprotein VI | TC(Ser219Pro) | rs1613662 |
| 50 | β 2AR4 | β 2-Adrenergic Receptor | C79G | rs1042714 |
| 51 | GP1a1 | Glycoprotien I a | C807T | rs1126643 |
| 52 | MMP71 | matrilysin promoter | A-181G | position 1022 of L22525 |
| 53 | β 2AR-1 | β 2-Adrenergic Receptor | A46G(Arg16Gly) | rs1042713 |
| 54 | IL62 | Interleukin-6 | C-634G | rs1800796 |
| 55 | MPO | myeloperoxidase | G-463A(rs2333227) | rs2333227 |
| 56 | HANP1 | Human Atrial Natriuuretic Peptide | T2238C | rs5065 |
| 57 | 2J2-2 | CYP2J2*2 | 14,487A>G(Thr 143 Ala | 14,487A>G, position 20518 of AF272142 |
| 58 | 2J2-3 | CYP2J2*3 | 14,532C>T(Arg 158 Cy | 14,532C>T, position 20563 of AF272142 |
| 59 | 2J2-4 | CYP2J2*4 | 15,028T>A(Ile 192 Asn) | 15,028T>A, position 21059 of AF272142 |

[FIG. 37-B]

| | | | | |
|---|---|---|---|---|
| 60 | ABCC6 | ABCC6 | C3421T(R1141X) | position 27135 of AC025778 |
| 61 | 2J2-6 | CYP2J2*6 | 25,661A>T(Asn 404 Ty | 25,661A>T、position 31692 of AF272142 |
| 62 | APE3 | Apolipoprotein E ε3 in exon 4 | Arg158Cys (C/T) | rs7412 |
| 63 | HANP2 | Human Atrial Natriuuretic Peptide | C708T | rs5064 |
| 64 | TNFa2 | Tumor necrosis factor-α | G-308A | rs1800629 |
| 65 | CF5 | Factor V | G1691A | rs6025 |
| 66 | β2AR5 | β2-Adrenergic Receptor | C491T(Thr164Ile) | rs1800888 |
| 67 | 1A2 | Glycoprotien I a | G873A | rs1062535 |
| 68 | BKR1 | bradykinin B2receptor | C-58T | rs1799722 |
| 69 | CD181 | CD 18 | C1323T | rs235326 |
| 70 | APM2 | adiponectin | T94G | rs2241766 |
| 71 | REG1 | Resistin | ATG 6 repeat | rs3833230 |
| 72 | AMPD1 | adenosine monophosphate deamina | C34T | position 274 of M37921 |
| 73 | VEGF5 | VEGF | C-634G | rs2010963 |
| 74 | LTA1 | Lymphotoxin-alfa | A252G | position 1069 of M16441 |
| 75 | LTA2 | Lymphotoxin-alfa | Thr26Asn/C804A | rs1041981 |
| 76 | TS41 | Thrombospondin-4 | G1186C(Ala387Pro) | rs1866389 |
| 77 | APM12 | ADIPONECTIN | G276T | rs1501299, IMS-JST013728 |
| 78 | ET1 | Endothelin-1 | G5665T/Lys198Asn | position 161 of M25380 |
| 79 | RAGE3 | RAGE | Gly82Ser (G/A) | rs2070600 |
| 80 | CCR2 | C-C chemokine receptor2 | G190A(Val64Ile) | rs1799864 |
| 81 | TS11 | Thrombospondin-1 | A2210G(Asn700Ser) | position 55322 of AC037198 |
| 82 | IL103 | Interleukin-10 | G-1082A | rs1800896 |
| 83 | 2C9-3 | CYP2C9*3 | Leu359Ile(A1075C) | rs1057910 |
| 84 | IL1B2 | IL-1β | C3953T | rs1143634 |
| 85 | 5178 | Mitochondria | A5178C | position 5178 of V00662 |
| 86 | IL-181 | Interleukin-18 | C-607A | rs1946518 |
| 87 | MS1 | Methionine synthase | A2756G(Asp919Gly) | rs1805087 |
| 88 | VWF2 | von Willebrand Factor | G-1051A | rs7965413 |
| 89 | IL102 | Interleukin-10 | C-819T | rs1800871 |
| 90 | IL-182 | Interleukin-18 | G-137C | rs187238 |
| 91 | MT12026 | Mitochondria | A12026G | position 12026 of V00662 |
| 92 | SRB11 | Scavenger receptor B I =CLA-1 | G4A(Gly2Ser) | rs4238001 |
| 93 | TPO1 | Thrombopoietin | A5713G | rs6141 |
| 94 | CYP3A4 | CYP3A4 | A-290G | rs2740574 |
| 95 | EPHX22 | soluble epoxide hydrolase(EPHX2) | Arg402-403insJn exon13 ((CGT)ins after 1206) | rs2234887 |
| 96 | LRP1 | LDL receptor related protein | C766T | position 516 of AF058399 |
| 97 | MT1310 | Mitochondria | C1310T | position 1310 of V00662 |
| 98 | PGC11 | Peroxisome proliferation activated receptor γ coactivator-1(PGC-1) | G1302A(Thr394Thr) | rs2970847 |
| 99 | PGC12 | Peroxisome proliferation activated receptor γ coactivator-1(PGC-1) | G1564A(Gly482Ser) | position 592 of AF108200 |

[FIG. 38]

| SNP-No | Genotype | Gene code | SNP-No | Genotype | Gene code | kai | odds |
|---|---|---|---|---|---|---|---|
| 1 | 1 | ABCA1 | 20 | 23 | GSY | 7 | -2.9 |
| 3 | 23 | ACE ID | 81 | 23 | TS11 | 90.8 | -4.5 |
| 3 | 23 | ACE ID | 86 | 3 | IL-181 | 32.8 | -2.4 |
| 8 | 12 | PONA2 | 11 | 23 | PAR2 | 7.7 | -99 |
| 11 | 23 | PAR2 | 64 | 12 | TNFa2 | 6.7 | -99 |
| 21 | 23 | CX37 | 99 | 1 | PGC12 | 7.1 | -2.2 |
| 25 | 3 | PAI | 90 | 12 | IL-182 | 8.8 | -4.2 |
| 27 | 3 | N10 | 64 | 12 | TNF2 | 6.7 | -99 |
| 35 | 23 | ESL2 | 82 | 23 | IL103 | 7.2 | -6.8 |
| 40 | 12 | 3AR | 80 | 1 | CCR2 | 8 | -4.3 |
| 62 | 23 | APE3 | 90 | 12 | IL-182 | 7 | -6.7 |
| 67 | 1 | 1A2 | 68 | 3 | BKR1 | 6.8 | -2.7 |
| 74 | 12 | LTA1 | 81 | 23 | TS11 | 84.5 | -4.4 |
| 74 | 12 | LTA1 | 86 | 3 | IL-181 | 31.6 | -2.4 |
| 75 | 23 | LTA2 | 81 | 23 | TS11 | 82.8 | -4.3 |
| 75 | 23 | LTA2 | 86 | 3 | IL-181 | 31 | -2.4 |
| 77 | 12 | APM12 | 81 | 23 | TS11 | 79.8 | -4 |
| 77 | 12 | APM12 | 86 | 3 | IL-181 | 29.1 | -2.3 |
| 79 | 23 | RAGE3 | 81 | 23 | TS11 | 100.8 | -4.8 |
| 79 | 23 | RAGE3 | 86 | 3 | IL-181 | 38.2 | -2.6 |
| 81 | 23 | TS11 | 93 | 23 | TPO1 | 57.9 | -3.4 |
| 81 | 23 | TS11 | 98 | 23 | PGC11 | 107.8 | -5.1 |
| 86 | 3 | IL-181 | 98 | 23 | PGC11 | 40.9 | -2.6 |

[FIG. 39]

| SNP-No | Genotype | Gene code | SNP-No | Genotype | Gene code | kai | odds |
|---|---|---|---|---|---|---|---|
| 3 | 23 | ACE ID | 81 | 23 | TS11 | 90.8 | -4.5 |
| 8 | 12 | PONA2 | 11 | 23 | PAR2 | 7.7 | -99 |
| 11 | 23 | PAR2 | 64 | 12 | TNFa2 | 6.7 | -99 |
| 25 | 3 | PAI | 90 | 12 | IL-182 | 8.8 | -4.2 |
| 27 | 3 | N10 | 64 | 12 | TNFa2 | 6.7 | -99 |
| 35 | 23 | ESL2 | 82 | 23 | IL103 | 7.2 | -6.8 |
| 40 | 12 | 3AR | 80 | 1 | CCR2 | 8 | -4.3 |
| 62 | 23 | APE3 | 90 | 12 | IL-182 | 7 | -6.7 |
| 74 | 12 | LTA1 | 81 | 23 | TS11 | 84.5 | -4.4 |
| 75 | 23 | LTA2 | 81 | 23 | TS11 | 82.8 | -4.3 |
| 77 | 12 | APM12 | 81 | 23 | TS11 | 79.8 | -4 |
| 79 | 23 | RAGE3 | 81 | 23 | TS11 | 100.8 | -4.8 |
| 81 | 23 | TS11 | 93 | 23 | TPO1 | 57.9 | -3.4 |
| 81 | 23 | TS11 | 98 | 23 | PGC11 | 107.8 | -5.1 |

[FIG. 40]

| SNP-No | Genotype | Gene code | SNP-No | Genotype | Gene code | kai | odds |
|---|---|---|---|---|---|---|---|
| 3 | 23 | ACE ID | 81 | 23 | TS11 | 90.8 | -4.5 |
| 8 | 12 | PONA2 | 11 | 23 | PAR2 | 7.7 | -99 |
| 11 | 23 | PAR2 | 64 | 12 | TNFa2 | 6.7 | -99 |
| 25 | 3 | PAI | 90 | 12 | IL-182 | 8.8 | -4.2 |
| 27 | 3 | N10 | 64 | 12 | TNFa2 | 6.7 | -99 |
| 35 | 23 | ESL2 | 82 | 23 | IL103 | 7.2 | -6.8 |
| 40 | 12 | 3AR | 80 | 1 | CCR2 | 8 | -4.3 |
| 62 | 23 | APE3 | 90 | 12 | IL-182 | 7 | -6.7 |
| 74 | 12 | LTA1 | 81 | 23 | TS11 | 84.5 | -4.4 |
| 75 | 23 | LTA2 | 81 | 23 | TS11 | 82.8 | -4.3 |
| 77 | 12 | APM12 | 81 | 23 | TS11 | 79.8 | -4 |
| 79 | 23 | RAGE3 | 81 | 23 | TS11 | 100.8 | -4.8 |
| 81 | 23 | TS11 | 98 | 23 | PGC11 | 107.8 | -5.1 |

[FIG. 41]

| SNP-No | Genotype | Gene code | SNP-No | Genotype | Gene code | kai | odds |
|---|---|---|---|---|---|---|---|
| 8 | 12 | PONA2 | 11 | 23 | PAR2 | 7.7 | -99 |
| 11 | 23 | PAR2 | 64 | 12 | TNFa2 | 6.7 | -99 |
| 27 | 3 | N10 | 64 | 12 | TNFa2 | 6.7 | -99 |
| 35 | 23 | ESL2 | 82 | 23 | IL103 | 7.2 | -6.8 |
| 62 | 23 | APE3 | 90 | 12 | IL-182 | 7 | -6.7 |
| 81 | 23 | TS11 | 98 | 23 | PGC11 | 107.8 | -5.1 |

[FIG. 42]

| SNP-No | Genotype | Gene code | SNP-No | Genotype | Gene code | kai | odds |
|---|---|---|---|---|---|---|---|
| 8 | 12 | PONA2 | 11 | 23 | PAR2 | 7.7 | -99 |
| 11 | 23 | PAR2 | 64 | 12 | TNFa2 | 6.7 | -99 |
| 27 | 3 | N10 | 64 | 12 | TNFa2 | 6.7 | -99 |
| 35 | 23 | ESL2 | 82 | 23 | IL103 | 7.2 | -6.8 |
| 62 | 23 | APE3 | 90 | 12 | IL-182 | 7 | -6.7 |

[FIG. 43]

| SNP-No | Genotype | Gene code | SNP-No | Genotype | Gene code | kai | odds |
|---|---|---|---|---|---|---|---|
| 8 | 12 | PONA2 | 11 | 23 | PAR2 | 7.7 | -99 |
| 11 | 23 | PAR2 | 64 | 12 | TNFa2 | 6.7 | -99 |
| 27 | 3 | N10 | 64 | 12 | TNFa2 | 6.7 | -99 |

[FIG. 44]

| SNP-No | GeneType |
|---|---|
| 81 | 23 |
| 86 | 3 |
| 38 | 3 |
| 5 | 12 |
| 90 | 12 |
| 56 | 3 |
| 71 | 3 |
| 62 | 12 |
| 18 | 23 |
| 76 | 3 |
| 73 | 1 |
| 25 | 23 |
| 13 | 3 |
| 35 | 23 |
| 36 | 1 |
| 98 | 12 |
| 8 | 23 |
| 23 | 23 |
| 24 | 12 |
| 83 | 12 |
| 2 | 12 |
| 11 | 12 |
| 10 | 23 |
| 15 | 12 |
| 47 | 23 |
| 49 | 12 |
| 29 | 23 |
| 50 | 12 |
| 79 | 23 |
| 12 | 23 |
| 30 | 12 |
| 45 | 12 |
| 52 | 12 |
| 82 | 12 |
| 7 | 12 |
| 20 | 12 |
| 26 | 12 |
| 37 | 12 |
| 43 | 12 |
| 96 | 12 |
| 55 | 23 |
| 87 | 12 |
| 21 | 12 |
| 31 | 23 |
| 42 | 12 |
| 19 | 12 |
| 40 | 23 |
| 28 | 12 |
| 69 | 12 |
| 80 | 23 |
| 78 | 12 |
| 54 | 12 |
| 77 | 12 |
| 3 | 23 |
| 70 | 23 |
| 89 | 23 |
| 32 | 12 |
| 4 | 23 |
| 33 | 23 |
| 74 | 12 |
| 75 | 23 |
| 51 | 12 |
| 48 | 12 |
| 67 | 23 |
| 16 | 23 |
| 68 | 3 |
| 53 | 12 |
| 27 | 23 |
| 99 | 23 |
| 88 | 23 |
| 17 | 23 |
| 22 | 12 |
| 93 | 23 |
| 1 | 23 |
| 64 | 12 |

[FIG. 45]

| SNP-No | GenoType |
|---|---|
| 81 | 23 |
| 98 | 23 |
| 5 | 12 |
| 90 | 12 |
| 69 | 3 |
| 86 | 3 |
| 35 | 23 |
| 25 | 3 |
| 83 | 23 |
| 68 | 3 |
| 28 | 3 |
| 62 | 23 |
| 80 | 1 |
| 8 | 12 |
| 15 | 23 |
| 10 | 12 |
| 18 | 23 |
| 70 | 1 |
| 20 | 23 |
| 11 | 23 |
| 96 | 23 |
| 40 | 12 |
| 82 | 23 |
| 32 | 1 |
| 33 | 1 |
| 73 | 1 |
| 21 | 23 |
| 31 | 12 |
| 78 | 3 |
| 52 | 23 |
| 27 | 3 |
| 13 | 3 |
| 24 | 23 |
| 53 | 3 |
| 43 | 3 |
| 77 | 23 |
| 30 | 23 |
| 89 | 12 |
| 45 | 23 |
| 93 | 3 |
| 42 | 23 |
| 36 | 1 |
| 38 | 3 |
| 55 | 12 |
| 64 | 12 |
| 50 | 23 |
| 75 | 12 |
| 99 | 1 |
| 1 | 1 |
| 67 | 1 |
| 7 | 23 |
| 16 | 3 |
| 48 | 3 |
| 26 | 23 |
| 49 | 23 |
| 37 | 23 |
| 54 | 23 |
| 76 | 3 |
| 4 | 3 |
| 51 | 3 |
| 74 | 12 |
| 39 | 3 |
| 19 | 3 |
| 56 | 3 |
| 29 | 12 |
| 84 | 12 |
| 71 | 3 |
| 87 | 23 |
| 12 | 3 |
| 88 | 3 |
| 79 | 12 |
| 3 | 23 |
| 2 | 23 |
| 23 | 3 |
| 47 | 12 |
| 22 | 1 |
| 17 | 3 |

[FIG. 46]

| SNP-No | GenoType |
|---|---|
| 81 | 23 |
| 90 | 12 |
| 25 | 3 |
| 33 | 1 |
| 5 | 12 |
| 80 | 1 |
| 40 | 12 |
| 62 | 23 |
| 35 | 23 |
| 82 | 23 |
| 67 | 1 |
| 2 | 12 |
| 8 | 23 |
| 10 | 23 |
| 15 | 12 |
| 23 | 23 |
| 24 | 12 |
| 29 | 23 |
| 49 | 12 |
| 50 | 12 |
| 79 | 23 |
| 83 | 12 |
| 11 | 12 |
| 17 | 23 |
| 22 | 12 |
| 26 | 12 |
| 45 | 12 |
| 47 | 23 |
| 52 | 12 |
| 12 | 23 |
| 86 | 23 |
| 20 | 12 |
| 30 | 12 |
| 98 | 23 |
| 7 | 12 |
| 37 | 12 |
| 43 | 12 |
| 96 | 12 |
| 31 | 23 |
| 36 | 1 |
| 42 | 12 |
| 19 | 12 |
| 87 | 12 |
| 28 | 12 |
| 21 | 12 |
| 99 | 1 |
| 3 | 23 |
| 54 | 12 |
| 77 | 12 |
| 78 | 12 |
| 70 | 23 |
| 89 | 23 |
| 18 | 23 |
| 38 | 23 |
| 48 | 12 |
| 13 | 23 |
| 32 | 12 |
| 73 | 23 |
| 75 | 23 |
| 4 | 23 |
| 74 | 12 |
| 76 | 3 |
| 1 | 1 |
| 16 | 23 |
| 53 | 12 |
| 71 | 3 |
| 64 | 12 |
| 93 | 3 |
| 55 | 3 |
| 88 | 3 |
| 51 | 1 |
| 68 | 3 |
| 39 | 3 |
| 27 | 3 |
| 69 | 3 |
| 84 | 1 |
| 56 | 3 |

[FIG. 47]

| SNP-No | GenoType |
|---|---|
| 81 | 23 |
| 98 | 23 |
| 5 | 12 |
| 90 | 12 |
| 69 | 3 |
| 86 | 3 |
| 35 | 23 |
| 25 | 3 |
| 83 | 23 |
| 68 | 3 |
| 28 | 3 |
| 62 | 23 |
| 80 | 1 |
| 8 | 12 |
| 15 | 23 |
| 10 | 12 |
| 18 | 23 |
| 70 | 1 |
| 20 | 23 |
| 11 | 23 |
| 96 | 23 |
| 40 | 12 |
| 82 | 23 |
| 32 | 1 |
| 33 | 1 |
| 73 | 1 |
| 21 | 23 |
| 31 | 12 |
| 78 | 3 |
| 52 | 23 |
| 27 | 3 |
| 13 | 3 |
| 24 | 23 |
| 53 | 3 |
| 43 | 3 |
| 77 | 23 |
| 30 | 23 |
| 89 | 12 |
| 45 | 23 |
| 93 | 3 |
| 42 | 23 |
| 36 | 1 |
| 38 | 3 |
| 55 | 12 |
| 64 | 12 |
| 50 | 23 |
| 75 | 12 |
| 99 | 1 |
| 1 | 1 |
| 67 | 1 |
| 7 | 23 |
| 16 | 3 |
| 48 | 3 |
| 26 | 23 |
| 49 | 23 |
| 37 | 23 |
| 54 | 23 |
| 76 | 3 |
| 4 | 3 |
| 51 | 3 |
| 74 | 12 |
| 39 | 3 |
| 19 | 3 |
| 56 | 3 |
| 29 | 12 |
| 84 | 12 |
| 71 | 3 |
| 87 | 23 |
| 12 | 3 |
| 88 | 3 |
| 79 | 12 |
| 3 | 23 |
| 2 | 23 |
| 23 | 3 |
| 47 | 12 |
| 22 | 1 |
| 17 | 3 |

[FIG. 48-A]

| SNP-No | Genotype | Gene code | SNP-No | Genotype | Gene code | kai | odds |
|---|---|---|---|---|---|---|---|
| 1 | 23 | ABCA1 | 81 | 1 | TS11 | 73.4 | 3.8 |
| 1 | 23 | ABCA1 | 86 | 12 | IL-181 | 21.6 | 2 |
| 2 | 12 | DRD2 | 79 | 1 | RAGE3 | 9.1 | 12 |
| 3 | 1 | ACE ID | 16 | 12 | HL1 | 8.5 | 2.1 |
| 3 | 1 | ACE ID | 19 | 1 | MMP9 | 6.7 | 2.2 |
| 3 | 1 | ACE ID | 27 | 12 | N10 | 8.8 | 2.3 |
| 3 | 1 | ACE ID | 42 | 1 | GCLM1 | 10 | 2.6 |
| 3 | 1 | ACE ID | 69 | 1 | CD181 | 8 | 2.5 |
| 4 | 23 | ESRa1 | 79 | 1 | RAGE3 | 7.2 | 10 |
| 4 | 23 | ESRa1 | 81 | 1 | TS11 | 80.5 | 4 |
| 4 | 23 | ESRa1 | 86 | 12 | IL-181 | 30.6 | 2.3 |
| 5 | 23 | N1 | 74 | 3 | LTA1 | 12.7 | 4.4 |
| 5 | 23 | N1 | 75 | 1 | LTA2 | 10.6 | 3.6 |
| 7 | 1 | N7 | 81 | 1 | TS11 | 73.6 | 3.7 |
| 7 | 1 | N7 | 86 | 12 | IL-181 | 26.1 | 2.1 |
| 8 | 3 | PONA2 | 74 | 3 | LTA1 | 10.5 | 2 |
| 8 | 3 | PONA2 | 75 | 1 | LTA2 | 10.6 | 2 |
| 8 | 3 | PONA2 | 81 | 1 | TS11 | 66.3 | 3.4 |
| 8 | 3 | PONA2 | 86 | 12 | IL-181 | 24 | 2.1 |
| 10 | 3 | GP3A | 79 | 1 | RAGE3 | 9.1 | 11.9 |
| 11 | 1 | PAR2 | 79 | 1 | RAGE3 | 9.7 | 99 |
| 11 | 1 | PAR2 | 98 | 1 | PGC11 | 9.2 | 3.5 |
| 12 | 3 | Tbm3 | 81 | 1 | TS11 | 52.4 | 3 |
| 13 | 23 | CF12 | 79 | 1 | RAGE3 | 7.1 | 9.8 |
| 13 | 23 | CF12 | 81 | 1 | TS11 | 72.8 | 3.6 |
| 15 | 12 | MMP12 | 79 | 1 | RAGE3 | 8.1 | 10.8 |
| 16 | 12 | HL1 | 79 | 1 | RAGE3 | 11.8 | 99 |
| 16 | 12 | HL1 | 81 | 1 | TS11 | 67 | 3.5 |
| 17 | 3 | FR1 | 79 | 1 | RAGE3 | 9 | 11.8 |
| 17 | 3 | FR1 | 86 | 12 | IL-181 | 26.4 | 2.1 |
| 18 | 12 | S2AR | 79 | 1 | RAGE3 | 8 | 10.8 |
| 18 | 12 | S2AR | 81 | 1 | TS11 | 64 | 3.5 |
| 19 | 1 | MMP9 | 81 | 1 | TS11 | 44.3 | 2.8 |
| 20 | 1 | GSY | 81 | 1 | TS11 | 62.9 | 3.3 |
| 20 | 1 | GSY | 86 | 12 | IL-181 | 25.6 | 2.1 |
| 21 | 1 | CX37 | 79 | 1 | RAGE3 | 7.8 | 99 |
| 21 | 1 | CX37 | 81 | 1 | TS11 | 43 | 2.8 |
| 21 | 1 | CX37 | 98 | 1 | PGC11 | 9.2 | 3.7 |
| 22 | 1 | ESL1 | 86 | 12 | IL-181 | 27 | 2.2 |
| 23 | 23 | IRS1 | 79 | 1 | RAGE3 | 8 | 10.8 |
| 24 | 1 | AGTR1-3 | 81 | 1 | TS11 | 77 | 3.8 |
| 24 | 1 | AGTR1-3 | 98 | 1 | PGC11 | 7.9 | 3 |
| 25 | 12 | PAI | 81 | 1 | TS11 | 88.9 | 4.3 |
| 25 | 12 | PAI | 98 | 1 | PGC11 | 7.9 | 3.2 |
| 26 | 1 | NOS3 | 81 | 1 | TS11 | 77.7 | 3.9 |
| 26 | 1 | NOS3 | 86 | 12 | IL-181 | 21.8 | 2 |
| 27 | 12 | N10 | 79 | 1 | RAGE3 | 8.8 | 99 |
| 27 | 12 | N10 | 81 | 1 | TS11 | 76.9 | 4 |
| 28 | 1 | AGT1 | 81 | 1 | TS11 | 59.6 | 3.4 |
| 28 | 1 | AGT1 | 86 | 12 | IL-181 | 23.8 | 2.1 |
| 29 | 3 | G1A3 | 86 | 12 | IL-181 | 22.5 | 2 |
| 30 | 23 | FGB3 | 37 | 23 | GP1ba | 7.9 | 2.7 |
| 30 | 23 | FGB3 | 74 | 3 | LTA1 | 7.3 | 3.1 |
| 30 | 23 | FGB3 | 75 | 1 | LTA2 | 7.5 | 3.2 |
| 31 | 12 | APE2 | 51 | 23 | GP1a1 | 9.9 | 2.1 |
| 32 | 12 | MTHFR | 79 | 1 | RAGE3 | 8.1 | 10.8 |
| 32 | 12 | MTHFR | 81 | 1 | TS11 | 64.2 | 3.4 |
| 33 | 23 | MCP1 | 81 | 1 | TS11 | 75 | 3.8 |
| 33 | 23 | MCP1 | 86 | 12 | IL-181 | 24.7 | 2.1 |
| 35 | 1 | ESL2 | 38 | 12 | PONA1 | 11.4 | 2.1 |
| 36 | 23 | FGA1 | 79 | 1 | RAGE3 | 7.9 | 99 |
| 36 | 23 | FGA1 | 81 | 1 | TS11 | 48.1 | 3 |
| 36 | 23 | FGA1 | 98 | 1 | PGC11 | 8.7 | 3.6 |
| 38 | 12 | PONA1 | 74 | 3 | LTA1 | 9.5 | 2.3 |
| 38 | 12 | PONA1 | 75 | 1 | LTA2 | 9 | 2.3 |
| 39 | 23 | CRP1 | 74 | 3 | LTA1 | 11.2 | 3.5 |
| 39 | 23 | CRP1 | 75 | 1 | LTA2 | 9.1 | 3 |
| 40 | 3 | 3AR | 81 | 1 | TS11 | 49.6 | 3.1 |
| 42 | 1 | GCLM1 | 81 | 1 | TS11 | 46.5 | 2.9 |
| 43 | 1 | MTP1 | 81 | 1 | TS11 | 52.4 | 3.1 |

[FIG. 48-B]

| | | | | | | | |
|---|---|---|---|---|---|---|---|
| 45 | 1 | LPL3 | 74 | 3 | LTA1 | 10.4 | 2.1 |
| 45 | 1 | LPL3 | 79 | 1 | RAGE3 | 11.7 | 99 |
| 45 | 1 | LPL3 | 81 | 1 | TS11 | 72.5 | 3.7 |
| 45 | 1 | LPL3 | 86 | 12 | IL-181 | 29.6 | 2.3 |
| 48 | 12 | ICAM1 | 81 | 1 | TS11 | 66.4 | 3.5 |
| 49 | 12 | GP61 | 79 | 1 | RAGE3 | 9.1 | 11.9 |
| 50 | 1 | β2AR4 | 81 | 1 | TS11 | 72.6 | 3.7 |
| 50 | 1 | β2AR4 | 86 | 12 | IL-181 | 21.9 | 2 |
| 51 | 23 | GP1a1 | 81 | 1 | TS11 | 62.2 | 3.6 |
| 51 | 23 | GP1a1 | 86 | 12 | IL-181 | 29.7 | 2.4 |
| 52 | 1 | MMP71 | 81 | 1 | TS11 | 79 | 3.9 |
| 52 | 1 | MMP71 | 86 | 12 | IL-181 | 25.6 | 2.1 |
| 54 | 12 | IL62 | 79 | 1 | RAGE3 | 7.3 | 10 |
| 54 | 12 | IL62 | 81 | 1 | TS11 | 64.2 | 3.4 |
| 55 | 3 | MPO | 81 | 1 | TS11 | 52.7 | 3.1 |
| 55 | 3 | MPO | 86 | 12 | IL-181 | 22.6 | 2.1 |
| 56 | 23 | HANP1 | 74 | 3 | LTA1 | 10.7 | 3.4 |
| 56 | 23 | HANP1 | 75 | 1 | LTA2 | 9.2 | 3 |
| 62 | 1 | APE3 | 74 | 3 | LTA1 | 10.7 | 3.7 |
| 62 | 1 | APE3 | 75 | 1 | LTA2 | 8.2 | 3 |
| 64 | 3 | TNFa2 | 74 | 3 | LTA1 | 11.6 | 3.6 |
| 64 | 3 | TNFa2 | 75 | 1 | LTA2 | 9.4 | 3.1 |
| 67 | 23 | 1A2 | 79 | 1 | RAGE3 | 9.3 | 12.1 |
| 67 | 23 | 1A2 | 81 | 1 | TS11 | 56.4 | 3.2 |
| 68 | 12 | BKR1 | 81 | 1 | TS11 | 68.1 | 3.8 |
| 69 | 1 | CD181 | 74 | 3 | LTA1 | 8.6 | 2.2 |
| 69 | 1 | CD181 | 75 | 1 | LTA2 | 7 | 2 |
| 69 | 1 | CD181 | 81 | 1 | TS11 | 35.1 | 2.8 |
| 70 | 3 | APM2 | 74 | 3 | LTA1 | 6.6 | 2 |
| 70 | 3 | APM2 | 81 | 1 | TS11 | 34.5 | 2.6 |
| 71 | 3 | REG1 | 74 | 3 | LTA1 | 9 | 3.2 |
| 71 | 3 | REG1 | 75 | 1 | LTA2 | 7.3 | 2.7 |
| 73 | 23 | VEGF5 | 79 | 1 | RAGE3 | 7.8 | 99 |
| 73 | 23 | VEGF5 | 81 | 1 | TS11 | 88.1 | 4.3 |
| 73 | 23 | VEGF5 | 86 | 12 | IL-181 | 27.9 | 2.2 |
| 74 | 3 | LTA1 | 76 | 23 | TS41 | 11.4 | 3.6 |
| 74 | 3 | LTA1 | 78 | 23 | ET1 | 7.7 | 2.3 |
| 74 | 3 | LTA1 | 84 | 1 | IL1B2 | 11.5 | 3.8 |
| 74 | 3 | LTA1 | 86 | 12 | IL-181 | 16 | 3.4 |
| 74 | 3 | LTA1 | 90 | 3 | IL-182 | 10.4 | 4.3 |
| 74 | 3 | LTA1 | 99 | 23 | PGC12 | 11 | 2.2 |
| 75 | 1 | LTA2 | 76 | 23 | TS41 | 9.7 | 3.1 |
| 75 | 1 | LTA2 | 78 | 23 | ET1 | 8 | 2.3 |
| 75 | 1 | LTA2 | 84 | 1 | IL1B2 | 9.2 | 3.2 |
| 75 | 1 | LTA2 | 86 | 12 | IL-181 | 15.1 | 3.2 |
| 75 | 1 | LTA2 | 90 | 3 | IL-182 | 10.4 | 4.3 |
| 75 | 1 | LTA2 | 99 | 23 | PGC12 | 10.4 | 2.2 |
| 78 | 23 | ET1 | 93 | 1 | TPO1 | 7.4 | 2 |
| 79 | 1 | RAGE3 | 80 | 23 | CCR2 | 11.9 | 99 |
| 79 | 1 | RAGE3 | 83 | 12 | 2C9-3 | 9 | 11.8 |
| 79 | 1 | RAGE3 | 87 | 1 | MS1 | 7.7 | 99 |
| 79 | 1 | RAGE3 | 89 | 23 | IL102 | 8.3 | 11.1 |
| 80 | 23 | CCR2 | 86 | 12 | IL-181 | 33 | 2.4 |
| 80 | 23 | CCR2 | 98 | 1 | PGC11 | 9.6 | 3.6 |
| 81 | 1 | TS11 | 86 | 12 | IL-181 | 68.2 | 3.6 |
| 81 | 1 | TS11 | 87 | 1 | MS1 | 55.8 | 3.3 |
| 81 | 1 | TS11 | 88 | 23 | VWF2 | 56.9 | 3.2 |
| 81 | 1 | TS11 | 89 | 23 | IL102 | 76.7 | 3.8 |
| 81 | 1 | TS11 | 96 | 1 | LRP1 | 77.9 | 3.9 |
| 81 | 1 | TS11 | 99 | 23 | PGC12 | 64 | 3.4 |
| 82 | 1 | IL103 | 86 | 12 | IL-181 | 30.6 | 2.3 |
| 86 | 12 | IL-181 | 88 | 23 | VWF2 | 21.1 | 2 |
| 86 | 12 | IL-181 | 89 | 23 | IL102 | 30.3 | 2.3 |
| 86 | 12 | IL-181 | 96 | 1 | LRP1 | 27 | 2.2 |
| 86 | 12 | IL-181 | 99 | 23 | PGC12 | 27.9 | 2.2 |
| 89 | 23 | IL102 | 98 | 1 | PGC11 | 9.9 | 3.6 |
| 93 | 1 | TPO1 | 98 | 1 | PGC11 | 8.9 | 99 |
| 96 | 1 | LRP1 | 98 | 1 | PGC11 | 6.8 | 2.9 |

[FIG. 49-A]

| SNP-No | Genotype | Gene code | SNP-No | Genotype | Gene code | kai | odds |
|---|---|---|---|---|---|---|---|
| 1 | 23 | ABCA1 | 81 | 1 | TS11 | 73.4 | 3.8 |
| 1 | 12 | DRD2 | 79 | 1 | RAGE3 | 9.1 | 12 |
| 2 | 23 | ESRa1 | 79 | 1 | RAGE3 | 7.2 | 10 |
| 3 | 23 | ESRa1 | 81 | 1 | TS11 | 80.5 | 4 |
| 3 | 23 | N1 | 74 | 3 | LTA1 | 12.7 | 4.4 |
| 3 | 23 | N1 | 75 | 1 | LTA2 | 10.6 | 3.6 |
| 3 | 1 | N7 | 81 | 1 | TS11 | 73.6 | 3.7 |
| 3 | 3 | PONA2 | 81 | 1 | TS11 | 66.3 | 3.4 |
| 4 | 3 | GP3A | 79 | 1 | RAGE3 | 9.1 | 11.9 |
| 4 | 1 | PAR2 | 79 | 1 | RAGE3 | 9.7 | 99 |
| 4 | 1 | PAR2 | 98 | 1 | PGC11 | 9.2 | 3.5 |
| 5 | 3 | Tbm3 | 81 | 1 | TS11 | 52.4 | 3 |
| 5 | 23 | CF12 | 79 | 1 | RAGE3 | 7.1 | 9.8 |
| 7 | 23 | CF12 | 81 | 1 | TS11 | 72.8 | 3.6 |
| 7 | 12 | MMP12 | 79 | 1 | RAGE3 | 8.1 | 10.8 |
| 8 | 12 | HL1 | 79 | 1 | RAGE3 | 11.8 | 99 |
| 8 | 12 | HL1 | 81 | 1 | TS11 | 67 | 3.5 |
| 8 | 3 | FR1 | 79 | 1 | RAGE3 | 9 | 11.8 |
| 8 | 12 | S2AR | 79 | 1 | RAGE3 | 8 | 10.8 |
| 10 | 12 | S2AR | 81 | 1 | TS11 | 64 | 3.5 |
| 11 | 1 | GSY | 81 | 1 | TS11 | 62.9 | 3.3 |
| 11 | 1 | CX37 | 79 | 1 | RAGE3 | 7.8 | 99 |
| 12 | 1 | CX37 | 98 | 1 | PGC11 | 9.2 | 3.7 |
| 13 | 23 | IRS1 | 79 | 1 | RAGE3 | 8 | 10.8 |
| 13 | 1 | AGTR1-3 | 81 | 1 | TS11 | 77 | 3.8 |
| 15 | 1 | AGTR1-3 | 98 | 1 | PGC11 | 7.9 | 3 |
| 16 | 12 | PAI | 81 | 1 | TS11 | 88.9 | 4.3 |
| 16 | 12 | PAI | 98 | 1 | PGC11 | 7.9 | 3.2 |
| 17 | 1 | NOS3 | 81 | 1 | TS11 | 77.7 | 3.9 |
| 17 | 12 | N10 | 79 | 1 | RAGE3 | 8.8 | 99 |
| 18 | 12 | N10 | 81 | 1 | TS11 | 76.9 | 4 |
| 18 | 1 | AGT1 | 81 | 1 | TS11 | 59.6 | 3.4 |
| 19 | 23 | FGB3 | 74 | 3 | LTA1 | 7.3 | 3.1 |
| 20 | 23 | FGB3 | 75 | 1 | LTA2 | 7.5 | 3.2 |
| 20 | 12 | MTHFR | 79 | 1 | RAGE3 | 8.1 | 10.8 |
| 21 | 12 | MTHFR | 81 | 1 | TS11 | 64.2 | 3.4 |
| 21 | 23 | MCP1 | 81 | 1 | TS11 | 75 | 3.8 |
| 21 | 23 | FGA1 | 79 | 1 | RAGE3 | 7.9 | 99 |
| 22 | 23 | FGA1 | 81 | 1 | TS11 | 48.1 | 3 |
| 23 | 23 | FGA1 | 98 | 1 | PGC11 | 8.7 | 3.6 |
| 24 | 23 | CRP1 | 74 | 3 | LTA1 | 11.2 | 3.5 |
| 24 | 23 | CRP1 | 75 | 1 | LTA2 | 9.1 | 3 |
| 25 | 3 | 3AR | 81 | 1 | TS11 | 49.6 | 3.1 |
| 25 | 1 | MTP1 | 81 | 1 | TS11 | 52.4 | 3.1 |
| 26 | 1 | LPL3 | 79 | 1 | RAGE3 | 11.7 | 99 |
| 26 | 1 | LPL3 | 81 | 1 | TS11 | 72.5 | 3.7 |
| 27 | 12 | ICAM1 | 81 | 1 | TS11 | 66.4 | 3.5 |
| 27 | 12 | GP61 | 79 | 1 | RAGE3 | 9.1 | 11.9 |
| 28 | 1 | β2AR4 | 81 | 1 | TS11 | 72.6 | 3.7 |
| 28 | 23 | GP1a1 | 81 | 1 | TS11 | 62.2 | 3.6 |
| 29 | 1 | MMP71 | 81 | 1 | TS11 | 79 | 3.9 |
| 30 | 12 | IL62 | 79 | 1 | RAGE3 | 7.3 | 10 |
| 30 | 12 | IL62 | 81 | 1 | TS11 | 64.2 | 3.4 |
| 30 | 3 | MPO | 81 | 1 | TS11 | 52.7 | 3.1 |
| 32 | 23 | HANP1 | 74 | 3 | LTA1 | 10.7 | 3.4 |
| 32 | 23 | HANP1 | 75 | 1 | LTA2 | 9.2 | 3 |
| 33 | 1 | APE3 | 74 | 3 | LTA1 | 10.7 | 3.7 |
| 33 | 1 | APE3 | 75 | 1 | LTA2 | 8.2 | 3 |
| 35 | 3 | TNFa2 | 74 | 3 | LTA1 | 11.6 | 3.6 |

[FIG. 49-B]

| | | | | | | | |
|---|---|---|---|---|---|---|---|
| 36 | 3 | TNFa2 | 75 | 1 | LTA2 | 9.4 | 3.1 |
| 36 | 23 | 1A2 | 79 | 1 | RAGE3 | 9.3 | 12.1 |
| 36 | 23 | 1A2 | 81 | 1 | TS11 | 56.4 | 3.2 |
| 38 | 12 | BKR1 | 81 | 1 | TS11 | 68.1 | 3.8 |
| 43 | 3 | REG1 | 74 | 3 | LTA1 | 9 | 3.2 |
| 45 | 23 | VEGF5 | 79 | 1 | RAGE3 | 7.8 | 99 |
| 45 | 23 | VEGF5 | 81 | 1 | TS11 | 88.1 | 4.3 |
| 48 | 3 | LTA1 | 76 | 23 | TS41 | 11.4 | 3.6 |
| 50 | 3 | LTA1 | 84 | 1 | IL1B2 | 11.5 | 3.8 |
| 50 | 3 | LTA1 | 86 | 12 | IL-181 | 16 | 3.4 |
| 51 | 3 | LTA1 | 90 | 3 | IL-182 | 10.4 | 4.3 |
| 52 | 1 | LTA2 | 76 | 23 | TS41 | 9.7 | 3.1 |
| 54 | 1 | LTA2 | 84 | 1 | IL1B2 | 9.2 | 3.2 |
| 54 | 1 | LTA2 | 86 | 12 | IL-181 | 15.1 | 3.2 |
| 55 | 1 | LTA2 | 90 | 3 | IL-182 | 10.4 | 4.3 |
| 56 | 1 | RAGE3 | 80 | 23 | CCR2 | 11.9 | 99 |
| 62 | 1 | RAGE3 | 83 | 12 | 2C9-3 | 9 | 11.8 |
| 62 | 1 | RAGE3 | 87 | 1 | MS1 | 7.7 | 99 |
| 64 | 1 | RAGE3 | 89 | 23 | IL102 | 8.3 | 11.1 |
| 67 | 23 | CCR2 | 98 | 1 | PGC11 | 9.6 | 3.6 |
| 67 | 1 | TS11 | 86 | 12 | IL-181 | 68.2 | 3.6 |
| 68 | 1 | TS11 | 87 | 1 | MS1 | 55.8 | 3.3 |
| 69 | 1 | TS11 | 88 | 23 | VWF2 | 56.9 | 3.2 |
| 69 | 1 | TS11 | 89 | 23 | IL102 | 76.7 | 3.8 |
| 69 | 1 | TS11 | 96 | 1 | LRP1 | 77.9 | 3.9 |
| 70 | 1 | TS11 | 99 | 23 | PGC12 | 64 | 3.4 |
| 73 | 23 | IL102 | 98 | 1 | PGC11 | 9.9 | 3.6 |
| 74 | 1 | TPO1 | 98 | 1 | PGC11 | 8.9 | 99 |

[FIG. 50]

| SNP-No | Genotype | Gene code | SNP-No | Genotype | Gene code | kai | odds |
|---|---|---|---|---|---|---|---|
| 1 | 12 | DRD2 | 79 | 1 | RAGE3 | 9.1 | 12 |
| 2 | 23 | ESRa1 | 79 | 1 | RAGE3 | 7.2 | 10 |
| 3 | 23 | ESRa1 | 81 | 1 | TS11 | 80.5 | 4 |
| 3 | 23 | N1 | 74 | 3 | LTA1 | 12.7 | 4.4 |
| 4 | 3 | GP3A | 79 | 1 | RAGE3 | 9.1 | 11.9 |
| 4 | 1 | PAR2 | 79 | 1 | RAGE3 | 9.7 | 99 |
| 5 | 23 | CF12 | 79 | 1 | RAGE3 | 7.1 | 9.8 |
| 7 | 12 | MMP12 | 79 | 1 | RAGE3 | 8.1 | 10.8 |
| 8 | 12 | HL1 | 79 | 1 | RAGE3 | 11.8 | 99 |
| 8 | 3 | FR1 | 79 | 1 | RAGE3 | 9 | 11.8 |
| 8 | 12 | S2AR | 79 | 1 | RAGE3 | 8 | 10.8 |
| 11 | 1 | CX37 | 79 | 1 | RAGE3 | 7.8 | 99 |
| 13 | 23 | IRS1 | 79 | 1 | RAGE3 | 8 | 10.8 |
| 16 | 12 | PAI | 81 | 1 | TS11 | 88.9 | 4.3 |
| 17 | 12 | N10 | 79 | 1 | RAGE3 | 8.8 | 99 |
| 18 | 12 | N10 | 81 | 1 | TS11 | 76.9 | 4 |
| 20 | 12 | MTHFR | 79 | 1 | RAGE3 | 8.1 | 10.8 |
| 21 | 23 | FGA1 | 79 | 1 | RAGE3 | 7.9 | 99 |
| 26 | 1 | LPL3 | 79 | 1 | RAGE3 | 11.7 | 99 |
| 27 | 12 | GP61 | 79 | 1 | RAGE3 | 9.1 | 11.9 |
| 30 | 12 | IL62 | 79 | 1 | RAGE3 | 7.3 | 10 |
| 36 | 23 | 1A2 | 79 | 1 | RAGE3 | 9.3 | 12.1 |
| 45 | 23 | VEGF5 | 79 | 1 | RAGE3 | 7.8 | 99 |
| 45 | 23 | VEGF5 | 81 | 1 | TS11 | 88.1 | 4.3 |
| 51 | 3 | LTA1 | 90 | 3 | IL-182 | 10.4 | 4.3 |
| 55 | 1 | LTA2 | 90 | 3 | IL-182 | 10.4 | 4.3 |
| 56 | 1 | RAGE3 | 80 | 23 | CCR2 | 11.9 | 99 |
| 62 | 1 | RAGE3 | 83 | 12 | 2C9-3 | 9 | 11.8 |
| 62 | 1 | RAGE3 | 87 | 1 | MS1 | 7.7 | 99 |
| 64 | 1 | RAGE3 | 89 | 23 | IL102 | 8.3 | 11.1 |
| 74 | 1 | TPO1 | 98 | 1 | PGC11 | 8.9 | 99 |

[FIG. 51]

| SNP-No | Genotype | Gene code | SNP-No | Genotype | Gene code | kai | odds |
|---|---|---|---|---|---|---|---|
| 1 | 12 | DRD2 | 79 | 1 | RAGE3 | 9.1 | 12 |
| 2 | 23 | ESRa1 | 79 | 1 | RAGE3 | 7.2 | 10 |
| 4 | 3 | GP3A | 79 | 1 | RAGE3 | 9.1 | 11.9 |
| 4 | 1 | PAR2 | 79 | 1 | RAGE3 | 9.7 | 99 |
| 5 | 23 | CF12 | 79 | 1 | RAGE3 | 7.1 | 9.8 |
| 7 | 12 | MMP12 | 79 | 1 | RAGE3 | 8.1 | 10.8 |
| 8 | 12 | HL1 | 79 | 1 | RAGE3 | 11.8 | 99 |
| 8 | 3 | FR1 | 79 | 1 | RAGE3 | 9 | 11.8 |
| 8 | 12 | S2AR | 79 | 1 | RAGE3 | 8 | 10.8 |
| 11 | 1 | CX37 | 79 | 1 | RAGE3 | 7.8 | 99 |
| 13 | 23 | IRS1 | 79 | 1 | RAGE3 | 8 | 10.8 |
| 17 | 12 | N10 | 79 | 1 | RAGE3 | 8.8 | 99 |
| 20 | 12 | MTHFR | 79 | 1 | RAGE3 | 8.1 | 10.8 |
| 21 | 23 | FGA1 | 79 | 1 | RAGE3 | 7.9 | 99 |
| 26 | 1 | LPL3 | 79 | 1 | RAGE3 | 11.7 | 99 |
| 27 | 12 | GP61 | 79 | 1 | RAGE3 | 9.1 | 11.9 |
| 30 | 12 | IL62 | 79 | 1 | RAGE3 | 7.3 | 10 |
| 36 | 23 | 1A2 | 79 | 1 | RAGE3 | 9.3 | 12.1 |
| 45 | 23 | VEGF5 | 79 | 1 | RAGE3 | 7.8 | 99 |
| 56 | 1 | RAGE3 | 80 | 23 | CCR2 | 11.9 | 99 |
| 62 | 1 | RAGE3 | 83 | 12 | 2C9-3 | 9 | 11.8 |
| 62 | 1 | RAGE3 | 87 | 1 | MS1 | 7.7 | 99 |
| 64 | 1 | RAGE3 | 89 | 23 | IL102 | 8.3 | 11.1 |
| 74 | 1 | TPO1 | 98 | 1 | PGC11 | 8.9 | 99 |

[FIG. 52]

| SNP-No | GenoType |
|---|---|
| 81 | 1 |
| 86 | 12 |
| 74 | 3 |
| 75 | 1 |
| 3 | 1 |
| 98 | 1 |
| 51 | 23 |
| 8 | 3 |
| 99 | 23 |
| 45 | 1 |
| 89 | 23 |
| 96 | 1 |
| 52 | 1 |
| 7 | 1 |
| 50 | 1 |
| 4 | 23 |
| 93 | 1 |
| 26 | 1 |
| 20 | 1 |
| 33 | 23 |
| 73 | 23 |
| 27 | 12 |
| 1 | 23 |
| 79 | 1 |
| 88 | 23 |
| 37 | 23 |
| 69 | 1 |
| 31 | 12 |
| 55 | 3 |
| 30 | 23 |
| 16 | 12 |
| 28 | 1 |
| 38 | 12 |
| 54 | 12 |
| 78 | 23 |
| 42 | 1 |
| 24 | 1 |
| 25 | 12 |
| 53 | 1 |
| 77 | 3 |
| 80 | 23 |
| 19 | 1 |
| 13 | 23 |
| 17 | 3 |
| 32 | 12 |
| 21 | 1 |
| 70 | 3 |
| 22 | 1 |
| 29 | 3 |
| 82 | 1 |
| 48 | 12 |
| 12 | 3 |
| 36 | 23 |
| 67 | 23 |
| 18 | 12 |
| 43 | 1 |
| 35 | 1 |
| 62 | 1 |
| 87 | 1 |
| 40 | 3 |
| 68 | 12 |
| 11 | 1 |
| 83 | 12 |
| 2 | 12 |
| 15 | 12 |
| 23 | 23 |
| 64 | 3 |
| 49 | 12 |
| 10 | 3 |
| 76 | 23 |
| 84 | 1 |
| 47 | 23 |
| 56 | 23 |
| 5 | 23 |
| 39 | 23 |
| 71 | 3 |
| 90 | 3 |

[FIG. 53]

| SNP-No | GenoType |
|---|---|
| 81 | 1 |
| 74 | 3 |
| 75 | 1 |
| 98 | 1 |
| 86 | 1 |
| 3 | 1 |
| 25 | 12 |
| 24 | 1 |
| 79 | 1 |
| 89 | 23 |
| 77 | 3 |
| 13 | 23 |
| 38 | 23 |
| 52 | 1 |
| 4 | 23 |
| 37 | 1 |
| 7 | 1 |
| 8 | 3 |
| 54 | 12 |
| 50 | 1 |
| 32 | 12 |
| 73 | 23 |
| 45 | 1 |
| 30 | 1 |
| 26 | 1 |
| 20 | 1 |
| 33 | 23 |
| 96 | 1 |
| 31 | 3 |
| 67 | 23 |
| 48 | 12 |
| 16 | 12 |
| 12 | 3 |
| 99 | 23 |
| 1 | 23 |
| 27 | 12 |
| 18 | 12 |
| 53 | 12 |
| 88 | 23 |
| 43 | 1 |
| 55 | 3 |
| 51 | 23 |
| 87 | 1 |
| 28 | 1 |
| 68 | 12 |
| 40 | 3 |
| 78 | 23 |
| 93 | 1 |
| 22 | 23 |
| 23 | 12 |
| 5 | 3 |
| 64 | 3 |
| 76 | 23 |
| 84 | 1 |
| 35 | 12 |
| 56 | 23 |
| 39 | 23 |
| 36 | 3 |
| 90 | 3 |
| 62 | 1 |
| 80 | 23 |
| 11 | 1 |
| 69 | 12 |
| 2 | 1 |
| 21 | 1 |
| 71 | 3 |
| 29 | 12 |
| 42 | 3 |
| 15 | 23 |
| 10 | 3 |
| 17 | 3 |
| 47 | 3 |
| 49 | 12 |
| 82 | 12 |
| 83 | 12 |
| 19 | 12 |

[FIG. 54]

| SNP-No | GenoType |
|---|---|
| 81 | 1 |
| 25 | 12 |
| 73 | 23 |
| 86 | 1 |
| 74 | 3 |
| 77 | 3 |
| 75 | 1 |
| 98 | 1 |
| 3 | 1 |
| 79 | 1 |
| 67 | 1 |
| 90 | 3 |
| 23 | 12 |
| 93 | 1 |
| 32 | 3 |
| 51 | 3 |
| 5 | 3 |
| 16 | 3 |
| 38 | 1 |
| 54 | 3 |
| 24 | 23 |
| 12 | 12 |
| 1 | 3 |
| 21 | 3 |
| 22 | 23 |
| 31 | 12 |
| 78 | 23 |
| 30 | 1 |
| 87 | 23 |
| 26 | 23 |
| 33 | 1 |
| 96 | 23 |
| 13 | 1 |
| 15 | 23 |
| 42 | 3 |
| 36 | 1 |
| 99 | 12 |
| 20 | 3 |
| 53 | 1 |
| 70 | 12 |
| 2 | 12 |
| 7 | 12 |
| 8 | 23 |
| 10 | 3 |
| 17 | 3 |
| 29 | 23 |
| 37 | 12 |
| 40 | 23 |
| 43 | 12 |
| 45 | 1 |
| 47 | 3 |
| 49 | 12 |
| 50 | 12 |
| 52 | 12 |
| 80 | 23 |
| 82 | 12 |
| 83 | 12 |
| 11 | 1 |
| 18 | 12 |
| 19 | 12 |
| 28 | 12 |
| 69 | 12 |
| 88 | 12 |
| 89 | 23 |
| 4 | 23 |
| 55 | 23 |
| 27 | 12 |
| 39 | 23 |
| 62 | 12 |
| 84 | 12 |

[FIG. 55]

| SNP-No | GenoType |
|---|---|
| 81 | 1 |
| 74 | 3 |
| 75 | 1 |
| 86 | 1 |
| 3 | 1 |
| 5 | 3 |
| 98 | 1 |
| 54 | 3 |
| 23 | 12 |
| 25 | 1 |
| 79 | 1 |
| 13 | 1 |
| 12 | 12 |
| 32 | 3 |
| 1 | 3 |
| 21 | 3 |
| 77 | 3 |
| 70 | 3 |
| 76 | 12 |
| 15 | 23 |
| 42 | 3 |
| 16 | 1 |
| 96 | 23 |
| 26 | 23 |
| 33 | 1 |
| 51 | 3 |
| 99 | 12 |
| 22 | 23 |
| 20 | 3 |
| 35 | 1 |
| 67 | 1 |
| 36 | 1 |
| 93 | 1 |
| 2 | 12 |
| 7 | 12 |
| 8 | 23 |
| 10 | 3 |
| 17 | 3 |
| 24 | 12 |
| 29 | 23 |
| 30 | 12 |
| 31 | 23 |
| 37 | 12 |
| 40 | 23 |
| 43 | 12 |
| 45 | 1 |
| 47 | 3 |
| 49 | 12 |
| 50 | 12 |
| 52 | 12 |
| 80 | 23 |
| 82 | 12 |
| 83 | 12 |
| 18 | 12 |
| 19 | 12 |
| 28 | 12 |
| 53 | 23 |
| 69 | 12 |
| 78 | 12 |
| 88 | 12 |
| 89 | 23 |
| 4 | 23 |
| 11 | 1 |
| 38 | 23 |
| 27 | 12 |
| 55 | 23 |
| 73 | 23 |
| 87 | 1 |

[FIG. 56]

| SNP-No | Genotype | Gene code | SNP-No | Genotype | Gene code | SNP-No | Genotype | Gene code | kai | odds |
|---|---|---|---|---|---|---|---|---|---|---|
| 8 | 23 | PONA2 | 81 | 23 | TS11 | | | | 113.4 | -4.2 |
| 8 | 23 | PONA2 | 86 | 3 | IL-181 | | | | 59.9 | -2.5 |
| 17 | 23 | FR1 | 81 | 23 | TS11 | | | | 111.8 | -4.1 |
| 17 | 23 | FR1 | 86 | 3 | IL-181 | | | | 57.8 | -2.4 |
| 22 | 1 | ESL1 | 86 | 3 | IL-181 | | | | 58.4 | -2.5 |
| 32 | 12 | MTHFR | 81 | 23 | TS11 | | | | 89.8 | -3.9 |
| 45 | 12 | LPL3 | 81 | 23 | TS11 | | | | 111.1 | -4.1 |
| 45 | 12 | LPL3 | 86 | 3 | IL-181 | | | | 58.3 | -2.5 |
| 52 | 1 | MMP71 | 86 | 3 | IL-181 | | | | 56.1 | -2.5 |
| 54 | 3 | IL62 | 74 | 1 | LTA1 | | | | 7 | -2.8 |
| 54 | 3 | IL62 | 75 | 3 | LTA2 | | | | 7 | -2.9 |
| 63 | 1 | HANP2 | 81 | 23 | TS11 | | | | 113.8 | -4.2 |
| 63 | 1 | HANP2 | 86 | 3 | IL-181 | | | | 60 | -2.5 |
| 74 | 1 | LTA1 | 84 | 23 | IL1B2 | | | | 7.1 | -3.5 |
| 75 | 3 | LTA2 | 84 | 23 | IL1B2 | | | | 7.2 | -3.5 |
| 80 | 23 | CCR2 | 86 | 3 | IL-181 | | | | 53.9 | -2.4 |
| 81 | 23 | TS11 | 96 | 12 | LRP1 | | | | 110.3 | -4.1 |
| 82 | 1 | IL103 | 86 | 3 | IL-181 | | | | 56.6 | -2.5 |
| 83 | 1 | 2C9-3 | 86 | 3 | IL-181 | | | | 58.7 | -2.5 |
| 86 | 3 | IL-181 | 96 | 12 | LRP1 | | | | 54.9 | -2.4 |

[FIG. 57]

| SNP-No | Genotype | Gene code | SNP-No | Genotype | Gene code | SNP-No | Genotype | Gene code | kai | odds |
|---|---|---|---|---|---|---|---|---|---|---|
| 8 | 23 | PONA2 | 81 | 23 | TS11 | | | | 113.4 | -4.2 |
| 17 | 23 | FR1 | 81 | 23 | TS11 | | | | 111.8 | -4.1 |
| 32 | 12 | MTHFR | 81 | 23 | TS11 | | | | 89.8 | -3.9 |
| 45 | 12 | LPL3 | 81 | 23 | TS11 | | | | 111.1 | -4.1 |
| 63 | 1 | HANP2 | 81 | 23 | TS11 | | | | 113.8 | -4.2 |
| 74 | 1 | LTA1 | 84 | 23 | IL1B2 | | | | 7.1 | -3.5 |
| 75 | 3 | LTA2 | 84 | 23 | IL1B2 | | | | 7.2 | -3.5 |
| 81 | 23 | TS11 | 96 | 12 | LRP1 | | | | 110.3 | -4.1 |

[FIG. 58]

| SNP-No | Genotype | Gene code | SNP-No | Genotype | Gene code | SNP-No | Genotype | Gene code | kai | odds |
|---|---|---|---|---|---|---|---|---|---|---|
| 8 | 23 | PONA2 | 81 | 23 | TS11 | | | | 113.4 | -4.2 |
| 17 | 23 | FR1 | 81 | 23 | TS11 | | | | 111.8 | -4.1 |
| 45 | 12 | LPL3 | 81 | 23 | TS11 | | | | 111.1 | -4.1 |
| 63 | 1 | HANP2 | 81 | 23 | TS11 | | | | 113.8 | -4.2 |
| 81 | 23 | TS11 | 96 | 12 | LRP1 | | | | 110.3 | -4.1 |

[FIG. 59]

| SNP-No | GenoType |
|---|---|
| 86 | 3 |
| 81 | 23 |
| 93 | 3 |
| 76 | 12 |
| 62 | 23 |
| 18 | 1 |
| 23 | 12 |
| 8 | 12 |
| 13 | 1 |
| 28 | 3 |
| 75 | 3 |
| 84 | 23 |
| 54 | 3 |
| 74 | 1 |
| 2 | 1 |
| 7 | 1 |
| 40 | 1 |
| 88 | 1 |
| 11 | 12 |
| 10 | 23 |
| 15 | 12 |
| 17 | 23 |
| 24 | 12 |
| 47 | 23 |
| 50 | 12 |
| 29 | 23 |
| 49 | 12 |
| 63 | 1 |
| 12 | 23 |
| 45 | 12 |
| 30 | 12 |
| 20 | 12 |
| 26 | 12 |
| 37 | 12 |
| 43 | 12 |
| 96 | 12 |
| 21 | 12 |
| 55 | 23 |
| 19 | 12 |
| 31 | 23 |
| 87 | 12 |
| 42 | 12 |
| 79 | 23 |
| 98 | 23 |
| 69 | 12 |
| 70 | 23 |
| 77 | 12 |
| 78 | 12 |
| 38 | 23 |
| 80 | 23 |
| 89 | 23 |
| 25 | 12 |
| 51 | 12 |
| 33 | 23 |
| 4 | 23 |
| 3 | 23 |
| 67 | 23 |
| 48 | 12 |
| 73 | 23 |
| 16 | 23 |
| 27 | 23 |
| 99 | 23 |
| 36 | 12 |
| 71 | 3 |
| 90 | 12 |
| 53 | 12 |
| 1 | 23 |
| 64 | 12 |
| 83 | 1 |
| 22 | 1 |
| 82 | 1 |
| 52 | 1 |
| 32 | 12 |
| 68 | 23 |

[FIG. 60]

| SNP-No | GenoType |
|---|---|
| 81 | 23 |
| 62 | 23 |
| 13 | 1 |
| 76 | 12 |
| 84 | 23 |
| 93 | 3 |
| 28 | 3 |
| 40 | 1 |
| 88 | 1 |
| 18 | 1 |
| 35 | 1 |
| 86 | 1 |
| 90 | 12 |
| 23 | 12 |
| 74 | 1 |
| 75 | 3 |
| 24 | 23 |
| 64 | 12 |
| 67 | 1 |
| 10 | 23 |
| 17 | 23 |
| 22 | 12 |
| 50 | 12 |
| 2 | 12 |
| 8 | 23 |
| 15 | 12 |
| 29 | 23 |
| 47 | 23 |
| 49 | 12 |
| 83 | 12 |
| 11 | 12 |
| 26 | 12 |
| 52 | 12 |
| 63 | 1 |
| 82 | 12 |
| 7 | 12 |
| 12 | 23 |
| 45 | 12 |
| 96 | 12 |
| 37 | 12 |
| 55 | 23 |
| 79 | 23 |
| 20 | 12 |
| 30 | 12 |
| 43 | 12 |
| 42 | 12 |
| 19 | 12 |
| 21 | 12 |
| 31 | 23 |
| 87 | 12 |
| 98 | 23 |
| 69 | 12 |
| 54 | 12 |
| 78 | 12 |
| 70 | 23 |
| 77 | 12 |
| 89 | 23 |
| 38 | 23 |
| 33 | 23 |
| 25 | 12 |
| 51 | 12 |
| 48 | 12 |
| 32 | 12 |
| 4 | 23 |
| 80 | 23 |
| 73 | 23 |
| 27 | 23 |
| 99 | 23 |
| 3 | 23 |
| 36 | 12 |
| 16 | 23 |
| 53 | 12 |
| 68 | 12 |
| 1 | 23 |
| 71 | 3 |

[FIG. 61]

| SNP-No | GenoType |
|---|---|
| 81 | 23 |
| 62 | 23 |
| 13 | 12 |
| 28 | 3 |
| 40 | 1 |
| 88 | 1 |
| 76 | 12 |
| 90 | 12 |
| 84 | 23 |
| 86 | 1 |
| 23 | 12 |
| 24 | 23 |
| 64 | 12 |
| 67 | 1 |
| 2 | 12 |
| 10 | 23 |
| 17 | 23 |
| 22 | 12 |
| 47 | 23 |
| 50 | 12 |
| 15 | 12 |
| 26 | 12 |
| 8 | 23 |
| 29 | 23 |
| 52 | 12 |
| 63 | 1 |
| 82 | 12 |
| 12 | 23 |
| 45 | 12 |
| 37 | 12 |
| 96 | 12 |
| 79 | 23 |
| 30 | 12 |
| 55 | 23 |
| 19 | 12 |
| 11 | 1 |
| 75 | 23 |
| 74 | 12 |
| 51 | 12 |
| 36 | 3 |
| 93 | 23 |
| 99 | 23 |
| 27 | 23 |
| 49 | 12 |
| 83 | 12 |
| 7 | 12 |
| 20 | 12 |
| 16 | 23 |
| 43 | 12 |
| 39 | 3 |
| 42 | 12 |
| 31 | 23 |
| 87 | 12 |
| 21 | 12 |
| 68 | 12 |
| 98 | 23 |
| 80 | 23 |
| 54 | 12 |
| 69 | 12 |
| 78 | 12 |
| 77 | 12 |
| 70 | 23 |
| 3 | 23 |
| 89 | 23 |
| 38 | 23 |
| 33 | 23 |
| 25 | 12 |
| 48 | 12 |
| 32 | 12 |
| 73 | 23 |
| 4 | 23 |
| 53 | 12 |
| 5 | 3 |
| 18 | 3 |
| 1 | 3 |

[FIG. 62]

| SNP-No | GenoType |
|---|---|
| 81 | 3 |
| 99 | 3 |
| 28 | 23 |
| 86 | 3 |
| 96 | 23 |
| 24 | 23 |
| 54 | 3 |
| 18 | 1 |
| 62 | 23 |
| 37 | 23 |
| 93 | 3 |
| 12 | 12 |
| 33 | 1 |
| 23 | 12 |
| 40 | 1 |
| 69 | 3 |
| 52 | 23 |
| 13 | 12 |
| 88 | 1 |
| 1 | 3 |
| 76 | 12 |
| 53 | 1 |
| 48 | 23 |
| 78 | 1 |
| 29 | 12 |
| 8 | 12 |
| 36 | 3 |
| 84 | 23 |
| 20 | 23 |
| 11 | 23 |
| 74 | 1 |
| 32 | 3 |
| 43 | 23 |
| 27 | 3 |
| 75 | 3 |
| 17 | 12 |
| 98 | 1 |
| 77 | 3 |
| 90 | 12 |
| 70 | 12 |
| 67 | 1 |
| 3 | 1 |
| 5 | 3 |
| 89 | 1 |
| 87 | 3 |
| 25 | 3 |
| 2 | 23 |
| 68 | 3 |
| 19 | 23 |
| 16 | 3 |
| 51 | 3 |
| 50 | 23 |
| 64 | 12 |
| 21 | 3 |
| 4 | 3 |
| 38 | 3 |
| 73 | 1 |
| 55 | 12 |
| 30 | 23 |
| 31 | 12 |
| 7 | 23 |
| 42 | 3 |
| 79 | 3 |
| 56 | 3 |
| 80 | 12 |
| 83 | 23 |
| 26 | 23 |
| 71 | 12 |
| 45 | 1 |
| 35 | 1 |
| 95 | 3 |
| 39 | 12 |
| 49 | 12 |
| 15 | 1 |

[FIG. 63]

| SNP-No | Genotype | Gene code | SNP-No | Genotype | Gene code | SNP-No | Genotype | Gene code | kai | odds |
|---|---|---|---|---|---|---|---|---|---|---|
| 1 | 12 | ABCA1 | 81 | 1 | TS11 | | | | 67.7 | 2.5 |
| 3 | 23 | ACE ID | 64 | 12 | TNFa2 | | | | 7.2 | 2.4 |
| 4 | 12 | ESRa1 | 81 | 1 | TS11 | | | | 45.9 | 2.1 |
| 7 | 23 | N7 | 45 | 3 | LPL3 | | | | 9.2 | 12.7 |
| 11 | 1 | PAR2 | 64 | 12 | TNFa2 | | | | 8.6 | 2.6 |
| 12 | 3 | Tbm3 | 86 | 12 | IL-181 | | | | 49.7 | 2.2 |
| 15 | 23 | MMP12 | 81 | 1 | TS11 | | | | 6.6 | 2.3 |
| 16 | 12 | HL1 | 81 | 1 | TS11 | | | | 67.4 | 2.5 |
| 17 | 1 | FR1 | 73 | 23 | VEGF5 | | | | 8.4 | 99 |
| 18 | 12 | S2AR | 81 | 1 | TS11 | | | | 42 | 2 |
| 19 | 1 | MMP9 | 81 | 1 | TS11 | | | | 40.3 | 2 |
| 20 | 1 | GSY | 64 | 12 | TNFa2 | | | | 6.7 | 2.3 |
| 20 | 1 | GSY | 86 | 12 | IL-181 | | | | 49.5 | 2.2 |
| 21 | 1 | CX37 | 81 | 1 | TS11 | | | | 49.3 | 2.2 |
| 25 | 1 | PAI | 45 | 3 | LPL3 | | | | 8.6 | 7.4 |
| 25 | 1 | PAI | 81 | 1 | TS11 | | | | 36.2 | 2 |
| 27 | 12 | N10 | 81 | 1 | TS11 | | | | 72.8 | 2.6 |
| 30 | 1 | FGB3 | 63 | 23 | HANP2 | | | | 9.2 | 12.8 |
| 30 | 1 | FGB3 | 81 | 1 | TS11 | | | | 54.2 | 2.2 |
| 31 | 3 | APE2 | 64 | 12 | TNFa2 | | | | 10.5 | 3.4 |
| 32 | 3 | MTHFR | 39 | 12 | CRP1 | | | | 8.5 | 11.6 |
| 32 | 3 | MTHFR | 48 | 1 | ICAM1 | | | | 14.1 | 2.2 |
| 36 | 23 | FGA1 | 81 | 1 | TS11 | | | | 78.4 | 2.7 |
| 36 | 23 | FGA1 | 86 | 12 | IL-181 | | | | 48.9 | 2.1 |
| 38 | 12 | PONA1 | 81 | 1 | TS11 | | | | 42 | 2 |
| 40 | 3 | 3AR | 81 | 1 | TS11 | | | | 52.6 | 2.2 |
| 40 | 3 | 3AR | 83 | 23 | 2C9-3 | | | | 7.7 | 2.6 |
| 42 | 1 | GCLM1 | 81 | 1 | TS11 | | | | 49.2 | 2.2 |
| 43 | 1 | MTP1 | 81 | 1 | TS11 | | | | 50.1 | 2.2 |
| 51 | 1 | GP1a1 | 81 | 1 | TS11 | | | | 35.6 | 2 |
| 53 | 23 | β 2AR-1 | 81 | 1 | TS11 | | | | 62.6 | 2.4 |
| 55 | 3 | MPO | 76 | 1 | TS41 | | | | 6.7 | 99 |
| 55 | 3 | MPO | 86 | 12 | IL-181 | | | | 40.7 | 2 |
| 64 | 12 | TNFa2 | 67 | 23 | 1A2 | | | | 14.4 | 4.3 |
| 64 | 12 | TNFa2 | 74 | 23 | LTA1 | | | | 10.8 | 4.7 |
| 64 | 12 | TNFa2 | 78 | 23 | ET1 | | | | 7.7 | 3.3 |
| 68 | 12 | BKR1 | 81 | 1 | TS11 | | | | 78.9 | 2.7 |
| 69 | 23 | CD181 | 81 | 1 | TS11 | | | | 38.8 | 2 |
| 70 | 12 | APM2 | 81 | 1 | TS11 | | | | 49.2 | 2.2 |
| 73 | 23 | VEGF5 | 86 | 12 | IL-181 | | | | 45.6 | 2.1 |
| 74 | 23 | LTA1 | 81 | 1 | TS11 | | | | 74.9 | 2.6 |
| 74 | 23 | LTA1 | 86 | 12 | IL-181 | | | | 47.3 | 2.1 |
| 75 | 12 | LTA2 | 81 | 1 | TS11 | | | | 73.8 | 2.6 |
| 77 | 23 | APM12 | 81 | 1 | TS11 | | | | 40.2 | 2 |
| 79 | 3 | RAGE3 | 81 | 1 | TS11 | | | | 52.4 | 2.2 |
| 81 | 1 | TS11 | 82 | 23 | IL103 | | | | 12.7 | 2.1 |
| 81 | 1 | TS11 | 88 | 12 | VWF2 | | | | 51.7 | 2.2 |
| 81 | 1 | TS11 | 89 | 12 | IL102 | | | | 43 | 2.1 |
| 81 | 1 | TS11 | 93 | 12 | TPO1 | | | | 89.1 | 2.9 |
| 81 | 1 | TS11 | 99 | 12 | PGC12 | | | | 45.2 | 2.1 |
| 5 | 12 | N1 | 7 | 23 | N7 | 62 | 1 | APE3 | 6.6 | 2.5 |
| 13 | 3 | CF12 | 28 | 23 | AGT1 | 49 | 23 | GP61 | 9 | 12.6 |
| 24 | 1 | AGTR1-3 | 52 | 23 | MMP71 | 78 | 23 | ET1 | 10.3 | 2.2 |
| 26 | 23 | NOS3 | 29 | 12 | G1A3 | 50 | 1 | β 2AR4 | 7.6 | 4.6 |
| 26 | 23 | NOS3 | 37 | 23 | GP1ba | 90 | 12 | IL-182 | 7.8 | 99 |
| 26 | 23 | NOS3 | 42 | 1 | GCLM1 | 52 | 23 | MMP71 | 9.2 | 3.6 |
| 26 | 23 | NOS3 | 52 | 23 | MMP71 | 70 | 12 | APM2 | 9.2 | 3.8 |
| 26 | 23 | NOS3 | 52 | 23 | MMP71 | 78 | 23 | ET1 | 15.5 | 11.2 |
| 28 | 23 | AGT1 | 33 | 12 | MCP1 | 35 | 23 | ESL2 | 7.3 | 6.4 |
| 28 | 23 | AGT1 | 35 | 23 | ESL2 | 47 | 3 | TNF α 1 | 6.7 | 3.3 |
| 28 | 23 | AGT1 | 35 | 23 | ESL2 | 62 | 1 | APE3 | 8.4 | 4.6 |
| 28 | 23 | AGT1 | 35 | 23 | ESL2 | 71 | 3 | REG1 | 6.8 | 3.6 |
| 28 | 23 | AGT1 | 35 | 23 | ESL2 | 98 | 3 | PGC11 | 7.8 | 5.2 |
| 33 | 12 | MCP1 | 35 | 23 | ESL2 | 93 | 12 | TPO1 | 7 | 2.7 |
| 35 | 23 | ESL2 | 51 | 1 | GP1a1 | 56 | 3 | HANP1 | 6.6 | 2.8 |
| 35 | 23 | ESL2 | 51 | 1 | GP1a1 | 62 | 1 | APE3 | 10.1 | 4.1 |
| 35 | 23 | ESL2 | 51 | 1 | GP1a1 | 71 | 3 | REG1 | 8.5 | 3.5 |
| 37 | 23 | GP1ba | 77 | 23 | APM12 | 80 | 1 | CCR2 | 7 | 10.3 |
| 50 | 1 | β 2AR4 | 52 | 23 | MMP71 | 78 | 23 | ET1 | 9.6 | 2.2 |

[FIG. 64]

| SNP-No | Genotype | Gene code | SNP-No | Genotype | Gene code | SNP-No | Genotype | Gene code | kai | odds |
|---|---|---|---|---|---|---|---|---|---|---|
| 7 | 23 | N7 | 45 | 3 | LPL3 | | | | 9.2 | 12.7 |
| 17 | 1 | FR1 | 73 | 23 | VEGF5 | | | | 8.4 | 99 |
| 25 | 1 | PAI | 45 | 3 | LPL3 | | | | 8.6 | 7.4 |
| 30 | 1 | FGB3 | 63 | 23 | HANP2 | | | | 9.2 | 12.8 |
| 31 | 3 | APE2 | 64 | 12 | TNFa2 | | | | 10.5 | 3.4 |
| 32 | 3 | MTHFR | 39 | 12 | CRP1 | | | | 8.5 | 11.6 |
| 55 | 3 | MPO | 76 | 1 | TS41 | | | | 6.7 | 99 |
| 64 | 12 | TNFa2 | 67 | 23 | 1A2 | | | | 14.4 | 4.3 |
| 64 | 12 | TNFa2 | 74 | 23 | LTA1 | | | | 10.8 | 4.7 |
| 64 | 12 | TNFa2 | 78 | 23 | ET1 | | | | 7.7 | 3.3 |
| 13 | 3 | CF12 | 28 | 23 | AGT1 | 49 | 23 | GP61 | 9 | 12.6 |
| 26 | 23 | NOS3 | 29 | 12 | G1A3 | 50 | 1 | β2AR4 | 7.6 | 4.6 |
| 26 | 23 | NOS3 | 37 | 23 | GP1ba | 90 | 12 | IL-182 | 7.8 | 99 |
| 26 | 23 | NOS3 | 42 | 1 | GCLM1 | 52 | 23 | MMP71 | 9.2 | 3.6 |
| 26 | 23 | NOS3 | 52 | 23 | MMP71 | 70 | 12 | APM2 | 9.2 | 3.8 |
| 26 | 23 | NOS3 | 52 | 23 | MMP71 | 78 | 23 | ET1 | 15.5 | 11.2 |
| 28 | 23 | AGT1 | 33 | 12 | MCP1 | 35 | 23 | ESL2 | 7.3 | 6.4 |
| 28 | 23 | AGT1 | 35 | 23 | ESL2 | 47 | 3 | TNFα1 | 6.7 | 3.3 |
| 28 | 23 | AGT1 | 35 | 23 | ESL2 | 62 | 1 | APE3 | 8.4 | 4.6 |
| 28 | 23 | AGT1 | 35 | 23 | ESL2 | 71 | 3 | REG1 | 6.8 | 3.6 |
| 28 | 23 | AGT1 | 35 | 23 | ESL2 | 98 | 3 | PGC11 | 7.8 | 5.2 |
| 35 | 23 | ESL2 | 51 | 1 | GP1a1 | 62 | 1 | APE3 | 10.1 | 4.1 |
| 35 | 23 | ESL2 | 51 | 1 | GP1a1 | 71 | 3 | REG1 | 8.5 | 3.5 |
| 37 | 23 | GP1ba | 77 | 23 | APM12 | 80 | 1 | CCR2 | 7 | 10.3 |

[FIG. 65]

| SNP-No | Genotype | Gene code | SNP-No | Genotype | Gene code | SNP-No | Genotype | Gene code | kai | odds |
|---|---|---|---|---|---|---|---|---|---|---|
| 7 | 23 | N7 | 45 | 3 | LPL3 | | | | 9.2 | 12.7 |
| 17 | 1 | FR1 | 73 | 23 | VEGF5 | | | | 8.4 | 99 |
| 25 | 1 | PAI | 45 | 3 | LPL3 | | | | 8.6 | 7.4 |
| 30 | 1 | FGB3 | 63 | 23 | HANP2 | | | | 9.2 | 12.8 |
| 32 | 3 | MTHFR | 39 | 12 | CRP1 | | | | 8.5 | 11.6 |
| 55 | 3 | MPO | 76 | 1 | TS41 | | | | 6.7 | 99 |
| 64 | 12 | TNFa2 | 67 | 23 | 1A2 | | | | 14.4 | 4.3 |
| 64 | 12 | TNFa2 | 74 | 23 | LTA1 | | | | 10.8 | 4.7 |
| 13 | 3 | CF12 | 28 | 23 | AGT1 | 49 | 23 | GP61 | 9 | 12.6 |
| 26 | 23 | NOS3 | 29 | 12 | G1A3 | 50 | 1 | β2AR4 | 7.6 | 4.6 |
| 26 | 23 | NOS3 | 37 | 23 | GP1ba | 90 | 12 | IL-182 | 7.8 | 99 |
| 26 | 23 | NOS3 | 52 | 23 | MMP71 | 78 | 23 | ET1 | 15.5 | 11.2 |
| 28 | 23 | AGT1 | 33 | 12 | MCP1 | 35 | 23 | ESL2 | 7.3 | 6.4 |
| 28 | 23 | AGT1 | 35 | 23 | ESL2 | 62 | 1 | APE3 | 8.4 | 4.6 |
| 28 | 23 | AGT1 | 35 | 23 | ESL2 | 98 | 3 | PGC11 | 7.8 | 5.2 |
| 35 | 23 | ESL2 | 51 | 1 | GP1a1 | 62 | 1 | APE3 | 10.1 | 4.1 |
| 37 | 23 | GP1ba | 77 | 23 | APM12 | 80 | 1 | CCR2 | 7 | 10.3 |

[FIG. 66]

| SNP-No | Genotype | Gene code | SNP-No | Genotype | Gene code | SNP-No | Genotype | Gene code | kai | odds |
|---|---|---|---|---|---|---|---|---|---|---|
| 7 | 23 | N7 | 45 | 3 | LPL3 | | | | 9.2 | 12.7 |
| 17 | 1 | FR1 | 73 | 23 | VEGF5 | | | | 8.4 | 99 |
| 25 | 1 | PAI | 45 | 3 | LPL3 | | | | 8.6 | 7.4 |
| 30 | 1 | FGB3 | 63 | 23 | HANP2 | | | | 9.2 | 12.8 |
| 32 | 3 | MTHFR | 39 | 12 | CRP1 | | | | 8.5 | 11.6 |
| 55 | 3 | MPO | 76 | 1 | TS41 | | | | 6.7 | 99 |
| 13 | 3 | CF12 | 28 | 23 | AGT1 | 49 | 23 | GP61 | 9 | 12.6 |
| 26 | 23 | NOS3 | 37 | 23 | GP1ba | 90 | 12 | IL-182 | 7.8 | 99 |
| 26 | 23 | NOS3 | 52 | 23 | MMP71 | 78 | 23 | ET1 | 15.5 | 11.2 |
| 28 | 23 | AGT1 | 33 | 12 | MCP1 | 35 | 23 | ESL2 | 7.3 | 6.4 |
| 28 | 23 | AGT1 | 35 | 23 | ESL2 | 98 | 3 | PGC11 | 7.8 | 5.2 |
| 37 | 23 | GP1ba | 77 | 23 | APM12 | 80 | 1 | CCR2 | 7 | 10.3 |

[FIG. 67]

| SNP-No | Genotype | Gene code | SNP-No | Genotype | Gene code | SNP-No | Genotype | Gene code | kai | odds |
|---|---|---|---|---|---|---|---|---|---|---|
| 7 | 23 | N7 | 45 | 3 | LPL3 | | | | 9.2 | 12.7 |
| 17 | 1 | FR1 | 73 | 23 | VEGF5 | | | | 8.4 | 99 |
| 25 | 1 | PAI | 45 | 3 | LPL3 | | | | 8.6 | 7.4 |
| 30 | 1 | FGB3 | 63 | 23 | HANP2 | | | | 9.2 | 12.8 |
| 32 | 3 | MTHFR | 39 | 12 | CRP1 | | | | 8.5 | 11.6 |
| 55 | 3 | MPO | 76 | 1 | TS41 | | | | 6.7 | 99 |
| 13 | 3 | CF12 | 28 | 23 | AGT1 | 49 | 23 | GP61 | 9 | 12.6 |
| 26 | 23 | NOS3 | 37 | 23 | GP1ba | 90 | 12 | IL-182 | 7.8 | 99 |
| 26 | 23 | NOS3 | 52 | 23 | MMP71 | 78 | 23 | ET1 | 15.5 | 11.2 |
| 28 | 23 | AGT1 | 33 | 12 | MCP1 | 35 | 23 | ESL2 | 7.3 | 6.4 |
| 37 | 23 | GP1ba | 77 | 23 | APM12 | 80 | 1 | CCR2 | 7 | 10.3 |

[FIG. 68]

| SNP-No | Genotype | Gene code | SNP-No | Genotype | Gene code | SNP-No | Genotype | Gene code | kai | odds |
|---|---|---|---|---|---|---|---|---|---|---|
| 7 | 23 | N7 | 45 | 3 | LPL3 | | | | 9.2 | 12.7 |
| 17 | 1 | FR1 | 73 | 23 | VEGF5 | | | | 8.4 | 99 |
| 25 | 1 | PAI | 45 | 3 | LPL3 | | | | 8.6 | 7.4 |
| 30 | 1 | FGB3 | 63 | 23 | HANP2 | | | | 9.2 | 12.8 |
| 32 | 3 | MTHFR | 39 | 12 | CRP1 | | | | 8.5 | 11.6 |
| 55 | 3 | MPO | 76 | 1 | TS41 | | | | 6.7 | 99 |
| 13 | 3 | CF12 | 28 | 23 | AGT1 | 49 | 23 | GP61 | 9 | 12.6 |
| 26 | 23 | NOS3 | 37 | 23 | GP1ba | 90 | 12 | IL-182 | 7.8 | 99 |
| 26 | 23 | NOS3 | 52 | 23 | MMP71 | 78 | 23 | ET1 | 15.5 | 11.2 |
| 37 | 23 | GP1ba | 77 | 23 | APM12 | 80 | 1 | CCR2 | 7 | 10.3 |

[FIG. 69]

| SNP-No | Genotype | Gene code | SNP-No | Genotype | Gene code | SNP-No | Genotype | Gene code | kai | odds |
|---|---|---|---|---|---|---|---|---|---|---|
| 7 | 23 | N7 | 45 | 3 | LPL3 | | | | 9.2 | 12.7 |
| 17 | 1 | FR1 | 73 | 23 | VEGF5 | | | | 8.4 | 99 |
| 30 | 1 | FGB3 | 63 | 23 | HANP2 | | | | 9.2 | 12.8 |
| 32 | 3 | MTHFR | 39 | 12 | CRP1 | | | | 8.5 | 11.6 |
| 55 | 3 | MPO | 76 | 1 | TS41 | | | | 6.7 | 99 |
| 13 | 3 | CF12 | 28 | 23 | AGT1 | 49 | 23 | GP61 | 9 | 12.6 |
| 26 | 23 | NOS3 | 37 | 23 | GP1ba | 90 | 12 | IL-182 | 7.8 | 99 |
| 26 | 23 | NOS3 | 52 | 23 | MMP71 | 78 | 23 | ET1 | 15.5 | 11.2 |
| 37 | 23 | GP1ba | 77 | 23 | APM12 | 80 | 1 | CCR2 | 7 | 10.3 |

[FIG. 70]

| SNP-No | GenoType |
|---|---|
| 81 | 1 |
| 86 | 12 |
| 36 | 23 |
| 74 | 23 |
| 20 | 1 |
| 55 | 3 |
| 30 | 1 |
| 73 | 23 |
| 12 | 3 |
| 16 | 12 |
| 27 | 12 |
| 68 | 12 |
| 53 | 23 |
| 1 | 12 |
| 40 | 3 |
| 93 | 12 |
| 42 | 1 |
| 79 | 3 |
| 43 | 1 |
| 18 | 12 |
| 21 | 1 |
| 88 | 12 |
| 99 | 12 |
| 19 | 1 |
| 4 | 12 |
| 75 | 12 |
| 38 | 12 |
| 70 | 12 |
| 77 | 23 |
| 89 | 12 |
| 69 | 23 |
| 25 | 1 |
| 64 | 12 |
| 51 | 1 |
| 32 | 3 |
| 48 | 1 |
| 82 | 23 |
| 83 | 23 |
| 52 | 23 |
| 8 | 1 |
| 45 | 3 |
| 78 | 23 |
| 28 | 23 |
| 22 | 23 |
| 26 | 23 |
| 80 | 1 |
| 87 | 23 |
| 63 | 23 |
| 50 | 1 |
| 90 | 12 |
| 17 | 1 |
| 33 | 12 |
| 15 | 23 |
| 7 | 23 |
| 37 | 23 |
| 29 | 12 |
| 3 | 23 |
| 11 | 1 |
| 67 | 23 |
| 96 | 3 |
| 24 | 1 |
| 31 | 3 |
| 76 | 1 |
| 2 | 23 |
| 13 | 3 |
| 39 | 12 |
| 54 | 12 |
| 98 | 3 |
| 35 | 23 |
| 5 | 12 |
| 47 | 3 |
| 62 | 1 |
| 71 | 3 |
| 84 | 1 |
| 10 | 12 |
| 23 | 12 |
| 49 | 23 |
| 56 | 3 |
| 95 | 3 |

[FIG. 71]

| SNP-No | GenoType |
|---|---|
| 81 | 1 |
| 93 | 12 |
| 55 | 23 |
| 64 | 12 |
| 18 | 3 |
| 51 | 1 |
| 43 | 3 |
| 4 | 1 |
| 26 | 23 |
| 90 | 12 |
| 16 | 1 |
| 2 | 23 |
| 19 | 23 |
| 33 | 12 |
| 83 | 23 |
| 7 | 23 |
| 99 | 1 |
| 38 | 1 |
| 82 | 23 |
| 15 | 23 |
| 30 | 23 |
| 70 | 12 |
| 11 | 23 |
| 86 | 1 |
| 5 | 12 |
| 45 | 3 |
| 35 | 23 |
| 74 | 3 |
| 75 | 1 |
| 36 | 3 |
| 96 | 23 |
| 22 | 23 |
| 50 | 23 |
| 12 | 12 |
| 53 | 1 |
| 29 | 12 |
| 28 | 23 |
| 80 | 1 |
| 48 | 1 |
| 52 | 23 |
| 27 | 1 |
| 32 | 23 |
| 89 | 3 |
| 21 | 23 |
| 78 | 23 |
| 68 | 1 |
| 8 | 12 |
| 42 | 1 |
| 49 | 23 |
| 39 | 12 |
| 1 | 3 |
| 13 | 3 |
| 24 | 23 |
| 77 | 23 |
| 25 | 3 |
| 40 | 1 |
| 63 | 23 |
| 79 | 12 |
| 87 | 23 |
| 17 | 1 |
| 76 | 1 |
| 69 | 23 |
| 98 | 3 |
| 3 | 1 |
| 67 | 23 |
| 54 | 23 |
| 31 | 3 |
| 73 | 3 |
| 88 | 23 |
| 84 | 23 |
| 37 | 23 |
| 71 | 3 |
| 47 | 12 |
| 20 | 23 |
| 95 | 3 |
| 56 | 3 |
| 62 | 12 |
| 10 | 12 |
| 23 | 12 |

[FIG. 72]

| SNP-No | GenoType |
|---|---|
| 64 | 12 |
| 86 | 1 |
| 18 | 3 |
| 26 | 23 |
| 99 | 1 |
| 90 | 12 |
| 38 | 1 |
| 82 | 23 |
| 75 | 1 |
| 16 | 1 |
| 7 | 23 |
| 19 | 23 |
| 51 | 3 |
| 43 | 3 |
| 2 | 23 |
| 36 | 3 |
| 5 | 12 |
| 53 | 1 |
| 15 | 23 |
| 11 | 23 |
| 31 | 12 |
| 83 | 23 |
| 35 | 23 |
| 4 | 1 |
| 1 | 3 |
| 45 | 3 |
| 29 | 12 |
| 28 | 23 |
| 24 | 23 |
| 27 | 1 |
| 70 | 12 |
| 73 | 1 |
| 22 | 23 |
| 81 | 1 |
| 80 | 1 |
| 69 | 23 |
| 79 | 12 |
| 13 | 3 |
| 50 | 23 |
| 3 | 1 |
| 39 | 12 |
| 67 | 23 |
| 89 | 1 |
| 49 | 23 |
| 93 | 1 |
| 52 | 23 |
| 33 | 12 |
| 30 | 23 |
| 25 | 3 |
| 40 | 1 |
| 87 | 23 |
| 68 | 1 |
| 32 | 3 |
| 21 | 23 |
| 42 | 1 |
| 77 | 3 |
| 12 | 12 |
| 63 | 23 |
| 76 | 1 |
| 17 | 1 |
| 78 | 23 |
| 48 | 1 |
| 54 | 23 |
| 8 | 1 |
| 74 | 3 |
| 96 | 23 |
| 20 | 23 |
| 55 | 3 |
| 71 | 12 |
| 98 | 1 |
| 84 | 23 |
| 88 | 23 |
| 37 | 23 |
| 62 | 1 |
| 10 | 12 |
| 47 | 12 |
| 23 | 12 |
| 56 | 3 |
| 95 | 3 |

[FIG. 73]

| SNP-No | GenoType |
|---|---|
| 86 | 1 |
| 82 | 23 |
| 64 | 12 |
| 7 | 23 |
| 26 | 23 |
| 74 | 3 |
| 99 | 1 |
| 75 | 1 |
| 27 | 1 |
| 36 | 3 |
| 43 | 3 |
| 38 | 1 |
| 19 | 23 |
| 18 | 3 |
| 5 | 12 |
| 90 | 12 |
| 31 | 12 |
| 11 | 23 |
| 16 | 1 |
| 29 | 12 |
| 53 | 1 |
| 2 | 23 |
| 15 | 23 |
| 22 | 23 |
| 83 | 23 |
| 45 | 3 |
| 51 | 3 |
| 28 | 23 |
| 67 | 1 |
| 4 | 1 |
| 49 | 23 |
| 39 | 12 |
| 35 | 23 |
| 1 | 1 |
| 69 | 3 |
| 70 | 12 |
| 93 | 1 |
| 52 | 23 |
| 73 | 1 |
| 48 | 3 |
| 24 | 23 |
| 50 | 23 |
| 33 | 12 |
| 3 | 1 |
| 40 | 1 |
| 25 | 3 |
| 12 | 12 |
| 32 | 3 |
| 87 | 23 |
| 77 | 3 |
| 80 | 1 |
| 78 | 23 |
| 68 | 3 |
| 21 | 23 |
| 76 | 1 |
| 79 | 12 |
| 63 | 23 |
| 84 | 23 |
| 89 | 1 |
| 30 | 1 |
| 17 | 1 |
| 8 | 1 |
| 71 | 12 |
| 13 | 3 |
| 20 | 23 |
| 42 | 1 |
| 54 | 3 |
| 55 | 12 |
| 81 | 1 |
| 37 | 23 |
| 88 | 23 |
| 96 | 23 |
| 98 | 12 |
| 23 | 12 |
| 47 | 12 |
| 10 | 12 |
| 56 | 3 |
| 62 | 12 |
| 95 | 3 |

[FIG. 74]

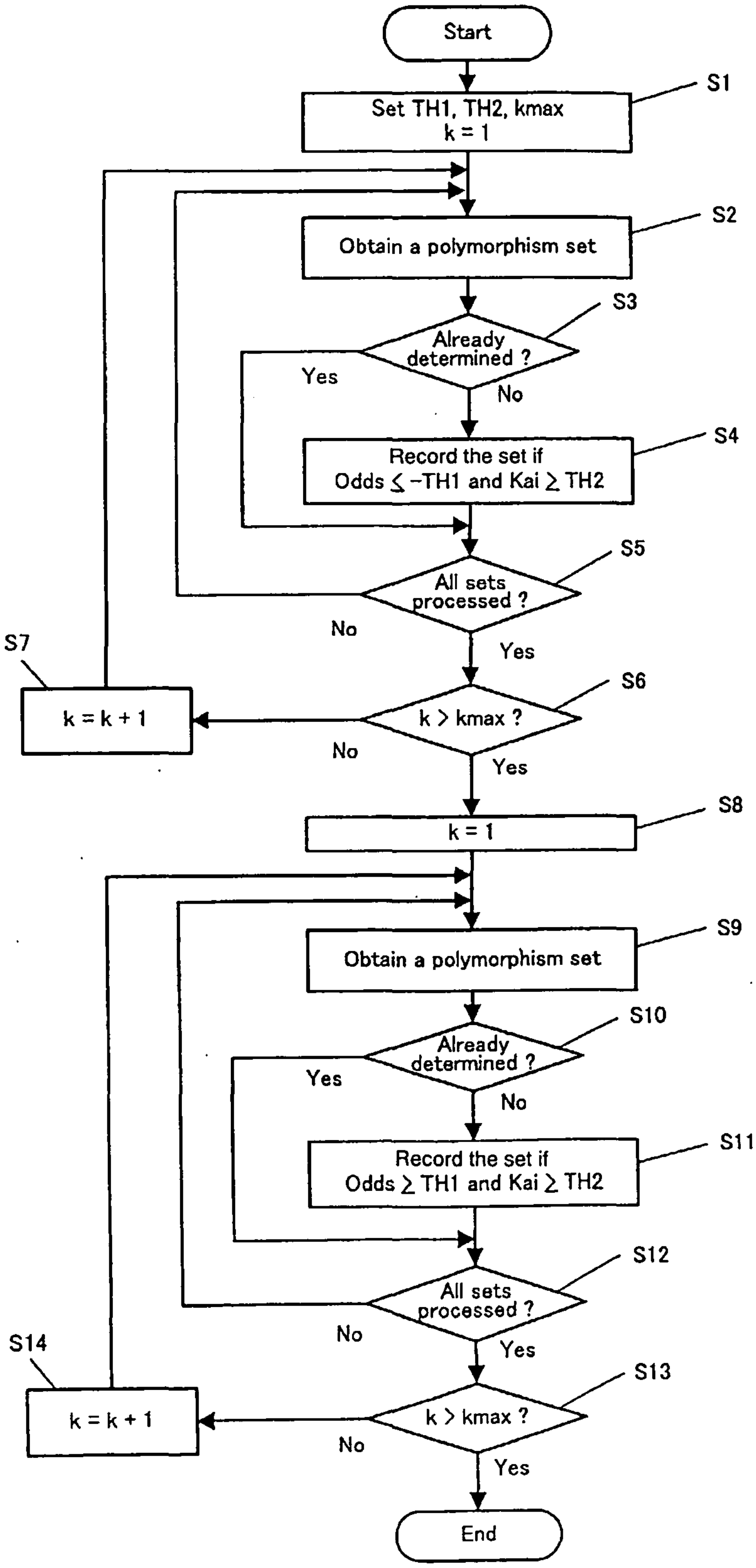
Start
Set TH1, TH2, kmax
k = 1
S1
Obtain a polymorphism set
S2
Already determined ?
S3
Yes
No
Record the set if
Odds ≤ -TH1 and Kai ≥ TH2
S4
All sets processed ?
S5
No
Yes
k > kmax ?
S6
S7
k = k + 1
No
Yes
k = 1
S8
Obtain a polymorphism set
S9
Already determined ?
S10
Yes
No
Record the set if
Odds ≥ TH1 and Kai ≥ TH2
S11
All sets processed ?
S12
No
Yes
k > kmax ?
S13
S14
k = k + 1
No
Yes
End

[FIG. 75]

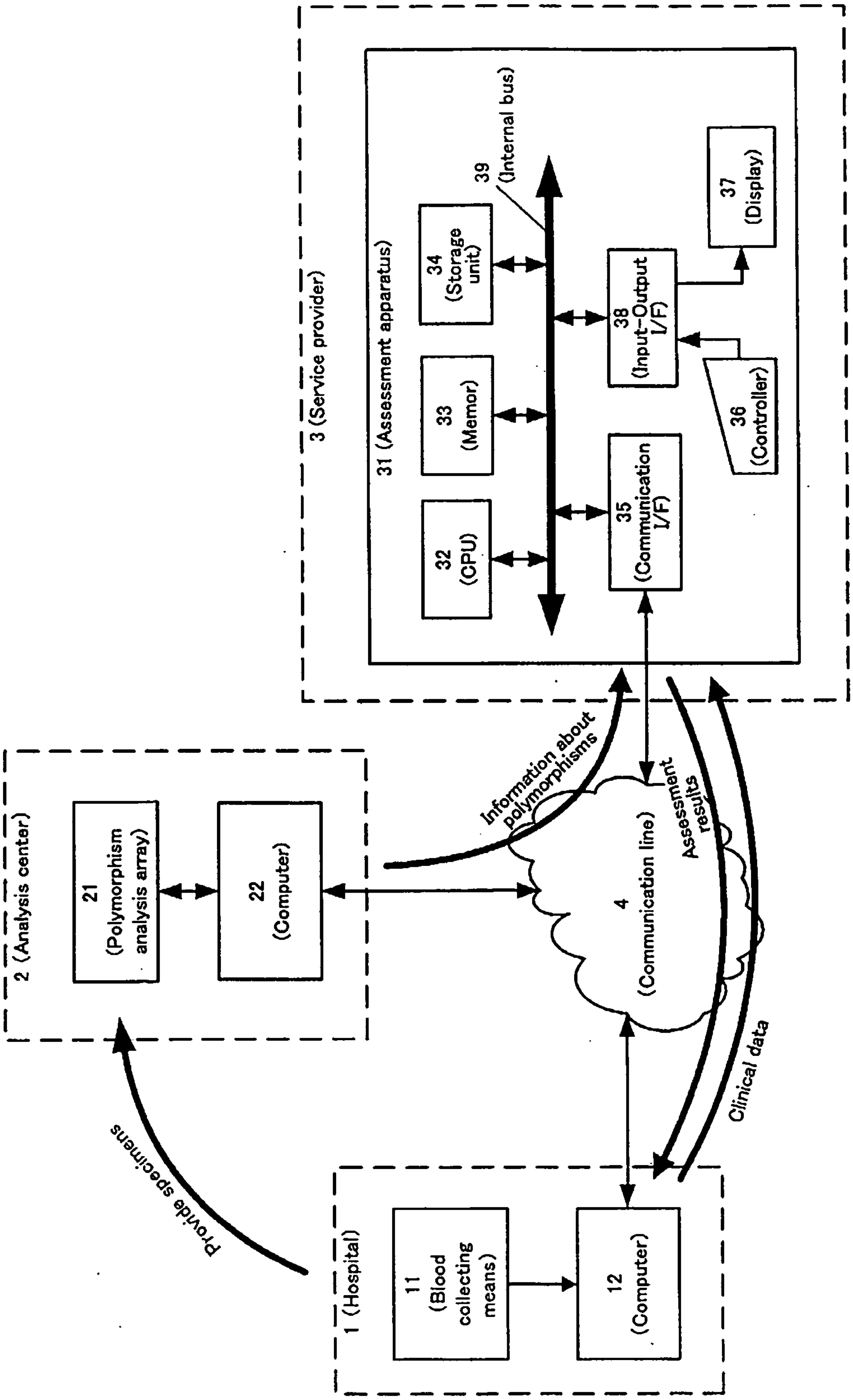
3 (Service provider)
31 (Assessment apparatus)
32 (CPU)
33 (Memor)
34 (Storage unit)
35 (Communication I/F)
36 (Controller)
37 (Display)
38 (Input-Output I/F)
39 (Internal bus)
2 (Analysis center)
21 (Polymorphism analysis array)
22 (Computer)
4 (Communication line)
Information about polymorphisms
Assessment results
Clinical data
Provide specimens
1 (Hospital)
11 (Blood collecting means)
12 (Computer)

[FIG. 76]

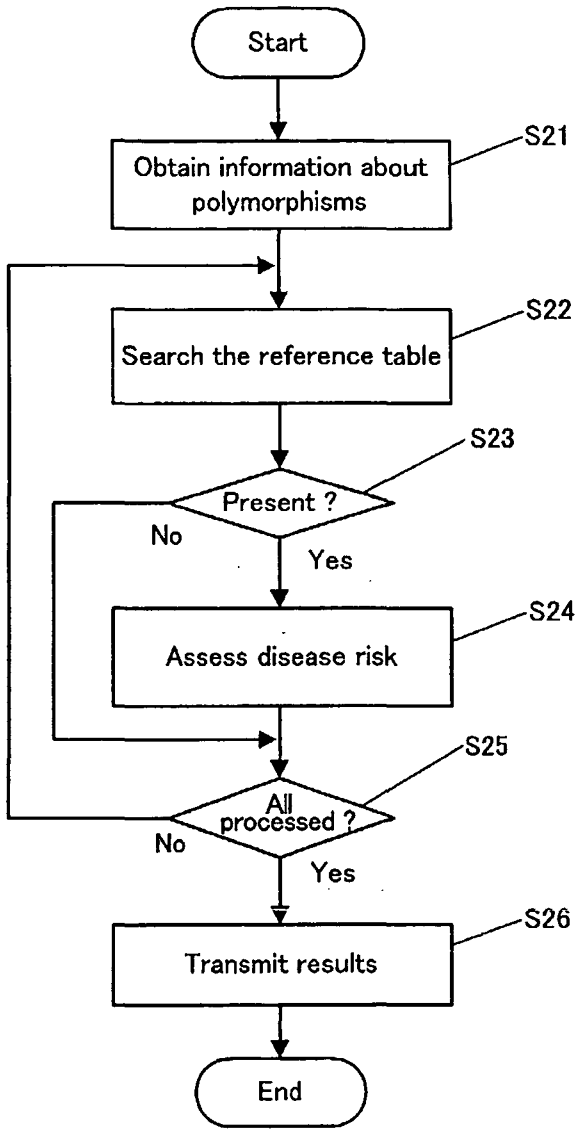
Start
Obtain information about polymorphisms
S21
Search the reference table
S22
Present ?
S23
No
Yes
Assess disease risk
S24
All processed ?
S25
No
Yes
Transmit results
S26
End

[FIG. 77]

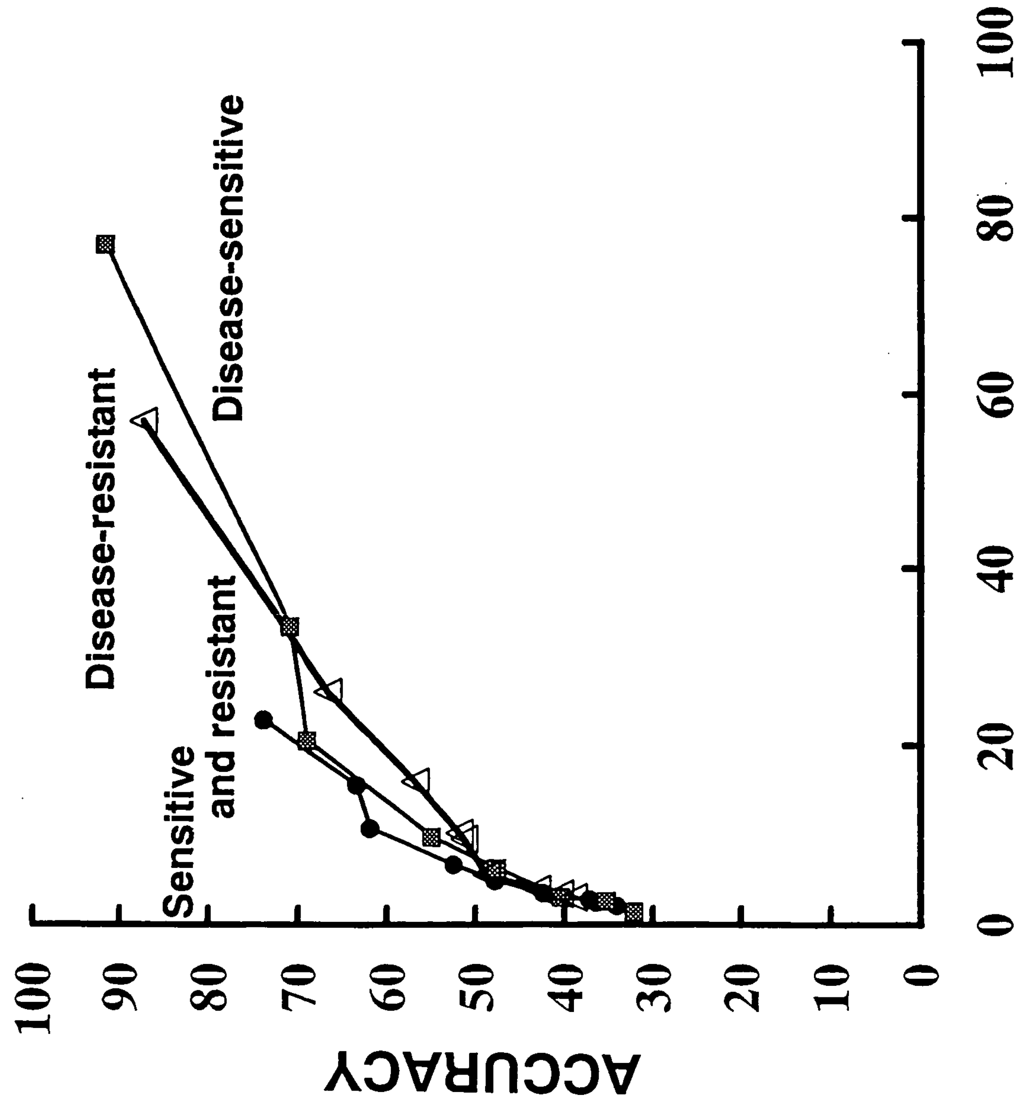
Disease-resistant
Disease-sensitive
Sensitive
and resistant
ACCURACY
INACCURACY
0
10
20
30
40
50
60
70
80
90
100
0
20
40
60
80
100

**INTERNATIONAL SEARCH REPORT**

International application No.
PCT/JP2004/015292

A. CLASSIFICATION OF SUBJECT MATTER
Int.Cl[7] G06F19/00

According to International Patent Classification (IPC) or to both national classification and IPC

B. FIELDS SEARCHED

Minimum documentation searched (classification system followed by classification symbols)
Int.Cl[7] G06F19/00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)
JICST FILE(JOIS), WPI, INSPEC(DIALOG)

C. DOCUMENTS CONSIDERED TO BE RELEVANT

| Category* | Citation of document, with indication, where appropriate, of the relevant passages | Relevant to claim No. |
|---|---|---|
| Y | WO 02/061659 A2 (SCIONA LTD.), 08 August, 2002 (08.08.02), Whole Claims; pages 52 to 54 & CA 2435682 A1 & EP 1395938 A2 & JP 2004-525448 A & US 2003-023387 A1 | 1-53 |
| Y | JP 2003-061677 A (Olympus Optical Co., Ltd.), 04 March, 2003 (04.03.03), Claims; Par Nos. [0043] to [0044], [0054] to [0065], [0093] to [0106] (Family: none) | 1-53 |

[X] Further documents are listed in the continuation of Box C.

[ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 January, 2005 (13.01.05) | 01 February, 2005 (01.02.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/015292

C (Continuation). DOCUMENTS CONSIDERED TO BE RELEVANT

| Category* | Citation of document, with indication, where appropriate, of the relevant passages | Relevant to claim No. |
|---|---|---|
| Y | IZAWA, H. et al., Prediction of Genetic Risk for Hypertension, 24 March, 2003 (24.03.03) [retrieved on 05 January, 2005 (05.01.05)], Retrieved from the internet: <URL:http://hyper.ahajournals.org/cgi/content/full/41/5/1035 doi:10.1161/01.HYP.0000065618.56368.24> | 1-53 |

Form PCT/ISA/210 (continuation of second sheet) (January 2004)

**INTERNATIONAL SEARCH REPORT**

International application No.
PCT/JP2004/015292

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

It appears that there is a technical relationship involving "special technical features" among claims 1-11, 20-28, 29-37, 38-44 and 45 in the use of a negative relationship between genetic polymorphism and disease index; among claims 12-15, 46, 47, 50 and 51 in the use of genetic polymorphism set shown in any of Figs. 1-9; and among claims 16-19, 48, 49, 52 and 53 in the use of genetic polymorphism set shown in any of Figs. 56-58. However, among these three claim groups, it does not appear that there is a technical relationship involving "special technical features".

1. ☐ As all required additional search fees were timely paid by the applicant, this international search report covers all searchable claims.
2. ☒ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest.

☐ No protest accompanied the payment of additional search fees.

Form PCT/ISA/210 (continuation of first sheet (2)) (January 2004)

SEQUENCE LISTING

<110> SIGNPOST CORPORATION
<120> Method of determining genetic polymorphisms for assessing disease risk, method of assessing disease risk, an array for assessing disease risk
<130> P04-130
<150> JP 2003-355716
<151> 2003-10-15
<160> 4
<170> PatentIn version 3.1

<210> 1
<211> 14
<212> DNA
<213> Artificial Sequence
<220>
<221> Primer
<400> 1
ctggagacca ctcccatcct ttct 14

<210> 2
<211> 25
<212> DNA
<213> Artificial Sequence
<220>
<221> Primer
<400> 2
gatgtggcca tcacattcgt cagat 25

<210> 3
<211> 21
<212> DNA
<213> Artificial Sequence
<220>
<221> Probe
<400> 3
ttacaggcgt gatacagtca c 21

<210> 4
<211> 23
<212> DNA
<213> Artificial Sequence
<220>
<221> Probe
<400> 4
gcctatacag tcacttttat gtg 23